# EUROPEAN PATENT SPECIFICATION

(11) **EP 1 924 579 B1**
(45) Date of publication and mention of the grant of the patent: **09.02.2011**
(21) Application number: 06778310.0
(22) Date of filing: 21.08.2006
(51) Int. Cl.: C07D 413/12, C07D 413/14, C07D 498/04, C07D 417/12, C07D 417/14, A61P 3/10, A61P 3/04, A61P 17/06, A61P 19/02, A61K 31/428, A61K 31/437, A61K 31/423

(54) **BENZOOXAZOLE, OXAZOLOPYRIDINE, BENZOTHIAZOLE AND THIAZOLOPYRIDINE DERIVATIVES**
BENZOXAZOL-, OXAZOLOPYRIDIN-, BENZOTHIAZOL- UND THIAZOLOPYRIDINDERIVATE
DERIVES DE BENZOOXAZOLE, OXAZOLOPYRIDINE, BENZOTHIAZOLE ET THIAZOLOPYRIDINE

(30) Priority: 02.09.2005 EP 05108035
(43) Date of publication of application: 28.05.2008
(73) Proprietor: F.Hoffmann-La Roche AG, 4070 Basel (CH)
(72) Inventor: BINGGELI, Alfred, CH-4102 Binningen (CH); CHRIST, Andreas, D., CH-4144 Arlesheim (CH); GREEN, Luke, CH-4057 Basel (CH); GUBA, Wolfgang, 79379 Muellheim (DE); MAERKI, Hans, P., CH-4059 Basel (CH); MARTIN, Rainer, E., CH-4056 Basel (CH); MOHR, Peter, CH-4054 Basel (CH)
(74) Representative: Klostermeyer-Rauber, Dörte
(86) International application number: PCT/EP2006/065522
(87) International publication number: WO 2007/025897

(56) References cited:
- WO-A-2005/003127
- WO-A-2006/094682
- WO-A1-2004/014885
- CONTOUR-GALCERA M-O ET AL: "Synthesis of substituted imidazopyrazines as ligands for the human somatostatin receptor subtype 5" BIOORGANIC & MEDICINAL CHEMISTRY LETTERS, OXFORD, GB, vol. 11, no. 5, 12 March 2001 (2001-03-12), pages 741-745, XP004230102 ISSN: 0960-894X

## Description

The present invention is concerned with novel benzooxazole, oxazolopyridine, benzothiazole and thiazolopyridine derivatives, their manufacture, pharmaceutical compositions containing them and their use as medicaments. The active compounds of the present invention are useful in the prevention and/or treatment of diabetes mellitus and other disorders.

In particular, the present invention is concerned with compounds of the general formula I 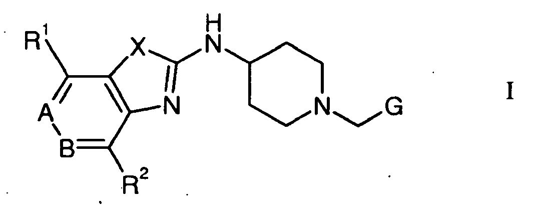 wherein
- X: is S or O;
- A: is CR³ and B is CR⁴, or Ais N or N⁺-O⁻ and B is CR⁴, or B is N or N⁺-O⁻ and A is CR³;
- R¹ and R²: are independently selected from the group consisting of hydrogen, halogen, cyano, nitro, C₁₋₇-alkyl, hydroxy-C₁₋₇-alkyl, C₁₋₇-alkoxy-C₁₋₇-alkyl, C₁₋₇-alkoxy, hydroxy-C₂₋₇-alkoxy, dihydroxy-C₃₋₇-alkoxy, carboxy-C₁₋₇-alkoxy, C₁₋₇-alkoxycarbonyl-C₁₋₇-alkoxy, carboxy-C₁₋₇-alkyl, C₁₋₇-alkoxycarbonyl-C₁₋₇-alkyl, 1*H*-tetrazol-5-yl-C₁₋₇-alkoxy, pyridinyl-C₁₋₇-alkoxy, -NR⁵R⁶, -NHCOR⁷, -NHSO₂R⁸, -SO₂NR⁹R¹⁰, 1*H*-tetrazol-5-yl, unsubstituted phenyl and phenyl substituted by one to three substituents selected from C₁₋₇alkyl, C₃₋₇-cycloalkyl, halogen, halogen-C₁₋₇-alkyl and C₁₋₇-alkoxy; and
- R²: can additionally also be selected from the group consisting of carboxy, C₁₋₇-alkoxycarbonyl and -CONR¹¹R¹²;
- R⁵ and R⁶: independently from each other are selected from the group consisting of hydrogen, C₁₋₇-alkyl, hydroxy-C₁₋₇-alkyl and C₃₋₇-cycloalkyl;
- R⁷: is selected from the group consisting of C₁₋₇-alkyl, C₃₋₇-cycloalkyl, halogen-C₁₋₇-alkyl, hydroxy-C₁₋₇-alkyl, C₁₋₇alkoxy-C₁₋₇-alkyl, carboxy-C₁₋₇-alkyl, C₁₋₇-alkoxycarbonyl-C₁₋₇-alkyl, 1*H*-tetrazol-5-yl-C₁₋₇-alkyl, unsubstituted phenyl, phenyl substituted by one to three groups selected from the group consisting of C₁₋₇-alkyl, C₃₋₇-cycloalkyl, C₁₋₇-alkoxy, halogen-C₁₋₇-alkyl and halogen, unsubstituted heteroaryl, heteroaryl substituted by one or two groups selected from C₁₋₇-alkyl, C₃₋₇-cycloalkyl, C₁₋₇-alkoxy, halogen-C₁₋₇-alkyl or halogen; unsubstituted heteroaryl-C₁₋₇-alkyl and heteroaryl-C₁₋₇-alkyl, wherein the heteroaryl is substituted by one or two groups selected from C₁₋₇-alkyl, C₃₋₇-cycloalkyl, C₁₋₇-alkoxy, halogen-C₁₋₇-alkyl and halogen;
- R⁸: is selected from the group consisting of C₁₋₇-alkyl, C₃₋₇-cycloalkyl, unsubstituted heteroaryl and heteroaryl substituted by one or two groups selected from C₁₋₇-alkyl, C₃₋₇-cycloalkyl, C₁₋₇-alkoxy, halogen-C₁₋₇-alkyl or halogen;
- R⁹: and R¹⁰ independently from each other are selected from the group consisting of hydrogen, C₁₋₇-alkyl, C₃₋₇-cycloalkyl, unsubstituted heteroaryl and heteroaryl substituted by one or two groups selected from C₁₋₇-alkyl, C₃₋₇-cycloalkyl, C₁₋₇-alkoxy, halogen-C₁₋₇-alkyl or halogen; or R⁹ and R¹⁰ together with the nitrogen atom they are attached to form a pyrrolidine or a piperidine ring;
- R¹¹: is selected from the group consisting of hydrogen, C₁₋₇-alkyl and C₃₋₇-cycloalkyl;
- R¹²: is selected from the group consisting of hydrogen, C₁₋₇-alkyl, C₃₋₇-cycloalkyl, halogen-C₁₋₇-alkyl, hydroxy-C₁₋₇-alkyl, C₁₋₇-alkoxy-C₁₋₇alkyl, carboxy-C₁₋₇-alkyl, C₁₋₇-alkoxycarbonyl-C₁₋₇-alkyl, unsubstituted phenyl, phenyl substituted by one to three groups selected from the group consisting of C₁₋₇-alkyl, C₃₋₇-cycloalkyl, C₁₋₇-alkoxy, halogen -C₁₋₇-alkyl and halogen, unsubstituted heteroaryl, heteroaryl substituted by one or two groups selected from C₁₋₇-alkyl, C₃₋₇-cycloalkyl, C₁₋₇-alkoxy, halogen-C₁₋₇-alkyl or halogen;
- one of R³ and R⁴: is selected from the group consisting of hydrogen, C₁₋₇-alkyl and C₁₋₇- alkoxy, or is absent in case one of A or B is N or N⁺-O⁻, and
- the other one of R³ and R⁴: is selected from the group consisting of hydrogen, C₁₋₇-alkyl, C₁₋₇-alkoxy, hydroxy, hydroxy-C₁₋₇-alkyl, cyano-C₁₋₇-alkoxy, C₃₋₇-cycloalkyloxy wherein the cycloalkyl group is substituted by carboxy or C₁₋₇-alkoxy-carbonyl, carboxy, C₁₋₇-alkoxy-carbonyl, carboxy-C₁₋₇-alkoxy, C₁₋₇-alkoxy-carbonyl-C₁₋₇-alkyl, carboxy-C₁₋₇-alkyl, C₁₋₇-alkoxy-carbonyl-C₁₋₇-alkoxy, 1*H*-tetrazol-5-yl-C₁₋₇-alkoxy, triazolyl-C₁₋₇-alkoxy, C₁₋₇-alkylsulfonyloxy, C₁₋₇-alkylsulfonyl-C₁₋₇-alkoxy, hydroxy-C₂₋₇-alkoxy, dihydroxy-C₃₋₇-alkoxy, C₁₋₇-alkoxy-C₁₋₇-alkoxy, - (CH₂)ₙ-NR¹³R¹⁴, -(CH₂)ₙ-NHCOR¹⁵, amino-C₁₋₇-alkoxy, aminocarbonyl-C₁₋₇-alkoxy, C₁₋₇-alkylaminocarbonyl-C₁₋₇alkoxy and 1*H*-tetrazol-5-yl;
- R¹³ and R¹⁴: independently from each other are selected from the group consisting of hydrogen, C₁₋₇-alkyl and C₃₋₇-cycloalkyl;
- R¹⁵: is selected from the group consisting of C₁₋₇-alkyl, C₃₋₇-cycloalkyl, halogen-C₁₋₇-alkyl, hydroxy-C₁₋₇-alkyl, C₁₋₇-alkoxy-C₁₋₇-alkyl, carboxy-C₁₋₇-alkyl and C₁₋₇-alkoxycarbonyl-C₁₋₇-alkyl;
- n: is an integer selected from 1, 2 and 3;
provided that benzooxazoles and benzothiazoles wherein R¹, R², R³ and R⁴ are hydrogen are excluded;
- G: is selected from the groups 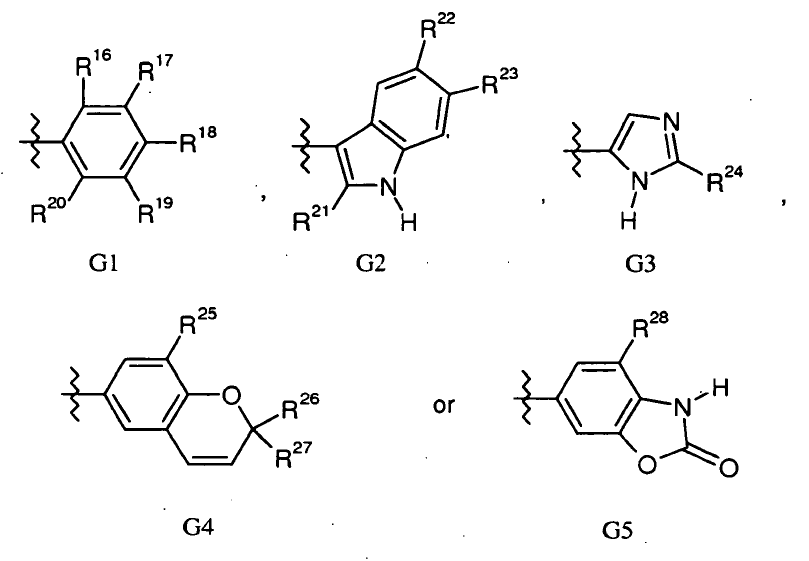
wherein
- R¹⁶: is hydrogen or halogen;
- R¹⁷: is selected from the group consisting of C₁₋₇-alkoxy, C₂₋₇-alkenyloxy, C₃₋₇-cycloalkyloxy, -NR²⁹R³⁰, halogen-C₁₋₇-alkoxy, C₁₋₇-alkoxy-C₁₋₇-alkoxy and C₁₋₇-alkoxy-C₁₋₇-alkyl; R²⁹ and R³⁰ independently from each other are hydrogen or C₁₋₇-alkyl;
- R¹⁸: is selected from the group consisting of hydrogen, C₁₋₇-alkyl, halogen-C₁₋₇-alkyl, hydroxy, C₁₋₇-alkoxy, halogen-C₁₋₇-alkoxy, C₃₋₇-cycloalkyloxy, halogen, pyrrolyl, imidazolyl, triazolyl, -CO₂R³¹, -NR³²R³³, -SOR³⁴; unsubstituted phenyl and phenyl substituted by one to two groups selected from the group consisting of C₁₋₇alkyl, halogen-C₁₋₇-alkyl, halogen-C₁₋₇-alkoxy, C₁₋₇-alkoxy and halogen;
- R³¹: is hydrogen or C₁₋₇-alkyl;
- R³² and R³³: independently from each other are hydrogen or C₁₋₇-alkyl;
- R³⁴: is C₁₋₇-alkyl;
- R¹⁹: is selected from the group consisting of hydrogen, C₁₋₇-alkyl, halogen, C₁₋₇-alkoxy, C₂₋₇-alkenyloxy, -O-tetrahydropyranyl, C₃₋₇-cycloalkyloxy, halogen-C₁₋₇-alkoxy, C₁₋₇-alkoxy-C₁₋₇-alkoxy, and C₁₋₇-alkoxy-C₁₋₇-alkyl;
- R¹⁰: is hydrogen or halogen;
- R²¹: is hydrogen or C₁₋₇-alkyl;
- R²² and R¹³: independently from each other are selected from the group consisting of hydrogen, C₁₋₇-alkyl, C₁₋₇-alkoxy and nitro;
- R²⁴: is unsubstituted phenyl or phenyl substituted by one to two groups selected from the group consisting of C₁₋₇-alkyl, C₁₋₇-alkoxy and halogen;
- R¹⁵: is C₁₋₇-alkoxy;
- R²⁶ and R²⁷: independently from each other are C₁₋₇-alkyl;
- R²⁸: is C₁₋₇-alkoxy;
and pharmaceutically acceptable salts thereof.

The compounds of formula I possess pharmaceutical activity, in particular they are modulators of somatostatine receptor activity. More particularly, the compounds are antagonists of the somatostatine receptor subtype 5 (SSTR5).

Diabetes mellitus is a systemic disease characterized by metabolic disorders involving insulin, carbohydrates, fats and proteins, and disorders in the structure and function of blood vessels. The primary symptom of acute diabetes is hyperglycemia, often accompanied by glucosuria, the presence in urine of large amounts of glucose, and polyuria, the excretion of large volumes of urine. Additional symptoms arise in chronic diabetes, including degeneration of the walls of blood vessels. Although many different human organs are affected by these vascular changes, the eyes and kidneys appear to be the most susceptible. As such, long-standing diabetes mellitus, even when treated with insulin, is a leading cause of blindness.

There are three recognized types of diabetes mellitus. Type I diabetes or insulin dependent diabetes mellitus (IDDM) is typically of juvenile onset; ketosis develops early in life with much more severe symptoms and has a near-certain prospect of later vascular involvement. Control of Type I diabetes is difficult and requires exogenous insulin administration. Type II diabetes or non-insulin dependent diabetes mellitus (NIDDM) is ketosis-resistant, generally develops later in life, is milder and has a more gradual onset. Gestational diabetes is related to type II diabetes and associated with an increased risk of later development of that disease. Type III diabetes is malnutrition-related diabetes.

NIDDM is a condition that poses a major threat to the health of the citizens of the western world. NIDDM accounts for over 85% of diabetes incidence worldwide and about 160 million people are suffering from NIDDM. The incidence is expected to increase considerably within the next decades, especially in developing countries. NIDDM is associated with morbidity and premature mortality resulting from serious complications, *e.g*. cardiovascular disease (G. C. Weir, J.L. Leahy, 1994, Pathogenesis of non-insulin dependent (Type II) diabetes mellitus. Joslin's Diabetes Mellitus 13th Ed. (Eds. C. R. Kahn, G. C. Weir), Lea & Febiger, Malvern, PA, pp. 240-264). NIDDM is characterized by both fasting and post-prandial hyperglycemia resulting from abnormalities in insulin secretion and insulin action (G. C. Weir *et al., vide supra),*

The hyperglycemia in patients suffering from NIDDM can usually be initially treated by dieting, but eventually most NIDDM patients have to take oral antidiabetic agents and/or insulin injections to normalize their blood glucose levels. The introduction of orally effective hypoglycemic agents was an important development in the treatment of hyperglycemia by lowering blood glucose levels. Currently, the most widely used oral antidiabetic agents are the sulfonylureas, which act by increasing the secretion of insulin from the pancreas (H.E. Lebovitz, 1994, Oral antidiabetic agents. Joslin's Diabetes Mellitus 13th Ed. (Eds. C. R. Kahn, G. C. Weir), Lea & Febiger, Malvern, PA, pp. 508-529), the biguanides (*e.g*., metformin) which act on the liver and periphery by unknown mechanisms (C. J. Bailey, M. R. C. Path, R. C. Turner N. Engl. J. Med. 1996, 334, 574-579) and the thiazolidinediones (*e.g*., rosiglitazone / Avandia®) which enhance the effects of insulin at peripheral target sites (G. L.Plosker, D. Faulds Drugs 1999, 57, 409-438). These existing therapies which comprise a wide variety of biguanide, sulfonylurea and thiazolidinedione derivatives have been used clinically as hypoglycemic agents. However, all three classes of compound have side effects. The biguanides, for example metformin, are unspecific and in certain cases has been associated with lactic acidosis, and need to be given over a longer period of time, *i.e*. they are not suitable for acute administration (Bailey et al., vide supra). The sulfonylureas, though having good hypoglycemic activity, require great care during use because they frequently cause serious hypoglycemia and are most effective over a period of circa ten years. The thiazolidinediones may cause weight gain following chronic administration (Plosker and Faulds, vide supra) and troglitazone has been associated with the occurrence of serious hepatic dysfunction.

Thus, there is a significant and rising need for antidiabetic drugs that have novel mechanisms of action, thereby avoiding side effects produced by known therapies. The hormone somatostatin (SST) is primarily produced in the intestinal tract and in the pancreas. In addition it acts as a neurotransmitter. The hormone is involved through its receptors in the regulation of several other hormones and in immunoregulation. In particular, SST suppresses the secretion of insulin by pancreatic β cells and the secretion of glucagon-like peptide 1 (GLP-1) by L cells. GLP-1 in turn is one of the most potent stimulators of insulin production and secretion and is a trophic factor for β cells. β and L cells express SST receptor subtype 5 (SSTRS) and agonizing this receptor suppresses insulin and GLP-1 secretion in humans and in animal models (*e.g*., Y. Zambre, Z. Ling, M.-C. Chen, X. Hou, C.-W. Woon, M. Culler, J. E. Taylor, D. H. Coy, C. van Schravendijk, F. Schuit, D. G. Pipeleers and D. L Eizirik, Inhibition of human pancreatic islet insulin release by receptor-selective somatostatin analogs directed to somatostatin receptor subtype 5 in Biochem. Pharmacol, 1999, 57, 11.59-1164; S. P. Fagan, A. Azizzadeh, S. Moldovan, M. K. Ray, T. E. Adrian, X. Ding, D. H. Coy and F. C. Brunicardi, Insulin secretion is inhibited by subtype five somatostatin receptor in the mouse in Surgery 1998, 124, 254-258; M. Norman, S. Moldovan, V. Seghers, X.-P. Wang, F. J. DeMayo and F. C. Brunicardi, Sulfonylurea receptor knockout causes glucose intolerance in mice that is not alleviated by concomitant somatostatin subtype receptor 5 knockout in Ann. Surg. 2002, 235, 767-774; T.A. Tirone, M. A. Norman, S. Moldovan, F. J. DeMayo, X.-P. Whang, F. C. Brunicardi, Pancreatic somatostatin inhibits insulin secretion via SSTR-5 in the isolated perfused mouse pancreas model in Pancreas 2003, 26, e67-73; M. Z. Strowski, M. Köhler, H. Y. Chen, M. E. Trumbauer, Z. Li, D. Szalkowski, S. Gopal-Truter, J. K. Fisher, J. M. Schaeffer, A D. Blake, B. B. Zhang, H. A. Wilkinson, Somatostatin receptor subtype 5 regulates insulin secretion and glucose homeostasis in Mol. Endocrinol. 2003,17, 93-106).

Consequently, antagonizing the effect of SST would lead to higher plasma insulin concentrations. In patients suffering from impaired glucose tolerance and NIDDM, a higher plasma insulin concentration would moderate the dangerous hyperglycemia and accordingly reduce the risk of tissue damage. If such SST5 antagonists are sufficiently selective over the other four SST receptors, little influence is expected on secretion of other hormones. Particularly, selectivity over SST receptor subtype 2 avoids influences on glucagon secretion (K. Cejvan, D. H. Coy, S. Efendic, Intra-islet somatostatin regulates glucagon release via type 2 somatostatin receptors in rats in Diabetes 2003, 52, 1176-1181; M. Z. Strowski, R. M. Parmar, A. D. Blake, J. M. Schaeffer, Somatostatin inhibits insulin and glucagon secretion via two receptor subtypes: an in vitro study of pancreatic islets from somatostatin receptor 2 knockout mice in Endocrinology 2000, 141, 111-117). Advantageous over established therapies is the dual mechanism of action to increase insulin secretion: directly on pancreatic β cells and indirectly through GLP-1 release from L cells. Additionally, SSTR5 knockout mice demonstrated higher insulin sensitivity than littermates (Strowski, Kohler et al, vide supra). Therefore, SSTR5 antagonists could have the potential to beneficially influence insulin resistance in patients with NIDDM. In summary, SSTR5 antagonists are expected to beneficially influence NIDDM, the underlying impaired fasting glucose and impaired glucose tolerance, as well as complications of long-standing, insufficiently controlled diabetes mellitus.

GLP-1 is known as an endogenous regulator of food intake reducing appetite as shown in laboratory animals, healthy volunteers and patients with NIDDM (E. Näslund, B. Barkeling, N. King, M. Gutniak, J. E. Blundell, J. J. Holst, S. Rössner, P. M. Hellström Int. J. Obes. 1999,23,304-311; J.-P. Gutzwiller, B. Göke, J. Drewe, P. Hildebrand, S. Ketterer, D. Handschin, R. Winterhalder, D. Conen, C. Beglinger Gut 1999, 44, 81-88; J.-P. Gutzwiller, J. Drewe, B. Göke, H. Schmidt, B. Rohrer, J. Lareida, C. Beglinger Am. J. Physio/. 1999, 276, R1541-1544; M. D. Turton, D. O'Shea, I. Gunn, S. A. Beak, C. M. Edwards, K. Meeran, S. J. Choi, G. M. Taylor, M. M. Heath, P. D. Lambert, J. P. Wilding, D. M. Smith, M. A. Ghatei, J. Herbert, S. R. Bloom Nature 1996, 379, 69-72; A. Flint, A. Raben, A. Astrup, J. J. Holst J. Clin. Invest. 1998, 101, 515-520; M. B. Toft-Nielsen, S. Madsbad, J. J. Holst Diabetes Care 1999, 22, 1137-1143); thus, elevated GLP-1 will also counteract obesity, a typical condition associated with and leading to NIDDM.

GLP-1 is co-secreted with GLP-2 that is, consequently, also regulated by SST through SSTR5 (L. Hansen, B. Hartmann, T. Bisgaard, H. Mineo, P. N. Jørgensen, J. J. Holst Am. J. Phys. 2000, 278, E1010-1018). GLP-2 is enterotrophic and beneficial in patients with malabsorption of certain origins, such as short bowel syndrome (D. G. Burrin, B. Stoll, X. Guan Domest. Anim. Endocrino/, 2003, 24, 103-122; K. V. Haderslev, P. B. Jeppesen, B. Hartmann, J. Thulesen, H. A. Sorensen, J. Graff, B. S. Hansen, F. Tofteng, S. S. Poulsen, J. L. Madsen, J. J. Holst, M. Staun, P. B. Mortensen Scand. J. Gastroenterol. 2002, 37, 392-398; P. B: Jeppesen J. Nutr. 2003, 133, 3721-3724).

Moreover, there is increasing evidence for a role of SST on immune cells and expression of SSTR5 on activated T lymphocytes (T. Talme, J. Ivanoff, M. Hägglund, R. J. J. van Neerven, A. Ivanoff, K.G. Sundqvist Clin. Exp. Immunol. 2001, 125, 71-79; D. Ferone, P. M. van Haven, C. Semino, V. A. Dalm, A. Barreca, A. Colao, S. W. J. Lamberts, F. Minuto, L J. Hofland Dig. Liver Dis. 2004, 36, S68-77, C. E. Ghamrawy, C. Rabourdin-Combe, S. Krantic Peptides 1999, 20, 305-311). Consequently, SSTR5 antagonists could also prove valuable in treating diseases characterized by a disturbed immune system, such as inflammatory bowel disease.

It is therefore an object of the present invention to provide selective, directly acting SSTR5 antagonists. Such antagonists are useful as therapeutically active substances, particularly in the treatment and/or prevention of diseases which are associated with the modulation of SST receptors subtype 5.

Trisubstituted imidazopyrazines and dihydroimidazopyrazines have been disclosed as non-peptidic SST5 selective agonists by M.-O. Contour-Galcera et al. in Bioorganic & Medicinal Chemistry Letters 2001, 11(5), 741-745.

In the present description the term "alkyl", alone or in combination with other groups, refers to a branched or straight-chain monovalent saturated aliphatic hydrocarbon radical of one to twenty carbon atoms, preferably one to sixteen carbon atoms, more preferably one to ten carbon atoms.

The term "lower alkyl" or "C₁-C₇-alkyl", alone or in combination, signifies a straight-chain or branched-chain alkyl group with 1 to 7 carbon atoms, preferably a straight or branched-chain alkyl group with 1 to 4 carbon atoms. Examples of straight-chain and branched C₁-C₇ alkyl groups are methyl, ethyl, propyl, isopropyl, butyl, isobutyl, *tert-*butyl, the isomeric pentyls, the isomeric hexyls and the isomeric heptyls, preferably methyl and ethyl and most preferred the groups specifically exemplified herein.

The term "cycloalkyl" or "C₃₋₇-cycloalkyl" denotes a saturated carbocyclic group containing from 3 to 7 carbon atoms, such as cyclopropyl, cyclobutyl, cyclopentyl, cyclohexyl or cycloheptyl.

The term "alkoxy" refers to the group R'-O-, wherein R' is alkyl. The term "lower alkoxy" or "C₁-C₇-alkoxy" refers to the group R'-O-, wherein R' is lower alkyl and the term "lower alkyl" has the previously given significance. Examples of lower alkoxy groups are, *e. g.* methoxy, ethoxy, *n*-propoxy, isopropoxy, *n*-butoxy, isobutoxy, sec-butoxy and *tert-*butoxy, preferably methoxy and ethoxy and most preferred the groups specifically exemplified herein.

The term "cycloalkyloxy" or "C₃₋₇-cycloalkyloxy" refers to the group R"-O-, wherein R" is cycloalkyl as defined above. The cycloalkyl group can be further substituted by a carboxy group or a C₁-C₇-alkoxycarbonyl group. Preferred cycloalkoxy is cyclobutoxy.

The term "halogen" refers to fluorine, chlorine, bromine and iodine, with fluorine, chlorine and bromine being preferred.

The term "lower halogenalkyl" or "halogen-C₁₋₇-alkyl" refers to lower alkyl groups as defined above wherein at least one of the hydrogen atoms of the lower alkyl group is replaced by a halogen atom, preferably fluoro or chloro, most preferably fluoro. Among the preferred halogenated lower alkyl groups are trifluoromethyl, difluoromethyl, fluoromethyl and chloromethyl, with trifluoromethyl being especially preferred.

The term "lower halogenalkoxy" or "halogen-C₁₋₇-alkoxy" refers to lower alkoxy groups as defined above wherein at least one of the hydrogen atoms of the lower alkoxy group is replaced by a halogen atom, preferably fluoro or chloro, most preferably fluoro. Among the preferred halogenated lower alkyl groups are trifluoromethoxy, difluoromethoxy, fluormethoxy and chloromethoxy, with trifluoromethoxy being especially preferred.

The term "lower hydroxyalkoxy" or hydroxy-C₁₋₇-alkoxy" refers to lower alkoxy groups as defined above wherein at least one of the hydrogen atoms of the lower alkoxy group is replaced by a hydroxy group. Examples of lower hydroxyalkoxy groups are hydroxyethoxy or hydroxypropoxy.

The term "lower alkoxyalkyl" or "C₁₋₇-alkoxy-C₁₋₇-alkyl" refers to lower alkyl groups as defined above wherein at least one of the hydrogen atoms of the lower alkyl group is replaced by an alkoxy group as defined above. Among the preferred lower alkoxyalkyl groups are methoxymethyl, methoxyethyl and ethoxymethyl.

The term "lower alkoxyalkoxy" or "C₁₋₇-alkoxy-C₁₋₇-alkoxy" refers to lower alkoxy groups as defined above wherein at least one of the hydrogen atoms of the lower alkoxy group is replaced by an alkoxy group as defined above. Among the preferred lower alkoxyalkoxy groups are 2-methoxy-ethoxy and 3-methoxy-propoxy.

The term "cyano-C₁₋₇-alkoxy" refers to lower alkoxy groups as defined above wherein at least one of the hydrogen atoms of the lower alkoxy group is replaced by a cyano group. A preferred cyanoalkoxy group is cyanomethoxy.

The term "tetrazolyl-C₁₋₇-alkoxy" refers to a lower alkoxy group as defined above wherein at least one of the hydrogen atoms of the lower alkoxy group is replaced by a tetrazolyl group.

The term "heteroaryl" refers to an aromatic 5- or 6-membered ring which can comprise 1, 2 or 3 atoms selected from nitrogen, oxygen and/or sulphur such as furyl, pyrrolyl, thienyl, 1*H*-imidazolyl, 2*H*-imidazolyl, 4*H*-imidazolyl, 1*H*-pyrazoly 1,3*H-*pyrazolyl, 4*H*-pyrazolyl, 1,2-oxazolyl (isoxazolyl), 1,3-oxazolyl, 1*H*-[1,2,4]triazolyl, 4*H-*[1,2,4]triazolyl, 1*H*-[1,2,3]triazolyl, 2*H*-[1,2,3]triazolyl, 4*H*-[1,2,3]triazolyl, [1,2,4]oxadiazolyl, [1,3,4]oxadiazolyl, [1,2,3]oxadiazolyl, 1*H*-tetrazolyl, 2*H*-tetrazolyl, [1,2,3,4]oxatriazolyl, [1,2,3,5] oxatriazolyl, 1,3-thiazolyl, 1,2-thiazolyl (isothiazolyl), 1*H-*pentazolyl, pyridyl, pyrazinyl, pyrimidinyl, pyridazinyl, indolyl, quinolinyl and their dihydro derivatives. The term "heteroaryl" further refers to bicyclic aromatic groups comprising two 5- or 6-membered rings, in which one or both rings can contain 1, 2 or 3 atoms selected from nitrogen, oxygen or sulphur such as, *e. g*., indole or quinoline, or partially hydrogenated bicyclic aromatic groups such as, *e. g*., indolinyl. Preferred heteroaryl groups are pyridyl, pyrimidyl, tetrazolyl and imidazolyl, which can optionally be substituted as described above, preferably with C₁₋₇-alkyl.

The term "triazolyl" means a group selected from 1*H*-[1,2,4]triazolyl, 4*H-*[1,2,4]triazolyl, 1*H*-[1,2,3]triazolyl, 2*H*-[1,2,3]triazolyl and 4*H*-[1,2,3]triazolyl. Preferred is 1*H*-[1,2,4]triazolyl.

The term "carboxy" refers to the group -COOH.

The term "lower carboxyalkyl" or "carboxy-C₁₋₇-alkyl" refers to lower alkyl groups as defined above wherein at least one of the hydrogen atoms of the lower alkyl group is replaced by an carboxy group as defined above. Among the preferred lower carboxyalkyl groups are carboxymethyl, carboxyethyl and carboxypropyl.

The term "lower carboxyalkoxy" or "carboxy-C₁₋₇-alkoxy" refers to lower alkoxy groups as defined above wherein at least one of the hydrogen atoms of the lower alkoxy group is replaced by an carboxy group as defined above. An example for a lower carboxyalkoxy group is carboxyethoxy.

The term "alkoxycarbonyl" or "C₁₋₇-alkoxycarbonyl" refers to the group -CO-OR' wherein R' is lower alkyl and the term "lower alkyl" has the previously given significance. A preferred alkoxycarbonyl group is methoxycarbonyl.

The term "lower alkoxycarbonylalkyl" or "C₁₋₇-alkoxycarbonyl-C₁₋₇-alkyl" refers to lower alkyl groups as defined above wherein at least one of the hydrogen atoms of the lower alkyl group is replaced by an alkoxycarbonyl group as defined herein before. Preferred lower alkoxycarbonylalkyl groups are methoxycarbonylmethyl or tert-butoxycarbonylmethyl.

The term "lower alkoxycarbonylalkoxy" or "C₁₋₇-alkoxycarbonyl-C₁₋₇-alkoxy" refers to lower alkoxy groups as defined above wherein at least one of the hydrogen atoms of the lower alkoxy group is replaced by an alkoxycarbonyl group as defined herein before. An example for a lower alkoxycarbonylalkoxy group is methoxycarbonylmethoxy.

The term "alkylsulfonyl" refers to the group R'-SO₂-, wherein R' is alkyl. The term "lower alkylsulfonyl" or "C₁₋₇-alkylsulfonyl" refers to the group R'-SO₂-, wherein R' is lower alkyl. Examples of lower alkylsulfonyl groups are e.g. methylsulfonyl or ethylsulfonyl.

The term "lower alkylsulfonyloxy" " or "C₁₋₇-alkylsulfonyloxy" refers to the group R'-SO₂-O-, wherein R' is lower alkyl. Compounds containing lower alkylsulfonyloxy groups are for example the esters of methanesulfonic acid.

The term "lower alkylsulfonyl-alkoxy" or "C₁₋₇-alkylsulfonyl-C₁₋₇-alkoxy" refers to lower alkoxy groups as defined above wherein at least one of the hydrogen atoms of the lower alkoxy group is replaced by a lower alkylsulfonyl group as defined above. A preferred lower alkylsulfonyl-alkoxy group is methylsulfonylbutoxy.

The term amino refers to the group -NH₂.

The term "alkylamino" or "C₁₋₇-alkylamino" refers to the group -NHR', wherein R' is lower alkyl and the term "lower alkyl" has the previously given significance. A preferred alkylamino group is methylamino.

The term "amino-C₁₋₇-alkoxy" refers to lower alkoxy groups as defined above wherein at least one of the hydrogen atoms of the lower alkoxy group is replaced by an amino group. Examples for aminoalkoxy groups are aminomethoxy or 2-aminoethoxy.

The term "aminocarbonyl" refers to the group -CO-NH₂.

The term "alkylaminocarbonyl" or "C₁₋₇-alkylaminocarbonyl" refers to the group -CO-NHR' wherein R' is lower alkyl and the term "lower alkyl" has the previously given significance. A preferred alkylaminocarbonyl group is tert-butylaminocarbonyl.

The term "aminocarbonylalkoxy" or "aminocarbonyl-C₁₋₇-alkoxy" refers to lower alkoxy groups as defined above wherein at least one of the hydrogen atoms of the lower alkoxy group is replaced by an aminocarbonyl group as defined herein before. A preferred lower alkoxycarbonylalkyl group is aminocarbonylmethoxy.

The term "alkylaminocarbonylalkoxy" or "C₁₋₇-alkylaminocarbonyl-C₁₋₇-alkoxy" refers to lower alkoxy groups as defined above wherein at least one of the hydrogen atoms of the lower alkoxy group is replaced by an alkylaminocarbonyl group as defined herein before. A preferred alkylaminocarbonylalkoxy group is tert-butylaminocarbonylmethoxy.

The term "pharmaceutically acceptable salts" refers to those salts which retain the biological effectiveness and properties of the free bases or free acids, which are not biologically or otherwise undesirable. The salts are formed with inorganic acids such as hydrochloric acid, hydrobromic acid, sulfuric acid, nitric acid, phosphoric acid and the like, preferably hydrochloric acid, and organic acids such as acetic acid, propionic acid, glycolic acid, pyruvic acid, oxylic acid, maleic acid, malonic acid, salicylic acid, succinic acid, fumaric acid, tartaric acid, citric acid, benzoic acid, cinnamic acid, mandelic acid, methanesulfonic acid, ethanesulfonic acid, *p*-toluenesulfonic acid, salicylic acid, *N-*acetylcystein and the like. In addition these salts may be prepared form addition of an inorganic base or an organic base to the free acid. Salts derived from an inorganic base include, but are not limited to, the sodium, potassium, lithium, ammonium, calcium, magnesium salts and the like. Salts derived from organic bases include, but are not limited to salts of primary, secondary, and tertiary amines, substituted amines including naturally occurring substituted amines, cyclic amines and basic ion exchange resins, such as isopropylamine, trimethylamine, diethylamine, triethylamine, tripropylamine, ethanolamine, lysine, arginine, *N*-ethylpiperidine, piperidine, polymine resins and the like. The compound of formula I can also be present in the form of zwitterions. Particularly preferred pharmaceutically acceptable salts of compounds of formula I are the hydrochloride salts.

The compounds of formula I can also be solvated, *e. g*., hydrated. The solvation can be effected in the course of the manufacturing process or can take place, *e. g*., as a consequence of hygroscopic properties of an initially anhydrous compound of formula I (hydration). The term pharmaceutically acceptable salts also includes physiologically acceptable solvates.

"Isomers" are compounds that have identical molecular formulae but that differ in the nature or the sequence of bonding of their atoms or in the arrangement of their atoms in space. Isomers that differ in the arrangement of their atoms in space are termed "stereoisomers". Stereoisomers that are not mirror images of one another are termed "diastereoisomers", and stereoisomers that are non-superimposable mirror images are termed "enantiomers", or sometimes optical isomers. A carbon atom bonded to four nonidentical substituents is termed a "chiral center".

In detail, the present invention relates to compounds of the general formula 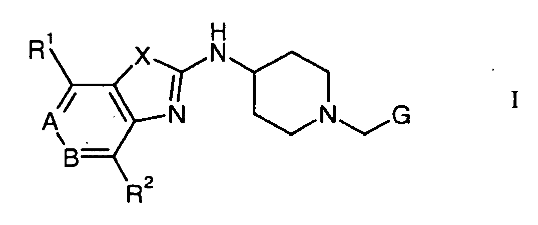 wherein
- X: is S or O;
- A: is CR³ and B is CR
⁴, or A is N or N⁺-O⁻ and B is CR⁴, or
Bis N or N⁺-O⁻ and A is CR³;
- R¹ and R²: are independently selected from the group consisting of hydrogen, halogen, cyano, nitro, C₁₋₇-alkyl, hydroxy-C₁₋₇-alkyl, C₁₋₇-alkoxy-C₁₋₇-alkyl, C₁₋₇-alkoxy, hydroxy-C₂₋₇-alkoxy, dihydroxy-C₁₋₇-alkoxy, carboxy-C₁₋₇-alkoxy, C₁₋₇₋alkoxycarbon yl-C₁₋₇-alkoxy, carboxy-C₁₋₇-alkyl, C₁₋₇-alkoxycarbonyl-C₁₋₇-alkyl, 1*H*-tetrazol-5-yl-C₁₋₇-alkoxy, pyridinyl-C₁₋₇-alkoxy, -NR⁵R⁶ -NHCOR⁷, -NHSO₂R⁸, -SO₂NR⁹R¹⁰, 1*H*-tetrazol-5-yl, unsubstituted phenyl and phenyl substituted by one to three substituents selected from C₁₋₇-alkyl, C₃₋₇-cycloalkyl, halogen, halogen-C₁₋₇-alkyl and C₁₋₇-alkoxy; and
- R²: can additionally also be selected from the group consisting of carboxy, C₁₋₇-alkoxycarbonyl and -CONR¹¹R¹²;
- R⁵ and R⁶: independently from each other are selected from the group consisting of hydrogen, C₁₋₇-alkyl, hydroxy-C₁₋₇-alkyl and C₃₋₇-cycloalkyl;
- R⁷: is selected from the group consisting of C₁₋₇-alkyl, C₃₋₇-cycloalkyl, halogen-C₁₋₇-alkyl, hydroxy-C₁₋₇-alkyl, C₁₋₇-alkoxy-C₁₋₇-alkyl, carboxy-C₁₋₇-alkyl. C₁₋₇-alkoxycarbonyl-C₁₋₇-alkyl, 1*H*-tetrazol-5-yl-C₁₋₇-alkyl, unsubstituted phenyl, phenyl substituted by one to three groups selected from the group consisting of C₁₋₇-alkyl, C₃₋₇-cycloalkyl, C₁₋₇-alkoxy, halogen-C₁₋₇-alkyl and halogen, unsubstituted heteroaryl, heteroaryl substituted by one or two groups
- R⁸: is selected from C₁₋₇-alkyl, C₃₋₇-cycloalkyl, C₁₋₇alkoxy, halogen-C₁₋₇-alkyl or halogen; unsubstituted heteroaryl-C₁₋₇-alkyl and heteroaryl-C₁₋₇-alkyl, wherein the heteroaryl is substituted by one or two groups selected from C₁₋₇-alkyl, C₃₋₇- cycloalkyl, C₁₋₇-alkoxy, halogen-C₁₋₇-alkyl and halogen; selected from the group consisting of C₁₋₇-alkyl, C₃₋₇-cycloalkyl, unsubstituted heteroaryl and heteroaryl substituted by one or two groups selected from C₁₋₇-alkyl, C₃₋₇-cycloalkyl, C₁₋₇-alkoxy, halogen-C₁₋₇-alkyl or halogen;
- R⁹ and R¹⁰: independently from each other are selected from the group consisting of hydrogen, C₁₋₇-alkyl, C₃₋₇-cycloalkyl, unsubstituted heteroaryl and heteroaryl substituted by one or two groups selected from C₁₋₇-alkyl, C₃₋₇-cycloalkyl, C₁₋₇-alkoxy, halogen- C₁₋₇-alkyl or halogen; or R⁹ and R¹⁰ together with the nitrogen atom they are attached to form a pyrrolidine or a piperidine ring;
- R¹¹: is selected from the group consisting of hydrogen, C₁₋₇-alkyl and C₃₋₇-cycloalkyl;
- R¹²: is selected from the group consisting of hydrogen, C₁₋₇-alkyl, C₃₋₇-cycloalkyl, halogen-C₁₋₇-alkyl, hydroxy-C₁₋₇-alkyl, C₁₋₇alkoxy-C₁₋₇alkyl, carboxy-C₁₋₇-alkyl, C₁₋₇-alkoxycarbonyl-C₁₋₇-alkyl, unsubstituted phenyl, phenyl substituted by one to three groups selected from the group consisting of C₁₋₇-alkyl, C₃₋₇-cycloalkyl, C₁₋₇-alkoxy, halogen-C₁₋₇-alkyl and halogen, unsubstituted heteroaryl, heteroaryl substituted by one or two groups selected from C₁₋₇-alkyl, C₃₋₇-cycloalkyl, C₁₋₇-alkoxy, halogen-C₁₋₇-alkyl or halogen;
- one of R³: and R⁴ is selected from the group consisting of hydrogen, C₁₋₇-alkyl and C₁₋₇- alkoxy, or is absent in case one of A or B is N or N⁺-O⁻, and
- the other one of R³ and R⁴: is selected from the group consisting of hydrogen, C₁₋₇-alkyl, C₁₋₇-alkoxy, hydroxy, hydroxy-C₁₋₇-alkyl, cyano-C₁₋₇-alkoxy, C₃₋₇-cycloalkyloxy wherein the cycloalkyl group is substituted by carboxy or C₁₋₇-alkoxy-carbonyl, carboxy, C₁₋₇-alkoxy-carbonyl, carboxy-C₁₋₇-alkoxy, C₁₋₇-alkoxy-carbonyl-C₁₋₇-alkyl, carboxy-C₁₋₇-alkyl, C₁₋₇-alkoxy-carbonyl-C₁₋₇-alkoxy, 1*H*-tetrazol-5-yl-C₁₋₇-alkoxy, triazolyl-C₁₋₇-alkoxy, C₁₋₇-alkylsulfonyloxy, C₁₋₇-alkylsulfonyl-C₁₋₇-alkoxy, hydroxy-C₁₋₇-alkoxy, dihydroxy-C₃₋₇-alkoxy, C₁₋₇-alkoxy-C₁₋₇-alkoxy, -(CH₂)ₙ-NR¹³R¹⁴, -(CH₂)ₙ-NHCOR¹⁵, amino-C₁₋₇-alkoxy, aminocarbonyl-C₁₋₇-alkoxy, C₁₋₇-alkylaminocarbonyl-C₁₋₇-alkoxy and 1*H*-tetrazol-5-yl;
- R¹³ and R¹⁴: independently from each other are selected from the group consisting of hydrogen, C₁₋₇-alkyl and C₃₋₇-cycloalkyl;
- R¹⁵: is selected from the group consisting of C₁₋₇-alkyl, C₃₋₇-cycloalkyl, halogen-C₁₋₇-alkyl, hydroxy-C₁₋₇-alkyl, C₁₋₇-alkoxy-C₁₋₇-alkyl, carboxy-C₁₋₇-alkyl and C₁₋₇-alkoxycarbonyl-C₁₋₇-alkyl;
- n: is an integer selected from 1, 2 and 3;
provided that benzooxazoles and benzothiazoles wherein R¹, R², R³ and R⁴ are hydrogen are excluded;
- G: is selected from the groups 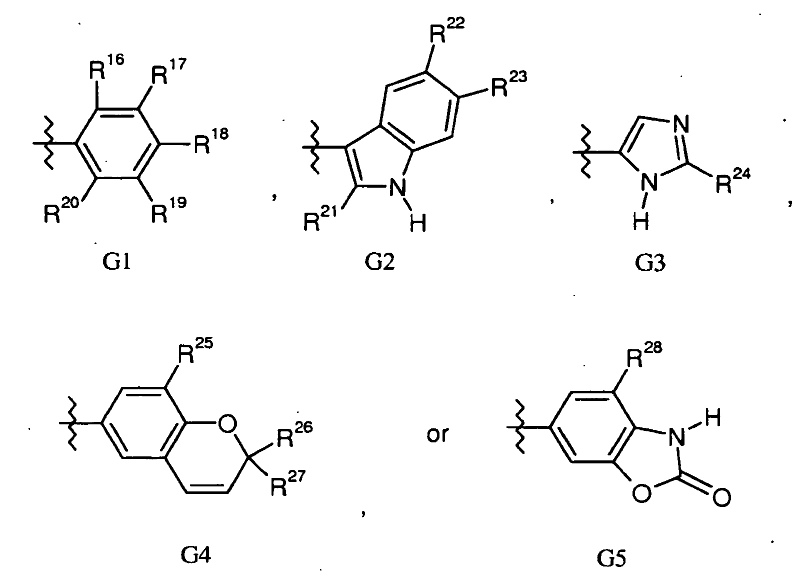
wherein
- R¹⁶: is hydrogen or halogen;
- R¹⁷: is selected from the group consisting of C₁₋₇-alkoxy, C₂₋₇-alkenyloxy, C₃₋₇-cycloalkyloxy, -NR²⁹R³⁰, halogen-C₁₋₇-alkoxy, C₁₋₇-alkoxy-C₁₋₇-alkoxy, C₁₋₇-alkoxy-C₁₋₇-alkyl; R¹⁹ and R³⁰ independently from each other are hydrogen or C₁₋₇-alkyl;
- R¹⁸: is selected from the group consisting of hydrogen, C₁₋₇-alkyl, halogen-C₁₋₇-alkyl, hydroxy, C₁₋₇-alkoxy, halogen-C₁₋₇-alkoxy, C₁₋₇-cycloalkyloxy, halogen, pyrrolyl, imidazolyl, triazolyl, -CO₂R³¹, -NR³²R³³, -SOR³⁴; unsubstituted phenyl and phenyl substituted by one to two groups selected from the group consisting of C₁₋₇-alkyl, halogen-C₁₋₇-alkyl, halogen-C₁₋₇-alkoxy, C₁₋₇-alkoxy and halogen;
- R³¹: is hydrogen or C₁₋₇-alkyl;
- R³² and R³³: independently from each other are hydrogen or C₁₋₇-alkyl;
- R³⁴: is C₁₋₇-alkyl;
- R¹⁹: is selected from the group consisting of hydrogen, C₁₋₇-alkyl, halogen, C₁₋₇-alkoxy, C₂₋₇-alkenyloxy, -O-tetrahydropyranyl, C₃₋₇-cycloalkyloxy, halogen-C₁₋₇-alkoxy, C₁₋₇-alkoxy-C₁₋₇-alkoxy, and C₁₋₇-alkoxy-C₁₋₇-alkyl;
- R²⁰: is hydrogen or halogen;
- R²¹: is hydrogen or C₁₋₇-alkyl;
- R²² and R²³: independently from each other are selected from the group consisting of hydrogen, C₁₋₇-alkyl, C₁₋₇-alkoxy and nitro;
- R²⁴: is unsubstituted phenyl or phenyl substituted by one to two groups selected from the group consisting of C₁₋₇-alkyl, C₁₋₇-alkoxy and halogen;
- R¹⁵: is C₁₋₇-alkoxy;
- R²⁶ and R²⁷: independently from each other are C₁₋₇-alkyl;
- R²⁸: is C₁₋₇-alkoxy;
and pharmaceutically acceptable salts thereof.

Preferred compounds of formula I according to the present invention are those, wherein X is O.

Also preferred are compounds of formula I according to the present invention, wherein A is CR³ and B is CR⁴ and wherein one of R³ and R⁴ is selected from the group consisting of hydrogen, C₁₋₇-alkyl and C₁₋₇-alkoxy and
the other one of R³ and R⁴ is selected from the group consisting of hydrogen, C₁₋₇-alkyl, C₁₋₇-alkoxy, hydroxy, hydroxy-C₁₋₇-alkyl, cyano-C₁₋₇-alkoxy, C₃₋₇-cycloalkyloxy wherein the cycloalkyl group is substituted by carboxy or C₁₋₇-alkoxy-carbonyl, carboxy, C₁₋₇-alkoxy-carbonyl, carboxy-C₁₋₇-alkoxy, C₁₋₇-alkoxy-carbonyl-C₁₋₇-alkyl, carboxy-C₁₋₇-alkyl, C₁₋₇-alkoxy-carbonyl-C₁₋₇-alkoxy, 1*H*-tetrazol-5-yl-C₁₋₇-alkoxy, triazolyl-C₁₋₇-alkoxy, C₁₋₇-alkylsulfonyloxy, C₁₋₇-alkylsulfonyl-C₁₋₇-alkoxy, hydroxy-C₂₋₇-alkoxy, dihydroxy-C₃₋₇-alkoxy, C₁₋₇-alkoxy-C₁₋₇-alkoxy,
-(CH₂)ₙ-NR¹³R¹⁴ -(CH₂)ₙ-NHCOR¹⁵, amino-C₁₋₇-alkoxy, aminocarbonyl-C₁₋₇-alkoxy, C₁₋₇-alkylaminocarbonyl-C₁₋₇-alkoxy and 1*H*-tetrazol-5-yl, and wherein R¹³ , R¹⁴, R¹⁵ and n are as defined herein before.

Furthermore, compounds of formula I according to the invention are preferred,
wherein A is CR³ and B is CR⁴ and wherein
one of R³ and R⁴ is selected from the group consisting of hydrogen, C₁₋₇-alkyl and C₁₋₇-alkoxy and
the other one of R³ and R⁴ is selected from the group consisting of hydrogen, C₁₋₇-alkyl, C₁₋₇-alkoxy, hydroxy-C₁₋₇-alkyl, carboxy, C₁₋₇-alkoxy-carbonyl, carboxy-C₁₋₇-alkoxy, C₁₋₇-alkoxy-carbonyl-C₁₋₇-alkyl, carboxy-C₁₋₇-alkyl, C₁₋₇-alkoxy-carbonyl-C₁₋₇-alkoxy, 1*H*-tetrazol-5-yl-C₁₋₇-alkoxy, hydroxy-C₂₋₇-alkoxy, dihydroxy-C₃₋₇-alkoxy, -(CH₂)ₙ-NR¹³R¹⁴, -(CH₂)ₙ-NHCOR¹⁵, and 1*H*-tetrazol-5-yl, and wherein R¹³ , R¹⁴, R¹⁵ and n are as defined herein before.

A preferred group of compounds of formula I of the present invention are those, wherein A is CR³, B is CR⁴, R³ is hydrogen and R⁴ is selected from the group consisting of C₁₋₇-alkyl, C₁₋₇-alkoxy, hydroxy, hydroxy-C₁₋₇-alkyl, cyano-C₁₋₇-alkoxy, C₃₋₇-cycloalkyloxy wherein the cycloalkyl group is substituted by carboxy or C₁₋₇-alkoxycarbonyl, carboxy, C₁₋₇-alkoxy-carbonyl, carboxy-C₁₋₇-alkoxy, C₁₋₇-alkoxy-carbonyl-C₁₋₇-alkyl, carboxy-C₁₋₇-alkyl, C₁₋₇-alkoxy-carbonyl-C₁₋₇-alkoxy, 1*H*-tetrazot-5-yl-C₁₋₇-alkoxy, triazolyl-C₁₋₇-alkoxy, C₁₋₇-alkylsulfonyloxy, C₁₋₇-alkylsulfon yl-C₁₋₇-alkoxy, hydroxy-C₂₋₇-alkoxy, dihydroxy-C₃₋₇-alkoxy, C₁₋₇-alkoxy-C₁₋₇-alkoxy, -(CH₂)ₙ-NR¹³R¹⁴, -(CH₂)ₙ-NHCOR¹⁵, amino-C₁₋₇-alkoxy, aminocarbonyl-C₁₋₇-alkoxy, C₁₋₇-alkylaminocarbonyl-C₁₋₇-alkoxy, and 1*H*-tetrazol-5-yl, and wherein R¹³, R¹⁴, R¹⁵ and n are as defined herein before.

Within this group, those compounds, wherein R⁴ is carboxy-C₁₋₇-alkoxy or C₁₋₇-alkoxy-carbonyl-C₁₋₇-alkoxy, are especially preferred: Also preferred are those compounds, wherein R⁴ is -(CH₂)ₙ-NR¹³R¹⁴, with compounds wherein R¹³ and R¹⁴ are hydrogen being especially preferred. Preferred n is 1. Also preferred are compounds, wherein R⁴ is selected from the group consisting of dihydroxy-C₃₋₇-alkoxy, carboxy, cyano C₁₋₇-alkoxy, aminocarbonyl-C₁₋₇-alkoxy and C₁₋₇-alkylaminocarbonyl-C₁₋₇-alkoxy.

Another group of preferred compounds of formula I according to the present invention are those, wherein R⁴ is hydrogen and R³ is selected from the group consisting of C₁₋₇-alkyl, C₁₋₇-alkoxy, hydroxy-C₁₋₇-alkyl, carboxy, C₁₋₇-alkoxy-carbonyl, carboxy-C₁₋₇-alkoxy, C₁₋₇-alkoxy-carbonyl-C₁₋₇-alkyl, carboxy-C₁₋₇-alkyl, C₁₋₇-alkoxy-carbonyl-C₁₋₇-alkoxy, 1*H*-tetrazol-5-yl-C₁₋₇-alkoxy, hydroxy-C₂₋₇-alkoxy, dihydroxy-C₃₋₇-alkoxy, -(CH₂)ₙ₋NR¹³R¹⁴, -(CH₂)ₙ-NHCOR¹⁵, and 1*H*-tetrazol-5-yl, and wherein R¹³, R¹⁴, R¹⁵ and n are as defined herein before.

Within this group, those compounds, wherein R³ is carboxy or C₁₋₇-alkoxycarbonyl, are especially preferred.

Furthermore, compounds of formula I are preferred, wherein R³ and R⁴ are hydrogen, provided that benzooxazoles and benzothiazoles wherein R¹, R², R³ and R⁴ are hydrogen are excluded.

A further group of preferred compounds of formula I according to the present invention are those, wherein A is N, B is CR⁴ and R⁴ is selected from the group consisting of hydrogen, C₁₋₇-alkyl, C₁₋₇-alkoxy, hydroxy, hydroxy-C₁₋₇-alkyl, cyano-C₁-₇-alkoxy, C₃₋₇-cycloalkyloxy wherein the cycloalkyl group is substituted by carboxy or C₁₋₇-alkoxy-carbonyl, carboxy, C₁₋₇-alkoxy-carbonyl, carboxy-C₁₋₇-alkoxy, C₁₋₇-alkoxy-carbonyl-C₁₋₇-alkyl, carboxy-C₁₋₇-alkyl, C₁₋₇-alkoxy-carbonyl-C₁₋₇-alkoxy, 1*H*-tetrazol-5-yl-C₁₋₇-alkoxy, triazolyl-C₁₋₇-alkoxy, C₁₋₇-alkylsulfonyloxy, C₁₋₇-alkylsufonyl-C₁₋₇-alkoxy, hydroxy-C₂₋₇-alkoxy, dihydroxy-C₃₋₇-alkoxy, C₁₋₇-alkoxy-C₁₋₇-alkoxy, -(CH₂)ₙ-NR¹³R¹⁴, -(CH₂)ₙ-NHCOR¹⁵, amino-C₁₋₇-alkoxy, aminocarbonyl-C₁₋₇-alkoxy, C₁₋₇-alkylaminocarbonyl-C₁₋₇-alkoxy and 1*H*-tetrazol-5-yl, and wherein R¹³, R¹⁴ R¹⁵ and n are as defined herein before.

Especially preferred are compounds of the present invention, wherein A is N and B is CH.

Furthermore, compounds of formula I are preferred, wherein A is N, B is CR⁴ and wherein R¹ and R² are hydrogen.

Also preferred are compounds of formula I according to the present invention,
wherein
- R¹: is selected from the group consisting of halogen, cyano, nitro, C₁₋₇-alkyl, hydroxy-C₁₋₇-alkyl, C₁₋₇-alkoxy-C₁₋₇-alkyl, C₁₋₇-alkoxy, hydroxy-C₂₋₇-alkoxy, dihydroxy-C₃₋₇-alkoxy, carboxy-C₁₋₇-alkoxy, C₁₋₇-alkoxycarbonyl-C₁₋₇-alkoxy, carboxy-C₁₋₇-alkyl, C₁₋₇-alkoxycarbonyl-C₁₋₇-alkyl, 1*H*-tetrazol-5-yl-C₁₋₇-alkcoxy, pyridinyl-C₁₋₇-alkoxy, -NR⁵R⁶, -NHCOR⁷, -NHSO₂R⁸, -SO₂NR⁹R¹⁰, 1*H*-tetrazol-5-yl, unsubstituted phenyl and phenyl substituted by one to three substituents selected from C₁₋₇-alkyl, C₃₋₇-cycloalkyl, halogen, hatogen-C₁₋₇-alkyl and C₁₋₇-alkoxy; and wherein R⁵ to R¹⁰ are as defined herein before, and
- R²: is hydrogen.

Within this group, those compounds of formula I are preferred, wherein R¹ is selected from the group consisting of halogen, nitro, C₁₋₇-alkyl, hydroxy-C₁₋₇-alkyl, C₁₋₇-alkoxy, dihydroxy-C₃₋₇-alkoxy, carboxy-C₁₋₇-alkoxy, C₁₋₇-alkoxycarbonyl-C₁₋₇-alkoxy, -NR⁵R⁶, -NHCOR⁷, -NHSO₂R⁸, -SO₂NR⁹R¹⁰, unsubstituted phenyl and phenyl substituted by one to three substituents selected from C₁₋₇-alkyl, C₃₋₇-cycloalkyl, halogen, halogen-C₁₋₇-alkyl and C₁₋₇-alkoxy; and wherein R⁵ to R¹⁰ are as defined herein before, with those compounds, wherein R¹ is -NR⁵R⁶ or -NHCOR⁷, and wherein R⁵ to R⁷ are as defined hereinbefore, being more preferred.

Especially preferred are compounds, wherein R¹ is -NR⁵R⁶ and R⁵ and R⁶ are hydrogen, or compounds, wherein R¹ is -NHCOR⁷ and R⁷ is selected from the group consisting ofC₁₋₇-alkoxy-C₁₋₇-alkyl, carboxy-C₁₋₇-alkyl and unsubstituted heteroaryl, preferably pyrimidinyl or pyridyl.

Also preferred are compounds, wherein R¹ is NHSO₂R⁸ and R⁸ is C₁₋₇-alkyl or imidazolyl substituted by one or two groups selected from selected from C₁₋₇-alkyl, C₃₋₇-cycloalkyl, C₁₋₇-alkoxy, halogen-C₁₋₇-alkyl or halogen.

Further preferred compounds are those, wherein R¹ is -SO₂NR⁹R¹⁰ and R⁹ and R¹⁰ independently from each other are selected from the group consisting of hydrogen or C₁₋₇-alkyl, or R⁹ and R¹⁰ together with the nitrogen atom they are attached to form a pyrrolidine or a piperidine ring.

Furthermore, a group of preferred compounds of formula I according to the present invention are those, wherein
- R¹: is hydrogen and
- R²: is selected from the group consisting of halogen, cyano, nitro, C₁₋₇-alkyl, hydroxy-C₁₋₇-alkyl, C₁₋₇-alkoxy-C₁₋₇-alkyl,C₁₋₇-alkoxy, hydroxy-C₂₋₇-alkoxy, dihydroxy-C₃₋₇-alkoxy, carboxy-C₁₋₇-alkoxy, C₁₋₇-alkoxycarbonyl-C₁₋₇-alkoxy, carboxy-C₁₋₇-alkyl, C₁₋₇-alkoxycarbonyl-C₁₋₇-alkyl,1*H*-tetrazol-5-yl-C₁₋₇-alkoxy, pyridinyl-C₁₋₇-alkoxy, -NR⁵R⁶, -NHCOR⁷, -NHSO₂R⁸, -SO₂NR⁹R¹⁰, carboxy, C₁₋₇-alkoxycarbonyl, -CONR¹¹R¹², 1*H*-tetrazol-5-yl, unsubstituted phenyl and phenyl substituted by one to three substituents selected from C₁₋₇-alkyl, C₃₋₇-cycloalkyl, halogen, halogen-C₁₋₇-alkyl and C₁₋₇-alkoxy; and wherein R⁵ to R¹² are as defined herein before.

Within this group, those compounds of formula I are preferred, whereinR² is selected from the group consisting of halogen, nitro, C₁₋₇-alkoxy, pyridinyl-C₁₋₇-alkoxy, -NR⁵R⁶, -NHCOR⁷, carboxy, C₁₋₇-alkoxycarbonyl, -CONR¹¹R¹²; and wherein R⁵ to R⁷, R¹¹ and R¹² are as defined herein before, with those compounds, wherein R² is -NHCOR⁷ and R⁷ is as defined herein before, being more preferred.

Especially preferred are those compounds of formula I, wherein R² is -NHCOR⁷ and R⁷ is selected from the group consisting of C₁₋₇alkoxy-C₁₋₇-alkyl, carboxy-C₁₋₇-alkyl, C₁₋₇-alkoxycarbonyl-C₁₋₇-alkyl, unsubstituted heteroaryl, preferably pyrimidinyl, and unsubstituted beteroaryl-C₁₋₇-alkyl, preferably, tetrazolyl-C₁₋₇-alkyl.

Also preferred are compounds, wherein R² is carboxy, C₁₋₇-alkoxycarbonyl and -CONR¹¹R¹² and R¹¹ and R¹² are as defined herein before. Especially preferred are those compounds of formula I, wherein R² is -CONR¹¹R¹², R¹¹ is hydrogen and R¹² is selected from the group consisting of hydroxy-C₁₋₇-alkyl, C₁₋₇-alkcoxy-C₁₋₇-alkyl, carboxy-C₁₋₇-alkyl, C₁₋₇-alkoxycarbonyl-C₁₋₇-alkyl and unsubsituted heteroaryl, preferably pyridyl.

Furthermore, compounds of formula I according to the present invention are preferred, wherein G is 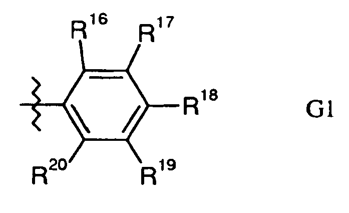 and wherein
- R¹⁶: is hydrogen or halogen;
- R¹⁷: is selected from the group consisting of C₁₋₇-alkoxy, C₂₋₇-alkenyloxy, C₃₋₇-cycloalkyloxy, -NR²⁹R³⁰, halogen-C₁₋₇-alkoxy, C₁₋₇-alkoxy-C₁₋₇-alkoxy, C₁₋₇-alkoxy-C₁₋₇-alkyl; R²⁹ and R³⁰ independently from each other are hydrogen or C₁₋₇-alkyl;
- R¹⁸: is selected from the group consisting of hydrogen, C₁₋₇-alkyl, halogen-C₁₋₇-alkyl, hydroxy, C₁₋₇-alkoxy, halogen-C₁₋₇-alkoxy, C₃₋₇-Cycloalkyloxy, halogen, pyrrolyl, imidazolyl, triazolyl, -CO₂R³¹, -NR³²R³³ , -SOR³⁴; unsubstituted phenyl and phenyl substituted by one to two groups selected from the group consisting of C₁₋₇-alkyl, halogen-C₁₋₇-alkyl, halogen-C₁₋₇-alkoxy, C₁₋₇-alkoxy and halogen; R³¹ is hydrogen or C₁₋₇-alkyl; R³² and R³³ independently from each other are hydrogen or C₁₋₇-alkyl; R³⁴ is C₁₋₇-alkyl;
- R¹⁹: is selected from the group consisting of hydrogen, C₁₋₇-alkyl, halogen, C₁₋₇-alkoxy, C₂₋₇-alkenyloxy, -O-tetrahydropyranyl, C₃₋₇-cycloalkyloxy, halogen-C₁₋₇-alkoxy, C₁₋₇-alkoxy-C₁₋₇-alkoxy, and C₁₋₇-alkoxy-C₁₋₇-alkyl; and
- R²⁰: is hydrogen or halogen. Within this group, those compounds are preferred, wherein R¹⁷ is C₁₋₇-alkoxy or halogen-C₁₋₇-alkoxy.

More preferably, R¹⁷ is ethoxy, isopropyloxy or isobutyloxy.

Preferred are furthermore compounds, wherein R¹⁸ is selected from the group consisting of hydrogen, C₁₋₇-alkyl, hydroxy, C₁₋₇-alkoxy, halogen, pyrrolyl, imidazolyl, triazolyl, -NR³²R³³ and -SOR³⁴, and R³² and R³³ are independently from each other hydrogen or C₁₋₇-alkyl, and R³⁴ is C₁₋₇-alkyl.

More preferably, R¹⁸ is selected from the group consisting of hydrogen, halogen, pyrrolyl, triazolyl and -NR³²R³³, wherein R³² and R³³ are hydrogen.

R¹⁶ is preferably hydrogen.

R¹⁹ is selected from the group consisting of hydrogen, C₁₋₇-alkyl, halogen, C₁₋₇-alkoxy, C₂₋₇-alkenyloxy, -O-tetrahydropyranyl, C₃₋₇-cycloalkyloxy, halogen-C₁₋₇-alkoxy, C₁₋₇-alkoxy-C₁₋₇-alkoxy, and C₁₋₇-alkoxy-C₁₋₇-alkyl. More preferably, R¹⁹ is hydrogen or C₁₋₇-alkoxy, and most preferably, R¹⁹ is ethoxy or isopropyloxy.

Preferably, R²⁰ is hydrogen.

Also preferred are compounds of the present invention, wherein G is selected from 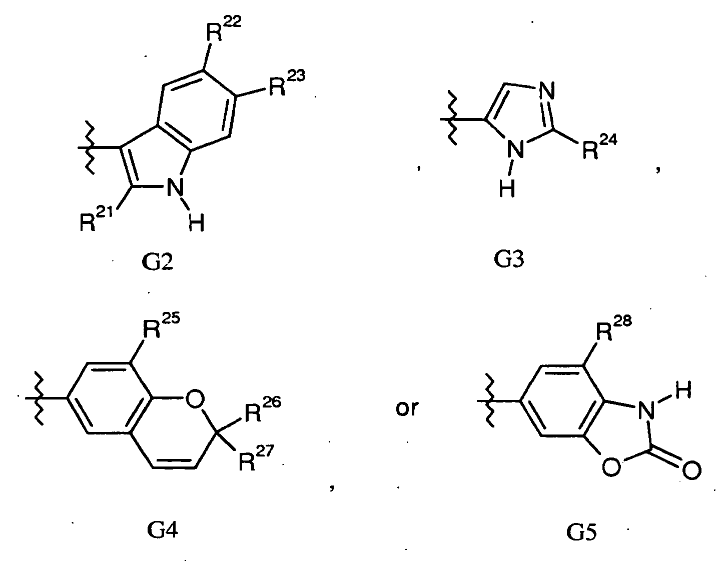 and wherein
- R²¹: is hydrogen or C₁₋₇-alkyl;
- R²² and R²³: independently from each other are selected from the group consisting of hydrogen, C₁₋₇-alkyl, C₁₋₇-alkoxy and nitro;
- R²⁴: is unsubstituted phenyl or phenyl substituted by one to two groups selected from the group consisting of C₁₋₇-alkyl, C₁₋₇-alkoxy and halogen;
- R²⁵: is C₁₋₇-alkoxy;
- R²⁶ and R²⁷: independently from each other are C₁₋₇-alkyl; and
- R²⁸: is C₁₋₇-alkoxy.

Preferred are further compounds of formula I according to the present invention, wherein
- X: is S or O;
- A: is CR³ and B is CR⁴, or
A is N or N⁺-O⁻ and B is CR⁴, or
B is N or N⁺-O⁻ and A is CR³
- R¹ and R²: are independently selected from the group consisting of hydrogen halogen, cyano, nitro, C₁₋₇-alkyl, hydroxy-C₁₋₇-alkyl, C₁₋₇-alkoxy-C₁₋₇-alkyl, C₁₋₇-alkoxy, hydroxy-C₁₋₇-alkoxy, dihydroxy-C₃₋₇-alkoxy, carboxy-C₁₋₇-alkoxy, C₁₋₇-alkoxycarbonyl-C₁₋₇-alkoxy, carboxy-C₁₋₇-alkyl, C₁₋₇-alkoxycarbonyl-C₁₋₇-alkyl, 1*H*-tetrazol-5-yl-C₁₋₇-alkoxy, pyridinyl-C₁₋₇-alkoxy, -NR⁵R⁶ -NHCOR⁷ -NHSO₂R⁸ -SO₂NR⁹R¹⁰ 1*H*-tetrazol-5-yl, unsubstituted phenyl and phenyl substituted by one to three substituents selected from C₁₋₇-alkyl, C₃₋₇-cycloalkyl, halogen, halocyen-C₁₋₇-alkyl and C₁₋₇-alkoxy; and
- R²: can additionally also be selected from the group consisting of carboxy, C₁₋₇-alkoxycarbonyl and -CONR¹¹R¹²;
- R⁵ and R⁶: independently from each other are selected from the group consisting of hydrogen, C₁₋₇-alkyl, hydroxy-C₁₋₇-alkyl and C₃₋₇-cycloalkyl;
- R⁷: is selected from the group consisting of C₁₋₇-alkyl, C₃₋₇-cycloalkyl, halogen-C₁₋₇-alkyl, hydroxy-C₁₋₇-alkyl, C₁₋₇-alkoxy-C₁₋₇-alkyl, carboxy-C₁₋₇-alkyl, C₁₋₇-alkoxycarbonyl-C₁₋₇-alkyl, 1*H*-tetrazol-5-yl-C₁₋₇- alkyl, unsubstituted phenyl, phenyl substituted by one to three groups selected from the group consisting of C₁₋₇-alkyl, C₃₋₇-cycloalkyl, C₁₋₇-alkoxy, halogen-C₁₋₇-alkyl and halogen, unsubstituted heteroaryl, heteroaryl substituted by one or two groups selected from C₁₋₇-alkyl, C₃₋₇-cycloalkyl, C₁₋₇-alkoxy, halogen-C₁₋₇-alkyl or halogen; unsubstituted heteroaryl-C₁₋₇-alkyl and heteroaryl-C₁₋₇-alkyl, wherein the heteroaryl is substituted by one or two groups selected from C₁₋₇-alkyl, C₃₋₇- cycloalkyl, C₁₋₇-alkoxy, halogen-C₁₋₇-alkyl and halogen;
- R⁸: is selected from the group consisting of C₁₋₇-alkyl, C₃₋₇-cycloalkyl, unsubstituted heteroaryl and heteroaryl substituted by one or two groups selected from C₁₋₇- alkyl, C₃₋₇-cycloalkyl, C₁₋₇-alkoxy, halogen-C₁₋₇-alkyl or halogen;
- R⁹ and R¹⁰: independently from each other are selected from the group consisting of hydrogen, C₁₋₇-alkyl, C₃₋₇-cycloalkyl, unsubstituted heteroaryl and heteroaryl substituted by one or two groups selected from C₁₋₇-alkyl, C₃₋₇-cydoalkyl, C₁₋₇- alkoxy, halogen-C₁₋₇-alkyl or halogen; or R⁹ and R¹⁰ together with the nitrogen atom they are attached to form a pyrrolidine or a piperidine ring;
- R¹¹: is selected from the group consisting of hydrogen, C₁₋₇-alkyl and C₃₋₇- cycloalkyl;
- R¹²: is selected from the group consisting of hydrogen, C₁₋₇-alkyl, C₃₋₇-cycloalkyl, halogen-C₁₋₇-aklyl, hydroxy-C₁₋₇-alkyl, C₁₋₇-alkoxy-C₁₋₇-alkyl, carboxy-C₁₋₇-alkyl, C₁₋₇-alkoxycarbonyl-C₁₋₇-alkyl, unsubstituted phenyl, phenyl substituted by one to three groups selected from the group consisting of C₁₋₇-alkyl, C₃₋₇-cycloalkyl, C₁₋₇-alkoxy, halogen-C₁₋₇-alkyl and halogen, unsubstituted heteroaryl, heteroaryl substituted by one or two groups selected from C₁₋₇-alkyl, C₃₋₇-cycloalkyl, C₁₋₇-alkoxy, halogen-C₁₋₇-alkyl or halogen;
- one of R³ and R⁴: is selected from the group consisting of hydrogen, C₁₋₇-alkyl and C₁₋₇- alkoxy, or is absent in case one of A or B is N or N⁺-O⁻, and
- the other one of R³ and R⁴: is selected from the group consisting of hydrogen, C₁₋₇-alkyl, C₁₋₇-alkoxy, hydroxy-C₁₋₇-alkyl, carboxy, C₁₋₇-alkoxy-carbonyl, carboxy-C₁₋₇-alkoxy, C₁₋₇-alkoxy-carbonyl-C₁₋₇-alkyl, carboxy-C₁₋₇-alkyl, C₁₋₇-alkoxy-carbonyl-C₁₋₇-alkoxy, 1*H*-tetrazol-5-yl-C₁₋₇-alkoxy, hydroxy-C₂₋₇-alkoxy, dihydroxy-C₃₋₇-alkoxy, -(CH₂)ₙ-NR¹³R¹⁴, -(CH₂)ₙ-NHCOR¹⁵, and 1*H*-tetrazol-5-yl;
- R¹³: and R¹⁴ independently from each other are selected from the group consisting of hydrogen, C₁₋₇-alkyl and C₃₋₇-cycloalkyl;
- R¹⁵: is selected from the group consisting of C₁₋₇-alkyl, C₃₋₇-cycloalkyl, halogen-C₁₋₇-alkyl, hydroxy-C₁₋₇-alkyl, C₁₋₇-alkoxy-C₁₋₇-alkyl, carboxy-C₁₋₇-alkyl and C₁₋₇-alkoxycarbonyl-C₁₋₇-alkyl;
- n: is an integer selected from 1, 2 and 3;
provided that benzooxazoles and benzothiazoles wherein R¹, R², R³ and R⁴ are hydrogen are excluded;
- G: is selected from the groups 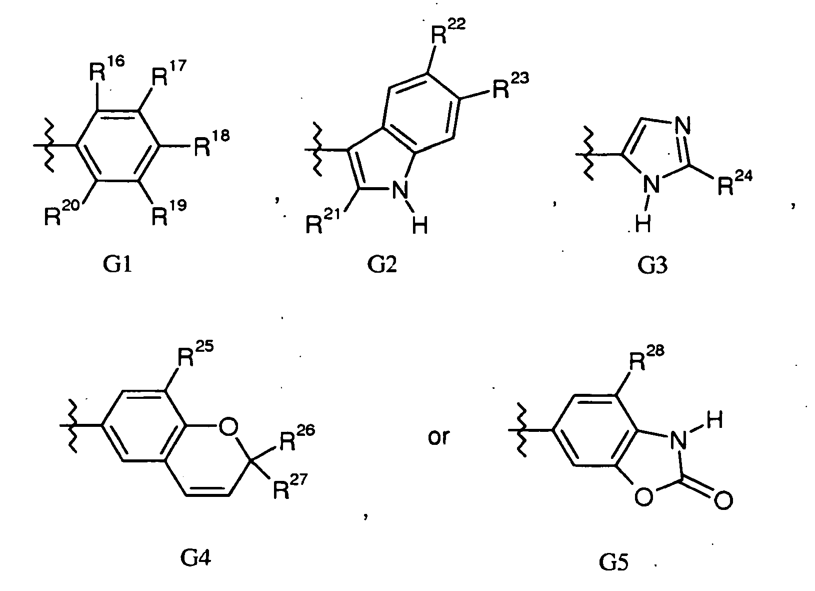
wherein
- R¹⁶: is hydrogen or halogen;
- R¹⁷: is selected from the group consisting of C₁₋₇-alkoxy. C₂₇-alkenyloxy, C₃₋₇-cycloalkyloxy, -NP²⁹R³⁰, halogen-C₁₋₇-alkoxy, C₁₋₇-alkoxy-C₁₋₇-alkoxy, C₁₋₇-alkoxy-C₁₋₇-alkyl; R²⁹ and R³⁰ independently from each other are hydrogen or C₁₋₇-alkyl;
- R¹⁸: is selected from the group consisting of hydrogen, C₁₋₇-alkyl, halogen-C₁₋₇-alkyl, hydroxy, C₁₋₇-alkoxy, halogen-C₁₋₇-alkoxy, C₃₋₇-cycloalkyloxy, halogen, pyrrolyl, -CO₂R³¹, -NR³¹R³³ , -SOR³⁴; unsubstituted phenyl and phenyl substituted by one to two groups selected from the group consisting of C₁₋₇-alkyl, halogen-C₁₋₇-alkyl, halooen-C₁₋₇-alkoxy, C₁₋₇-alkoxy and halogen;
- R³¹: is hydrogen or C₁₋₇-alkyl;
- R³² and R³³: independently from each other are hydrogen or C₁₋₇-alkyl;
- ^{.}R³⁴: is C₁₋₇-alkyl;
- R¹⁹: is selected from the group consisting of hydrogen, C₁₋₇-alkyl, halogen, C₁₋₇-alkoxy, C₂₋₇-alkenyloxy, -O-tetrahydropyranyl, C₃₋₇-cycloalkyloxy, halogen-C₁₋₇-alkoxy, C₁₋₇-atkoxy-C₁₋₇-alkoxy, and C₁₋₇-alkoxy-C₁₋₇-alkyl;
- R²⁰: is hydrogen or halogen;
- R²¹: is hydrogen or C₁₋₇-alkyl;
- R²² and R²³: independently from each other are selected from the group consisting of hydrogen, C₁₋₇-alkyl, C₁₋₇-alkoxy and nitro;
- R²⁴: is unsubstituted phenyl or phenyl substituted by one to two groups selected from the group consisting of C₁₋₇-alkyl, C₁₋₇-alkoxy and halogen;
- R²⁵: is C₁₋₇-alkoxy;
- R²⁶ and R²⁷: independently from each other are C₁₋₇-alkyl;
- R²⁸: is C₁₋₇-alkoxy;
and pharmaceutically acceptable salts thereof.

Examples of preferred compounds of formula I of the present invention are the following:
[1-(3-ethoxy-4-methoxy-benzyl)-piperidin-4-yl]-(7-nitro-benzooxazol-2-yl)-amine, [1-(3,5-diethoxy-4-fluoro-benzyl)-piperidin-4-yl]-(7-nitro-benzooxazol-2-yl)-amine,
*N²*-[1-(3,5-diethoxy-4-fluoro-benzyl)-piperidin-4-yl]-benzooxazole-2,7-diamine,
*N*-{2-[1-(3,5-diethoxy-4-fluoro-benzyl)-piperidin-4-ylamino]-benzooxazol-7-yl}-2-methoxy-acetam ide,
*N*-{2-[1-(3,5-diethoxy-4-fluoro-benzyl)-piperidin-4-ylamino]-benzooxazol-7-yl}-succinamic acid,
pyrimidine-5-carboxylic acid {2-[1-(3,5-diethoxy-4-fluoro-benzyl)-piperidin-4-ylamino]-benzooxazol-7-yl}-amide,
*N*-{2-[1-(3,5-diethoxy-4-fluoro-benzyl)-piperidin-4-ylamino]-benzooxazol-7-yl}-2-pyridin-3-yl-acetamide,
1-methyl-1H-imidazote-4-sulfonic acid {2-[1-(3,5-diethoxy-4-fluoro-benzyl)-piperidin-4-ylamino]-benzooxazol-7-yl}-amide,
2-[1-(3,5-diethoxy-4-fluoro-benzyl)-piperidin-4-ylamino]-benzooxazol-7-yl}-methanesulfonamide,
[1-(3,5-diethoxy-4-fluoro-benzyl)-piperidin-4-yl]-[7-(pyrrolidine-1-sulfonyl)-benzooxazol-2-yl]-amine,
[1-(3,5-diethoxy-benzyl)-piperidin-4-yl]-[7-(pyrrolidine-1-sulfonyl)-benzooxazol-2-yl)-amine,
[1-(3-ethoxy-4-methoxy-benzyl)-piperidin-4-yl]-[7-(pyrrolidine-1-sulfonyl)-benzooxazol-2-yl]-amine,
[1-(3,5-diethoxy-4-pyrrol-1-yl-benzyl)-piperidin-4-yl]-[7-(pyrrolidine-1-sulfonyl)-benzooxazol-2-yl]-amine,
[1-(4-chloro-3-ethoxy-benzyl)-piperidin-4-yl]-[7-(pyrrolidine-1-sulfonyl)-benzooxazol-2-yl]-amine,
[1-(3-ethoxy-4-methoxy-benzyl)-piperidin-4-yl]-(4-nitro-benzooxazol-2-yl)-amine,
[1-(3,5-diethoxy-4-pyrrol-1-yl-benzyl)-piperidin-4-yl]-(4-nitro-benzooxazol-2-yl)-amine,
[1-(3,5-diethoxy-4-fluoro-benzyl)-piperidin-4-yl]-(4-nitro-benzooxazol-2-yl)-amine,
*N*₂*-*[1-(3,5-diethoxy-4-fluoro-benzyl)-piperidin-4-yl]-benzooxazole-2,4-diamine,
*N*-{2-[1-(3,5-diethoxy-4-fluoro-benzyl)-piperidin-4-ylamino]-benzooxazol-4-yl}-2-methoxy-acetamide,
*N*-{2-[1-(3,5-diethoxy-4-fluoro-benzyl)-piperidin-4-ylamino]-benzooxazol-4-yl}-succinamic acid methyl ester,
*N*-{2-[1-(3,5-diethoxy-4-fluoro-benzyl)-piperidin-4-ylamino]-benzooxazol-4-yl}-succinamic acid,
pyrimidine-5-carboxylic acid {2-[1-(3,5-diethoxy-4-fluoro-benzyl)-piperidin-4-ylamino]-benzooxazol-4-yl}-amide,
*N*²-[1-(3,5-diethoxy-4-pyrrol-1-yl-benzyl)-piperidin-4-yl] -benzooxazole-2,4-diamine,
*N*- {2-[1-(3,5-diethoxy-4-pyrrol-1-yl-benzyl)-piperidin-4-ylamino]-benzooxazol-4-yl}-2-methoxy-acetamide,
*N*-{2-[1-(3,5-diethoxy-4-fluoro-benzyl)-piperidin-4-ylamino]-benzooxazol-4-yl}-2-(1H-tetrazol-5-yl)-acetamide,
*N*-{2-[1-(3,5-diethoxy-4-pyrrol-1-yl-benzyl)-piperidin-4-ylamino]-6enzooxazol-4-yl}-succinamic acid methyl ester,
*N*-{2-[1-(3,5-diethoxy-4-pyrrol-1-yl-benzyl)-piperidin-4-ylamino]-benzooxazol-4-yl}-succinamic acid,
[1-(3-ethoxy-4-methoxy-benzyl)-piperidin-4-yl]-[4-(pyridin-4-ylmethoxy)-benzooxazol-2-yl]-amine,
[1-(3,5-diethoxy-4-pyrrol-1-yl-benzyl)-piperidin-4-yl)-[4-(pyridin-4-ylmethoxy)-benzooxazol-2-yl]-amine,
[1-(3,5-diethoxy-4-fluoro-benzyl)-piperidin-4-yl]-[4-(pyridin-4-ylmethoxy)-benzooxazol-2-yl]-amine,
2-[1-(3-ethoxy-4-methoxy-benzyl)-piperidin-4-ylamino]-benzooxazole-4-carboxylic acid methyl ester;
2-[1-(3,5-diethoxy-4-pyrrol-1-yl-benzyl)-piperidin-4-ylamino]-benzooxazole-4-carboxylic acid methyl ester,
2-[1-(3,5-diethoxy-4-fluoro-benzyl)-piperidin-4-ylamino]-benzooxazole-4-carboxylic acid methyl ester,
2- [1-(3-ethoxy-4-methoxy-benzyl)-piperidin-4-ylamino]-benzooxazole-4-carboxylic acid,
2-[1-(3,5-diethoxy-4-pyrrol-1-yl-benzyl)-piperidin-4-ylamino]-benzooxazole-4-carboxylic acid,
2-[1-(3,5-diethoxy-4-fluoro-benzyl)-piperidin-4-ylamino]-benzooxazole-4-carboxylic acid,
2- 1-(3-ethoxy-4-methoxy-benzyl)-piperidin-4-ylamino]-benzooxazole-4-carboxylic acid pyridin-3-ylamide,
2-[1-(3,5-diethoxy-4-fluoro-benzyl)-piperidin-4-ylamino]-benzooxazole-4-carboxylic acid pyridin-3-ylamide,
2-[1-(3,5-diethoxy-4-pyrrol-1-yl-benzyl)-piperidin-4-ylamino]-benzooxazole-4-carboxylic acid pyridin-3-ylamide,
2-[1-(3-ethoxy-4-methoxy-benzyl)-piperidin-4-ylamino]-benzooxazole-4-carboxylic acid (2-hydroxy-ethyl)-amide,
2-[1-(3,5-diethoxy-4-fluoro-benzyl)-piperidin-4-ylamino]-benzooxazole-4-carboxylic acid (2-hydroxy-ethyl)-amide,
2-[1-(3,5-diethoxy-4-pyrrol-1-yl-benzyl)-piperidin-4-ylamino]-benzooxazole-4-carboxylic acid (2-hydroxy-ethyl)-amide,
({2-[1-(3-ethoxy-4-methoxy-benzyl)-piperidin-4-ylamino]-benzooxazole-4-carbonyl}-amino)-acetic acid methyl ester,
({2-[1-(3,5-diethoxy-4-fluoro-benzyl)-piperidin-4-ylamino]-benzooxazole-4-carbonyl}-amino)-acetic acid methyl ester,
({2-[1-(3,5-diethoxy-4-pyrrol-1-yl-benzyl)-piperidin-4-ylamino]-benzooxazole-4-carbonyl}-amino)-acetic acid methyl ester,
({2-[1-(3-ethoxy-4-methoxy-benzyl)-piperidin-4-ylamino]-benzooxazole-4-carbonyl}-amino)-acetic acid,
({2-[1-(3,5-diethoxy-4-fluoro-benzyl)-piperidin-4-ylamino]-benzooxazole-4-carbonyl}-amino)-acetic acid,
({2-[1-(3,5-diethoxy-4-pyrrol-1-yl-benzyl)-piperidin-4-ylamino]-benzooxazole-4-carbonyl}-amino)-acetic acid,
[1-(3-ethoxy-4-methoxy-benzyl)-piperidin-4-yl]-(4-iodo-benzooxazol-2-yl)-amine,
[1-(3,5-diethoxy-4-fluoro-benzyl)-piperidin-4-yl]-(4-iodo-benzooxazol-2-yl)-amine,
[1-(3,5-diethoxy-4-pyrrol-1-yl-benzyl)-piperidin-4-yl]-(4-iodo-benzooxazol-2-yl)-amine,
(7-bromo-benzooxazol-2-yl)-[1-(3-ethoxy-4-methoxy-benzyl)-piperidin-4-yl]-amine,
(7-bromo-benzooxazol-2-yl)-[1-(3,5-diethoxy-4-fluoro-benzyl)-piperidin-4-yl]-amine,
(7-bromo-benzooxazol-2-yl)-[1-(3,5-diethoxy-4-pyrrol-1-yl-benzyl)-piperidin-4-yl]-amine,
2-[1-(3-ethoxy-4-hydroxy-benzyl)-piperidin-4-ylamino]-benzooxazole-6-carboxylic acid methyl ester,
2-[1-(3-ethoxy-4-methoxy-benzyl)-piperidin-4-ylamino]-benzooxazole-6-carboxylic acid methyl ester,
2-[1-(8-ethoxy-2,2-dimethyl-2H*-*chromen*-*2H*-*ylmethyl)-piperidin-4-ylamino]-benzooxazole-6-carboxylic acid methyl ester,
2-1-(3-isobutoxy-4-methoxy-benzyl)-piperidin-4-ylamino]-benzooxazole-6-carboxylic acid methyl ester,
2-{1-[3-(2-fluoro-ethoxy)-4-methoxy-benzyl]-piperidin-4-ylamino}-benzooxazole-6-carboxylic acid methyl ester,
2-[1-(3-ethoxy-4-methyl-benzyl)-piperidin-4-ylamino]-benzooxazole-6-carboxylic acid methyl ester,
2-[1-(4-chloro-3-ethoxy-benzyl)-piperidin-4-ylamino]-benzooxazole-6-carboxylic acid methyl ester,
2-[1-(4-amino-3,5-diethoxy-benzyl)-piperidin-4-ylamino]-benzooxazole-6-carboxylic acid methyl ester,
2-[1-(3,5-diethoxy-4-fluoro-benzyl)-piperidin-4-ylamino]-benzooxazole-6-carboxylic acid methyl ester,
2-[1-(3-ethoxy-4-hydroxy-benzyl)-piperidin-4-ylamino]-benzooxazole-6-carboxylic acid,
2-[1-(3-ethoxy-4-methoxy-benzyl)-piperidin-4-ylamino]-benzooxazole-6-carboxylic acid,
2-[1-(8-ethoxy-2,2-dimethyl-2*H*-chromen-6-ylmethyl)-piperidin-4-ylamino]-benzooxazole-6-carboxylic acid,
2-[1-(3-isobutoxy-4-methoxy-benzyl)-piperidin-4-ylamino]-benzooxazole-6-carboxylic acid,
2-{1-[3-(2-fluoro-ethoxy)-4-methoxy-benzyl]-piperidin-4-ylamino)-benzooxazole-6-carboxylic acid,
2-[1-(3-ethoxy-4-methyl-benzyl)-piperidin-4-ylamino]-benzooxazole-6-carboxylic acid,
2-[1-(4-chloro-3-ethoxy-benzyl)-piperidin-4-ylamino]-benzooxazole-6-carboxylic acid,
2-[1-(4-amino-3,5-diethoxy-benzyl)-piperidin-4-ylamino]-benzooxazole-6-carboxylic acid,
2-[1-(3,5-diethoxy-4-fluoro-benzyl)-piperidin-4-ylamino]-benzooxazole-6-carboxylic acid,
[1-(3-ethoxy-4-fluoro-benzyl)-piperidin-4-yl]-(4-nitro-benzooxazol-2-yl)-amine,
[1-(4-chloro-3-ethoxy-benzyl)-piperidin-4-yl]-(4-nitro-benzooxazol-2-yl)-amine,
[1-(3-ethoxy-4-methyl-benzyl)-piperidin-4-yl]-(4-nitro-benzooxazol-2-yl)-amine,
2-[1-(3,5-diethoxy-4-fluoro-benzyl)-piperidin-4-ylamino]-benzooxazole-7-sulfonic acid amide,
2-[1-(3-ethoxy-4-fluoro-benzyl)-piperidin-4-ylamino]-benzooxazole-7-sulfonic acid amide,
2-[1-(3-ethoxy-4-methoxy-benzyl)-piperidin-4-ylamino]-benzooxazole-7-sulfonic acid amide,
[1-(3,5-diethoxy-4-fluoro-benzyl)-piperidin-4-yl]-(7-methoxy-benzooxazol-2-yl)-amine,
[1-(3,5-diethoxy-4-pyrrol-1-yl-benzyl)-piperidin-4-yl]-(7-methoxy-benzooxazol-2-yl)-amine,
[1-(3,5-diethoxy-4-fluoro-benzyl)-piperidin-4-yl]-oxazolo[5,4-*c*] pyridin-2-yl-amine,
[1-(4-chloro-3-ethoxy-benzyl)-piperidin-4-yl]-oxazolo[5,4-*c*]pyridin-2-yl-amine,
[1-(3-ethoxy-4-methyl-benzyl)-piperidin-4-yl]-oxazolo[5,4-*c*]pyridin-2-yl-amine,
[1-(3-ethoxy-4-methoxy-benzyl)-piperidin-4-yl]-oxazolo[5,4-*c*]pyridin-2-yl-amine,
[1-(3-isobutoxy-4-methoxy-benzyl)-piperidin-4-yl]-oxazolo[5,4-*c*]pyridin-2-yl-amine,
4-ethoxy-6-[4-(oxazolo[5,4-c] pyridin-2-ylamino)-piperidin-1-ylmethyl]-3H-benzooxazol-2-one,
[rac]-[1-(3,5-diethoxy-4-methanesulfinyl-benzyl)-piperidin-4-yl]-oxazolo[5,4-*c*] pyridin-2-yl-amine,
[1-(3-ethylamino-4-methoxy-benzyl)-piperidin-4-yl]-oxazolo[5,4-*c*]pyridin-2-yl-amine,
[1-(8-ethoxy-2,2-dimethyl-2*H*-chromen-6ylmethyl)-piperidin-4-yl]-oxazolo[5,4-c]pyridin-2-yl-amine,
[1-(4-methoxy-3-propoxy-benzyl)-piperidin-4,-yl]-oxazolo[5,4-*c*]pyridin-2-yl-amine,
{1-[3-(2-fluoro-ethoxy)-4-methoxy-benzyl]-piperidin-4-yl}-oxazolo[5,4-*c*]pyridin-2-yl-amine,
2-ethoxy-4-[4-(oxazolo[5,4-*c*] pyridin-2-ylamino)-piperidin-1-ylmethyl]-phenol,
[1-(4-amino-3,5-diethoxy-benzyl)-piperidin-4-yl]-oxazolo[5,4-*c*]pyridin-2-yl-amine,
[1-(3,5-diisopropoxy-benzyl)-piperidin-4-yl]-oxazolo[5,4-*c*]pyridin-2-yl-amine,
[1-(3-ethoxy-4-fluoro-benzyl)-piperidin-4-yl]-oxazolo[5,4-*c*] pyridin-2-yl-amine,
[1-(3-ethoxy-4-isopropoxy-benzyl)-piperidin-4-yl]-oxazolo[5,4-*c*] pyridin-2-yl-amine,
oxazolo[5,4-*c*]pyridin-2-yl-[1-(2-phenyl-3H-imidazol-4-ylmethyl)-piperidin-4-yl]-amine,
[1-(2-methyl-5-nitro-1H-indol-3-ylmethyl)-piperidin-4-yl]-oxazolo[5,4-*c*]pyridin-2-yl-amine,
{2-[1-(3-ethoxy-4-methoxy-benzyl)-piperidin-4-ylamino]-benzooxazol-5-yloxy}-acetic acid methyl ester,
{2-[1-(3,5-diethoxy-4-fluoro-benzyl)-piperidin-4-ylamino]-benzooxazol-5-yloxy}-acetic acid methyl ester,
{2-[1-(3,5-diethoxy-4-pyrrol-1-yl-benzyl)-piperidin-4-ylamino]-benzooxazol-5-yloxy}-acetic acid methyl ester,
{2-[1-(3-ethoxy-4-methoxy-benzyl)-piperidin-4-ylamino]-benzooxazol-5-yloxy}-acetic acid,
{2-[1-(3,5-diethoxy-4-fluoro-benzyl)-piperidin-4ylamino]-benzooxazol-5-yloxy}-acetic acid,
{2-[1-(3,5-diethoxy-4-pyrrol-1-yl-benzyl)-piperidin-4-ylamino]-benzooxazol-5-yloxy}-acetic acid,
[1-(3,5-dieth oxy-4-fluoro-benzyl)-piperidin-4-yl]-(7-phenyl-benzooxazol-2-yl)-amine,
{2-[1-(3-ethoxy-4-methoxy-benzyl)-piperidin-4-ylamino]-benzooxazol-7-yloxy}-acetic acid methyl ester,
{2-[1-(3,5-diethoxy-4-fluoro-benzyl)-piperidin-4-ylamino]-benzooxazol-7-yloxy}-acetic acid methyl ester,
{2-[1-(3,5-diethoxy-4-pyrrol-1-yl-benzyl)-piperidin-4-ylamino]-benzooxazol-7-yloxy}-acetic acid methyl ester,
{2-[1-(3-ethoxy-4-methoxy-benzyl)-piperidin-4-ylamino]-benzooxazol-7-yloxy}-acetic acid,
{2-[1-(3,5-diethoxy-4-fluoro-benzyl)-piperidin-4-ylamino]-benzooxazol-7-yloxy)-acetic acid,
{2-[1-(3,5-diethoxy-4-pyrrol-1-yl-benzyl)-piperidin-4-ylamino]-benzooxazol-7-yloxy}-acetic acid,
(5-aminomethyl-benzooxazol-2-yl)-[1-(3,5-diethoxy-4-fluoro-benzyl)-piperidin-4-yl]-amine,
4-{2-[1-(3,5-diethoxy-4-fluoro-benzyl)-piperidin-4-ylamino]-benzooxazol-7-yloxy}-butyric acid methyl ester,
4-{2-[1-(3,5-diethoxy-4-pyrrol-1-yl-benzyl)-piperidin-4-ylamino]-benzooxazol-7-yloxy}-butyric acid methyl ester,
4-{2-[1-(3-ethoxy-4-methoxy-benzyl)-piperidin-4-ylamino]-benzooxazol-7-yloxy}-butyric acid methyl ester,
4-{2-[1-(3,5-diethoxy-4-fluoro-benzyl)-piperidin-4-ylamino]-benzooxazol-7-yloxy}-butyric acid,
4-{2-[1-(3,5-diethoxy-4-pyrrol-1-yl-benzyl)-piperidin-4-ylamino]-benzooxazol-7-yloxy}-butyric acid,
4-{2-[1-(3-ethoxy-4-methoxy-benzyl)-piperidin-4-ylamino]-benzooxazol-7-yloxy}-butyric acid,
[rac]-3-{2-[1-(3,5-diethoxy-4-fluoro-benzyl)-piperidin-4-ylamino]-benzooxazol-7-yloxy}-propane-1,2-diol,
[rac]-3-{2-[1-(3,5-diethoxy-4-pyrrol-1-yl-benzyl)-piperidin-4-ylamino]-benzooxazol-7-yloxy}-propane-1,2-diol,
[rac]-3-{2-[1-(3-ethoxy-4-methoxy-benzyl)-piperidin-4-ylamino]-benzooxazol-7-yloxy}-propane-1,2-diol,
4-{2-[1-(3,5-diethoxy-4-fluoro-benzyl)-piperidin-4-ylamino]-benzooxazol-5-yloxy}-butyric acid methyl ester,
4-{2-[1-(3,5-diethoxy-4-pyrrol-l-yl-benzyl)-piperidin-4-ylamino]-benzooxazol-5-yloxy}-butyric acid methyl ester,
4-{2-[1-(3-ethoxy-4-methoxy-benzyl)-piperidin-4-ylamino]-benzooxazol-5-yloxy}-butyric acid methyl ester,
4-{2-[1-(3,5-diethoxy-4-fluoro-benzyl)-piperidin-4-ylamino]-benzooxazol-5-yloxy}-butyric acid,
4-{2-[1-(3,5-diethoxy-4-pyrrol-1-y1-benzyl)-piperidin-4-ylamino]-benzooxazol-5-yloxy}-butyric acid,
4-{2-[1-(3-ethoxy-4-methoxy-benzyl)-piperidin-4-ylamino]-benzooxazol-5-yloxy}-butyric acid,
1-{2-[1-(3,5-diethoxy-4-fluoro-benzyl)-piperidin-4-ylamino]-benzooxazol-5-yloxy}-cyclobutanecarboxylic acid ethyl ester,
1- {2-[1-(3,5-diethoxy-4-pyrrol-1-yl-benzyl)-piperidin-4-ylamino]-benzooxazol-5-yloxy}-cyclobutanecarboxylic acid ethyl ester,
1-{2-[1-(3,5-diethoxy-4-fluoro-benzyl)-piperidin-4-ylamino]-benzooxazol-5-yloxy}-cyclobutanecarboxylic acid,
1-{2-[1-(3,5-diethoxy-4-pyrrol-1-yl-benzyl)-piperidin-4-ylamino]-benzooxazol-5-yloxy}-cyclobutanecarboxylic acid,
*N*-{2-[1-(3,5-diethoxy-4-fluoro-benzyl)-piperidin-4-ylamino)-benzooxazol-5-ylmethyl}-acetamide,
*N*-{2-[1-(3,5-diethoxy-4-fluoro-benzyl)-piperidin-4-ylamino]-benzooxazol-5-ylmethyl}-malonamic acid ethyl ester,
(S)-N-{2-[1-(3,5-diethoxy-4-fluoro-benzyl)-piperidin-4-ylamino]-benzooxazol-5-ylmethyl}-2-hydroxy-propionamide,
[1-(3,5-diethoxy-4-fluoro-benzyl)-piperidin-4-yl]-(4-methyl-oxazolo[5,4-*c*]pyridin-2-yl)-amine,
[1-(3,5-diethoxy-4-pyrrol-1-yl-benzyl)-piperidin-4-yl]-(4-methyl-oxazolo[5,4-*c*]pyridin-2-yl)-amine,
[1-(3,5-diisopropoxy-benzyl)-piperidin-4-yl]-(4-methyl-oxazolo[5,4-*c*]pyridin-2-yl)-amine,
*N*-{2-[1-(3,5-diethoxy-4-fluoro-benzyl)-piperidin-4-ylamino]-benzooxazol-5-ylmethyl}-malonamic acid,
[rac]-3-{2-[1-(3,5-diethoxy-4-fluoro-benzyl)-piperidin-4-ylamino]-benzooxazol-5-yloxy}-propane-1,2-diol,
[rac]-3-{2-[1-(3,5-diethoxy-4-pyrrol-1-yl-benzyl)-piperidin-4-ylamino]-benzooxazol-5-yloxy}-propane-1,2-diol,
[rac]-3-{2-[1-(3,5-diisopropoxy-benzyl)-piperidin-4-ylamino]-benzooxazol-5-yloxy}-propane-1,2-diol,
[1-(3,5-diethoxy-4-pyrrol-1-yl-benzyl)-piperidin-4-yl]-[5-(1H-tetrazol-5-ylmethoxy)-benzooxazol-2-yl]-amine,
[1-(3,5-diethoxy-4-fluoro-benzyl)-piperidin-4-yl]-[5-(1H-tetrazol-5-ylmethoxy)-benzooxazol-2-yl]-amine,
{2-[1-(3,5-diethoxy-4-fluoro-benzyl)-piperidin-4-ylamino]-oxazolo[5,4-c]pyridin-4-yl}-methanol,
{2-[1-(3,5-diethoxy-4-pyrrol-1-yl-benzyl)-piperidin-4-ylamino]-oxazolo[5,4-c]pyridin-4-yl}-methanol,
{2-[1-(3,5-diisopropoxy-benzyl)-piperidin-4-ylamino]-oxazolo[5,4-*c*]pyridin-4-yl}-methanol,
{2-[1-(3,5-diethoxy-4-[1,2,4]triazol-1-yl-benzyl)-piperidin-4-ylamino]-oxazolo[5,4-*c*]pyridin-4-yl}-methanol,
{2-[1-(3,5-diethoxy-4-fluoro-benzyl)-piperidin-4-ylamino]-benzooxazol-5-yl}-acetic acid methyl ester,
{2-[1-(3,5-diethoxy-4-pyrrol-1-yl-benzyl)-piperidin-4-ylamino]-benzooxazol-5-yl}-acetic acid methyl ester,
{2-[1-(3,5-diisopropoxy-benzyl)-piperidin-4-ylamino]-benzooxazol-5-yl}-acetic acid methyl ester,
{2-[1-(3,5-diethoxy-4-[1,2,4]triazol-1-yl-benzyl)-piperidin-4-ylamino]-benzooxazol-5-yl}-acetic acid methyl ester,
{2-[1-(3,5-diethoxy-4-fluoro-benzyl)-piperidin-4-ylamino]-benzooxazol-5-yl}-acetic acid,
{2-[1-(3,5-diethoxy-4-pyrrol-1-yl-benzyl)-piperidin-4-ylamino]-benzooxazol-5-yl}-acetic acid,
{2-[1-(3,5-diisopropoxy-benzyl)-piperidin-4-ylamino]-benzooxazol-5-yl}-acetic acid, {2-[1-(3,5-diethoxy-4-[1,2,4]triazol-1-yl-benzyl)-piperidin-4-ylamino]-benzooxazol-5-yl}-acetic acid,
[1-(3,5-diethoxy-4-fluoro-benzyl)-piperidin-4-yl]-(5-methoxy-benzooxazol-2-yl)-amine,
[1-(3,5-diethoxy-4-pyrrol-1-yl-benzyl)-piperidin-4-yl]-(5-methoxy-benzooxazol-2-yl)-amine,
2-[1-(3,5-diethoxy-4-fluoro-benzyl)-piperidin-4-ylamino]-benzooxazole-5-carboxylic acid methyl ester,
2-[1-(3,5-diethoxy-4-pyrrol-1-yl-benzyl)-piperidin-4-ylamino]-benzooxazole-5-carboxylic acid methyl ester,
2-[1-(3,5-diisopropoxy-benzyl)-piperidin-4-ylamino]-benzooxazole-5-carboxylic acid methyl ester,
2-[1-(3,5-diethoxy-4-[1,2,4]triazol-1-yl-benzyl)-piperidin-4-ylamino]-benzooxazole-5-carboxylic acid methyl ester,
2-[1-(3,5-diethoxy-4-fluoro-benzyl)-piperidin-4-ylamino]-benzooxazole-5-carboxylic acid,
2-[1-(3,5-diethoxy-4-pyrrol-1-yl-benzyl)-piperidin-4-ylamino]-benzooxazole-5-carboxylic acid,
2-[1-(3,5-diisopropoxy-benzyl)-piperidin-4-ylamino]-benzooxazole-5-carboxylic acid,
2-[1-(3,5-diethoxy-4-[1,2,4]triazol-l-yl-benzy1)-piperidin-4-ylamino]-benzooxazole-5-carboxylic acid,
2-[1-(4-chloro-3,5-diethoxy-benzyl)-piperidin-4-ylamino]-benzooxazole-5-carboxylic acid methyl ester,
2-[1-(2,6-diethoxy-4'-fluoro-biphenyl-4-ylmethyl)-piperidin-4-ylamino]-benzooxazole-5-carboxylic acid methyl ester,
2-[1-(4-chloro-3,5-diethoxy-benzyl)-piperidin-4-ylamino]-benzooxazole-5-carboxylic acid,
2-[1-(2,6-diethoxy-4'-fluoro-biphenyl-4-ylmethyl)-piperidin-4-ylamino]-benzooxazole-5-carboxylic acid,
{2-[1-(3,5-diethoxy-4-fluoro-benzyl)-piperidin-4-ylamino]-benzooxazol-5-yloxy}-acetonitrile,
*N*-tert-butyl-2-{2-[1-(3,5-diethoxy-4-fluoro-benzyl)-piperidin-4-ylamino]-benzooxazol-5-yloxy}-acetamide,
{2-[1-(3,5-diisopropoxy-benzyl)-piperidin-4-ylamino]-benzooxazol-5-yloxy}-acetonitrile,
*N*-tert-butyl-2-{2-[1-(3,5-diisopropoxy-benzyl)-piperidin-4-ylamino]-benzooxazol-5-yloxy}-acetamide,
{2-[1-(4-chloro-3,5-diethoxy-benzyl)-piperidin-4-ylamino]-benzooxazol-5-yloxy}-aceton itrile,
*N*-tert-butyl-2-{2-[1-(4-chloro-3,5-diethoxy-benzyl)-piperidin-4-ylamino]-benzooxazol-5-yloxy}-acetamide,
{2-[1-(2,6-diethoxy-4'-fluoro-biphenyl-4-ylmethyl)-piperidin-4-ylamino]-benzooxazol-5-yloxyl-acetonitrile,
*N*-tert-butyl-2-{2-[1-(2,6-diethoxy-4'-fluoro-biphenyl-4-ylmethyl)-piperidin-4-ylamino]-benzooxazol-5-yloxyl-acetamide,
2-{2-[1-(2,6-diethoxy-4'-fluoro-biphenyl-4-ylmethyl)-piperidin-4-ylamino]-benzooxazol-5-yloxy)-acetamide,
2-{2-[1-(3,5-diisopropoxy-benzyl)-piperidin-4-ylamino]-benzooxazol-5-yloxy}-acetamide,
2-{2-[1-(4-chloro-3,5-diethoxy-benzyl)-piperidin-4-ylamino]-benzooxazol-5-yloxy}-acetamide,
2-{2-[1-(3,5-diethoxy-4-fluoro-benzyl)-piperidin-4-ylamino]-benzooxazol-5-yloxy}-acetamide,
[rac]-3-{2-[1-(2,6-diethoxy-4'-fluoro-biphenyl-4-ylmethyl)-piperidin-4-ylamino]-benzooxazol-5-yloxy}-propane-1,2-diol,
[rac]-3-{2-[1-(4-chloro-3,5-diethoxy-benzyl)-piperidin-4-ylamino]-benzooxazol-5-yloxy}-propane-1,2-diol,
2-{2-[1-(3,5-diisopropoxy-benzyl)-piperidin-4-ylamino]-benzooxazol-5-yl}-ethanol,
2-{2-[1-(3,5-diethoxy-4-fluoro-benzyl)-piperidin-4-ylamino]-benzooxazol-5-yl}-ethanol,
{2-[1-(3,5-diethoxy-4-fluoro-benzyl)-piperidin-4-ylamino]-benzooxazol-5-yl}-methanol,
[5-(2-amino-ethoxy)-benzooxazol-2-yl] [1-(3,5-diethoxy-4-fluoro-benzyl)-piperidin-4-yl]-amine,
2-[1-(2,6-diethoxy-4'-fluoro-biphenyl-4-ylmethyl)-piperidin-4-ylamino]-benzooxazole-6-carboxylic acid,
2-[1-(4-chloro-3,5-diethoxy-benzyl)-piperidin-4-ylamino]-benzooxazole-6-carboxylic acid,
2-[1-(3,5-diisopropoxy-benzyl)-piperidin-4-ylamino]-benzooxazole-6-carboxylic acid,
2-[1-(4-chloro-3,5-diethoxy-benzyl)-piperidin-4-ylamino]-benzooxazol-5-ol,
3-{2-[1-(3,5-diethoxy-4-fluoro-benzyl)-piperidin-4-ylamino]-benzooxazol-5-yloxy}-propan-1-ol,
3-{2-[1-(3,5-diisopropoxy-benzyl)-piperidin-4-ylamino]-benzooxazol-5-yloxy}-propan-1-ol,
3-{2-[1-(4-chloro-3,5-diethoxy-benzyl)-piperidin-4-ylamino]-benzooxazol-5-yloxy}-propan-1-ol,
3-{2-[1-(2,6-diethoxy-4'-fluoro-biphenyl-4-ylmethyl)-piperidin-4-ylamino]-benzooxazol-5-yloxy}-propan-1-ol,
2-{2-[1-(3,5-diethoxy-4-fluoro-benzyl)-piperidin-4-ylamino]-benzooxazol-5-yloxy}-ethanol,
2-{2-[1-(2,6-diethoxy-4'-fluoro-biphenyl-4-ylmethyl)-piperidin-4-ylamino]-benzooxazol-5-yloxy}-ethanol,
[1-(3,5-diethoxy-4-fluoro-benzyl)-piperidin-4-yl]-[5-(3-methoxy-propoxy)-benzooxazol-2-yl]-amine,
[1-(3,5-diisopropoxy-benzyl)-piperidin-4-yl]-[5-(3-methoxy-propoxy)-benzooxazol-2-yl]-amine,
[1-(4-chloro-3,5-diethoxy-benzyl)-piperidin-4-yl]-[5-(3-methoxy-propoxy)-benzooxazol-2-yl]-amine,
[1-(2,6-diethoxy-4'-fluoro-biphenyl-4-ylmethyl)-piperidin-4-yl]-[5-(3-methoxy-propoxy)-benzooxazol-2-yl]-amine,
[1-(4-chloro-3,5-diethoxy-benzyl)-piperidin-4-yl]-[5-(3-methanesulfonyl-propoxy)-benzooxazol-2-yl] -amine,
methanesulfonic acid 2-[1-(3,5-diethoxy-4-fluoro-benzyl)-piperidin-4-ylamino]-benzooxazol-5-yl ester,
methanesulfonic acid 2-[1-(3,5-diisopropoxy-benzyl)-piperidin-4-ylamino]-benzooxazol-5-yl ester,
methanesulfonic acid 2-[1-(4-chloro-3,5-diethoxy-benzyl)-piperidin-4-ylamino]-benzooxazol-5-yl ester,
methanesulfonic acid 2-[1-(2,6-diethoxy-4'-fluoro-biphenyl-4-ylmethyl)-piperidin-4-ylamino]-benzooxazol-5-yl ester,
[1-(3,5-diethoxy-4-fluoro-benzyl)-piperidin-4-yl)-[5-(3-[1,2,4] triazol-1-yl-propoxy)-benzooxazol-2-yl]-amine,
[1-(3,5-diisopropoxy-benzyl)-piperidin-4-yl]-[5-(3-[1,2,4] triazol-1-yl-propoxy)-benzooxazol-2-yl]-amine,
[1-(4-chloro-3,5-diethoxy-benzyl)-piperidin-4-yl]-[5-(3-[1,2,4]triazol-1-yl-propoxy)-benzooxazol-2-yl]-amine,
[1-(2,6-diethoxy-4'-fluoro-biphenyl-4-ylmethyl)-piperidin-4-yl]-[5-(3-[1,2,4]triazol-1-yl-propoxy)-benzooxazol-2-yl]-amine,
2-[1-(3,5-diethoxy-4-fluoro-benzyl)-piperidin-4-ylamino]-benzothiazole-6-carboxylic acid ethyl ester,
2-[1-(3,5-diisopropoxy-benzyl)-piperidin-4-ylamino]-benzothiazole-6-carboxylic acid ethyl ester,
2-[1-(4-chloro-3,5-diethoxy-benzyl)-piperidin-4-ylamino]-benzothiazole-6-carboxylic acid ethyl ester,
2-[1-(2,6-diethoxy-4'-fluoro-biphenyl-4-ylmethyl)-piperidin-4-ylamino]-benzothiazole-6-carboxylic acid ethyl ester,
2-[1-(3,5-diethoxy-4-pyrrol-1-yl-benzyl)-piperidin-4-ylamino]-benzothiazole-6-carboxylic acid ethyl ester,
2-[1-(3,5-diethoxy-4-fluoro-benzyl)-piperidin-4-ylamino]-benzothiazole-6-carboxylic acid,
2-[1-(3,5-diisopropoxy-benzyl)-piperidin-4-ylamino]-benzothiazole-6-carboxylic acid, 2-[1-(4-chloro-3,5-diethoxy-benzyl)-piperidin-4-ylamino]-benzothiazole-6-carboxylic acid,
2-[1-(2,6-diethoxy-4'-fluoro-biphenyl-4-ylmethyl)-piperidin-4-ylamino]-benzothiazole-6-carboxylic acid,
2-[1-(3,5-diethoxy-4-pyrrol-1-yl-benzyl)-piperidin-4-ylamino]-benzothiazole-6-carboxylic acid,
2-[1-(3,5-diisopropoxy-benzyl)-piperidin-4-ylamino]-benzothiazole-5-carboxylic acid methyl ester,
2-[1-(4-chloro-3,5-diethoxy-benzyl)-piperidin-4-ylamino]-benzothiazole-5-carboxylic acid methyl ester,
2-[1-(2,6-diethoxy-4'-fluoro-biphenyl-4-ylmethyl)-piperidin-4-ylamino]-benzothiazole-5-carboxylic acid methyl ester,
2-[1-(3,5-diethoxy-4-pyri-ol-1-yl-benzyl)-piperidin-4-ylamino]-benzothiazole-5-carboxylic acid methyl ester,
2-[1-(3,5-diisopropoxy-benzyl)-piperidin-4-ylamino]-benzothiazole-5-carboxylic acid, 2-[1-(4-chloro-3,5-diethoxy-benzyl)-piperidin-4-ylamino]-benzothiazole-5-carboxylic acid,
2-[1-(2,6-diethoxy-4'-fluoro-biphenyl-4-ylmethyl)-piperidin-4-ylamino]-benzothiazole-5-carboxylic acid,
2-[1-(3,5-diethoxy-4-pyrrol-1-yl-benzyl)-piperidin-4-ylamino]-benzothiazole-5-carboxylic acid,
{2-[1-(2,6-diethoxy-4'-fluoro-biphenyl-4-ylmethyl)-piperidin-4-ylamino]-oxazolo[5,4-*c*]pyridin-4-yl}-methanol,
{2-[1-(4-chloro-3,5-diethoxy-benzyl)-piperidin-4-ylamino]-oxazolo[5,4-*c*]pyridin-4-yl}-methanol,
   and pharmaceutically acceptable salts thereof.

Especially preferred are the following compounds of formula I of the present invention:
*N*²-[1-(3,5-diethoxy-4-fluoro-benzyl)-piperidin-4-yl]-benzooxazole-2,7-diamine,
*N*-{2-[1-(3,5-diethoxy-4-fluoro-benzyl)-piperidin-4-ylamino]-benzooxazol-7-yl}-succinamic acid,
*N*-{2-[1-(3,5-diethoxy-4-fluoro-benzyl)-piperidin-4-ylamino]-benzooxazol-4-yl}-succinamic acid,
2-[1-(4-chloro-3-ethoxy-benzyl)-piperidin-4-ylamino]-benzooxazole-6-carboxylic acid, 2-[1-(4-amino-3,5-diethoxy-benzyl)-piperidin-4-ylamino]-benzooxazole-6-carboxylic acid,
2-[1-(3,5-diethoxy-4-fluoro-benzyl)-piperidin-4-ylamino]-benzooxazole-6-carboxylic acid,
[1-(3,5-diethoxy-4-fluoro-benzyl)-piperidin-4-yl]-oxazolo[5,4-*c*] pyridin-2-yl-amine, [1-(3,5-diisopropoxy-benzyl)-piperidin-4-yl]-oxazolo[5,4-*c*]pyridin-2-yl-amine,
{2-[1-(3,5-diethoxy-4-fluoro-benzyl)-piperidin-4-ylamino]-benzooxazol-5-yloxy}-acetic acid,
{2-[1-(3,5-diethoxy-4-pyrrol-1-yl-benzyl)-piperidin-4-ylamino]-benzooxazol-5-yloxy}-acetic acid,
[1-(3,5-diethoxy-4-fluoro-benzyl)-piperidin-4-yl]-(7-phenyl-benzooxazol-2-yl)-amine,
4-{2-[1-(3,5-diethoxy-4-pyrrol-1-yl-benzyl)-piperidin-4-ylamino]-benzooxazol-7-yloxy}-butyric acid,
[rac]-3-{2-[1-(3,5-diethoxy-4-fluoro-benzyl)-piperidin-4-ylamino]-benzooxazol-7-yloxy}-propane-1,2-diol,
4-{2-[1-(3,5-diethoxy-4-fluoro-benzyl)-piperidin-4-ylamino]-benzooxazol-5-yloxy}-butyric acid,
[rac]-3-{2-[1-(3,5-diethoxy-4-fluoro-benzyl)-piperidin-4-ylamino]-benzooxazol-5-yloxy}-propane-1,2-diol,
{2-[1-(3,5-diisopropoxy-benzyl)-piperidin-4-ylamino]-oxazolo[5,4-*c*]pyridin-4-yl}-methanol,
2-[1-(4-chloro-3,5-diethoxy-benzyl)-piperidin-4-ylamino]-benzooxazole-5-carboxylic acid,
2-[1-(2,6-diethoxy-4'-fluoro-biphenyl-4-ylmethyl)-piperidin-4-ylamino]-benzooxazole-5-carboxylic acid,
{2-[1-(2,6-diethoxy-4'-fluoro-biphenyl-4-ylmethyl)-piperidin-4-ylamino]-benzooxazol-5-yloxy}-acetonitrile,
*N*-*tert*-butyl-2-{2-[1-(2,6-diethoxy-4'-fluoro-biphenyl-4-ylmethyl)-piperidin-4-ylamino]-benzooxazol-5-yloxy}-acetamide,
2-{2-[1-(2,6-diethoxy-4'-fluoro-biphenyl-4-ylmethyl)-piperidin-4-ylamino]-benzooxazol-5-yloxy}-acetamide,
[rac]-3-{2-[1-(2,6-diethoxy-4'-fluoro-biphenyl-4-ylmethyl)-piperidin-4-ylamino]-benzooxazol-5-yloxy}-propane-1,2-diol,
2-[1-(2,6-diethoxy-4'-fluoro-biphenyl-4-ylmethyl)-piperidin-4-ylamino]-benzooxazole-6-carboxylic acid,
2-[1-(4-chloro-3,5-diethoxy-benzyl)-piperidin-4-ylamino]-benzooxazole-6-carboxylic acid,
3-{2-[1-(2,6-diethoxy-4'-fluoro-biphenyl-4-ylmethyl)-piperidin-4-ylamino]-benzooxazol-5-yloxy}-propan-1-ol,
2-{2-[1-(2,6-diethoxy-4'-fluoro-biphenyl-4-ylmethyl)-piperidin-4-ylamino]-benzooxazol-5-yloxy}-ethanol,
[1-(2,6-diethoxy-4'-fluoro-biphenyl-4-ylmethyl)-piperidin-4-yl]-[5-(3-methoxy-propoxy)-benzooxazol-2-yl]-amine,
[1-(4-chloro-3,5-diethoxy-benzyl)-piperidin-4-yl]-[5-(3-methanesulfonyl-propoxy)-benzooxazol-2-yl]-amine,
methanesulfonic acid 2-[1-(2,6-diethoxy-4'-fluoro-biphenyl-4-ylmethyl)-piperidin-4-ylamino]-benzooxazol-5-yl ester,
[1-(4-chloro-3,5-diethoxy-benzyl)-piperidin-4-yl]-[5-(3-[1,2,4] triazol-1-yl-propoxy)-benzooxazol-2-yl]-amine,
[1-(2,6-diethoxy-4'-fluoro-biphenyl-4-ylmethyl)-piperidin-4-yl]-[5-(3-[1,2,4] triazol-1-yl-propoxy)-benzooxazol-2-yl]-amine,
2-[1-(4-chloro-3,5-diethoxy-benzyl)-piperidin-4-ylamino]-benzothiazole-6-carboxylic acid,
2-[1-(2,6-diethoxy-4'-fluoro-biphenyl-4-ylmethyl)-piperidin-4-ylamino]-benzothiazole-6-carboxylic acid,
2-[1-(4-chloro-3,5-diethoxy-benzyl)-piperidin-4-ylamino]-benzothiazole-5-carboxylic acid,
2-[1-(2,6-diethoxy-4'-fluoro-biphenyl-4-ylmethyl)-piperidin-4-ylamino]-benzothiazole-5-carboxylic acid,
   and pharmaceutically acceptable salts thereof.

The pharmaceutically acceptable salts of the compounds of formula I individually constitute preferred embodiments of the present invention.

Compounds of formula I can have one or more asymmetric carbon atoms and can exist in the form of optically pure enantiomers, mixtures of enantiomers such as, for example, racemates, optically pure diastereoisomers, mixtures of diastereoisomers, diastereoisomeric racemates or mixtures of diastereoisomeric racemates. The optically active forms can be obtained for example by resolution of the racemates, by asymmetric synthesis or asymmetric chromatography (chromatography with a chiral adsorbens or eluant). The invention embraces all of these forms.

It will be appreciated, that the compounds of general formula I in this invention may be derivatised at functional groups to provide derivatives which are capable of conversion back to the parent compound in vivo.

A further aspect of the present invention is the process for the manufacture of compounds of formula I as defined above, which process comprises
reacting a compound of the general formula 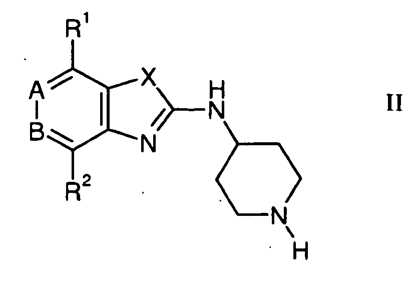 wherein A, B, X, R¹ and R² are as defined herein before, with an aldehyde of the formula 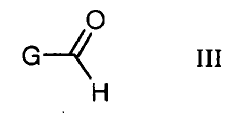 wherein G is as defined herein before, by employing a reducing agent to obtain a compound of the formula 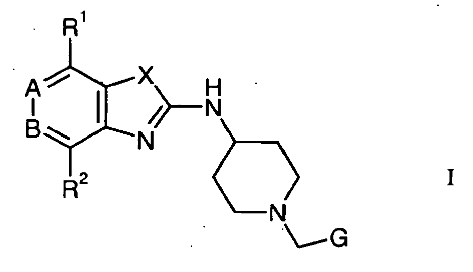 and, if desired,
converting the compound of formula I into a pharmaceutically acceptable salt.

The invention further relates to compounds of formula I as defined above, when manufactured according to a process as defined above.

Suitable reducing agents are preferably selected from the group consisting of pyridine-BH₃ complex, NaBH(OAc)₃ and NaCNBH₃. The reaction can be carried out under acidic conditions (*e.g*., acetic acid, formic acid), by using a Lewis acid (*e.g*., Ti(iPrO)₄, ZnCl₂) or under buffered conditions, *e.g*., in the presence of acetic acid and a tertiary amine like *N*-ethyl-diisopropylamine in a suitable solvent such as dichloromethane, dichloroethane, ethanol or isopropanol (or mixtures thereof) at ambient or elevated temperatures using conventional heating or heating by microwave irradiation.

The conversion of a compound of formula I, wherein R¹ or R² or R³ or R⁴ signifies an amino group, an alkyl-amino group, a cyano group, a nitro group or a carboxy group into a compound of formula I, wherein R¹ or R² or R³ or R⁴ signifies a group such as - NHCOR⁷ or -NHSO₂R⁸, -(CH₂)ₙNHCOR¹⁵, -1*H*-tetrazol-5-yl, -CH₂NH₂, -NH₂, - CONR¹¹R¹² is also embraced in the present invention.

As described above, the compounds of formula I of the present invention can be used as medicaments for the treatment and/or prevention of diseases which are associated with the modulation of SST receptors subtype 5.

Diseases which are associated with the modulation of SST receptors subtype 5" are such diseases as diabetes mellitus, particularly type 2 diabetes mellitus, impaired fasting glucose, impaired glucose tolerance, micro- and macrovascular diabetic complications, posttransplantation diabetes mellitus in patients having type 1 diabetes mellitus, gestational diabetes, obesity, inflammatory bowel diseases such as Crohn's disease or ulcerative colitis, malabsorption, autoimmune diseases such as rheumatoid arthritis, osteoarthritis, psoriasis and other skin disorders, and immunodeficiences. Microvascular diabetic complications include diabetic nephropathy, diabetic neuropathy and diabetic retinopathy, whereas macrovascular diabetes-associated complications lead to an increased risk for myocardial infarction, stroke and limb amputations.

The use as medicament for the treatment and/or prevention of diabetes mellitus, particularly type 2 diabetes mellitus, impaired fasting glucose or impaired glucose tolerance is preferred.

The invention therefore also relates to pharmaceutical compositions comprising a compound as defined above and a pharmaceutically acceptable carrier and/or adjuvant.

Further, the invention relates to compounds as defined above for use as therapeutically active substances, particularly as therapeutic active substances for the treatment and/or prevention of diseases which are associated with the modulation of SST receptors subtype 5.

In addition, the invention relates to the use of compounds as defined above for the preparation of medicaments for the treatment and/or prevention of diseases which are associated with the modulation of SST receptors subtype 5.

The compounds of formula (I) can be manufactured by the methods given below, by the methods given in the examples or by analogous methods. Appropriate reaction conditions for the individual reaction steps are known to a person skilled in the art. Starting materials are either commercially available or can be prepared by methods analogous to the methods given below, by methods described in references cited in the text or in the examples, or by methods known in the art.

The synthesis of compounds with the general structure I, particularly compounds according to formula I-a to I-m, is described in schemes 1 to 6.

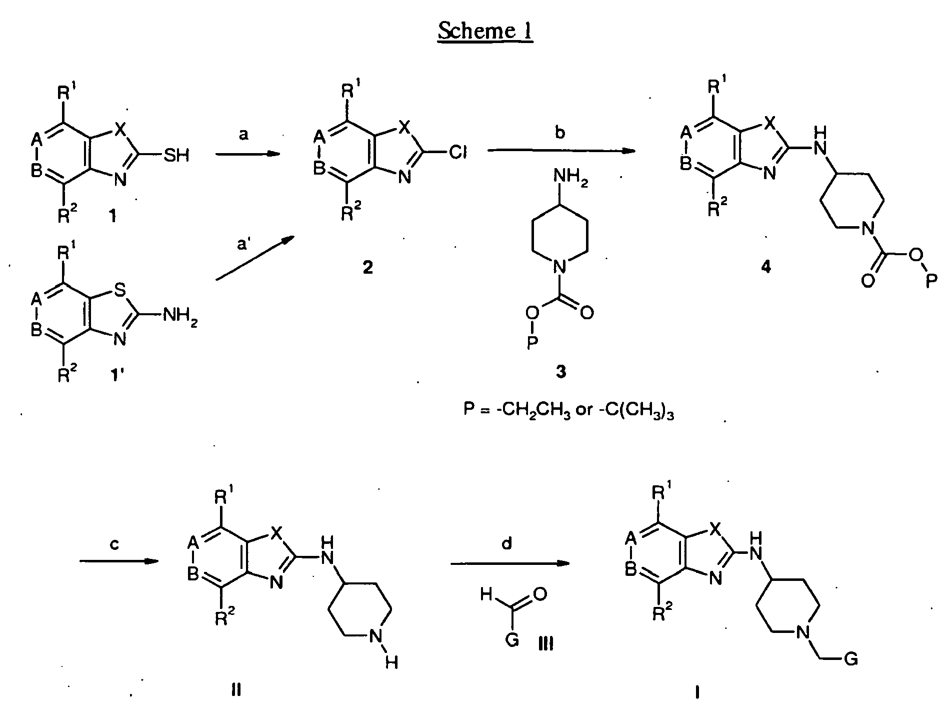

Chloro-thiazoles or oxazoles 2 (scheme 1), optionally substituted at the aryl or heteroaryl moiety attached to the azole heterocyles (A, B are N, N⁺-O-, CR³ or CR⁴ as defined herein before) are known, can be prepared by methods known in the art or can be prepared i) from precursor thiols 1 by treatment with thionyl chloride in the presence of a catalytic amount of *N,N*-dimethylformamide at temperatures between room temperature and the reflux temperature of the solvents (preferred method for the formation of chloro-oxazoles) or by treatment with sulfuryl chloride preferably at room temperature [as described in US 2469697 (1949, Eastman Kodak Co.)] (method for the formation of chloro-thiazoles) (step a) or ii) by treatment of 2-amino-thiazoles 1' with *tert*-butyl nitrite and copper (II)-chloride in a solvent like MeCN at temperatures around 65 °C (step a'). Chloro-thiazoles or oxazoles 2 (scheme 1) react with a suitably protected amino-piperidine derivative 3 in the presence of a base like *N*-ethyl-diisopropylamine and optionally an additional solvent like *N,N*-dimethylformamide or acetonitrile at temperatures preferably between room temperature and the reflux temperature of the solvents to give the amino azoles 4 (step b). The protecting group present in compounds 4 is then removed using, *e*. *g.,* 48% aqueous hydrogen bromide as reagent preferably at elevated temperatures to remove an ethyl carbamate or using trifluoroacetic acid in a solvent like dichloromethane preferable at room temperature to remove a BOC-protective group (step c). Secondary amines II then react with aldehydes III in the presence of a reducing agent such as pyridine-BH₃ complex, NaBH(OAc)₃ or NaCNBH₃ under acidic conditions (*e*.*g.,* acetic acid, formic acid), by using a Lewis acid (*e. g*., Ti(iPrO)₄, ZnCl₂) or under buffered conditions, *e. g*., in the presence of acetic acid and a tertiary amine like *N*-ethyl-diisopropylamine, in a suitable solvent such as dichloromethane, dichloroethane, ethanol or isopropanol (or mixtures thereof) at ambient or elevated temperatures using conventional heating or heating by microwave irradiation to give compounds of formula I (step d).

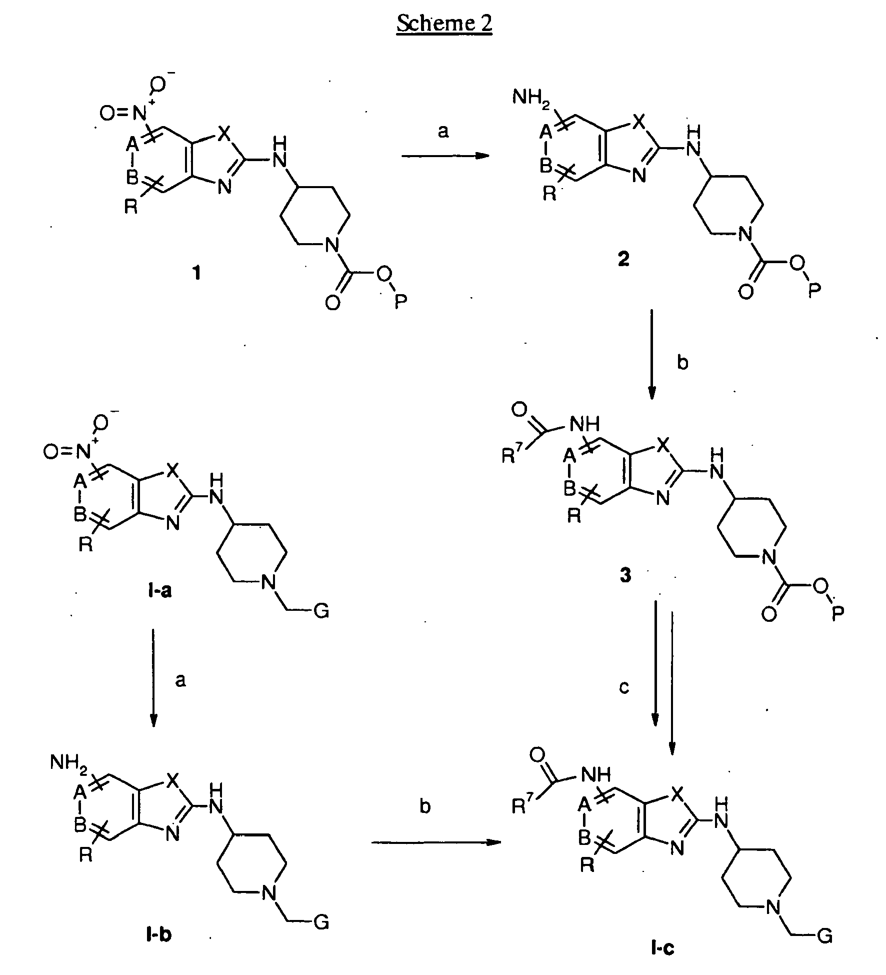

Nitro azoles 1 or I-a (substituted at any position of the aromatic ring attached to the azole moiety, scheme 2) can be reduced to the corresponding amino derivatives 2 or I-b either by catalytic hydrogenation, preferably with a platinum catalyst, if an *N*-benzyl moiety is present as in I-a (step a). Alternatively, chemical reductions using iron, zink or tin reagents can be used. Primary amino compounds 2 or I-b can be coupled to various types of acids or acid chlorides by well known coupling methods to give amides 3 or I-c (step b). The transformation of the amides 3 into compounds I-c can be performed as described for the transformation of compounds 4 (scheme 1) into compounds I. Compounds I-c (scheme 2) which contain an ester function in the amide substituent R⁷CONH-, can be used as such or optionally can be saponified, *e.g*., using lithium hydroxide in a solvent like tetrahydrofuran/water to give free acids I-c.

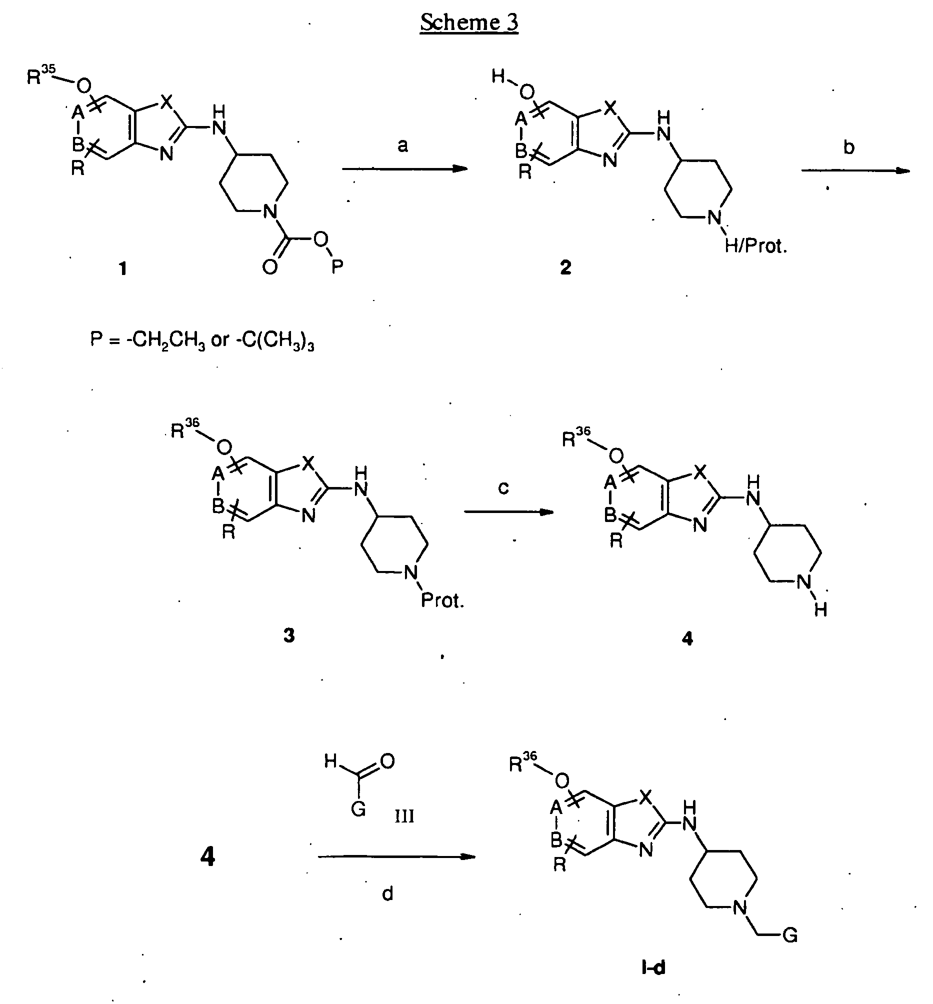

Alkoxy substituted azoles 1 (substituted at any position of the aromatic ring attached to the azole moiety, scheme 3), with R³⁵ preferably being a methyl or a benzyl group can be transformed into phenolic compounds 2 carrying a protective function at the secondary nitrogen group either directly, *e. g*., by catalytic hydrogenation of a benzyl ether function or indirectly by simultaneous cleavage of the methoxy or benzyloxy function and the nitrogen protective group and subsequent re-introduction of the latter (step a). Conditions which might need re-introduction of a protective group at the secondary nitrogen moiety are, *e. g*., use of 48% aqueous hydrobromic acid at elevated temperatures to cleave an aromatic methoxy function or the use of boron trifluoride-ethyletherate and dimethyl sulfide in a solvent like dichloromethane, preferably at reflux, to cleave an aromatic benzyl ether function. Intermediate 2 carrying a protective group at the secondary nitrogen function can react with suitable halides, mesylates, tosylates or alcohols transformed into any other suitable leaving group in a polar solvent such as *N,N*-dimethylformamide or acetone and a suitable base (*e. g.*, C_{S2}CO₃, K₂CO₃) at room temperature or elevated temperatures, by *Mitsunobu* reaction with alcohols activated by a mixture of triphenylphosphine and diethyl- or di-*tert*-butyl-azodicarboxylate, or by analogous alkylation reactions giving modified azole compounds 3 (step b). Alternatively, intermediates 2 can react with a sulfonyl chloride in a solvent like dichloromethane in the presence of base like *N*-ethyl diisopropylamine preferably at temperatures between 0 °C and room temperature to give sulfonate esters R³⁶O. Optionally, the substituent R³⁶O can be modified at any stage of the synthesis. Removal of the protective function in compounds 3 gives compounds 4 (step c). The transformation of compounds 4 (scheme 3) into compounds I-d can be performed in full analogy to the transformation of compounds II (scheme 1) into compounds I. Compounds I-d (scheme 3) which contain an ester function in the ether substituent R³⁶O, can be used as such or optionally can be saponified, *e. g*., using lithium hydroxide in a solvent like tetrahydrofuran/water to give free acids I-d.

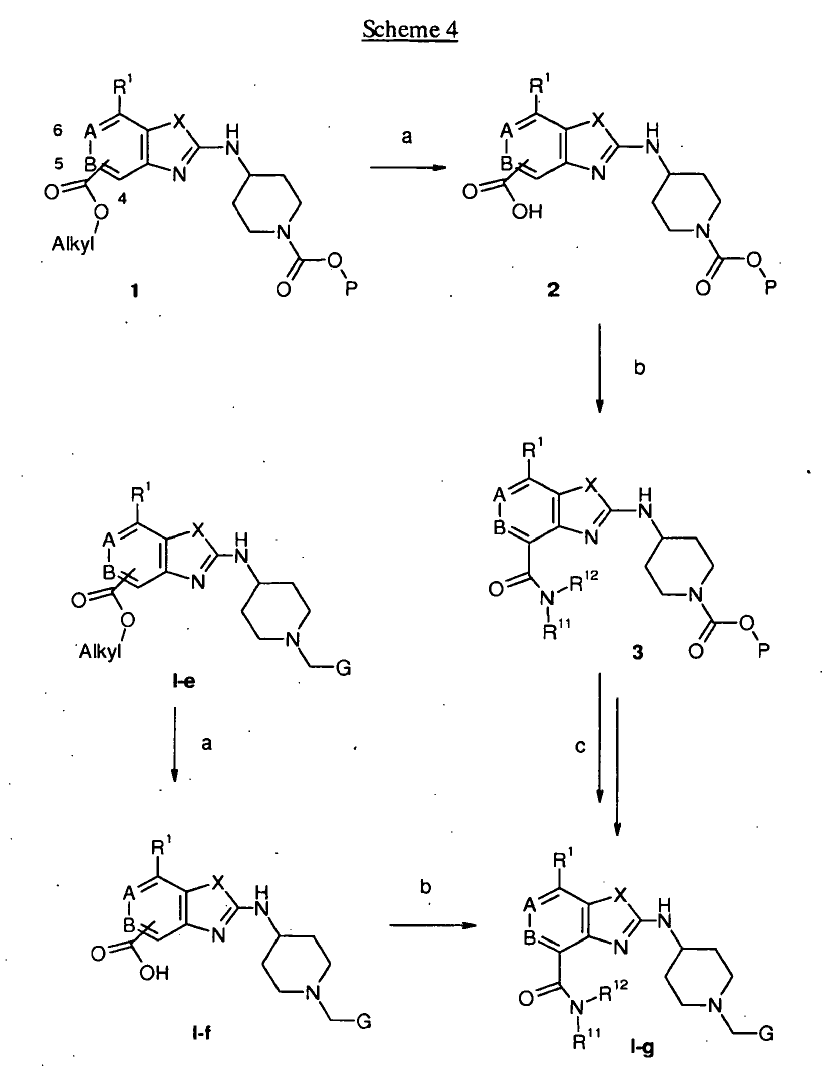

Compounds 1. or compounds I-e (scheme 4) carrying an ester function at position 4, 5 or 6 of the aromatic ring attached to the azole moiety, can be saponified, *e. g.,* using lithium hydroxide in a solvent like tetrahydrofuran/water to give free acids 2 or I-f (step a). Acids 2 or I-f, carrying the carboxy function at position 4, can then be coupled to various types of amines by well known coupling methods to give amides 3 or I-g (step b). The transformation of the amides 3 into compounds I-g can be performed as described for the transformation of compounds 4 (scheme 1) into compounds I. Compounds I-g (scheme 4) which contain an ester function in the amide substituent R¹¹R¹²NCO-, can be used as such or optionally can be saponified, *e. g*., using lithium hydroxide in a solvent like tetrahydrofuran/water to give free acids I-g.

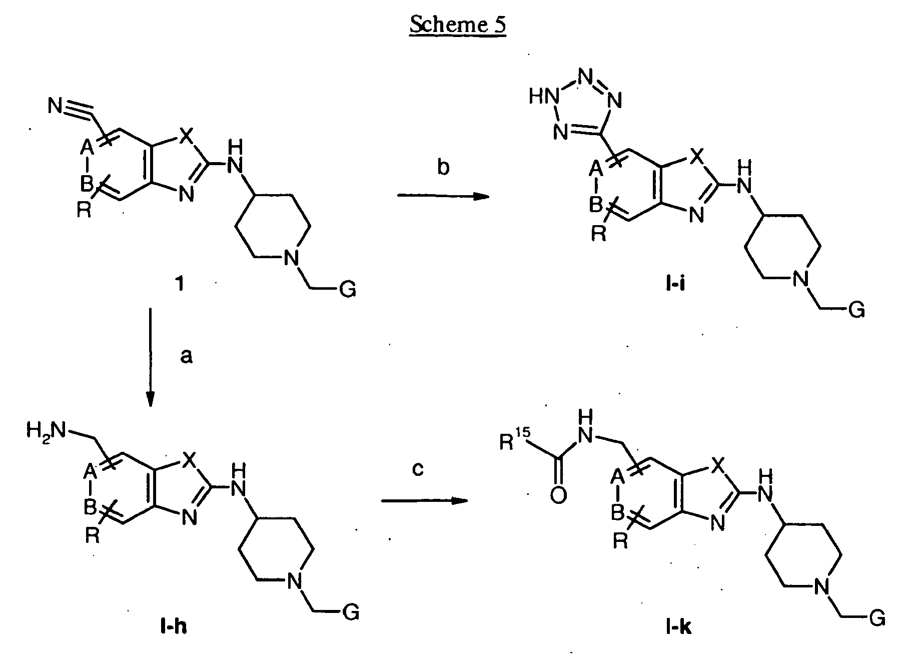

Nitriles 1 (residing at any position of the aromatic ring attached to the azole moiety, scheme 5) can be reduced to the primary amino compounds I-h, *e. g*., by using borane-dimethylsulfide in tetrahydrofuran preferably at reflux (step a); alternatively nitriles 1 can be converted into tetrazoles I-i, *e*. *g.,* by treatment with sodium azide in the presence of ammonium hydrochloride in a solvent like *N,N-*dimethyl-formamide at elevated temperature optionally in the presence of microwave irradiation (step b). Amines I-h can then be coupled to various types of acids or acid chlorides by well known coupling methods to give amides I-k (step c). Compounds I-k (scheme 5) which contain an ester function in the amide substituent R¹⁵CONHCH₂, can be used as such or optionally can be saponified using, *e.g*., lithium hydroxide in a solvent like tetrahydrofuran/water to give free acids I-k.

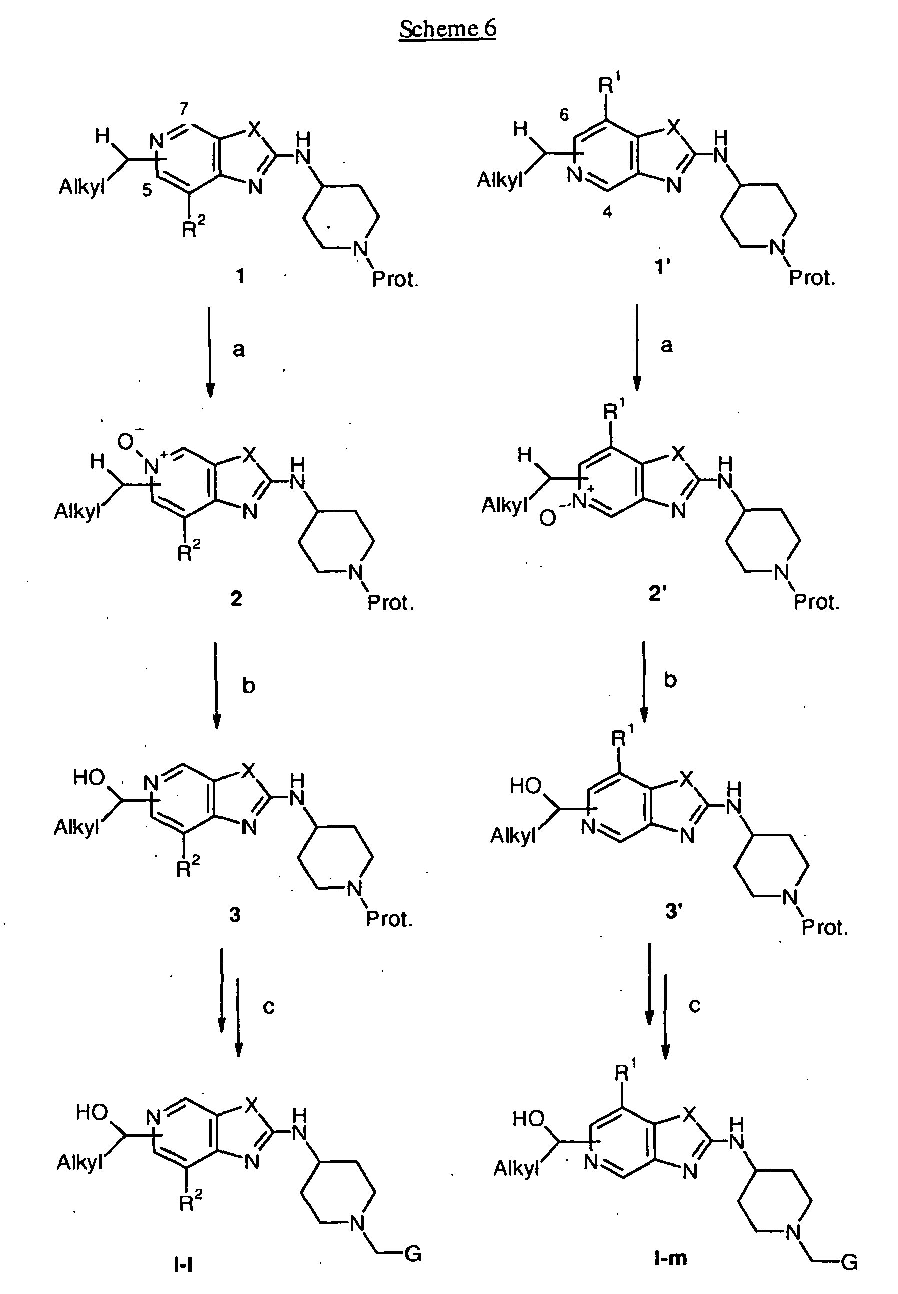

Alkyl pyridine azoles 1, substituted at position 5 or 7 of the aromatic ring attached to the azole moiety, or 1', substituted at postion 4 or 6 of the aromatic ring attached to the azole moiety, (scheme 6) can be oxidized to the corresponding N-oxides 2 or 2', *e.g*., by using hydrogen peroxide, *m*-chloroperbenzoic acid or peracetic acid in solvents such as dichloromethane or acetic or trifluoro acetic acid (step a). In case the protective group at the secondary nitrogen atom is removed under such reaction conditions, it can be reintroduced. Treatment of N-oxides 2 or 2' with trifluoro acetic anhydride or acetic anhydride in solvents like dichloromethane followed by mild saponification leads to alcohols 3 or 3' with or without concomitant loss of the protective group. Alternatively, N-oxides 2 or 2' can be treated with ClCOOEt in the presence of triethylamine in solvents like ethanol or dichloromethane giving rearranged carbonates and after mild saponification alcohols 3 or 3' in a sequence avoiding acidic conditions (step b). The transformation of the alcohols 3 or 3' into compounds I-l or I-m can be performed as described for the transformation of compounds 4 (scheme 1) into compounds I.

The synthesis of aldehydes of formula III can for example be carried out according to the procedures described in scheme 7.

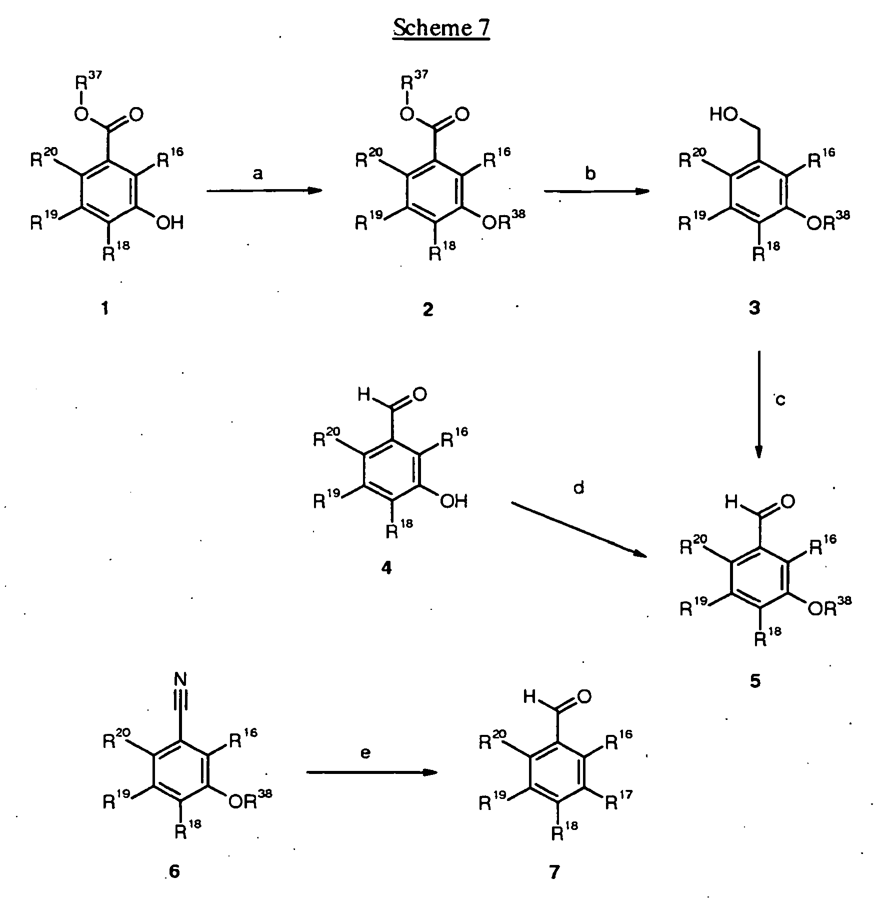

Aldehydes of the general formula III are either commercially available or can be derived by alkylation of the phenolic carboxylic esters or acids of formula 1 with alkyl halides, alkyl mesylates, alkyl tosylates or alcohols transformed into any other suitable leaving group in a polar solvent such as *N,N*-dimethylformamide or acetone and a suitable base (*e*. *g*., Cs₂CO₃, K₂CO₃) at room temperature or elevated temperatures, by *Mitsunobu* reaction with alcohols activated by a mixture of triphenylphosphine and diethylazodicarboxylate, or by analogous alkylation reactions (scheme 7, step a). The corresponding benzylic alcohols of formula 3 are provided by reduction of the esters of formula 2 by a suitable reducing agent (*e*. *g*., diisobutylaluminum hydride or by LiAlH₄) in a solvent such as THF (step b). These benzylic alcohols can then be oxidized to the aldehydes of formula 5, preferably with MnO₂ as oxidant in dichloromethane (step c). Alternatively the introduction of the side-chain can be accomplished by direct alkylation of the phenolic benzaldehydes of formula 4 providing the desired compounds of formula 5 directly (step d). A further well-established route towards the synthesis of benzylaldehydes of formula 7 consists in the reduction of the corresponding benzonitriles of formula 6 by a suitable reducing agent such as diisobutylaluminum hydride at low temperature in a non-protic polar solvent (*e. g.,* THF) (step e).

Additional syntheses of aldehydes of formula III are described in the examples.

As described hereinbefore, it has been found that the compounds of formula I possess pharmaceutical activity, in particular they are modulators of somatostatin receptor activity. More particularly, the compounds of the present invention have been found to be antagonists of the somatostatin receptor subtype 5 (SSTR5).

The following tests were carried out in order to determine the activity of the compounds of formula (I).

A CHO cell line stably transfected with a plasmid encoding the human subtype 5 somatostatin receptor (GenBank accession number D16827) was obtained from Euroscreen. Cells were cultured and used for binding and functional assays.

Membranes of these cells were prepared by sonication in the presence of protease inhibitors and subsequent fractionating centrifugation. The protein concentration in the membrane preparation was determined using a commercial kit (BCA kit, Pierce, USA). Membranes were stored at -80°C until use. After thawing, membranes were diluted in assay buffer (50 mM TRIS-HCl at pH 7.4, 5 mM MgCl₂ and 0.20 % BSA (bovine serum albumine)) and subjected to dounce homogenization.

For binding studies, 0.1 mL membrane suspension, corresponding to app. 6 x 10⁻¹⁵ mol receptor, was incubated for 1 hour at room temperature with 0.05 nM ¹²⁵I-labeled tracer (11-Tyr somatostatin-14, Perkin-Elmer) and either test compounds in varying concentrations or, for the determination of non-specific binding, 0.001 mM non-labeled somatostatin-14 (Sigma-Aldrich, Buchs, Switzerland). The incubation was stopped by filtration through GF/B glassfiber filters (Unifilter, Perkin-Elmer) and washing with ice-cold wash buffer (50 mM Tris-HCl at pH 7.4). The bound radioactivity was measured after application of a scintillation cocktail (Microscint 40, Perkin-Elmer) and expressed as disintegrations per minute (dpm).

The receptor concentration was determined in a prior saturation experiment where a fixed, arbitrary amount of membranes was incubated with a concentration range of radio-labeled tracer. This allows estimating the total number of specific binding sites per amount of protein (*i. e*., Bₘₐₓ), typically between 1 and 5 pmol/mg.

The concentration of the test compound required to result in half maximal inhibition of binding of the radio-labeled tracer (IC₅₀) was estimated from a concentration-versus-dpm graph, The binding affinity (Kᵢ) was calculated from the IC₅₀ by applying the Cheng-Prussoff equation for single binding sites.

For functional experiments, 50'000 cells were incubated in Krebs Ringer HEPES buffer (115 mM NaCl, 4.7 mM KCl, 2.56 mM CaCl₂, 1.2 mM KH₂PO₄, 1.2 mM MgSO₄, 20 mM NaHCO₃ and 16 mM HEPES, adjusted to pH 7.4) supplemented with 1 mM IBMX (3-isobutyl-1-methyl-xanthin) and 0.1% BSA, then stimulated with 0.004 mM forskolin. Simultaneously with forskolin, test compounds in varying concentrations were applied. Cells were then incubated for 20 minutes at 37 °C and 5% CO₂. Subsequently, cells were lysed and cAMP (cyclic adenosine monophosphate) concentration measured using a fluorescence-based commercial kit according to the manufacturer (HitHunter cAMP, DiscoverX).

The concentration of the test compound to induce a half maximal effect (i.e. EC₅₀) as well as the efficacy as compared to 0.15 nM somatostatin-14 were determined from concentration-versus-fluorescence (arbitrary units) graphs. For the determination of potential antagonism, 0.15 nM somatostatin-14 was applied together with the test compounds and the concentration of the test compounds to half maximally reverse the effect of somatostatin-14 (i.e. IC₅₀) were deduced from concentration-versus-fluorescence graphs.

The compounds of the present invention exhibit Kᵢ values of 0.1 nM to 10 µM, preferably Kᵢ values of 1 nM to 500 nM and more preferably 0.1 nM to 100 nM for human subtype 5 somatostatin receptor. The following table shows measured values for selected compounds of the present invention that are antagonists as assessed in functional experiments.

| | SSTR5 Kᵢ (nM) |
|---|---|
| Example 23 | 6 |
| Example 71 | 44 |
| Example 73 | 223 |

The compounds of formula (I) and their pharmaceutically acceptable salts and esters can be used as medicaments, *e.g*., in the form of pharmaceutical preparations for enteral, parenteral or topical administration. They can be administered, for example, perorally, *e*. *g.,* in the form of tablets, coated tablets, dragées, hard and soft gelatine capsules, solutions, emulsions or suspensions, rectally, *e. g*., in the form of suppositories, parenterally, *e. g*., in the form of injection solutions or infusion solutions, or topically, *e*. *g*., in the form of ointments, creams or oils.

The production of the pharmaceutical preparations can be effected in a manner which will be familiar to any person skilled in the art by bringing the described compounds of formula (I) and their pharmaceutically acceptable salts, into a galenical administration form together with suitable, non-toxic, inert, therapeutically compatible solid or liquid carrier materials and, if desired, usual pharmaceutical adjuvants.

Suitable carrier materials are not only inorganic carrier materials, but also organic carrier materials. Thus, for example, lactose, corn starch or derivatives thereof, talc, stearic acid or its salts can be used as carrier materials for tablets, coated tablets, dragées and hard gelatine capsules. Suitable carrier materials for soft gelatine capsules are, for example, vegetable oils, waxes, fats and semi-solid and liquid polyols (depending on the nature of the active ingredient no carriers are, however, requited in the case of soft gelatine capsules). Suitable carrier materials for the production of solutions and syrups are, for example, water, polyols, sucrose, invert sugar and the like. Suitable carrier materials for injection solutions are, for example, water, alcohols, polyols, glycerol and vegetable oils. Suitable carrier materials for suppositories are, for example, natural or hardened oils, waxes, fats and semi-liquid or liquid polyols. Suitable carrier materials for topical preparations are glycerides, semi-synthetic and synthetic glycerides, hydrogenated oils, liquid waxes, liquid paraffins, liquid fatty alcohols, sterols, polyethylene glycols and cellulose derivatives.

Usual stabilizers, preservatives, wetting and emulsifying agents, consistency-improving agents, flavour-improving agents, salts for varying the osmotic pressure, buffer substances, solubilizers, colorants and masking agents and antioxidants come into consideration as pharmaceutical adjuvants.

The dosage of the compounds of formula (I) can vary within wide limits depending on the disease to be controlled, the age and the individual condition of the patient and the mode of administration, and will, of course, be fitted to the individual requirements in each particular case. For adult patients a daily dosage of about 1 mg to about 1000 mg, especially about 1 mg to about. 100 mg, comes into consideration. Depending on the dosage it is convenient to administer the daily dosage in several dosage units.

The pharmaceutical preparations conveniently contain about 0.1-500 mg, preferably 0.5-100 mg, of a compound of formula (I).

The present invention will be further explained by reference to the following illustrative examples. They are, however, not intended to limit its scope in any manner.

### Examples

Abbreviation s:
AcOEt = ethyl acetate, Ar = argon, BuLi = butyllithium, DMF = *N,N-*dimethylformamide, h = hour(s), DMSO = dimethyl sulfoxide, HPLC = high performance liquid chromatography, Hyflo = infusorial earth, i. V. = *in vacuo,* LDA= lithium diisopropylamide, min = minunte(s), mL= mililiter, MeCl₂ = dichloromethane,
POCl₃ = phosphorous oxychloride, RT = room temperature, TFA = trifluoroacetic acid,
TFAA= trifluoroacetic anhydride, THF= tetrahydrofuran.

### Example 1

[1-(3-Ethoxy-4-methoxy-benzyl)-piperidin-4-yl]-(7-nitro-benzooxazol-2-yl)-amine A] 0.26 g (1.0 mMol) of (7-nitro-benzooxazol-2-yl)-piperidin-4-yl-amine and 0.19 g (1.05 eq.) of 3-ethoxy-4-methoxybenzaldehyde were dissolved under Ar in 2 mL of EtOH; 0.25 mL (0,19 g = 1.5 eq.) of *N*-ethyl-diisopropylamine and 0.11 mL (0,12 g = 2.0 eq.) of glacial acetic acid were added and the mixture then heated for 2 h at 50 °C. After cooling down to RT, 0.16 g (2.5 eq.) of sodium cyanoborohydride was added and the reaction mixture heated again for 1.5 h at 50 °C. It was then poured into crashed ice, the pH was adjusted to ∼ 11 with sodium carbonate solution and the mixture was extracted twice with MeCl₂; the organic phases were washed with water, dried over MgSO₄, filtered and evaporated i.v. The crude product was purified by chromatography (silicagel, eluent: gradient of MeCl₂ / MeOH) to yield 0.34 g of the title compound as yellow foam.
MS: 427.2 (M+H)⁺.
The (7-nitro-benzooxazol-2-yl)-piperidin-4-yl-amine used in 1A] was synthesized as follows:

### B] 2-Chloro-7-nitro-benzooxazole

5.0 g (25.5 mMol) of 7-nitro-benzooxazole-2-thiol [PCT Int. Appl. (1994), WO 9406782 A1] was dissolved under Ar in 28.5 mL (14 eq.) of thionyl chloride at RT; 0.1 mL of DMF was added as catalyst and the reaction then heated at reflux for 30 min. After cooling down to 50 °C, 100 mL of toluene was added and the mixture was evaporated i.v. to remove the excess of thionyl chloride. The residue was poured into cold water, toluene was added and it was extracted twice; the organic phases were washed with water, dried over MugsO4, filtered and evaporated i.v. to yield 4.04 g of the title compound as yellow solid.
MS: 198.0 (M)⁺, 1 Cl.

### C] 4-(7-Nitro-benzooxazol-2-ylamino)-piperidine-1-carboxylic acid tert-butyl ester

3.8 g (19.1 mMol) of 2-chloro-7-nitro-benzooxazole was suspended under Ar in 20 mL of MeCl₂ at RT; while stirring, 4.98 mL (3.79 g = 1.5 eq.) of *N*-ethyl
diisopropylamine was added, which resulted in a clear solution. 4.39 g (1.1 eq.) of 1*-tert-*butoxycarbonyl-4-aminopiperidine was added in small portions and the solution stirred for 1 h at RT. Then, the reaction mixture was poured into crashed ice and extracted twice with MeCl₂, the organic phases were washed with water, dried over MgSO₄, filtered and evaporated i.v. The crude product was purified by chromatography (silicagel, eluent: gradient of MeCl₂ / MeOH) to yield 6.11 g of the title compound as yellow solid.
MS: 363.0 (M+H)⁺.

### D] (7-Nitro-benzooxazol-2-yl)-niperidin-4-yl-amine

5.80 g (16 mMol) of 4-(7-nitro-benzooxazol-2-ylamino)-piperidine-1-carboxylic acid *tert*-butyl ester was dissolved under Ar in 100 mL of MeCl₂; to the stirred solution, 12.25 mL (18.25 g =10 eq.) of TFA was added in small portions and stirring continued at RT for 3 h. Then, the reaction mixture was evaporated i.v. , the residue was dissolved in MeCl₂ and water, the pH was adjusted to -12 with sodium hydroxide solution and the mixture was extracted twice with MeCl₂; the organic phases were washed with water, dried over MgSO₄, filtered and evaporated i.v. The crude product was purified by chromatography (silicagel, eluent: gradient of MeCl₂ / MeOH) to yield 3.71g of the title compound as yellow solid.
MS: 262.8 (M)⁺.

### Example 2

[1-(3,5-Diethoxy-4-fluoro-benzyl)-piperidin-4-yl]-(7-nitro-benzooxazol-2-yl)-amine A] In analogy to the procedure described in example 1A], (7-nitro-benzooxazol-2-yl)-piperidin-4-yl-amine (example 1D]) was reacted with 3,5-diethoxy-4-fluorobenzaldehyde (example 2H]), sodium cyanoborohydride, *N*-ethyl-diisopropylamine and acetic acid in ethanol at 50°C to yield the title compound as yellow solid.
MS: 459.2 (M+H)⁺.
The 3,5-diethoxy-4-fluoro-benzaldehyde used in 2A] was synthesized as follows:

### B] tert-Butyl-(4-fluoro-benzyloxy)-dimethyl-silane

To a solution of (4-fluoro-phenyl)-methanol (12.16 g, 96.4 mmol, 1.0 eq.) in anhydrous DMF (50 mL) at 0 °C under Ar was added imidazole (7.22 g, 106.1 mMol, 1.1 eq.) and *tert*-butyl-chloro-dimethyl-silane (15.99 g, 106.1 mMol, 1.1 eq.). After the addition was completed the cooling bath was removed and the reaction stirred for 18 h at RT. The reaction mixture was poured on ice, extracted with ethyl acetate (2 x 100 mL) and the combined organic phases washed with a sat. solution of sodium carbonate (2 x 100 mL) and sodium chloride (2 x 100 mL). The organic phase was dried over Na₂SO₄, concentrated by evaporation under reduced pressure yielding a brown oil that was purified by high vacuum destillation (bp 32-35 °C at 0.1 mbar) to give 23.0 g (99%) of the title compound. ¹H NMR (400 MHz, CDCl₃): δ 0.00 (s, 6H), 0.84 (s, 9H), 4.60 (s, 2H), 6.89-6.94 (m, 2H), 7.16-7.20 (m, 2H). MS: 183.1 [M-*tert*-Bu]⁺.

### C] 5-(tert-Butyl-dimethyl-silanyloxymethyl)-2-fluoro-phenol

To a solution of *tert*-butyl-(4-fluoro-benzyloxy)-dimethyl-silane (5.00 g, 20.8 mMol, 1.0 eq.) in anhydrous THF (20 mL) was added at -78 °C under Ar a solution of *sec-*BuLi (17.6 mL, 22.8 mmol, 1.1 eq., 1.3 M solution in hexane) within 30 min. Then a solution of trimethyl borate (2.37 mL, 2.20 g, 20.8 mMol, 1.0 eq.) in anhydrous THF (7.5 mL) was added slowly within 30 min and the cooling bath removed. A solution of conc. acetic acid (2.78 mL, 1.87 g, 31.2 mMol, 1.5 eq.) was slowly added followed by addition of 35% hydrogen peroxide (2.22 mL, 0.78 g, 22.9 mMol, 1.1 eq.) and the reaction mixture kept at 0 °C for 30 min. After stirring at RT for an additional 4 h, the reaction was extracted with diethyl ether (2 x 100 mL) and the combined organic phases washed with a solution of 10% sodium hydroxide (2 x 100 mL) and a sat. solution of sodium chloride (2 x 100 mL). The organic phase was dried over Na₂SO₄, concentrated by evaporation under reduced pressure and the crude material purified with column chromatography on silica eluting with hexane/ethyl acetate (19:1) providing 4.80 g (90%) of the title compound. ¹H NMR (400 MHz, CDCl₃): δ 0.00 (s, 6H), 0.84 (s, 9H), 4.56 (s, 2H), 4.97 (br s, 1H), 6.68-6.72 (m, 1H), 6.87-6.94 (m, 2H). MS: 256.2 [M]⁺.

### D] 2-(tert-Butyl-dimethyl-silanyloxy)-4-(tert-butyl-dimethyl-silanyloxymethyl)-1-fluoro-benzene

To a solution of 5-(*tert*-butyl-dimethyl-silanyloxymethyl)-2-fluoro-phenol (4.60 g, 17.9 mMol, 1.0 eq.) in anhydrous DMF (20 mL) at 0°C under Ar was added imidazole (1.34 g, 19.7 mMol, 1.1 eq.) and *tert*-butyl-chloro-dimethyl-silane (2.97 g, 19.7 mMol, 1.1 eq.). After the addition was completed the cooling bath was removed and the reaction stirred for 18 h at RT. The reaction mixture was poured on ice, extracted with ethyl acetate (2 x 100 mL) and the combined organic phases washed with a sat. solution of sodium carbonate (2 x 100 mL) and sodium chloride (2 x 100 mL). The organic phase was dried over Na₂SO₄ and concentrated by evaporation under reduced pressure yielding 4.50 g (68%) of the title compound. ¹H NMR (400 MHz, CDCl₃): δ 0.00 (s, 6H), 0.10 (s, 6H), 0.85 (s, 9H), 0.92 (s, 9H), 4.55 (s, 2H), 6.71-6.74 (m, 1H), 6.80-6.83 (m, 1H), 6.87-6.92 (m, 1H). MS: 370.2 [M]⁺.

### E] 3-(tert-Butyl-dimethyl-silanyloxy)-5-(tert-butyl-dimethyl-silanyloxymethyl)-2-fluorophenol

To a solution of 2-(*tert*-butyl-dimethyl-silanyloxy)-4-(*tert*-butyl-dimethyl-silanyloxymethyl)-1-fluoro-benzene (23.70 g, 63.9 mMol, 1.0 eq.) in anhydrous THF (130 mL) was added at -78 °C under Ar a solution of *sec*-BuLi (54.5 mL, 71.6 mmol, 1.1 eq., 1.3 M solution in hexane) within 30 min. Then a solution of trimethyl borate (7.13 mL, 6.64 g, 63.9 mMol, 1.0 eq.) in anhydrous THF (30 mL) was added slowly within 30 min and the cooling bath removed. A solution of conc. acetic acid (5.49 mL, 5.76 g, 95.9 mMol, 1.5 eq.) was slowly added followed by addition of 35% hydrogen peroxide (6.28 mL, 2.39 g, 70.3 mMol, 1.1 eq.) and the reaction mixture kept at 0 °C for 30 min. After stirring at RT for an additional 4 h, the reaction was extracted with diethyl ether (2 x 100 mL) and the combined organic phases washed with a solution of 10% sodium hydroxide (2 x 100 mL) and a sat. solution of sodium chloride (2 x 100 mL). The organic phase was dried over Na₂SO₄, concentrated by evaporation under reduced pressure and the crude material purified with column chromatography on silica eluting with hexane/ethyl acetate (19:1) providing 15.80 g (64%) of the title compound. ¹H NMR (400 MHz, CDCl₃): δ 0.00 (s, 6H), 0.10 (s, 6H), 0.85 (s, 9H), 0.91 (s, 9H), 4.50 (s, 2H), 4.93 (br s, 1H), 6.37 (d, *J*= 5.6 Hz, 1H), 6.47 (d, *J*= 5.6 Hz, 1H). MS: 329.2 [M-*tert*-Bu]⁺.

### F] tert-Butyl-(3,5-diethoxy-4-fluoro-benzyloxy)-dimethyl-silane

To a solution of 3-(*tert*-butyl-dimethyl-silanyloxy)-5-(*tert*-butyl-dimethyl-silanyloxymethyl)-2-fluoro-phenol (5.80 g, 15.0 mMol, 1.0 eq.) in DMF (60 mL) was added potassium carbonate (4.56 g, 33.0 mMol, 2.2 eq.) and ethyl bromide (2.46 mL, 3.60 g, 33.0 mMol, 2.2 eq.) and the reaction mixture stirred under Ar at 60 °C for 5 h. The potassium carbonate was removed by filtration, the crude reaction mixture concentrated by evaporation under reduced pressure, the residue extracted with ethyl acetate (3 x 100 mL), the combined organic phases washed with water (2 x 100 mL) and dried over Na₂SO₄. The solvent was removed by evaporation under reduced pressure and the crude material purified with column chromatography on silica eluting with hexane/ethyl acetate (99:1) providing 3.10 g (63%) of the title compound. ¹H NMR (400 MHz, CDCl₃): δ 0.00 (s, 6H), 0.85 (s, 9H), 1.33 (t, *J*= 7.0 Hz, 6H), 4.00 (q, *J*= 7.0 Hz, 4H), 4.55 (s, 2H), 6.47 (d, *J*= 6.8 Hz, 2H). MS: 329.3 [MH]⁺.

### G] (3,5-Diethoxy-4-fluoro-phenyl)-methanol

To a solution of *tert*-butyl-(3,5-diethoxy-4-fluoro-benzyloxy)-dimethyl-silane (1.20 g, 3.65 mMol, 1.0 eq.) in methanol (8 mL) was added Dowex 50W-X8 (0.33 g) and the reaction mixture stirred under Ar at RT for 22 h. The resin was removed by filtration and the reaction mixture concentrated by evaporation under reduced pressure yielding the title compound in quantitative yield (0.78 g). ¹H NMR (400 MHz, CDCl₃): δ 1.34 (t, *J* = 7.0 Hz, 6H), 1.57 (t, *J*= 5.4 Hz, 1H), 4.01 (q, *J*= 7.0 Hz, 4H), 4.51 (d, *J*= 5.4 Hz, 2H), 6.51 (d, *J*= 6.8 Hz, 2H). MS: 214.2 [M]⁺.

### H] 3,5-Diethoxy-4-fluoro-benzaldehyde

To a solution of (3,5-diethoxy-4-fluoro-phenyl)-methanol (2.30 g, 10.7 mMol, 1.0 eq.) in 1,2-dichloroethane (50 mL) was added MnO₂ (2.89 g, 33.3 mmol, 3.1 eq.). The reaction mixture was stirred for 21 h at 50 °C and filtered through Hyflo providing 1.90 g (83%) of the title compound. ¹H NMR (400 MHz, CDCl₃): δ 1.38 (t, *J*= 7.0 Hz, 6H), 4.09 (q, *J*= 7.0 Hz, 4H), 7.04 (d, *J*= 7.2 Hz, 2H), 9.75 (s, 1H). MS: 212.1 [M]⁺.

### Example 3

### N²-[1-(3,5-Diethoxy-4-fluoro-benzyl)-piperidin-4-yl]-benzooxazole-2,7-diamine

3.40 g (7.4 mMol) of [1-(3,5-diethoxy-4-fluoro-benzyl)-piperidin-4-yl]-(7-nitro-benzooxazol-2-yl)-amine (example 2]) was dissolved under Ar in 100 mL of THF followed by 100 mL of MeOH; 0.34 g of platinum(IV) oxide (0.2 eq.) was added and the mixture then hydrogenated at RT and 1 bar H₂. After 1 h, the reaction mixture was filtered with the aid of dicalite; then, the solvent was evaporated i.v. The crude product was purified by chromatography (silicagel, eluent: gradient of MeCb / MeOH) to yield 3.02 g of the title compound as off-white solid.
MS: 429.3 (M+H)⁺.

### Example 4

### N-{2-[1-(3,5-Diethoxy-4-fluoro-benzyl)-piperidin-4-ylamino]-benzooxazol-7-yl}-2-methoxy-acetamide

0.40 g (0.94 mMol) of *N*²-[1-(3,5-diethoxy-4-fluoro-benzyl)-piperidin-4-yl]-benzooxazole-2,7-diamine (example 3]) was suspended in 10 mL of MeCl₂ at RT; then, 0.24 mL (0.185 g = 1.5 eq.) of *N*-ethyl diisopropylamine was added to result in a clear solution; 0.11 mL (0.125 g = 1.2 eq.) of methoxyacetyl chloride was added drop by drop. After 16 h stirring at RT, the reaction mixture was stirred at 45 °C for 5 h to complete the conversion. The reaction mixture was poured into crashed ice and extracted twice with MeCl₂; the organic phases were washed with water, dried over MgSO₄, filtered and evaporated i.v. The crude product was purified by chromatography (silicagel, eluent: gradient of MeCl₂ / MeOH) to yield 0.11 g of the title compound as colorless solid.
MS: 501.2 (M+H)⁺.

### Example 5

### N-{2-[1-(3,5-Diethoxy-4-fluoro-benzyl)-piperidin-4-ylamino]-benzooxazol-7-yl}-succinamic acid

0.54 g(1.0 mMol) of crude *N*-{2-1-(3,5-diethoxy-4-fluoro-benzyl)-piperidin-4-ylamino]-benzooxazol-7-yl}-succinamic acid methyl ester (prepared from *N*²-[1-(3,5-diethoxy-4-fluoro-benzyl)-piperidin-4-yl]-benzooxazole-2,7-diamine (example 3]) and 3-carbomethoxypropionylchloride in analogy to the procedure described in example 4]) was reacted with 1.0 mL of LiOH / water (1.0 molar) in 15 mL of THF/MeOH (2:1) at 0 °C to RT. After 5 h, the reaction mixture was poured into crashed ice, the pH was adjusted to ∼7.0 with aq. HCl (1N) and the reaction mixture was extracted twice with MeCl₂; the organic phases were washed with water, dried over MgSO₄, filtered and evaporated i.v. The crude product was purified by chromatography (silicagel, eluent: gradient of MeCl₂ / MeOH) to yield 0.135 g of the title compound as yellow solid.
MS: 527.1 (M-H)-..

### Example 6

### Pyrimidine-5-carboxylic acid {2-[1-(3,5-diethoxy-4-fluoro-benzyl)-piperidin-4-ylamino]-benzooxazol-7-yl}-amide

0.40 g (0.94 mMol) of *N*²-[1-(3,5-diethoxy-4-fluoro-benzyl)-piperidin-4-yl]-benzooxazole-2,7-diamine (example 3]) was suspended in 10 mL of MeCl₂ at RT under Ar; then,-0.116 g (1.0 eq.) of pyrimidine-5-carboxylic acid, 0.219 g (1.20 eq.) of N-(3-dimethylaminopropyl)-N'-ethyl-carbodiimide hydrochloride and 0.151 g (1.30 eq.) of *N,N*-dimethyl-4-aminopyridine were added. The reaction mixture became a clear solution after stirring for 1 h at RT. After 16 h, the solution was evaporated i.v. and the residue was purified by chromatography (silicagel, eluent: gradient of MeCl₂ / MeOH) to yield 0.115 g of the title compound as colorless foam.
MS: 535.3 (M+H)⁺.

### Example 7

### N-{2-[1-(3,5-Diethoxy-4-fluoro-benzyl)-piperidin-4-ylamino]-benzooxazol-7-yl}-2-pyridin-3-yl-acetamide

In analogy to the procedure described in example 6], *N*²-[1-(3,5-diethoxy-4-fluorobenzyl)-piperidin-4-yl]-benzooxazole-2,7-diamine (example 3]) was reacted with pyridin-3-yl-acetic acid, *N*-(3-dimethylamino-propyl)-*N*'-ethyl-carbodiimide-hydrochloride and *N,N*-dimethyl-4-aminopyridine in dichloromethane to yield the title compound as yellow foam.
MS: 548.4 (M+H)⁺.

### Example 8

### 1-Methyl-1H-imidazole-4-sulfonic acid {2-[1-(3,5-diethoxy-4-fluoro-benzyl)-piperidin-4-ylamino]-benzooxazol-7-yl}-amide

0.40 g (0.94 mMol) of *N*²-[1-(3,5-diethoxy-4-fluoro-benzyl)-piperidin-4-yl]-benzooxazole-2,7-diamine (example 3]) was suspended in 10 mL of MeCl₂ at RT under Ar; then, 0.24 mL (1.85 g = 1.5 eq.) of *N*-ethyl-diisopropylamine was added to result in a clear solution; 0.20 g (1.2 eq.) of 1-methyl-1H-imidazole-4-sulfonyl chloride was added in small portions and the mixture was stirred for 16 h at RT; it was subsequently stirred at 45 °C for 5 h to complete the conversion. Then, the reaction mixture was poured into crashed ice and extracted twice with MeCl₂; the organic phases were washed with water,

dried over MgSO₄, filtered and evaporated i.V. The crude product was purified by chromatography (silicagel, eluent: gradient of MeCl₂, / MeOH) to yield 0.14 g of the title compound as colorless foam.
MS: 573.3 (M+H)⁺.

### Example 9

### 2-[1-(3,5-Diethoxy-4-fluoro-benzyl)-piperidin-4-ylamino]-benzooxazol-7-yl}-methanesulfonamide

0.26 g (0.61 mMol) of *N*²-[1-(3,5-diethoxy-4-fluoro-benzyl)-piperidin-4-yl]-benzooxazole-2,7-diamine (example 3]) and 0.05 mL(0.053 g= 1.1 eq.) of pyridine were dissolved in 5 mL of MeCl₂ at 0 °C under Ar; then, 0.05 mL (0.07 g = 1.0 eq.) of methanesulfonyl chloride was added slowly. After stirring at RT for 16 h, the reaction mixture was poured into crashed ice and extracted twice with MeCl₂; the organic phases were washed with water, dried over MgSO₄, filtered and evaporated i.V. The crude product was purified by chromatography (silicagel, eluent: gradient of MeCl₂ / MeOH) to yield 0.033 g of the title compound as yellow solid.
MS: 507.3 (M+H)⁺.

### Example 10

### [1-(3,5-Diethoxy-4-fluoro-benzyl)-piperidin-4-yl]-[7-(pyrrolidine-1-sulfonyl)-benzooxazol-2-yl]-amine

A] In analogy to the procedure described in example 1A], piperidin-4-yl-[7-(pyrrolidine-1-sulfonyl)-benzooxazol-2-yl]-amine (example 10F]) was reacted with 3,5-diethoxy-4-fluoro-benzaldehyde (example 2H]), sodium cyanoborohydride, *N*-ethyl-diisopropylamine and acetic acid in ethanol at 50 °C to yield the title compound as light yellow solid.
MS: 547.3 (M+H)⁺.
The piperidin-4-yl-[7-(pyrrolidine-1-sulfonyl)-benzooxazol-2-yl]-amine used in 10A] was synthesized as follows:

### B] 2-Hydroxy-3-nitro-benzenesulfonyl chloride

2.30 g (14.9 mmol) of 2-am ino-6-nitro-phenol [PCT Int. Appl. WO 9611917 A1] was suspended in 7.8 mL aq. HCl (37%) and 3.2 mL of acetic acid and the mixture was cooled down to - 5°C; then, a solution of 1.13 a (1.1 eq.) of sodium nitrite in 1.8 mL water was added drop by drop. After stirring at 0 °C for 45 min, this reaction mixture was dropped into a saturated solution of sulfur dioxide in acetic acid at 0 °C, which contained 0.46 a (0.3 eq.) of copper(I)-chloride. Afterwards, the reaction mixture was warmed up to RT. 1 hour later, the mixture was poured into crashed ice and extracted three times with AcOEt; the organic phases were washed with water, dried over MgSO₄, filtered and evaporated i.V. to yield 2.81 g of the title compound as brown oil.
MS: 237.0 (M)⁺, 1 Cl.

### C] 2-Nitro-6-(pyrrolidine-1-sulfonyl)-phenol

2.80 g (11.8 mMol) of 2-hydroxy-3-nitro-benzenesulfonyl chloride and 6.13 mL (4.66 g = 35 mmol) of *N-*ethyl-diisopropylamine were dissolved in 56 mL of MeCl₂ and the mixture was cooled down to 0 °C; while stirring, 2.34 mL (2.01 g = 2.4 eq.) of pyrrolidine was added drop by drop and it was warmed up to RT. After 1 h, the reaction mixture was poured into crashed ice, acidified with aq. HCl (IN) and extracted twice with MeCl₂; the organic phases were washed with water, dried over MgSO₄ filtered and evaporated i.V. The crude product was purified by chromatography (silicagel, eluent: gradient of n-heptane / MeCl₂) to yield 2.33 g of the title compound as yellow solid.
MS: 271.1 (M-H)⁻.

### D] 2-Amino-6-(pyrrolidine-1-sulfonyl)-phenol

2.30 g (8.5 mMol) of 2-nitro-6-(pyrrolidine-1-sulfonyl)-phenol was dissolved under Ar in 100 mL of THF; 0.20 g of Pd on activated carbon (10%) was added and the mixture then hydrogenated at RT and 1 bar H₂. After 2 h, it was filtered with the aid of dicalite; then, the solvent was evaporated i.V. The crude product was purified by chromatography (silicagel, eluent: gradient of n-heptane / EtOAc) to yield 1.60 g of the title compound as yellow solid.
MS: 243.0 (M+H)⁺.

### E] 7-(Pyrlolidine-1-sulfonyl)-benzooxazole-2-thiol

1.55 g (6.4 mMol) of 2-amino-6-(pyrrolidine-1-sulfonyl)-phenol was dissolved in 25 mL of MeOH, 1.15 g (1.1 eq.) of potassium ethyl xanthogenate was added and the mixture was heated at reflux for 7 h; after cooling down to RT, the solvent was removed by evaporation i.V. The residue was dissolved in water, acidified with aq. HCl (IN) and subsequently, the product precipitated. Filtration, washing with water and drying over P₂O₅ gave 1.68 g of the title compound as off-white solid.
MS: 284.1 (M)⁺.

### F] Piperidin-4-yl-[7-(pyrrolidine-1-sulfonyl)-benzooxazol-2-yl]-amine

In analogy to the procedures described in examples 1B-D], 7-(pyrrolidine-1-sulfonyl)-benzooxazole-2-thiol was reacted with thionyl chloride to give 2-chloro-7-(pyrrolidine-1-sulfonyl)-benzooxazole, which was condensed with 4-amino-piperidine-carboxylic acid *tert*-butyl ester to give 4-[7-(pyrrolidine-1-sulfonyl)-benzooxazol-2-ylamino]-piperidine-1-carboxylic acid *tert*-butyl ester. Subsequent Boc removal resulted in the title compound which was obtained as colorless solid.
MS: 351.2 (M+H)⁺.

### Example 11

### [1-(3,5-Diethoxy-benzyl)-piperidin-4-yl]-[7-(pyrrolidine-1-sulfonyl)-benzooxazol-2-yl]-amine

In analogy to the procedure described in example 1A], piperidin-4-yl-[7-(pyrrolidine-1-sulfonyl)-benzooxazol-2-yl]-amine (example 10F]) was reacted with 3,5-diethoxy-benzaldehyde (prepared in analogy to the procedure described in example 2F], by reaction of 3,5-dihydroxybenzaldehyde with ethyl iodide in DMF using K₂CO₃ as base), sodium cyanoborohydride, *N-*ethyl-diisopropylamine and acetic acid in ethanol at 50°C to yield the title compound as light yellow foam.
MS: 529.2 (M+H)⁺.

### Example 12

### [1-(3-Ethoxy-4-methoxy-benzyl)-piperidin-4-yl]-[7-(pyrrolidine-1-sulfonyl)-benzooxazol-2-yl]-amine

In analogy to the procedure described in example 1A], piperidin-4-yl-[7-(pyrrolidine-1-sulfonyl)-benzooxazol-2-yl]-amine (example 10F]) was reacted with 3-ethoxy-4-methoxy-benzaldehyde, sodium cyanoborohydride, *N-*ethyl-diisopropylamine and acetic acid in ethanol at 50 °C to yield the title compound as light yellow foam.
MS: 515.2 (M+H)⁺.

### Example 13

### [1-(3,5-Diethoxy-4-pyrrol-1-yl-benzyl)-piperidin-4-yl]-[7-(pyrrolidine-1-sulfonyl)-benzooxazol-2-yl]-amine

A] In analogy to the procedure described in example 1A], piperidin-4-yl-[7-(pyrrolidine-1-sulfonyl)-benzooxazol-2-yl]-amine (example 10F]) was reacted with 3,5-diethoxy-4-pyrrol-1-yl-benzaldehyde (example 13C]), sodium cyanoborohydride, *N-*ethyl-diisopropylamine and acetic acid in ethanol at 50 °C to yield the title compound as light yellow solid.
MS: 594.3 (M+H)⁺.
The 3,5-diethoxy-4-pyrrol-1-yl-benzaldehyde used in 13A] was synthesized as follows:

### B] 3.5-Diethoxy-4-pyrrol-1-yl-benzoic acid ethyl ester

To a solution of 4-amino-3,5-diethoxy-benzoic acid ethyl ester (3.0 g, 11.84 mMol, 1.0 eq.; prepared as described in Helv. Chim. Acta 1977, 60, 3025-3034) in heptane (10 mL) and conc. acetic acid (0.2 mL) was added 2,5-dimethoxy-tetrahydro-furan (1.88 g, 14.21 mMol, 1.2 eq.). After heating to reflux for 5 h, a Dean-Stark apparatus was attached and the reaction mixture heated for an additional time period of 5h. Filtration of the crude reaction mixture and crystallisation at 0 °C from heptane provided 2.94 g (82%) of the title compound. ¹H NMR (300 MHz, DMSO): δ 1.15 (t, *J*= 7.0 Hz, 6H), 1.27 (t, *J*= 7.1 Hz, 3H), 3.98 (q, *J*= 7.0 Hz, 4H), 4.28 (q, *J*= 7.1 Hz, 2H), 6.07-6.08 (m, 2H), 6.73-6.74 (m, 2H), 7.22 (s, 2H). ¹³C NMR (75 MHz, DMSO): δ 14.11, 14.35, 61.06, 64.57, 106.87, 107.64, 122.61, 123.33, 129.29, 153.75, 165.06. MS: 303.4 [M+H]⁺.

### C] 3,5-Diethoxy-4-pyrrol-1-yl-benzaldehvde

To a solution of 3,5-diethoxy-4-pyrrol-1-yl-benzoic acid ethyl ester (1.51 g, 4.98 mMol, 1.0 eq.) in toluene (5 mL) was added slowly over a time periode of 15 min under slight cooling to 20°C a solution of diisobutylaluminium hydride (8.9 mL, 12.45 mMol, 2.5 eq.; 20% solution in toluene). After 1 h, the excess hydride was quenched by cautious addition of water (10 mL) and a 28% solution of sodium hydoxide (2 mL). The mixture was stirred for 30 min and the organic phase filtered over Hyflo. The aqueous layer was extracted with toluene (2 x 50 mL), the combined organic phases washed with a sat. solution of sodium chloride (2 x 50 mL) and concentrated by evaporation under reduced pressure to afford 1.30 g (100%) of (3,5-diethoxy-4-pyrrol-1-yl-phenyl)-methanol. The crude alcohol (1.30 g, 4.98 mMol, 1.0 eq.) was dissolved in toluene (20 mL) and MnO₂ (7.79 g, 89.5 mMol, 18.0 eq.) was added. The reaction mixture was heated to reflux for 7 h, after which time the reaction was filtered through Hyflo and concentrated yielding 1.15 g (89% yield) of the title compound. ¹H NMR (300 MHz, DMSO): δ 1.17 (t, *J*= 7.0 Hz, 6H), 4.02 (q, *J*= 7.0 Hz, 4H), 6.08-6.09 (m, 2H), 6.75-6.76 (m, 2H), 7.25 (s, 2H), 9.89 (s, 1H). MS: 260.1 [M+H]⁺.

### Example 14

### [1-(4-Chloro-3-ethoxy-benzyl)-piperidin-4-yl]-[7-(pyrrolidine-1-sulfonyl)-benzooxazol-2-yl]-amine

A] In analogy to the procedure described in example 1A], piperidin-4-yl-[7-(pyrrolidine-1-sulfonyl)-benzooxazol-2-yl]-amine (example 10F]) was reacted with 4-chloro-3-ethoxy-benzaldehyde (example 14B]), sodium cyanoborohydride, *N-*ethyl-diisopropylamine and acetic acid in ethanol at 50 °C to yield the title compound as light yellow solid.
MS: 519.2 (M+H)⁺, 1 Cl.
The 4-chloro-3-ethoxy-benzaldehyde used in 14A] was synthesized as follows:

### B] 4-Chloro-3-ethoxy-benzaldehyde

To a solution of 4-chloro-3-hydroxy-benzoic acid (3.0 g, 17.4 mMol, 1.0 eq.) in DMF (15 mL) was added K₂CO₃ (4.81 g, 34.8 mMol, 2.0 eq.) and ethyl iodide (4.03 mL, 5.97 g, 38.2 mMol, 2.2 eq.). The reaction mixture was stirred for 6 h at RT, diluted with water (20 mL) and extracted with ethyl acetate (3 x 50 mL). The organic phases were dried over Na₂SO₄ and concentrated to afford 3.6 g (91%) of 4-chloro-3-ethoxy-benzoic acid ethyl ester. The crude ester was then dissolved in THF (20 mL) and cooled to -78°C under Ar. A solution of diisobutylaluminium hydride (95 mL, 95.0 mMol, 6.0 eq.; 1 M solution in THF) was slowly added over a time periode of 15 min, the cooling bath removed after completion of addition and the reaction allowed to reach 0°C. After 1 h, the reaction was cooled to -78°C and the excess hydride quenched by cautious addition of a solution of 1 M HCl (10 mL). The mixture was brought to RT, the organic phase separated and the aqueous layer extracted with ethyl acetate (3 x 100 mL). The combined organic phases were dried over Na₂SO₄ and concentrated by evaporation under reduced pressure to afford 2.94 g (100%) of 4-chloro-3-ethoxy-benzyl alcohol. The crude alcohol (2.94 g, 15.75 mMol, 1.0 eq.) was dissolved in dichloromethane (15 mL) and MnO₂ (5.48 g, 63.0 mMol, 4.0 eq.) was added. The reaction mixture was stirred for 16 h, after which time the reaction was filtered through Hyflo and concentrated. The residue was purified by flash column chromatography on silica eluting with heptane/ethyl acetate (4:1) to yield 1.51 g (52% yield) of the title compound. ¹H NMR (300 MHz, CDCl₃): δ 1.51 (t, *J* = 7.1 Hz, 3H ), 4.19 (q, *J*= 7.1 Hz, 2H), 7.37-7.42 (m, 2H), 7.55 (d, *J*= 9.0 Hz, 1H), 9.94 (s, 1H).

### Example 15

### [1-(3-Ethoxy-4-methoxy-benzyl)-piperidin-4-yl]-(4-nitro-benzooxazo1-2-yl)-amine

A] In analogy to the procedure described in example 1A], (4-nitro-benzooxazol-2-yl)-piperidin-4-yl-amine (example 15E]) was reacted with 3-ethoxy-4-methoxybenzaldehyde, sodium cyanoborohydride, *N-*ethyl-diisopropylamine and acetic acid in ethanol at 50 °C to yield the title compound as yellow foam.
MS: 427.2 (M+H)⁺.
The (4-nitro-benzooxazol-2-yl)-piperidin-4-yl-amine used in 15A] was synthesized as follows:

### B] 4-Nitro-benzooxazole-2-thiol

12.1 g (78.5 mMol) of 2-amino-3-nitro-phenol was suspended in 150 mL of MeCl₂ under Ar; 30.2 mL (22.78 g = 2.2 eq.) of *N-*ethyl-diisopropylamine was added at RT, followed by 6.78 ml (10.24 g = 1.1 eq.) of thiophosgene. After 2 h, 15 ml of EtOH was added drop by drop and the reaction mixture was stirred at RT for 16 h. The major part of the solvent was removed by evaporation i.v., the residue formed was dissolved in water and extracted twice with EtOAc; the organic phases were washed with water, dried over MgSO₄, filtered and evaporated i.v. to give 44.6 g (crude product) of the of the title compound as dark oil.
MS: 196.1 (M)⁺.

### C] 2-Methylsulfanyl-4-nitro-benzooxazole

15.4 g (78.5 mMol) of 4-nitro-benzooxazole-2-thiol was dissolved under Ar in 200 mL of DMF; while stirring, 54.8 g (5.0 eq.) of potassium carbonate was added, followed by 17.28 mL (39.39 g = 3.5 eq.) of methyl iodide. After 3 h stirring at RT, the reaction mixture was poured into crashed ice and extracted three times with ether; the organic phases were washed with water, dried over MgSO₄, filtered and evaporated i.v. to yield 11.63 g of the title compound as light brown solid.
MS: 210.1 (M)⁺.

### D] 4-(4-Nitro-benzooxazol-2-ylamino-piperidine-1-carboxylic acid tert-butyl ester

7.84 g (37.3 mMol) of 2-methylsulfanyl-4-nitro-benzooxazole and 7.78 g of 4-amino-piperidine carboxylic acid *tert*-butylester were dissolved under Ar in 97.7 mL (73.78 g (15 eq.) of *N-*ethyl-diisopropylamine and the mixture was stirred at 100 °C for 4.5 h. After cooling down to RT, it was poured into crashed ice and extracted three times with ether; the organic phases were washed with water, dried over MgSO₄, filtered and evaporated i.v. to yield 9.67 g of the title compound as yellow solid.
MS: 363.0 (M+H)⁺.

### E] (4-Nitro-benzooxazol-2-yl)-piperidin-4-yl-amine

In analogy to the procedure described in example 1D], 4-(4-nitro-benzooxazol-2-ylamino)-piperidine-1-carboxylic acid *tert*-butyl ester was reacted with trifluoroacetic acid in dichloromethane to yield the title compound as yellow solid:
MS: 262.8 (M)⁺.

### Example 16

### [1-(3,5-Diethoxy-4-pyrrol-1-yl-ben zyl)-piperidin-4-yl]-(4-nitro-benzooxazol-2-yl)-amine

In analogy to the procedure described in example 1A], (4-nitro-benzooxazol-2-yl)-piperidin-4-yl-amine (example 15E]) was reacted with 3,5-diethoxy-4-pyrrol-1-yl-benzaldehyde (example 13C]), sodium cyanoborohydride, *N-*ethyl-diisopropylamine and acetic acid in ethanol at 50 °C to yield the title compound as yellow solid.
MS: 506.2 (M+H)⁺.

### Example 17

### [1-(3,5-Diethoxy-4-fluoro-benzyl)-piperidin-4-yl]-(4-nitro-benzooxazol-2-yl)-amine

In analogy to the procedure described in example 1A], (4-nitro-benzooxazol-2-yl)-piperidin-4-yl-amine (example 15E]) was reacted with 3,5-diethoxy-4-fluoro-benzaldehyde (example 2H]), sodium cyanoborohydride, *N-*ethyl-diisopropylamine and acetic acid in ethanol at 50°C to yield the title compound as yellow foam.
MS: 459.2 (M+H)⁺.

### Example 18

### N² -[1-(3,5-Diethoxy-4-fluoro-benzyl)-piperidin-4-yl]-benzooxazole-2,4-diamine

In analogy to the procedure described in example 3], [1-(3,5-diethoxy-4-fluorobenzyl)-piperidin-4-yl]-(4-nitro-benzooxazol-2-yl)-amine (example 17) was hydrogenated with platinum on activated carbon to yield the title compound as yellow foam.
MS: 429.3 (M+H)⁺.

### Example 19.

### N-{2-[1-(3,5-Diethoxy-4-fluoro-benzyl)-piperidin-4-ylamino]-benzooxazol-4-yl}-2-methoxy-acetamide

0.215 g (0.5 mMol) of *N²*-[1-(3,5-diethoxy-4-fluoro-benzyl)-piperidin-4-yl]-benzooxazole-2,4-diamine (example 18]) was dissolved in 5 mL of DMF under Ar and the reaction mixture was cooled down to 3°C; 0.006 g (0.1 eq.) of *N*,*N-*dimethyl-4-aminopyridine was added, followed by 0.05 mL (0.062 g = 1.1 eq.) of methoxyacetylchloride. The reaction mixture was warmed up to RT. After 3 h, it was poured into crashed ice and extracted twice with EtOAc; the organic phases were washed with water, dried over MugsO4. filtered and evaporated i.v. The crude product was purified by chromatography (silicagel, eluent: gradient of Mecl₂/ MeOH) to yield 0.162 g of the title compound as light yellow solid.
MS: 501.1 (M+H)⁺.

### Example 20

### N-{2-[1-(3,5-Diethoxy-4-fluoro-benzyl)-piperidin-4-ylamino]-benzooxazol-4-yl}-succinamic acid methyl ester

In analogy to the procedure described in example 19], *N²*-[1-(3,5-diethoxy-4-fluoro-benzyl)-piperidin-4-yl]-benzooxazole-2,4-diamine (example 18]) was reacted with 3-carbomethoxypropionylchloride, *N,N*-dimethyl-4-aminopyridine in *N,N-*dimethylformamide to yield the title compound as yellow solid.
MS: 543.3 (M+H)⁺.

### Example 21

### N- {2-[1-(3,5-Diethoxy-4-fluoro-benzyl)-piperidin-4-ylamino]-benzooxazol-4-yl}-succinamic acid

In analogy to the procedure described in example 5, *N-*{2-[1-(3,5-diethoxy-4-fluoro-benzyl)-piperidin-4-ylamino]-benzooxazol-4-yl}-succinamic acid methyl ester (example 20]) was saponified to yield the title compound as colorless solid.
MS: 527.2 (M-H)-.

### Example 22

### Pyrimidine-5-carboxylic acid {2-[1-(3,5-diethoxy-4-fluoro-benzyl)-piperidin-4-ylamino]-benzooxazol-4-yl}-amide

0.055 g (0.45 mMol) of pyrimidine-5-carboxylic acid and 0.078g (0.45 mMol) of 2-chloro-4,6-dimethoxy-1,3,5-triazine were dissolved under Ar in 5 mL of MeCN at 0 °C; then, 0.10 mL (0.092 g = 2.0 eq.) of N-methylmorpholine was added and the mixture was stirred at 0 °C for 2 h. A solution of 0.191 g (0.45 mMol) *N²*-[1-(3,5-diethoxy-4-fluoro-benzyl)-piperidin-4-yl]-benzooxazole-2,4-diamine (example 18]) in 10 mL of MeCN was added drop by drop and the reaction was then warmed up to RT. After stirring for 16 h, it was poured into crashed ice and extracted twice with MeCl₂; the organic phases were washed with water, dried over MgSO₄, filtered and evaporated i.v. The crude product was purified by HPLC (silicagel, eluent: gradient of n-heptane/ EtOH / 2-propanol / MeCN) to yield 0.11 g of the title compound as light yellow solid.
MS: 535.4 (M+H)⁺.

### Example 23

### N²-[1-(3,5-Diethoxy-4-pyrrol-1-yl-benzyl)-piperidin-4-yl]-benzooxazole-2,4-diarnine

2.54 g (5.0 mmol) of [1-(3,5-diethoxy-4-pyrrol-1-yl-benzyl)-piperidin-4-yl]-(4-nitro-benzooxazol-2-yl)-amine [example 16]) was dissolved in 50mL of MeOH under Ar; then, 1.18 g of platinum on activated carbon was added and the mixture was hydrogenated at 50 °C and 3 bar H₂ for 2 h. It was then filtered with the aid of dicalite; the solvent was evaporated i.v. and the crude product was purified by chromatography (silicagel, eluent: gradient of Mecl₂ / MeOH / NH₄OH conc.) to yield 1.48 g of the title compound as yellow foam.
MS: 476.0 (M+H)⁺.

### Example 24

### N-12-[1-(3,5-Diethoxy-4-pyrrol-1-yl-benzyl)-piperidin-4-ylamino]-benzooxazol-4-yl}-2-methoxy-acetamide

In analogy to the procedure described in example 19], *N*²-[1-(3,5-diethoxy-4-pyrrol-1-yl-benzyl)-piperidin-4-yl]-benzooxazole-2,4-diamine (example 23]) was reacted with methoxyacetylchloride, *N*,*N-*dimethyl-4-aminopyridine in *N,N*-dimethylformamide to yield the title compound as light yellow solid.
MS: 548.3 (M+H)⁺.

### Example 25

### N-{2-[1-(3,5-Diethoxy-4-fluoro-benzyl)-piperidin-4-ylamino]-benzooxazol-4-yl}-2-(1H-tetrazol-5-yl)-acetamide

0.215 g (0.50 mMol) of *N*²-[1-(3,5-diethoxy-4-fluoro-benzyl)-piperidin-4-yl]-benzooxazole-2,4-diamine (example 18]) and 0.067 a (1.05 eq.) of 1H-tetrazole-5-acetic acid were suspended under Ar in 10 mL of MeCl₂, while stirring, 0.22 mL (0.165 g = 2.5 eq.) of *N-*ethyl-diisopropylamine was added drop by drop and the mixture was cooled down to 5 °C; 0.145 g (1.1 eq.) of BOP-Cl [bis(2-oxo-3-oxazolidinyl)phosphinic chloride] was added and the reaction was warmed up to RT. After 16 h, it was poured into crashed ice and extracted twice with MeCl₂ / 2-propanol (4:1); the organic phases were evaporated i.v. The crude product was purified by chromatography (silicagel, eluent: gradient of MeCl₂ / MeOH / NH₄OH conc.) to yield 0.137 g of the title compound as light yellow solid.
MS: 539.3 (M+H)⁺.

### Example 26

### N-{2-[1-(3,5-Diethoxy-4-pyrrol-1-yl-benzyl)-piperidin-4-ylamino]-benzooxazol-4-yl}-succinamic acid methyl ester

In analogy to the procedure described in example 19], *N²*-[1 1-(3,5-diethoxy-4-pyrrol-1-yl-benzyl)-piperidin-4-yl]-benzooxazole-2,4-diamine (example 23]) was reacted with 3-carbomethoxypropionylchloride, *N*,*N-*dimethyl-4-aminopyridine in *N,N-*dimethylformamide to yield the title compound as yellow solid.
MS: 590.4 (M+H)⁺.

### Example 27

### N-{2-[1-(3,5-Diethoxy-4-pyrrol-1-yl-benzyl)-piperidin-4-ylamino]-benzooxazol-4-yl}-succinamic acid

In analogy to the procedure described in example 5, *N-*{2-[1-(3,5-diethoxy-4-pyrro)-1-yl-benzyl)-piperidin-4-ylamino]-benzooxazol-4-yl}-succinamic acid methyl ester (example 26]) was saponified to yield the title compound as light yellow solid.
MS: 576.2 (M-H)⁺.

### Example 28

### [1-(3-Ethoxy-4-methoxy-benzyl)-piperidin-4-yl]-[4-(pyridin-4-ylmethoxy)-benzooxazol-2-yl]-amine

A] In analogy to the procedure described in example 1A], piperidin-4-yl-[4-(pyridin-4-ylmethoxy)-benzooxazol-2-yl]-amine (example 28H]) was reacted with 3-ethoxy-4-methoxy-benzaldehyde, sodium cyanoborohydride, *N-*ethyl-diisopropylantine and acetic acid in ethanol at 50°C to yield the title compound as light yellow oil.
MS: 489.2 (M+H)⁺.
The piperidin-4-yl-[4-(pyridin-4-ylmethoxy)-benzooxazol-2-yl]-amine as used in example 28A] was synthesized as follows:

### B] Benzoic acid 3-hydroxy-2-nitro-phenyl ester

24.0 g (0.155 Mol) of 2,6-dihydroxy nitrobenzene was dissolved in 500 mL of DMF under Ar; then, 58.27 mL (44.0 g = 2.2 eq.) of *N-*ethyl-diisopropylamine was added and the reaction mixture cooled down to - 55°C; a solution of 19.8 mL (23.9 g = 1.1 eq.) of benzoyl chloride in 100 mL DMF was added during 1 h and the mixture then warmed up to RT; after 16 h, it was poured into crashed ice, the pH was adjusted to > 10 with sodium hydroxide solution (2N in water) and it was extracted three times with ether; the organic phases were washed once with water; the combined water phases were acidified with aq. HCl (25%) to pH ~3 and also extracted three times with ether; the organic phases were washed once with water, dried over MgSO₄, filtered and evaporated i.v. ; the crude product was recrystallised from MeCl₂ / n-heptane to give 23.22 g of the title compound as light yellow solid.
MS: 258 (M-H)⁻.

### C] Benzoic acid 3-benzyloxy-2-nitro-phenyl ester

23.3 g (89.9 mMol) of benzoic acid 3-hydroxy-2-nitro-phenyl ester, 9.72 mL (10.21 g = 1.05 eq.) of benzyl alcohol and 28.29 g (1.20 eq.) of triphenylphosphine were dissolved in 400 ml of THF. The stirred reaction mixture was cooled down to 0 °C and a solution of 21.73 g (1.05 eq.) of di-*tert*-butyl azodicarboxylate in 100 ml of THF was added drop by drop. Then, the reaction mixture was warmed up to ambient temperature. After 20 h, the solvent was evaporated and the residue (43.3 g) was purified by chromatography (SiO₂, heptane / AcOEt = 95:5 to 4:1) to yield 34.61 g of the title compound as orange solid.
MS: 367.3 (M+NH₄)⁺.

### D] 3-Ben zyloxy-2-nitro-phenol

24.8 g (71 mMol) of benzoic acid 3-benzyloxy-2-nitro-phenyl ester was dissolved in 300 mL of THF / MeOH (2:1) and the mixture was cooled down to 0 °C; while stirring, 75 mL of a solution of sodium hydroxide in water (2N) was added drop by drop and the reaction was warmed up to RT. After 16 h, it was poured into crashed ice, the pH was adjusted to ~4 with aq. HCl (2N) and it was extracted twice with EtOAc; the organic phases were washed once with water, dried over MugSO₄, filtered and evaporated i.v. ; the crude product (23.57 g) was purified by chromatography (SiO₂, n-heptane / AcOEt = 95:5 to 4: 1) to yield 18.63 g of the title compound as yellow oil.
MS: 244.3 (M-H)-.

### E] 2-Amino-3-benzyloxy-phenol

18.63 g (76 mMol) of 3-benzyloxy-2-nitro-phenol was dissolved in 200 mL of MeOH under Ar; 1.48 g of Pt (0) powder was added and the well stirred reaction mixture was hydrogenated at RT and 1 bar H₂. The reaction mixture was filtered with the aid of dicalite; the solvent was evaporated i.v. to yield 13.32 g of the title compound as light brown solid.
MS: 215.2 (M)⁺.

### F] 4-(4-Hydroxy-benzooxazol-2-ylamino)-piperidine-1-carboxylic acid tell-butyl ester

2.67 g (6.3 mMol) of 4-(4-benzyloxy-benzooxazol-2-ylamino)-piperidine-1-carboxylic acid *tert*-butyl ester (prepared in analogy to the procedures described in example 10E] and example 1B-C], from 2-amino-3-benzyloxy-phenol and potassium ethyl xanthogenate in methanol at reflux to give 4-benzyloxy-benzooxazole-2-thiol, followed by treatment with thionylchloride / DMF at reflux and condensation of the crude 4-benzyloxy-2-chloro-benzooxazole formed with 4-amino-piperidine carboxylic acid *tert*-butyl ester in *N-*ethyl-diisopropylamine at 90 °C) was dissolved in 100 mL of MeCl₂, 11.64 mL (9.89 g = 25 eq.) of dimethyl sulfide were added to the stirred solution, followed by 8.25 mL (9.32 g = 5 eq.) boron trifluoride-ethyietherate. This reaction mixture was stirred over night at RT and then for 5 h at reflux. To the cooled reaction mixture, 50 mL of water was added; it was then stirred vigorously for 30 min, then neutralized with sodium hydrogen carbonat solution. The MeCl₂ was removed by evaporation i.v. 50 mL of dioxane was added to the remaining aqueous solution and followed by 7 g of solid sodium hydrogen carbonate. 7.02 g (5 eq.) of di-ter-butyl bicarbonate was added in small portions to this well stirred solution, which was then stirred for 72 h at RT. The reaction mixture was subsequently poured into crashed ice and extracted twice with EtOAc; the organic phases were washed once with water, dried over MagsO4, filtered and evaporated i.v. ; the crude product was purified by chromatography (SiO₂, MeCl₂ / MeOH) to yield 1.35 g of the title compound as light brown foam.
MS: 334.1 (M+H)⁺.

### G] 4-[4-(Pyridin-4-ylmethoxy)-benzooxazol-2-ylaminol-piperidine-1-carboxylic acid tert-butyl ester

1.01 g (3.0 mMol) of 4-(4-hydroxy-benzooxazol-2-ylamino)-piperidine-1-carboxylic acid *tert*-butyl ester, 0.37 g (1.10eq.) of 4-pyidylcarbinol and 1.04 g (1.30 eq.) of triphenylphosphine were dissolved in 20 mL of THF and the reaction mixture was cooled down to 0 °C; a solution of 0.89 g (1.25 eq.) of di-*tert*-butyl azodicarboxylate in 10 mL) of THF was added drop by drop. Then, the reaction mixture was warmed up to ambient temperature. After 20 h, the solvent was evaporated i.v. and the residue (3.65 g) was purified by chromatography (SiO₂, MeCl₂ / MeOH) to yield 1.05 g of the title compound as off-white solid.
MS: 424.2 (M)⁺.

### H] Piperidin-4-yl-[4-(pyrridin-4-ylmethoxy)-benzooxazol-2-yl]-amine

1.10 g (2.6 mMol) of 4-[4-(pyridin-4-ylmethoxy)-benzooxazol-2-ylamino]-piperidine-1-carboxylic acid *tert*-butyl ester was dissolved in 20 mL of MeOH and 7.77 mL (12 eq.) of HCl solution in MeOH (4 molar) was added; the reaction mixture was stirred for 2 h at 55°C, cooled down to RT and poured into crashed ice, the pH was adjusted to ~9 with aq. NH₄OH (25%) and the mixture was extracted twice with MeCl₂; the organic phases were washed with water, dried over MugSO₄, filtered and evaporated i.v. to yield 0.855 g of the title compound as off-white solid.
MS: 325.1 (M+H)⁺.

### Example 29

### [1-(3,5-Diethoxy-4-pyrrol-1-yl-benzyl)-piperidin-4-yl]-[4-(pyridin-4-ylmethoxy)-benzooxazol-2-yl]-amine

In analogy to the procedure described in example 1A], piperidin-4-yl-[4-(pyridin-4-ylmethoxy)-benzooxazol-2-yl]-amine (example 28H]) was reacted with 3,5-diethoxy-4-pyrrol-1-yl-benzaldehyde (example 13C]), sodium cyanoborohydride, *N-*ethyl-diisopropylamine and acetic acid in ethanol at 50 °C to yield the title compound as yellow oil.
MS: 568.4 (M+H)⁺.

### Example 30

### [1-(3,5-Diethoxy-4-fluoro-benzyl)-piperidin-4-yl]-[4-(pyridin-4-ylmethoxy)-benzooxazol-2-yl]-amine

In analogy to the procedure described in example 1A], piperidin-4-yl-[4-(pyridin-4-ylmethoxy)-benzooxazol-2-yl]-amine (example 28H]) was reacted with 3,5-diethoxy-4-fluoro-benzaldehyde (example 2H]), sodium cyanoborohydride, *N-*ethyl-diisopropylamine and acetic acid in ethanol at 50°C to yield the title compound as amorphous light yellow solid.
MS: 521.4 (M+H)⁺.

### Example 31

### 2-[1-(3-Ethoxy-4-methoxy-benzyl)-piperidin-4-ylamino]-benzooxazole-4-carboxylic acid methyl ester

In analogy to the procedure described in example 1A], 2-(piperidin-4-ylamino)-benzooxazole-4-carboxylic acid methyl ester (prepared by i) treatment of 2-mercapto-benzooxazole-4-carboxylic acid methyl ester [EP 1020 451 A1, publication date July 19, 2000; filing date March 25, 1998] with thionylchloride /DMF at reflux in analogy to the procedure described in example 1B]; ii) by subsequent condensation of the crude 2-chloro-benzooxazole-4-carboxylic acid methyl ester with 4-amino-piperidine carboxylic acid *tert*-butyl ester in *N-*ethyl-diisopropylamine at RT in analogy to the procedure described in example 1C] yielding 2-(1-*tert*-butoxycarbonyl-piperidin-4-ylamino)-benzooxazole-4-carboxylic acid methyl ester; and iii) subsequent Boc cleavage in with trifluoroacetic acid in analogy to the procedure described in example 1D]) was reacted with 3-ethoxy-4-methoxy-benzaldehyde, sodium cyanoborohydride, *N-*ethyl-diisopropylamine and acetic acid in ethanol at 50 °C to yield the title compound as colorless oil.
MS: 440.4 (M+H)⁺.

### Example 32

### 2-[1-(3,5-Diethoxy-4-pyrrol-1-yl-benzyl)-piperidin-4-ylamino]-benzooxazole-4-carboxylic acid methyl ester

In analogy to the procedure described in example 1A], 2-(piperidin-4-ylamino)-benzooxazole-4-carboxylic acid methyl ester (example 31]) was reacted with 3,5-diethoxy-4-pyrrol-1-yl-benzaldehyde (example 13C]), sodium cyanoborohydride, *N-*ethyl-diisopropylamine and acetic acid in ethanol at 50°C to yield the title compound as colorless oil.
MS: 519.5 (M+H)⁺.

### Example 33

### 2-[1-(3,5-Diethoxy-4-fluoro-benzyl)-piperidin-4-ylamino]-benzooxazole-4-carboxylic acid methyl ester

In analogy to the procedure described in example 1A], 2-(piperidin-4-ylamino)-benzooxazole-4-carboxylic acid methyl ester (example 31]) was reacted with 3,5-diethoxy-4-fluoro-benzaldehyde (example 2H]), sodium cyanoborohydride, *N-*ethyl-diisopropylamine and acetic acid in ethanol at 50 °C to yield the title compound as colorless oil.
MS: 472.3 (M+H)⁺.

### Example 34

### 2-[1-(3-Ethoxy-4-methoxy-benzyl)-piperidin-4-ylamino]-benzooxazole-4-carboxylic acid

In analogy to the procedure described in example 5, 2-[1-(3-ethoxy-4-methoxybenzyl)-piperidin-4-ylamino]-benzooxazole-4-carboxylic acid methyl ester (example 31]) was saponified to yield the title compound as colorless solid.
MS:426.1 (M+H)⁺.

### Example 35

### 2-[1-(3,5-Diethoxy-4-pyrrol-1-yl-benzyl)-piperidin-4-ylamino]-benzooxazole-4-carboxylic acid

In analogy to the procedure described in example 5, 2-[1-(3,5-diethoxy-4-pyrrol-1-yl-benzyl)-piperidin-4-ylamino]-benzooxazole-4-carboxylic acid methyl ester (example 32]) was saponified to yield the title compound as colorless oil.
MS: 505.2 (M+H)⁺.

### Example 36

### 2-[1-(3,5-Diethoxy-4-fluoro-benzyl)-piperidin-4-ylamino]-benzooxazole-4-carboxylic acid

In analogy to the procedure described in example 5, 2-[1-(3,5-diethoxy-4-fluorobenzyl)-piperidin-4-ylamino]-benzooxazole-4-carboxylic acid methyl ester (example 33]) was saponified to yield the title compound as colorless solid.
MS: 458.2, (M+H)⁺.

### Example 37

### 2-[1-(3-Ethoxy-4-methoxy-benzyl)-piperidin-4-ylamino]-benzooxazole-4-carboxylic acid pyridin-3-ylamide

A] In analogy to the procedure described in example 1A], 2-(piperidin-4-ylamino)-benzooxazole-4-carboxylic acid pyridin-3-ylamide (example 37B]) was reacted with 3-ethoxy-4-methoxy-benzaldehyde, sodium cyanoborohydride, *N-*ethyl-diisopropylamine and acetic acid in ethanol at 50 °C to yield the title compound as colorless solid.
MS: 502.2 (M+H)⁺.

2-(Piperidin-4-ylamino)-benzooxazole-4-carboxylic acid pyridin-3-ylamide as used in example 37A] was synthesized as follows:

### B] 2-(Piperidin-4-ylamino)-benzooxazole-4-carboxylic acid pyridin-3-ylamide

The title compound has been prepared by the following reaction sequence: i) saponification of 2-(1-*tert*-butoxycarbonyl-piperidin-4-ylamino)-benzooxazole-4-carboxylic acid methyl ester (example 31) in analogy to to the procedure described in example 5; ii) coupling of the thus formed 2-(1-*tert*-butoxycarbonyl-piperidin-4-ylamino)-benzooxazole-4-carboxylic acid with 3-amino-pyridine using 2-chloro-4,6-dimethoxy-1,3,5-triazine, N-methylmorpholine in MeCN at RT in analogy to the procedure described in example 22 to yield 4-[4-(pyridin-3-ylcarbamoyl)-benzooxazol-2-ylamino]-piperidine-1-carboxylic acid *tert*-butyl ester; iii) Boc cleavage in analogy to the procedure described in example 1D].

### Example 38

### 2-[1-(3,5-Diethoxy-4-fluoro-benzyl)-piperidin-4-ylamino]-benzooxazole-4-carboxylic acid pyridin-3-ylamide

In analogy to the procedure described in example 1A], 2-(piperidin-4-ylamino)-benzooxazole-4-carboxylic acid pyridin-3-ylamide (example 37 B]) was reacted with 3,5-diethoxy-4-fluoro-benzaldehyde (example 2H]), sodium cyanoborohydride, *N-*ethyl-diisopropylamine and acetic acid in ethanol at 50 °C to yield the title compound as colorless solid.
MS: 534.4 (M+H)⁺.

### Example 39

### 2-[1-(3,5-Diethoxy-4-pyrrol-1-yl-benzyl)-piperidin-4-ylamino]-benzooxazole-4-carboxylic acid pyridin-3-ylamide

In analogy to the procedure described in example 1A], 2-(piperidin-4-ylamino)-benzooxazole-4-carboxylic acid pyridin-3-ylamide (example 37 B]) was reacted with 3,5-diethoxy-4-pyrrol-1-yl-benzaldehyde (example 13C]), sodium cyanoborohydride, *N-*ethyl-diisopropylamine and acetic acid in ethanol at 50°C to yield the title compound as colorless solid.
MS: 581.3 (M+H)⁺.

### Example 40

### 2-[1-(3-Ethoxy-4-methoxy-benzyl)-piperidin-4-ylamino]-benzooxazole-4-carboxylic acid (2-hydroxy-ethyl)-amide

A] In analogy to the procedure described in example 1A], 2-(piperidin-4-ylamino)-benzooxazole-4-carboxylic acid (2-hydroxy-ethyl)-amide (example 40B]) was reacted with 3-ethoxy-4-methoxy-benzaldehyde, sodium cyanoborohydride, *N-*ethyl-diisopropylamine and acetic acid in ethanol at 50°C to yield the title compound as colorless solid.
MS: 469.2 (M+H)⁺.

2-(Piperidin-4-ylamino)-benzooxazole-4-carboxylic acid (2-hydroxy-ethyl)-amide as used in example 40A] was synthesized as follows:

### B] 2-(Piveridin-4-ylamino)-benzooxazole-4-carboxylic acid (2-hydroxy-ethyl)-amide

The title compound has been prepared by the following reaction sequence: i) saponification of 2-( 1-*tert*-butoxycarbonyl-piperidin-4-ylamino)-benzooxazole-4-carboxylic acid methyl ester (example 31) in analogy to to the procedure described in example 5; ii) coupling of the thus formed 2-(1-*tert*-butoxycabonyl-piperidin-4-ylamino)-benzooxazole-4-carboxylic acid with 2-amino-ethanol using 2-chloro-4,6-dimethoxy-1,3,5-triazine, N-methylmorpholine in MeCN at RT in analogy to the procedure described in example 22 to yield 4-[4-(2-hydroxy-ethylcarbamoyl)-benzooxazol-2-ylamino]-piperidine-1-carboxylic acid *tert*-butyl ester; iii) Boc cleavage in analogy to the procedure described in example 1D].

### Example 41

### 2-[1-(3,5-Diethoxy-4-fluoro-benzyl)-piperidin-4-ylamino]-benzooxazole-4-carboxylic acid (2-hydroxy-ethyl)-amide

In analogy to the procedure described in example 1A], 2-(piperidin-4-ylamino)-benzooxazole-4-carboxylic acid (2-hydroxy-ethyl)-amide (example 40B]) was reacted with 3,5-diethoxy-4-fluoro-benzaldehyde (example 2H]), sodium cyanoborohydride, *N-*ethyl-diisopropylamine and acetic acid in ethanol at 50 °C to yield the title compound as colorless foam.
MS: 501.2 (M+H)⁺.

### Example 42

### 2-[1-(3,5-Diethoxy-4-pyrrol-1-yl-benzyl)-piperidin-4-ylamino]-benzooxazole-4-carboxylic acid (2-hydroxy-ethyl)-amide

In analogy to the procedure described in example 1A], 2-(piperidin-4-ylamino)-benzooxazole-4-carboxylic acid (2-hydroxy-ethyl)-amide (example 408]) was reacted with 3,5-diethoxy-4-pyrrol-1-yl-benzaldehyde (example 13C]), sodium cyanoborohydride, *N-*ethyl-diisopropylamine and acetic acid in ethanol at 50°C to yield the title compound as colorless solid.
MS: 548.4 (M+H)⁺.

### Example 43

### ({2-[1-(3-Ethoxy-4-methoxy-benzyl)-piperidin-4-ylamino]-benzooxazole-4-cwbonyl}-amino)-acetic acid methyl ester

A] In analogy to the procedure described in example 1A], ([2-(piperidin-4-ylamino)-benzooxazole-4-carbonyl]-amino}-acetic acid methyl ester (example 43B]) was reacted with 3-ethoxy-4-methoxy-benzaldehyde, sodium cyanoborohydride, *N-*ethyl-diisopropylamine and acetic acid in ethanol at 50 °C to yield the title compound as colorless solid.
MS: 497.1 (M+H)⁺.
The {[2-(piperidin-4-ylamino)-benzooxazole-4-carbonyl]-amino}-acetic acid methyl ester used in example 43A] was synthesized as follows:

### B] {[2-(Piperidin-4-ylamino)-benzooxazole-4-carbonyll-amino}-acetic acid methyl ester

The title compound has been prepared by the following reaction sequence: i) saponification of 2-(1-*tert*-butoxycarbonyl-piperidin-4-ylamino)-benzooxazole-4-carboxylic acid methyl ester (example 31) in analogy to to the procedure described in example 5; ii) coupling of the thus formed 2-(1-*tert*-butoxycarbonyl-piperidin-4-ylamino)-benzooxazole-4-carboxylic acid with glycine methyl ester hydrochloride using 2-chloro-4,6-dimethoxy-1,3,5-triazine, N-methylmorpholine in MeCN at RT in analogy to the procedure described in example 22 to yield 4-[4-(methoxycarbonylmethyl-carbamoyl)-benzooxazol-2-ylamino]-piperidine-1-carboxylic acid *tert*-butyl ester; iii) Boc cleavage in analogy to the procedure described in example 1D].

### Example 44

### ({2-[1-(3,5-Diethoxy-4-fluoro-benzyl)-piperidin-4-ylamino]-benzooxazole-4-carbonyl}-amino)-acetic acid methyl ester

In analogy to the procedure described in example 1A], {[2-(piperidin-4-ylamino)-benzooxazole-4-carbonyl]-amino]-acetic acid methyl ester (example 43B]) was reacted with 3,5-diethoxy-4-fluoro-benzaldehyde (example 2H]), sodium cyanoborohydride, *N-*ethyl-diisopropylamine and acetic acid in ethanol at 50 °C to yield the title compound as colorless solid.
MS: 529.3 (M+H)⁺.

### Example 45

### ({2-[1-(3,5-Diethoxy-4-pyrrol-1-yl-benzyl)-piperidin-4-ylamino]-benzooxazole-4-carbonyl}-amino)-acetic acid methyl ester

In analogy to the procedure described in example 1A], {[2-(piperidin-4-ylamino)-benzooxazole-4-carbonyl]-amino}-acetic acid methyl ester (example 43B]) was reacted with 3,5-diethoxy-4-pyrrol-1-yl-benzaldehyde (example 13C]), sodium cyanoborohydride, *N-*ethyl-diisopropylamine and acetic acid in ethanol at 50°C to yield the title compound as light yellow solid.
MS: 576.2 (M+H)⁺.

### Example 46

### ({2-[1-(3-Ethoxy-4-methoxy-benzyl)-piperidin-4-ylamino]-benzooxazole-4-carbonyl}-amino)-acetic acid

In analogy to the procedure described in example 5, ({2-[1-(3-ethoxy-4-methoxybenzyl)-piperidin-4-ylamino]-benzooxazole-4-carbonyl}-amino)-acetic acid methyl ester (example 43]) was saponified to yield the title compound as colorless solid.
MS: 483.3 (M+H)⁺.

### Example 47

### ({2-[1-(3,5-Diethoxy-4-fluoro-benzyl)-piperidin-4-ylamino]-benzooxazole-4-carbonyl}-amino)-acetic acid

In analogy to the procedure described in example 5, ({2-[1-(3,5-diethoxy-4-fluorobenzyl)-piperidin-4-ylamino]-benzooxazole-4-carbonyl}-amino)-acetic acid methyl ester (example 44]) was saponified to yield the title compound as colorless solid.
MS:513.3(M-H)⁻.

### Example 48

### ({2-[1-(3,5-Diethoxy-4-pyrrol-1-yl-ben zyl)-piperidin-4-ylamino]-benzooxazole-4-carbonyl}-amino)-acetic acid

In analogy to the procedure described in example 5, ({2-[1-(3,5-diethoxy-4-pyrrol-l-yl-benzyl)-piperidin-4-ylamino]-benzooxazole-4-carbonyl}-amino)-acetic acid methyl ester (example 45]) was saponified to yield the title compound as colorless solid.
MS: 562.5 (M+H)⁺.

### Example 49

### [1-(3-Ethoxy-4-methoxy-benzyl)-piperidin-4-yl]-(4-iodo-benzooxazol-2-yl)-amine

A] In analogy to the procedure described in example 1A], (4-iodo-benzooxazol-2-yl)-piperidin-4-yl-amine (example 49B]) was reacted with 3-ethoxy-4-methoxybenzaldehyde, sodium cyanoborohydride, *N-*ethyl-diisopropylamine and acetic acid in ethanol at 50°C to yield the title compound as off-withe foam.
MS:508.1(M+H)⁺.
The (4-iodo-benzooxazol-2-yl)-piperidin-4-yl-amine used in example 49A] was synthesized as follows:

### B] (4-Iodo-benzooxazol-2-yl)-piperidin-4-yl-armine

The title compound has been prepared by the following reaction sequence: i) treatment of 2-amino-3-iodo-phenol with potassium ethyl xanthogenate in MeOH at reflux in analogy to the procedure described in example 10E]; ii) reaction of the thus formed 4-iodo-benzooxazole-2-thiol with thionylchloride and DMF at reflux in analogy to the procedure described in example 1B] to yield 2-chloro-4-iodo-benzooxazole; iii) condensation with 4-amino-piperidine-carboxylic acid *tert*-butyl ester in *N-*ethyl-diisopropylamine at reflux in analogy to the procedure described in example 1C] to yield 4-(4-iodo-benzooxazol-2-ylamino)-piperidine-1-carboxylic acid *tert*-butyl ester; iv) Boc cleavage in analogy to the procedure described in example 1D].

### Example 50

### [1-(3,5-Diethoxy-4-fluoro-benzyl)-piperidin-4-yl]-(4-iodo-benzooxazol-2-yl)-amine

In analogy to the procedure described in example 1A], (4-iodo-benzooxazol-2-yl)-piperidin-4-yl-amine (example 49B]) was reacted with 3,5-diethoxy-4-fluorobenzaldehyde (example 2H]), sodium cyanoborohydride, *N-*ethyl-diisopropylamine and acetic acid in ethanol at 50 °C to yield the title compound as off-withe solid.
MS: 540.2 (M+H)⁺.

### Example 51

### [1-(3,5-Diethoxy-4-pyrrol-1-yl-benzyl)-piperidin-4-yl]-(4-iodo-benzooxazol-2-yl)-amine

In analogy to the procedure described in example 1A], (4-iodo-benzooxazol-2-yl)-piperidin-4-yl-amine (example 49B]) was reacted with 3.5-diethoxy-4-pyrrol-1-yl-benzaldehyde (example 13C]), sodium cyanoborohydride, *N-*ethyl-diisopropylamine and acetic acid in ethanol at 50 °C to yield the title compound as off-white foam.
MS: 587.3 (M+H)⁺.

### Example 52

### (7-Bromo-benzooxazol-2-yl)-[1-(3-ethoxy-4-methoxy-benzyl)-piperidin-4-yl]-amine

A] In analogy to the procedure described in example 1A], (7-bromo-benzooxazol-2-yl)-piperidin-4-yl-amine (example 52B]) was reacted with 3-ethoxy-4-methoxybenzaldehyde, sodium cyanoborohydride, *N-*ethyl-diisopropylamine and acetic acid in ethanol at 50°C to yield the title compound as light brown solid.
MS: 460.2 (M+H)⁺, 1Br.
The (7-bromo-benzooxazol-2-yl)-piperidin-4-yl-amine used in example 52A] was synthesized as follows:

### B] (7-Bromo-benzooxazol-2-yl)-piperidin-4-yl-amine

The title compound has been prepared by the following reaction sequence: i) treatment of 2-amino-6-bromo-phenol [Acta Ciencia Indica (1978), 4(1), 24-6] with potassium ethyl xanthogenate in MeOH at reflux in analogy to the procedure described in example 10E]; ii) reaction of the thus formed 7-brorno-benzooxazdle-2-thiol with thionylchloride and DMF at reflux in analogy to the procedure described in example 1B] to yield 7-bromo-2-chloro-benzooxazole; iii) condensation with 4-amino-piperidine-carboxylic acid *tert*-butyl ester in *N*-ethyl-diisopropylamine at RT followed by reflux in analogy to the procedure described in in example 1C] yielding 4-(7-bromo-benzooxazol-2-ylamino)-piperidine-1-carboxylic acid *tert*-butyl ester; iv) Boc cleavage in analogy to the procedure described in example 1D].

### Example 53

### (7-Bromo-benzooxazol-2-yl)-[1-(3,5-diethoxy-4-fluoro-benzyl)-piperidin-4-yl]-amine

In analogy to the procedure described in example 1A], (7-bromo-benzooxazol-2-yl)-piperidin-4-yl-amine (example 52B]) was reacted with 3,5-diethoxy-4-fluorobenzaldehyde (example 2H]), sodium cyanoborohydride, *N*-ethyl-diisopropylamine and acetic acid in ethanol at 50°C to yield the title compound as brown foam.
MS: 492.1 (M+H)⁺, 1Br.

### Example 54

### (7-Bromo-benzooxazol-2-yl)-[1-(3,5-diethoxy-4-pyrrol-1-yl-benzyl)-piperidin-4-yl]-amine

In analogy to the procedure described in example 1A], (7-bromo-benzooxazol-2-yl)-piperidin-4-yl-amine (example 52B]) was reacted with 3,5-diethoxy-4-pyrrol-1-yl-benzaldehyde (example 13C]), sodium cyanoborohydride, *N-*ethyl-diisopropylamine and acetic acid in ethanol at 50°C to yield the title compound as light brown foam.
MS: 539.3 (M+H)⁺, 1Br.

### Example 55

### 2-[1-(3-Ethoxy-4-hydroxy-benzyl)-piperidin-4-ylamino]-benzooxazole-6-carboxylic acid methyl ester

In analogy to the procedure described in example 1A], 2-(piperidin-4-ylamino)-benzooxazole-6-carboxylic acid methyl ester (prepared by i) treatment of 2-mercapto-benzooxazole-6-carboxylic acid methyl ester [PCT Int. Appl. WO 94 /06783 A1] with thionylchloride /DMF at reflux in analogy to the procedure described in example 1B]; ii) by subsequent condensation of the crude 2-chloro-benzooxazole-6-carboxylic acid methyl ester with 4-amino-piperidine carboxylic acid *tert*-butyl ester in *N,N-*dimethylformamide at 80°C in analogy to the procedure described in example 1C]: and iii) subsequent Boc cleavage in with trifluoroacetic acid in analogy to the procedure described in example 1D]) was reacted with 3-ethoxy-4-hydroxy-benzaldehyde, sodium cyanoborohydride, *N*-ethyl-diisopropylamine and acetic acid in ethanol at 50 °C to yield the title compound as light yellow amorphous solid.
MS: 426.3 (M+H)⁺.

### Example 56

### 2-[1-(3-Ethoxy-4-methoxy-benzyl)-piperidin-4-ylamino]-benzooxazole-6-carboxylic acid methyl ester

In analogy to the procedure described in example 1A], 2-(piperidin-4-ylamino)-benzooxazole-6-carboxylic acid methyl ester (example 55) was reacted with 3-ethoxy-4-methoxy-benzaldehyde, sodium cyanoborohydride, *N*-ethyl-diisopropylamine and acetic acid in ethanol at 50 °C to yield the title compound as colorless solid.
MS: 440.4 (M+H)⁺.

### Example 57

### 2-[1-(8-Ethoxy-2,2-dimethyl-2H-chromen-6-ylmethyl)-piperidin-4-ylamino]-benzooxazole-6-carboxylic acid methyl ester

In analogy to the procedure described in example 1A], 2-(piperidin-4-ylamino)-benzooxazole-6-carboxylic acid methyl ester (example 55) was reacted with 8-ethoxy-2,2-dimethyl-2*H*-chromene-6-carbaldehyde [WO 01/ 083 476 A1 (Hoffmann-La Roche AG)], sodium cyanoborohydride, *N*-ethyl-diisopropylamine and acetic acid in ethanol at 50°C to yield the title compound as light yellow oil.
MS: 492.5 (M+H)⁺.

### Example 58

### 2-[1-(3-Isobutoxy-4-methoxy-benzyl)-piperidin-4-ylamino]-benzooxazole-6-carboxylic acid methyl ester

In analogy to the procedure described in example 1A], 2-(piperidin-4-ylamino)-benzooxazole-6-carboxylic acid methyl ester (example 55) was reacted with 3-isobutoxy-4-methoxy-benzaldehyde [prepared by reaction of isovanillin with 1-bromo-2-methyl propane as described in WO 04/000 806 A1 (Elbion AG)], sodium cyanoborohydride, *N-*ethyl-diisopropylamine and acetic acid in ethanol at 50°C to yield the title compound as light yellow solid.
MS: 468.2 (M+H)⁺.

### Example 59

### 2-{1-[3-(2-Fluoro-ethoxy)-4-methoxy-benzyl]-piperidin-4-ylamino}-benzooxazole-6-carboxylic acid methyl ester

A] In analogy to the procedure described in example 1A], 2-(piperidin-4-ylamino)-benzooxazole-6-carboxylic acid methyl ester (example 55) was reacted with 3-(2-fluoro-ethoxy)-4-methoxy-benzaldehyde (example 59B]), sodium cyanoborohydride, *N*-ethyl-diisopropylamine and acetic acid in ethanol at 50 °C to yield the title compound as off-white solid. MS: 458.3 (M+H)⁺.
The 3-(2-fluoro-ethoxy)-4-methoxy-benzaldehyde used in 59A] was synthesized as follows:

### B] 3-(2-Fluoro-ethoxy)-4-methoxy-benzaldehyde

To a solution of 3-hydroxy-4-methoxy-benzaldehyde (10.0 g, 66.0 mMol, 1.0 eq.) in anhydrous DMF (40 mL) was added potassium carbonate (13.6 g, 99.0 mmol, 1.5 eq.) and 1-bromo-2-fluoro-ethane (9.2 g, 72.0 mMol, 1.1 eq.) and the mixture stirred at RT for 48 h. Potassium carbonate was removed by filtration and the organic phase concentrated under reduced pressure. To the crude reaction mixture was added a conc. solution of sodium chloride (100 mL) and the solution extracted with ethyl acetate (3 x 100 mL). The combined organic phases were dried over MgSO₄ and the product crystallized from a mixture of isopropanol/diethyl ether to yield 12.69 g (97%) of the title compound. ¹H NMR (300 MHz, DMSO): δ 3.89 (1H), 4.24-4.27 (m, 1H). 4.34-4.37 (m, 1H), 4.67-4.70 (m, 1H), 4.83-4.86 (m, 1H), 7.20 (d, *J*= 8.4 Hz, 1H), 7.43 (d, *J*= 1.9 Hz, 1H), 7.59 (dd, *J*= 8.4 Hz, *J*= 1.9 Hz, 1H), 9.84 (s, 1H). MS: 198.6 [M+H]⁺.

### Example 60

### 2-[1-(3-Ethoxy-4-methyl-benzyl)-piperidin-4-ylamino]-benzooxazole-6-carboxylic acid methyl ester

In analogy to the procedure described in example 1A], 2-(piperidin-4-ylamino)-benzooxazole-6-carboxylic acid methyl ester (example 55) was reacted with 3-ethoxy-4-methyl-benzaldehyde (prepared in analogy to the procedure described in example 2F], by reaction of 3-hydroxy-4-methyl-benzaldehyde with ethyl iodide in DMF using K₁CO₃ as base), sodium cyanoborohydride, *N*-ethyl-diisopropylamine and acetic acid in ethanol at 50°C to yield the title compound as off white solid. MS: 424.3 (M+H)⁺.

### Example 61

### 2-[1-(4-Chloro-3-ethoxy-benzyl)-piperidin-4-ylamino]-benzooxazole-6-carboxylic acid methyl ester

In analogy to the procedure described in example 1A], 2-(piperidin-4-ylamino)-benzooxazole-6-carboxylic acid methyl ester (example 55) was reacted with 4-chloro-3-ethoxy-benzaldehyde (example 14B]), sodium cyanoborohydride, *N*-ethyl-diisopropylamine and acetic acid in ethanol at 50 °C to yield the title compound as off-white solid. MS: 444.4 (M+H)⁺, 1Cl.

### Example 62

### 2-[1-(4-Amino-3,5-diethoxy-benzyl)-piperidin-4-ylamino]-benzooxazole-6-carboxylic acid methyl ester

A] In analogy to the procedure described in example 1A], 2-(piperidin-4-ylamino)-benzooxazole-6-carboxylic acid methyl ester (example 55) was reacted with 4-amine-3,5-diethoxy-benzaldehyde (example 62C]), sodium cyanoborohydride, *N*-ethyl-diisopropylamine and acetic acid in ethanol at 50°C to yield the title compound as light brown solid. MS: 469.4 (M+H)⁺.
The 4-amino-3,5-diethoxy-benzaldehyde used in 62A] was synthesized as follows:

### B] (4-Amino-3,5-diethoxy-phenyl)-methanol

To a solution of 4-amino-3,5-diethoxy-benzoic acid ethyl ester (2.8 g, 11.05 mMol, 1.0 eq.; prepared as described. in Help. Chim. Acta 1977, 60, 3025-3034) in dichloromethane (50 mL) at 0 °C under Ar was slowly added diisobutylaluminium hydride (27.6 mL, 27.64 mmol, 2.5 eq., 1 M solution in dichloromethane) over a time periode of 15 min and the cooling bath removed on completion of addition. After 18 h, the excess hydride was quenched by cautious addition of a sat solution of potassium sodium tartrate (10 mL). The solidified mixture was extracted with dichloromethane (5 x 200 mL) and THF (2 x 150 mL), the combined organic phases washed with water (3 x 100 mL), dried over MgSO₄, concentrated by evaporation under reduced pressure and the crude material purified by column chromatography on silica eluting with a gradient of heptane/ethyl acetate (4:1 - 1:1) providing 1.10 g (47%) of the title compound. ¹H NMR (300 MHz, CDCl₃): δ 1.42 (t, *J*= 7.0 Hz, 3H), 3.82 (br s, 2H), 4.05 (q, *J*= 7.0 Hz, 2H), 4.54 (s, 2H), 6.50 (s, 2H). ¹³C NMR (75 MHz, CDCl₃): δ 15.03, 64.21, 66.00, 104.51, 125.44, 129.89, 146.71. MS: 211.9 [M+H]⁺.

### C] 4-Amino-3,5-diethoxy-benzaldehyde

To a solution of (4-amino-3,5-diethoxy-phenyl)-methanol (0.79 g, 3.74 mmol, 1.0 eq.) in DMF (20 my) was added MnO₂ (1.63 g , 18.70 mMol, 5.0 eq.). The reaction mixture was stirred for 24 h at RT, filtered through Hyflo, the filtrate extracted with ethyl acetate (3 x 50 mL) and the combined organic phases dried over MgSO₄ providing 0.69 g (88%) of the title compound. ¹H NMR (300 MHz, DMSO): δ 1.46 (t, *J*= 7.0 Hz, 3H), 4.15 (q, *J*= 7.0 Hz, 2H), 4.50 (br s, 2H), 7.04 (s, 2H), 9.70 (s, 1H). MS: 210.0 [M+H]⁺.

### Example 63

### 2-[1-(3,5-Diethoxy-4-fluoro-benzyl)-piperidin-4-ylamino]-benzooxazole-6-carboxylic acid methyl ester

In analogy to the procedure described in example 1A], 2-(piperidin-4-ylamino)-benzooxazole-6-carboxylic acid methyl ester (example 55) was reacted with 3,5-diethoxy-4-fluoro-benzaldehyde (example 2H]), sodium cyanoborohydride, *N*-ethyl-diisopropylamine and acetic acid in ethanol at 50 °C to yield the title compound as light yellow solid.
MS: 472.3 (M+H)⁺.

### Example 64

### 2-[1-(3-Ethoxy-4-hydroxy-benzyl)-piperidin-4-ylamino]-benzooxazole-6-carboxylic acid

In analogy to the procedure described in example 5,2-[1-(3-ethoxy-4-hydroxybenzyl)-piperidin-4-ylamino]-benzooxazole-6-carboxylic acid methyl ester (example 55) was saponified to yield the title compound as off-white solid.
MS: 412.4 (M+H)⁺.

### Example 65

### 2-[1-(3-Ethoxy-4-methoxy-benzyl)-piperidin-4-ylamino]-benzooxazole-6-carboxylic acid

In analogy to the procedure described in example 5,2-[1-(3-ethoxy-4-methoxybenzyl)-piperidin-4-ylamino]-benzooxazole-6-carboxylic acid methyl ester (example 56) was saponified to yield the title compound as colorless solid.
MS: 426.3 (M+H)⁺.

### Example 66

### 2-[1-(8-Ethoxy-2,2-dimethyl-2H-chromen-6-ylmethyl)-piperidin-4-ylamino]-benzooxazole-6-carboxylic acid

In analogy to the procedure described in example 5,2-[1-(8-ethoxy-2,2-dimethyl-2*H*-chromen-6-ylmethyl)-piperidin-4-ylamino]-benzooxazole-6-carboxylic acid methyl ester (example 57) was saponified to yield the title compound as colorless solid.
MS: 478.2 (M+H)⁺.

### Example 67

### 2-[1-(3-Isobutoxy-4-methoxy-benzyl)-piperidin-4-ylamino]-benzooxazole-6-carboxylic acid

In analogy to the procedure described in example 5,2-[1-(3-isobutoxy-4-methoxybenzyl)-piperidin-4-ylamino]-benzooxazole-6-carboxylic acid methyl ester (example 58) was saponified to yield the title compound as colorless solid.
MS: 454.3 (M+H)⁺.

### Example 68

### 2-{1-[3-(2-Fluoro-ethoxy)-4-methoxy-benzyl]-piperidin-4-ylamino}-benzooxazole-6-carboxylic acid

In analogy to the procedure described in example 5, 2-{1-[3-(2-fluoro-ethoxy)-4-methoxy-benzyl]-piperidin-4-ylamino}-benzooxazole-6-carboxylic acid methyl ester (example 59) was saponified to yield the title compound as colorless solid.
MS: 444.3 (M+H)⁺.

### Example 69

### 2-[-(3-Ethoxy-4-methyl-benzyl)-piperidin-4-ylamino]-benzooxazole-6-carboxylic acid

In analogy to the procedure described in example 5, 2-[1-(3-ethoxy-4-methylbenzyl)-piperidin-4-ylamino]-benzooxazole-6-carboxylic acid methyl ester (example 60) was saponified to yield the title compound as colorless solid.
MS: 410.4 (M+H)⁺.

### Example 70

### 2-[1-(4-Chloro-3-ethoxy-benzyl)-piperidin-4-ylamino]-benzooxazole-6-carboxylic acid

In analogy to the procedure described in example 5, 2-[1-(4-chloro-3-ethoxy-benzyl)-piperidin-4-ylamino]-benzooxazole-6-carboxylic acid methyl ester (example 61) was saponified to yield the title compound as colorless solid.
MS: 430.3 (M+H)⁺, 1Cl.

### Example 71

### 2-[1-(4-Amino-3,5-diethoxy-ben zyl)-piperidin-4-ylamino]-benzooxazole-6-carboxylic acid

In analogy to the procedure described in example 5, 2-[1-(4-amino-3,5-diethoxy-benzyl)-piperidin-4-ylamino]-benzooxazole-6-carboxylic acid methyl ester (example 62) was saponified to yield the title compound as colorless solid.
MS: 455.3 (M+H)⁺.

### Example 72

### 1-(3,5-Diethoxy-4-fluoro-benzyl)-piperidin-4-ylamino]-benzooxazole-6-carboxylic acid

In analogy to the procedure described in example 5, 2-[1-(3,5-diethoxy-4-fluorobenzyl)-piperidin-4-ylamino]-benzooxazole-6-carboxylic acid methyl ester (example 63) was saponified to yield the title compound as colorless solid.
MS: 458.3 (M+H)⁺.

### Example 73

### [1-(3-Ethoxy-4-fluoro-benzyl)-piperidin-4-yl]-(4-nitro-benzooxazol-2-yl)-amine

In analogy to the procedure described in example 1A], (4-nitro-benzooxazol-2-yl)-piperidin-4-yl-amine (example 15E]) was reacted with 3-ethoxy-4-fluoro-benzaldehyde (prepared from 3-hydroxy-4-fluoro-benzoic acid in analogy to the procedure described for the synthesis of 4-chloro-3-ethoxy-benzaldehyde in example 14B]), sodium cyanoborohydride, *N*-ethyl-diisopropylamine and acetic acid in ethanol at 50°C to yield the title compound as yellow foam.
MS: 415.4 (M+H)⁺.

### Example 74

### [1-(4-Chloro-3-ethoxy-benzyl)-piperidin-4-yl]-(4-nitro-benzooxazol-2-yl)-amine

In analogy to the procedure described in example 1A], (4-nitro-benzooxazol-2-yl)-piperidin-4-yl-amine (example 15E]) was reacted with 4-chloro-3-ethoxy-benzaldehyde (example 14B]), sodium cyanoborohydride, *N*-ethyl-diisopropylamine and acetic acid in ethanol at 50°C to yield the title compound as yellow foam.
MS: 431.4 (M+H)⁺,1Cl.

### Example 75

### [1-(3-Ethoxy-4-methyl-benzyl)-piperidin-4-yl]-(4-nitro-benzooxazol-2-yl)-amine

In analogy to the procedure described in example 1A], (4-nitro-benzooxazol-2-yl)-piperidin-4-yl-amine (example 15E]) was reacted with 3-ethoxy-4-methyl-benzaldehyde (prepared in analogy to the procedure described in example 2F], by reaction of 3-hydroxy-4-methyl-benzaldehyde with ethyl iodide in DMF using K₂C₃ as base), sodium cyanoborohydride, *N*-ethyl-diisopropylamine and acetic acid in ethanol at 50 °C to yield the title compound as yellow foam,
MS: 411.5 (M+H)⁺.

### Example 76

### 2-[1-(3,5-Diethoxy-4-fluoro-benzyl)-piperidin-4-ylamino]-benzooxazole-7-sulfonic acid amide

A] In analogy to the procedure described in example 1A], 2-(piperidin-4-ylamino)-benzooxazole-7-sulfonic acid amide (example 76H]) was reacted with 3,5-diethoxy-4-fluoro-benzaldehyde (example 2H]), sodium cyanoborohydride, *N-*ethyl-diisopropylamine and acetic acid in ethanol at 50 °C to yield the title compound as colorless solid.
MS: 493.2 (M+H)⁺.
2-(Piperidin-4-ylamino)-benzooxazole-7-sulfonic acid amide as used in example 76A] was synthesized as follows:

### B1 5-Bromo-2-hydroxy-benzenesulfonyl chloride

15.00 g(86.7 mMol) of 4-bromophenol was added at 0 °C in small portions to 41 mL (71.4 g =7.0 eq.) of chlorosulfonic acid; this mixture was then stirred at RT for 16 h. It was subsequently added drop by drop to 200 mL of water between 15 °C and 20 °C.
The reaction mixture was then extracted three times with EtOAc; the organic phases were washed three times with water, dried over MgSO₄, filtered and evaporated i.v. ; the crude product was purified by chromatography (SiO₂, n-heptane / EtOAc) to yield 9.34 g of the title compound as light brown oil.
MS: 269.9 (M), 1Br, 1Cl.

### C] 5-Bromo-2-hydroxy-3-nitro-benzenesulfonyl chloride

9.34 g (34.4 mMol) of 5-bromo-2-hydroxy-benzenesulfonyl chloride was dissolved in 65 mL of MeCl₂ and the mixture was cooled down to 5 °C; while stirring, a mixture of 2.70 mL (3.75 g =59.6 mMol) of nitric acid and 2.70 mL (4.94 g = 50.4 mMol) of sulfuric acid was added drop by drop. The reaction mixture was warmed up to RT and after 2 h, poured into crashed ice and extracted twice with MeCl₂; the organic phases were washed once with water, dried over MgSO_{4,} filtered and evaporated i.v. ; the crude product was purified by chromatography (SiO₂, n-heptane / EtOAc) to yield 5.00 g of the title compound as yellow oil.
MS: 314.9 (M), 1Br, 1Cl.

### D] 5-Bromo-2-hydroxy-3-nitro-benzenesulfonamide

5.00 g (15.8 mMol) of 5-bromo-2-hydroxy-3-nitro-benzenesulfonyl chloride was dissolved in 20 mL of THF and this solution was added drop by drop to a solution of 3.65 mL (3.32 = 1.5 eq.) of ammonium hydroxide (25% in water) and 3.30 mL (2.40 g = 1.5 eq.) of triethylamine in 80-mL of THF at 0 °C. The reaction mixture was warmed up to RT. After 16 h, it was poured into crashed ice, acidified with aq. HCl (1N) and extracted twice with MeCl₂; the organic phases were washed with water, dried over MgSO4, filtered and evaporated i.V. The crude product was purified by recrystallisation from EtOAc / n-heptane to yield 3.70 g of the title compound as yellow solid.
MS: 295.0 (M-H)-, 1Br.

### E] 3-Amino-2-hydroxy-benzenesulfonamide

3.70 g (12.5 mMol) of 5-bromo-2-hydroxy-3-nitro-benzenesulfonamide was dissolved under Ar in 230 mL of MeOH; 0.74 g of Pd on activated carbon (10%) was added and the reaction mixture then hydrogenated at RT and 1 bar H₂. After 4 h, it was filtered with the aid of dicalite; then the solvent was evaporated i.v. to yield 2.40 g of the title compound as light brown solid.
MS: 187.0 (M-H)⁻.

### F] 2-Chloro-benzooxazole-7-sulfonic acid amide

1.90 g (8.3 mMol) of 2-mercapto-benzooxazole-7-sulfonic acid amide (prepared from 3-amino-2-hydroxy-benzenesulfonamide (example 76E]) and potassium ethyl xanthogenate in analogy to example 10]) were reacted with 8.40 mL (13.74 g = 14 eq.) of thionyl chloride in presence of 0.04 mL of DMF for 9 h at RT. The excess of thionyl chloride was removed by azeotropic inert gas distillation with toluene i.v. to yield 2.21 g of the title compound as light brown solid, which was used in the subsequent reaction step without further purification.
MS: 231.1 (M-H)-, 1Cl.

### G] 4-(7-Sulfamoyl-benzooxazol-2-ylamino)-piperidine-1-carboxylic acid tert-butyl ester

1.91 g (8.2 mMol) of 2-chloro-benzooxazole-7-sulfonic acid amide was suspended in 40 mL of MeCN under Ar at RT; 19.68 mL (14.85 g = 14 eq.) of *N*-ethyl-diisopropylamine was added to the reaction mixture, which then became a clear solution. 1.87g (1.1 eq.) of 4-amino-piperidine-carboxylic acid *tert-*butyl ester was added and the mixture stirred for 16 h at RT. It was subsequently poured into crashed ice, acidified with aq. HCl (1N) and extracted twice with MeCl₂; the organic phases were washed with water, dried over MugsO4, filtered and evaporated i.v.. The crude product was purified by chromatography (SiO₂ MeCl₂ / MeOH) to yield 0.90 g of the title compounds as brown solid.
MS: 396.2 (M).

### H] 2-(Piperidin-4-ylamino)-benzooxazole-7-sulfonic acid amide

In analogy to the procedure described in example 1D], 4-(7-sulfamoyl-benzooxazol-2-ylamino)-piperidine-1-carboxylic acid *tert*-butyl ester was treated with trifluoroacetic acid in dichloromethane to yield the title compound as off-white solid.
MS: 297.2 (M+H)⁺.

### Example 77

### 2-[1-(3-Ethoxy-4-fluoro-benzyl)-piperidin-4-ylamino]-benzooxazole-7-sulfonic acid amide

In analogy to the procedure described in example 1A], 2-(piperidin-4-ylamino)-benzooxazole-7-sulfonic acid amide (example 76H]) was reacted with 3-ethoxy-4-fluorobenzaldehyde (prepared from 3-hydroxy-4-fluoro-benzoic acid in analogy to the procedure described for the synthesis of 4-chloro-3-ethoxy-benzaldehyde in example 14B]), sodium cyanoborohydride, *N*-ethyl-diisopropylamine and acetic acid in ethanol at 50 °C to yield the title compound as colorless foam.
MS: 449.2 (M+H)⁺.

### Example 78

### 2-[1-(3-Ethoxy-4-methoxy-benzyl)-piperidin-4-ylamino]-benzooxazole-7-sulfonic acid amide

In analogy to the procedure described in example 1A], 2-(piperidin-4-ylamino)-benzooxazole-7-sulfonic acid amide (example 76H]) was reacted with 3-ethoxy-4-methoxy-benzaldehyde, sodium cyanoborohydride, *N*-ethyl-diisopropylamine and acetic acid in ethanol at 50°C to yield the title compound as colorless foam.
MS:461.1 (M+H)⁺.

### Example 79

### [1-(3,5-Diethoxy-4-fluoro-benzyl)-piperidin-4-yl]-(7-methoxy-benzooxazol-2-yl)-amine

A] In analogy to the procedure described in example 1A], (7-methoxy-benzooxazol-2-yl)-piperidin-4-yl-amine (example 79B]) was reacted with 3,5-diethoxy-4-fluoro-benzaldehyde (example 2H]), sodium cyanoborohydride, *N*-ethyl-diisopropylamine and acetic acid in ethanol at 50°C to yield the title compound as colorless solid.
MS: 444.2 (M+H)⁺.
The (7-methoxy-benzooxazol-2-yl)-piperidin-4-yl-amine used in example 79A] was synthesized as follows:

### B1 (7-Methoxy-benzooxazol-2-yl)-piperidin-4-yl-amine

The title compound has been prepared by the following reaction sequence: i) treatment of 2-amino-6-methoxy-phenol [Journal of Heterocyclic Chemistry (2002), 39(1), 163-171] with potassium ethyl xanthogenate in MeOH at reflux in analogy to the procedure described in example 10E]; ii) reaction of the thus formed 7-methoxy-benzooxazole-2-thiol with thionylchloride and DMF at RT in analogy to the procedure described in example 1B] to yield 2-chloro-7-methoxy-benzooxazole; iii) condensation with 4-amino-piperidine-carboxylic acid *tert*-butyl ester in *N*-ethyl-diisopropylamine/ MeCN at 50 °C in analogy to the procedure described in example 1C] to yield 4-(7-methoxy-benzooxazol-2-ylamino)-piperidine-1-carboxylic acid *tert-*butyl ester; iv) Boc cleavage in analogy to the procedure described in example 1D].

### Example 80

### [1-(3,5-Diethoxy-4-pyrrol-1-yl-benzyl)-piperidin-4-yl]-(7-methoxy-benzooxazol-2-yl)-amine

In analogy to the procedure described in example 1A], (7-methoxy-benzooxazol-2-yl)-piperidin-4-yl-amine (example 79B]) was reacted with 3,5-diethoxy-4-pyrrol-1-yl-benzaldehyde (example 13C]), sodium cyanoborohydride, *N*-ethyl-diisopropylamine and acetic acid in ethanol at 50 °C to yield the title compound as yellow oil.
MS: 491.2 (M+H)⁺.

### Example 81

### [1-(3,5-Diethoxy-4-fluoro-benzyl)-piperidin-4-yl]-oxazolo[5,4-c]pyridin-2-yl-amine

A] In analogy to the procedure described in example 1A], oxazolo[5,4-*c*]pyridin-2-yl-piperidin-4-yl-amine, dihydrochloride (example 81C]) was reacted with 3,5-diethoxy-4-fluoro-benzaldehyde (example 2H]), sodium cyanoborohydride, *N*-ethyl-diisopropylamine and acetic acid in ethanol at 50 °C to yield the title compound as colorless solid.
MS: 415.4 (M+H)⁺.
The oxazolo[5,4-*c*]pyridin-2-yl-piperidin-4-yl-amine, dihydrochloride used in example 81A] was synthesized as follows:

### B] 4-(Oxazolo[5,4-c]pyridin-2-ylamino)-piperidine-1-carboxylic acid tert-butyl ester

A mixture of 2-methylsulfanyl-oxazolo[5,4-*c*]pyridine (4.0 g, 24.07 mMol, 1.0 eq.; prepared as described in J. Org. Chem 1995, 60, 5721-5725) and 4-amino-piperidine-1-carboxylic acid *tert*-butyl ester (5.78 g, 28.88 mMol, 1.2 eq.) in DMF (10 mL) was heated under a constant flow of Ar to 140 °C for 18 h. The reaction mixture was cooled down, a solution of sat. sodium hydrogencarbonate (100 mL) was added and the mixture extracted with dichloromethane (3 x 100 mL). The combined organic phases were dried over MgSO₄, the solvent removed by evaporation under reduced pressure and the crude material purified with column chromatography on silica eluting with heptane/ethyl acetate (1:2 + 1% NEt₃) providing 5.82 g (76%) of the title compound.
MS: 318.9 (M+H)⁺.

### C] Oxazolo[5,4-c]pyridin-2-yl-piperidin-4-yl-amine,dihydrochloride

A solution of 4-(oxazolo[5,4-*c*]pyridin-2-ylamino)-piperidine-1-carboxylic acid *tert*-butyl ester (5.82 g, 18.28 mMol) in a mixture of ethanol (40 mL) and 4 M HCl in dioxane (100 mL) was stirred at RT for 18 h. The solvent mixture was removed under reduced pressure and the crude material directly used in the following reductive alkylation step.
MS:219.1 (M+H)⁺.

### Example 82

### [1-(4-Chloro-3-ethoxy-benzyl)-piperidin-4-yl]-oxazolo[5,4-c]pyridin-2-yl-amine

In analogy to the procedure described in example 1A], oxazolo[5,4-*c*]pyridin-2-yl-piperidin-4-yl-amine, dihydrochloride (example 81C]) was reacted with 4-chloro-3-ethoxy-benzaldehyde (example 14B]), sodium cyanoborohydride, *N*-ethyl-diisopropylamine and acetic acid in ethanol at 50 °C to yield the title compound as colorless solid.
MS: 387.2 (M+H)⁺, 1Cl.

### Example 83

### [1-(3-Ethoxy-4-methyl-benzyl)-piperidin-4-yl]-oxazolo[5,4-c]pyridin-2-yl-amine

In analogy to the procedure described in example 1A], oxazolo[5,4-*c*]pyridin-2-yl-piperidin-4-yl-amine, dihydrochloride (example 81C]) was reacted with 3-ethoxy-4-methyl-benzaldehyde (prepared in analogy to the procedure described in example 2F], by reaction of 3-hydroxy-4-methyl-benzaldehyde with ethyl iodide in DMF using K₂CO₃ as base), sodium cyanoborohydride, *N*-ethyl-diisopropylamine and acetic acid in ethanol at 50 °C to yield the title compound as colorless solid.
MS: 367.2 (M+H)⁺.

### Example 84

### [1-(3-Ethoxy-4-methoxy-benzyl)-piperidin-4-yl]-oxazolo[5,4-c]pyridin-2-yl-amine

In analogy to the procedure described in example 1A], oxazolo[5,4-*c*]pyridin-2-yl-piperidin-4-yl-amine, dihydrochloride (example 81C]) was reacted with 3-ethoxy-4-methoxy-benzaldehyde, sodium cyanoborohydride, *N*-ethyl-diisopropylamine and acetic acid in ethanol at 50°C to yield the title compound as colorless solid.
MS: 383.3 (M+H)⁺.

### Example 85

### [1-(3-Isobutoxy-4-methoxy-benzyl)-piperidin-4-yl]-oxazolo[5,4-c]pyridin-2-yl-amine

In analogy to the procedure described in example 1A], oxazolo[5,4-*c*]pyridin-2-yl-piperidin-4-yl-amine, dihydrochloride (example 81C]) was reacted with 3-isobutoxy-4-methoxy-benzaldehyde [prepared by reaction of isovanillin with 1-bromo-2-methyl propane as described in WO 04/000 806 A1 (Elbion AG)], sodium cyanoborohydride, *N-*ethyl-diisopropylamine and acetic acid in ethanol at 50 °C to yield the title compound as colorless amorphous solid.
MS: 411.5 (M+H)⁺.

### Example 86

### 4-Ethoxy-6-[4-(oxazolo[5,4-c]pyridin-2-ylamino)-piperidin-1-ylmethyl]-3H-benzooxazol-2-one

A] In analogy to the procedure described in example 1A], oxazolo[5,4-*c*]pyridin-2-yl-piperidin-4-yl-amine, dihydrochloride (example 81C]) was reacted with 4-ethoxy-2-oxo-2,3-dihydro-benzooxazole-6-carbaldehyde (example 86C]), sodium cyanoborohydride, *N*-ethyl-diisopropylamine and acetic acid in ethanol at 50 °C to yield the title compound as colorless solid.
MS: 410.3 (M+H)⁺.
The 4-ethoxy-2-oxo-2,3-dihydro-benzooxazole-6-carbaldehyde used in example 86A] was synthesized as follows:

### B] 4-Ethoxy-6-hydroxymethyl-3H-benzooxazol-2-one

To a solution of 4-ethoxy-2-oxo-2,3-dihydro-benzooxazole-6-carboxylic acid ethyl ester (1.1 g, 4.38 mMol, 1.0 eq.; prepared as described in Helv. Chim. Acta 1977, 60, 3025-3034) in a mixture of dichloromethane (20 mL) and THF (10 mL) at -78 °C under Ar was slowly added diisobutylaluminium hydride (14.0 mL, 14.02 mMol, 3.2 eq., 1M solution in dichloromethane) over a time periode of 15 min, the cooling bath removed on completion of addition and the reaction allowed to reach 0°C. After 1 h, the excess hydride was quenched by cautious addition of a sat. solution of potassium sodium tartrate (10 mL). The solidified mixture was extracted with hot THF, the combined organic phases concentrated by evaporation under reduced pressure and the crude material purified with column chromatography on silica eluting with hexane/ethyl acetate (1:2) providing 0.69 g (75%) of the title compound. ¹H NMR (300 MHz, DMSO): δ 1.37 (t, *J*= 7.0 Hz, 3H), 4.15 (q, *J*= 7.0 Hz, 2H), 4.49 (d, *J*= 5.6 Hz, 2H), 5.21 (t, *J*= 5.6 Hz, 1H), 6.82 (s, 1H), 6.85 (s, 1H), 11.64 (br s, 1H). ¹³C NMR (75 MHz, DMSO): δ 1.4.61, 62.89, 64.26, 100.74, 106.46,117.80, 137.50, 142.74, 144.05, 154.54. MS: 209.8 [M+H]⁺.

### C] 4-Ethoxy-2-oxo-2,3-dihydro-benzooxazole-6-carbaldeyde

To a solution of 4-ethoxy-6-hydroxymethyl-3*H*-benzooxazol-2-one (0.69 g, 3.30 mMol, 1.0 eq.) in a mixture of dichloromethane (40 mL) and ethanol (5 mL) was added MnO₂ (1.15 g, 13.2 mmol, 4.0 eq.). The reaction mixture was heated to 40°C for 2 h, filtered through Hyflo and concentrated by evaporation under reduced pressure. The residue was purified by flash column chromatography on silica eluting with heptane/ethyl acetate (1:1) to yield 0.53 g (78%) of the title compound. ¹H NMR (300 MHz, DMSO): δ 1.43 (t, *J*= 7.0 Hz, 3H), 4.23 (q, *J*= 7.0 Hz, 2H), 7.38 (s, 1H), 7.42 (s, 1H), 9.87 (s, 1H), 12.28 (br s, 1H). ¹³C NMR (75 MHz, DMSO): δ 14.41, 64.63, 104.07, 109.32, 125.20, 131.05, 143.13, 143.88, 154.21, 191.11. MS: 208.1 [M+H]⁺.

### Example 87

### [rac]-[1-(3,5-Diethoxy-4-methanesulfinyl-benzyl)-piperidin-4-yl]-oxazolo[5,4-c] pyridin-2-yl-amine

A] In analogy to the procedure described in example 1A], oxazolo[5,4-*c*]pyridin-2-yl-piperidin-4-yl-amine, dihydrochloride (example 81C]) was reacted with [rac]-3,5-diethoxy-4-methanesulfinyl-benzaldehyde (example 87C]), sodium cyanoborohydride, *N*-ethyl-diisopropylamine and acetic acid in ethanol at 50 °C to yield the title compound as colorless amorphous solid.
MS: 459.3 (M+H)⁺.
The [rac]-3,5-diethoxy-4-methanesulfinyl-benzaldehyde used in example 87A] was synthesized as follows:

### B1 3,5-Diethoxy-4-methylsulfanyl-benzaldehyde

To a suspension of sodium hydride (2.62 g, 60.0 mMol, 2.0 eq.; 55% free-flowing powder moistened with oil) in DMF (50 mL) under Ar was added carefully methanethiol (2.88 g, 60.0 mMol, 2.0 eq.). After 15 min, a solution of 4-bromo-3,5-diethoxy-benzaldehyde (8.2 g, 30.0 mmol, 1.0 eq.; prepared according to J. Am Chem. Soc. 2001, 123, 8033-8038) in DMF (30 mL) was added, and the reaction mixture stirred overnight. The mixture was acidified to pH 2 by addition of a solution of 1 M HCl and extracted with ethyl acetate (3 x 100 mL). The combined organic phases were dried over MgSO₄, the solvent removed by evaporation under reduced pressure and the crude material purified with column chromatography on silica eluting with heptane/ethyl acetate (1:1) providing 6.9 g (96%) of the title compound. ¹H NMR (300 MHz, CDCl₃): δ 1.50 (t, *J*= 7.0 Hz, 6H), 2.50 (s, 3H), 4.18 (q, *J*= 7.0 Hz, 4H), 7.02 (s, 2H), 9.88 (s, 1H). ¹³C NMR (75 MHz, CDCl₃): δ 14.66, 17.50, 64.88, 105.71, 122.00, 135.90, 159.54, 191.31. MS: 240.9 [M+H]+.

### C][rac]-3,5-Diethoxy-4-methanesulfinyl-benzaldehyde

To a solution of 3,5-diethoxy-4-methylsulfanyl-benzaldehyde (0.28 g, 1.16 mMol, 1.0 eq.) in conc. acetic acid (5 mL) was added hydrogen peroxide (0.15 mL, 0.051 g, 1.50 mMol, 1.3 eq.; 35% solution in water). After stirring the solution for 2 h at RT the reaction mixture was extracted with ethyl acetate (3 x 50 mL) and the combined organic phases dried over MgSO₄. The organic solvent was removed by evaporation under reduced pressure and the crude material purified with column chromatography on silica eluting with ethyl acetate (1% methanol) providing 0.25 g (84%) of the title compound. ¹H NMR (300 MHz, CDCl₃): δ 1.50 (t, *J*= 7.0 Hz, 6H), 3.11 (s, 3H), 4.15-4.26 (m, 4H), 7.07 (s, 2H), 9.93 (s, 1H). ¹³C NMR (75 MHz, CDCl₃): δ 14.50, 37.62, 65.34, 106. 10, 125.13, 139.95, 159.53, 191.07. MS: 257.1 [M+H]⁺.

### Example 88

### [1-(3-Ethylamino-4-methoxy-benzyl)-piperidin-4-yl]-oxazolo[5,4-c]pyridin-2-yl-amine

A] In analogy to the procedure described in example 1A], oxazolo[5,4*-c*]pyridin-2-yl-piperidin-4-yl-amine, dihydrochloride (example 81C]) was reacted with 3-ethylamino-4-methoxy-benzaldehyde (example 92B]), sodium cyanoborohydride, *N-*ethyl-diisopropylamine and acetic acid in ethanol at 50 °C to yield the title compound as light yellow oil.
MS: 382.3 (M+H)⁺.
The 3-ethylamino-4-methoxy-benzaldehyde used in example 88A] was synthesized as follows:

### B] 3-Ethylamino-4-methoxy-benzaidehyde

Through a solution of 2-(3-bromo-4-methoxy-phenyl)-[1,3]dioxolane (1.2 g, 4.63 mMol, 1.0 eq.; prepared as described in WO 01/74 775 A1, Sanofi-Synthelabo) in toluene (6 mL) was bubbled ethylamine for 10 min. To this solution was added sodium *tert-*butoxide (0.67 g, 6.95 mMol, 1.5 eq.), BINAP (0.029 g, 0.046 mmol, 0.01 eq.) and Pd₂(dba)₃ (*tris*(dibenzylideneacetone)dipalladium, 0.021 g,0.023 mMol, 0.005 eq.) and the solution heated to 110 °C under microwave irradiation for 20 min. A few drops of a solution of 37% HCl were added and the reaction mixture heated again to 100 °C under microwave irradiation for 5 min. Evaporation of the solvent and purification of the crude reaction product by column chromatography on silica eluting with hexane/ethyl acetate (7:3) provided 0.52 g (63%) of the title compound. ¹H NMR (300 MHz, CDCl₃) : δ 1.24 (t, *J*= 7.1 Hz, 3H), 3.16 (q, *J*= 7.1 Hz, 2H), 3.86 (s, 3H); 4.17 (br s, 1H), 6.78 (d, *J*= 8.1 Hz, 1H), 7.01 (d, *J*= 1.9 Hz, 1H), 7.13 (dd, *J*= 8.1 Hz, *J*= 1.9 Hz, 1H). MS: 179.9
[M+H]⁺.

### Example 89

### [1-(8-Ethoxy-2,2-dimethyl-2H-chromen-6-ylmethyl)-piperidin-4-yl]-oxazolo[5,4-c] pyridin-2-yl-amine

In analogy to the procedure described in example 1A], oxazolo[5,4*-c*]pyridin-2-yl-piperidin-4-yl-amine, dihydrochloride (example 81C]) was reacted with 8-ethoxy-2,2-dimethyl-2*H*-chromene-6-carbaldehyde [WO 01/ 083 476 A1 (Hoffmann-La Roche AG)], sodium cyanoborohydride, *N*-ethyl-diisopropylamine and acetic acid in ethanol at 50 °C to yield the title compound as off-white amorphous solid.
MS: 435.5 (M+H)⁺.

### Example 90

### [1-(4-Methoxy-3-propoxy-benzyl)-piperidin-4-yl]-oxazolo[5,4-c]pyridin-2-yl-amine

In analogy to the procedure described in example 1A], oxazolo[5,4*-c*]pyridin-2-yl-piperidin-4-yl-amine, dihydrochloride (example 81C]) was reacted with 4-methoxy-3-propoxy-benzaldehyde [prepared by reaction of isovanillin with propyl iodide in DMF and potassium carbonate as base as described in J. Med. Chem. 1994, 37, 1696-1703], sodium cyanoborohydride, *N*-ethyl-diisopropylamine and acetic acid in ethanol at 50 °C to yield the title compound as colorless solid.
MS: 397.3 (M+H)⁺.

### Example 91

### {1-[3-(2-Fluoro-ethoxy)-4-methoxy-benzyl)-piperidin-4-yl}-oxazo10 [5,4-c]pyridin-2-yl-amine

In analogy to the procedure described in example 1A], oxazolo[5,4-*c*]pyridin-2-yl-piperidin-4-yl-amine, dihydrochloride (example 81C]) was reacted with 3-(2-fluoro-ethoxy)-4-methoxy-benzaldehyde (example 59B]), sodium cyanoborohydride, *N*-ethyl-diisopropylamine and acetic acid in ethanol at 50 °C to yield the title compound as colorless amorphous solid.
MS: 401.3 (M+H)+^{.}

### Example 92

### 2-Ethoxy-4-[4-(oxazolo[5,4-c]pyridin-2-ylamino)-piperidin-1-ylmethyl]-phenol

In analogy to the procedure described in example 1A], oxazolo[5,4-*c*]pyridin-2-yl-piperidin-4-yl-amine, dihydrochloride (example 81C]) was reacted with 3-ethoxy-4-hydroxy-benzaldehyde, sodium cyanoborohydride, *N*-ethyl-diisopropylamine and acetic acid in ethanol at 50°C to yield the title compound as colorless oil.
MS: 369.3 (M+H)⁺.

### Example 93

### [1-(4-Amino-3,5-diethoxy-benzyl)-piperidin-4-yl]-oxazolo[5,4-c] pyridin-2-yl-amine

In analogy to the procedure described in example 1A], oxazolo[5,4-*c*]pyidin-2-yl-piperidin-4-yl-amine, dihydrochloride (example 81C]) was reacted with 4-amino-3,5-diethoxy-benzaldehyde (example 62C]), sodium cyanoborohydride, *N*-ethyl-diisopropylamine and acetic acid in ethanol at 50 °C to yield the title compound as light yellow oil.
MS: 412.4 (M+H)⁺.

### Example 94

### [1-(3,5-Diisopropoxy-benzyl)-piperidin-4-yl]-oxazolo[5,4-c]pyridin-2-yl-amine

In analogy to the procedure described in example 1A], oxazolo[5,4-c]pyridin-2-yl-piperidin-4-yl-amine, dihydrochloride (example 81C]) was reacted with 3,5-diisopropoxy-benzaldehyde (prepared in analogy to the procedure described in example 2F], by reaction of 3,5-dihydroxybenzaldehyde with 2-iodo-propane in DMF using K₂CO₃ as base), sodium cyanoborohydride, *N*-ethyl-diisopropylamine and acetic acid in ethanol at 50 °C to yield the title compound as colorless solid.
MS: 425.4 (M+H)⁺.

### Example 95

### [1-(3-Ethoxy-4-fluoro-benzyl)-piperidin-4-yl]-oxazolo[5,4-c]pyridin-2-yl-amine

In analogy to the procedure described in example 1A], oxazolo[5,4-*c*]pyridin-2-yl-piperidin-4-yl-amine, dihydrochloride (example 81C]) was reacted with 3-ethoxy-4-fluoro-benzaldehyde (prepared from 3-hydroxy-4-fluoro-benzoic acid in analogy to the procedure described for the synthesis of 4-chloro-3-ethoxy-benzaldehyde in example 14B]), sodium cyanoborohydride, *N*-ethyl-diisopropylamine and acetic acid in ethanol at 50°C to yield the title compound as off-white solid.
MS: 371.1 (M+H)⁺.

### Example 96

### [1-(3-Ethoxy-4-isopropoxy-benzyl)-piperidin-4-yl]-oxazolo[5,4-c]pyridin-2-yl-amine

In analogy to the procedure described in example 1A], oxazolo[5,4-*c*]pyridin-2-yl-piperidin-4-yl-amine, dihydrochloride (example 81C]) was reacted with 3-ethoxy-4-isopropoxy-benzaldehyde, sodium cyanoborohydride, *N*-ethyl-diisopropylamine and acetic acid in ethanol at 50 °C to yield the title compound as off white solid.
MS: 411.4 (M+H)⁺.

### Example 97

### Oxazolo[5,4-c]pyridin-2-yl-[1-(2-phenyl-3H-imidazol-4-ylmethyl)-piperidin-4-yl]-amine

To a solution of oxazolo[5,4*-c*]pyridin-2-yl-piperidin-4-yl-amine, dihydrochloride (example 81C]; 100 mg, 0.34 mMol, 1.0 eq.) and 2-phenyl-1*H*-imidazole-4-carbaldehyde (commercially available, 71.0 mg, 0.41 mMol, 1.2 eq.) in ethanol (2 mL) was added *N-*ethyl diisopropylamine (79.6 µL, 88.1 mg, 0.68 mMol, 2.0 eq.) and acetic acid (61.2 mg, 1.0 mMol, 3.0 eq.) and the mixture heated to 100 °C under microwave irradiation. After 10 min, sodium cyano borohydride (25.8 mg, 0.41 mmol, 1.2 eq.) was added and the mixture stirred for an additional 15 min at 100 °C under microwave irradiation. Removal of the solvent under reduced pressure and purification by preparative HPLC on reversed phase elating with a gradient of acetonitrile/water provided 11.9 mg (9%) of the title compound as colorless solid.
MS: 375.3 (M+H)⁺.

### Example 98

### [1-(2-Methyl-5-nitro-1H-indol-3-ylmethyl)-piperidin-4-yl]-oxazolo[5,4-c]pyridin-2-yl-amine

In analogy to the procedure described in example 1A], oxazolo[5,4-*c*]pyridin-2-yl-piperidin-4-yl-amine, dihydrochloride (example 81C]) was reacted with 2-methyl-5-nitro-1*H*-indole-3-carbaldehyde, sodium cyanoborohydride, *N*-ethyl-diisopropylamine and acetic acid in ethanol at 50 °C to yield the title compound as light yellow solid.
MS: 407.3 (M+H)⁺.

### Example 99

### {2-[1-(3-Ethoxy-4-methoxy-benzyl)-piperidin-4-ylamino]-benzooxazol-5-yloxy}-acetic acid methyl ester

A] In analogy to the procedure described in example 1A], [2-(piperidin-4-ylamino)-benzooxazol-5-yloxy]-acetic acid methyl ester (prepared from 4-(5-methoxycarbonyl-methoxy-benzooxazol-2-ylamino)-piperidine-1-carboxylic acid *tert-*butyl ester (example 99C]) by cleavage of the Boc protecting group with trifluoroacetic acid in analogy to the procedure described in example 1D]) was reacted with 3-ethoxy-4-methoxybenzaldehyde, sodium cyanoborohydride, *N*-ethyl-diisopropylamine and acetic acid in ethanol at 50°C to yield the title compound as light brown foam.
MS:470.1 (M+H)⁺.
4-(5-Methoxycarbonylmethoxy-benzooxazol-2-ylamino)-piperidine-1-carboxylic acid *tert*-butyl ester as used in example 99A] was synthesized as follows:

### B] 4-(5-Hydroxy-benzooxazol-2-ylamino)-piperidine-1-carboxylic acid tert-butyl ester

3.25 g (13.1 mMol) of (5-methoxy-benzooxazol-2-yl)-piperidin-4-yl-amine (prepared by i) reaction of 2-chloro-5-methoxy-benzooxazole [Journal of Organic Chemistry (1996), 61(10), 3289-97] with 4-amino-piperidine-carboxylic acid *tert*-butyl ester in *N*-ethyl-diisopropylamine/MeCN at 50 °C to give 4-(5-methoxy-benzooxazol-2-ylamino)-piperidine-1-carboxylic acid *tert*-butyl ester; ii) cleavage of the Boc protecting group with trifluoroacetic acid; in analogy to the procedures described in examples 1C-D]) was added in small portions while stirring to 29.5 mL (44.3 g = 20 eq.) of HBr in water (48%) at RT and the reaction mixture was subsequently warmed up to 100°C. After 9h, it was cooled down to ∼ 50°C, 160 ml of toluene was added in two portions and it was evaporated i.V.; the residue, crude 2-(piperidin-4-ylamino)-benzooxazol-5-ol hydrobromide, was dissolved in 30 mL of water, 5.98 g (5.0 eq.) of solid sodium hydrogen carbonate and 10.37 g (3.3 eq.) of di-*tert*-butyl dicarbonate was added, followed by 30 mL of dioxane. This reaction mixture was stirred for 72 h at RT. It was then poured into crashed ice and extracted twice with EtOAc; the organic phases were washed with water, dried over MgSO₄, filtered and evaporated i.v. and the residue (5.13 g) was purified by chromatography (SiO₂, MeCl₂ / MeOH) to yield 2.86 g of the title compound as dark-brown amorphous solid.
MS: 334.1 (M+H)⁺.

### C] 4-(5-Methoxycarbonylmethoxy-benzooxazol-2-ylamino)-piperidine-1-carboxylic acid tert-butyl ester

2.09 g (6.3 mMol) of 4-(5-hydroxy-benzooxazol-2-ylamino)-piperidine-1-carboxylic acid *tert*-butyl ester was dissolved in 100 ml of MeCN and cooled down to 0 °C; 2.19 g (15.7 mmol) of anhydrous potassium carbonate was added, followed by 0.63 mL (1.04 g = 6.6 mMol) of methyl bromo acetate. The reaction mixture was stirred for 18 hours at RT and to complete the reaction, it was subsequently stirred at 50 °C for 90 min. The reaction mixture was then poured into crashed ice and extracted twice with EtOAc. The organic phases were washed with water and brine, dried over MgSO₄ filtered and evaporated i.v. and the residue (2.76 g) was purified by chromatography (SiO₂, MeCl₂ / MeOH) to yield 2.22 g of the title compound as light brown solid.
MS: 406.2 (M+H)⁺.

### Examples 100

### {2-[1-(3,5-Diethoxy-4-fluoro-benzyl)-piperidin-4-ylamino]-benzooxazol-5-yloxy}-acetic acid methyl ester

In analogy to the procedure described in example 1A]; [2-(piperidin-4-ylamino)-benzooxazol-5-yloxy]-acetic acid methyl ester (example 99) was reacted with 3,5-diethoxy-4-fluoro-benzaldehyde (example 2H]), sodium cyanoborohydride, *N*-ethyl-diisopropylamine and acetic acid in ethanol at 50 °C to yield the title compound as off-white oil.
MS: 502.1(M+H)⁺.

### Example 101

### {2-[1-(3,5-Diethoxy-4-pyrrol-1-yl-benzyl)-piperidin-4-ylamino]-benzooxazol-5-yloxy}-acetic acid methyl ester

In analogy to the procedure described in example 1A], [2-(piperidin-4-ylamino)-benzooxazol-5-yloxy]-acetic acid methyl ester (example 99) was reacted with 3,5-diethoxy-4-pyrrol-1-yl-benzaldehyde (example 13C]), sodium cyanoborohydride, N-ethyl-diisopropylamine and acetic acid in ethanol at 50°C to yield the title compound as off-white oil.
MS: 549.3 (M+H)⁺.

### Example 102

### {2-[1-(3-Ethoxy-4-methoxy-benzyl)-piperidin-4-ylamino]-benzooxazol-5-yloxy}-acetic acid

In analogy to the procedure described in example 5, {2-[1-(3-ethoxy-4-methoxybenzyl)-piperidin-4-ylamino]-benzooxazol-5-yloxy}-acetic acid methyl ester (example 99) was saponified to yield the title compound as off-white solid.
MS: 454.4 (M-H)⁻.

### Example 103

### {2-[1-(3,5-Diethoxy-4-fluoro-ben zyl)-piperidin-4-ylamino]-benzooxazol-5-yloxy }-acetic acid

In analogy to the procedure described in example 5, {2-[1-(3,5-diethoxy-4-fluorobenzyl)-piperidin-4-ylamino]-benzooxazol-5-yloxy}-acetic acid methyl ester (example 100) was saponified to yield the title compound as off-white solid.
MS: 486.4 (M-H)⁻.

### Example 104

### {2-[1-(3,5-Diethoxy-4-pyrrol-1-yl-benzyl)-piperidin-4-ylamino]-benzooxazol-5-yloxy}-acetic acid

In analogy to the procedure described in example 5, {2-[1-(3,5-diethoxy-4-pyrrol-1-yl-benzyl)-piperidin-4-ylamino]-benzooxazol-5-yloxy}-acetic acid methyl ester (example 101) was saponified to yield the title compound as off-white solid.
MS: 533.4 (M-H)⁻.

### Example 105

### [1-(3,5-Diethoxy-4-fluoro-benzyl)-piperidin-4-yl]-(7-phenyl-benzooxazol-2-yl)-amine

A] In analogy to the procedure described in example 1A], (7-phenyl-benzooxazol-2-yl)-piperidin-4-yl-amine (prepared from 4-[*tert*-butoxycarbonyl-(7-phenyl-benzooxazol-2-yl)-amino]-piperidine-1-carboxylic acid *tert*-butyl ester (example 105C]) by cleavage of the Boc protecting group with trifluoroacetic acid in analogy to the procedure described in example 1D]) was reacted with 3,5-diethoxy-4-fluorobenzaldehyde (example 2H]), sodium cyanoborohydride, *N*-ethyl-diisopropylamine and acetic acid in ethanol at 50 °C to yield the title compound as off-white oil.
MS: 490.3 (M+H)⁺.
4-[*tert*-Butoxycarbonyl-(7-phenyl-benzooxazol-2-yl)-amino]-piperidine-1-carboxylic acid *tert*-butyl ester as used in example 105A] was synthesized as follows:

### B]4-[(7-Bromo-benzooxazol-2-yl)-tert-butoxycarbonyl-amino]-piperidine-1-carboxylic acid tert-butyl ester

4.60g (11.6mMol) of 4-(7-bromo-benzooxazol-2-ylamino)-piperidine-1-carboxylic acid *tert*-butyl ester [example 52B] was dissolved in 100 mL of MeCN under Ar at RT; while stirring, 5.57 g (2.2 eq.) of di-*tert*-butly-dicarbonate was added in several small portions, followed by 0.28 g (0.2 eq.) of *N,N*-dimethyl-4-aminopyridine. The reaction mixture was stirred for 72 h at RT, poured into crashed ice and extracted three times with EtOAc; the organic phases were washed with water and brine, dried over MgSO₄, filtered and evaporated i.v. and the residue (6.00 g) was purified by chromatography (SiO₂, MeCl₂ / MeOH) to yield 4.24 g of the title compound as brown foam.
MS: 496.3 (M+H)⁺, 1Br.

### C]4-tert-Butoxycarbonyl-(7-phenyl-benzooxazol-2-yl)-amino]-piperidine-1-carboxylic acid tert-butyl ester

0.90 g (1.8 mMol) of 4-[(7-bromo-benzooxazol-2-yl)-*tert*-butoxycarbonyl-amino]-piperidine-1-carboxylic acid *tert*-butyl ester, 0.34 g (1.5 eq.) of phenyl boronic acid, 0.32 g (3.0 eq.) of potassium fluoride, 0.04 g (0.1 eq.) of palladium acetate and 0.10 g (0.2 eq.) of 2-(di-*tert*-butylphosphino)biphenyl were suspended in 5 mL of THF at RT; then, the reaction mixture was warmed up to reflux. After 34 h, it was poured into crashed ice and extracted three times with EtOAc; the organic phases were washed with water, dried over MgSO₄, filtered and evaporated i.v. and the residue (1.04 g) was purified by chromatography (SiO₂, n-heptane / EtOAc) to yield 0.23 g of the title compound as off-white oil.
MS: 494.5 (M+H)⁺.

### Example 106

### {2-[1-(3-Ethoxy-4-methoxy-benzyl)-piperidin-4-ylamino]-benzooxazol-7-yloxy}-acetic acid methyl ester

A] In analogy to the procedure described in example 1A], [2-(piperidin-4-ylamino)-benzooxazol-7-yloxy]-acetic acid methyl ester (example 106B]) was reacted with 3-ethoxy-4-methoxybenzaldehyde, sodium cyanoborohydride, *N*-ethyl-diisopropylamine and acetic acid in ethanol at 50 °C to yield the title compound as colorless oil.
MS: 470.1 (M+H)⁺.
The [2-(piperidin-4-ylamino)-benzooxazol-7-yloxy]-acetic acid methyl ester used in example 106A] was synthesized as follows:

### B] [2-(Piperidin-4-ylamino)-benzooxazol-7-yloxy]-acetic acid methyl ester

The title compound has been prepared by the following reaction sequence: i) in analogy to example 99B], (7-methoxy-benzooxazol-2-yl)-piperidin-4-yl-amine (example 79B]) was reacted with HBr (48% in water) at 100 °C to give 2-(piperidin-4-ylamino)-benzooxazol-7-ol hydrobromide; ii) in analogy to example 99B], 2-(piperidin-4-ylamino)-benzooxazol-7-ol hydrobromide was reacted with di-*tert*-butyl dicarbonate to give 4-(7-hydroxy-benzooxazol-2-ylamino)-piperidine-1-carboxylicacid *tert*-butyl ester; iii) in analogy to example 99C], 4-(7-hydroxy-benzooxazol-2-ylamino)-piperidine-1-carboxylic acid *tert*-butyl ester was reacted with methyl bromo acetate and potassium carbonate in MeCN (RT to 50 °C) to yield 4-(7-methoxycarbonylmethoxy-benzooxazol-2-ylamino)-piperidine-1-carboxylic acid *tert*-butyl ester; iv) in analogy to example 1D], 4-(7-methoxycarbonylmethoxy-benzooxazol-2-ylamino)-piperidine-1-carboxylic acid *tert*-butyl ester was reacted with trifluoroacetic acid to finally give the title compound.

### Example 107

### {2-[1-(3,5-Diethoxy-4-fluoro-ben zyl)-piperidin-4-ylamino]-ben zooxazol-7-yloxy }-acetic acid methyl ester

In analogy to the procedure described in example 1A], [2-(piperidin-4-ylamino)-benzooxazol-7-yloxy]-acetic acid methyl ester (example 106B]) was reacted with 3,5-diethoxy-4-fluoro-benzaldehyde (example 2H]), sodium cyanoborohydride, *N*-ethyl-diisopropylamine and acetic acid in ethanol at 50 °C to yield the title compound as colorless foam.
MS: 502.1 (M+H)⁺.

### Example 108

### {2-[1-(3,5-Diethoxy-4-pyrrol-1-yl-benzyl)-piperidin-4-ylamino]-benzooxazol-7-yloxy acetic acid methyl ester

In analogy to the procedure described in example 1A], [2-(piperidin-4-ylamino)-benzooxazol-7-yloxy]-acetic acid methyl ester (example 106B]) was reacted with 3,5-diethoxy-4-pyrrol-1-yl-benzaldehyde (example 13C]), sodium cyanoborohydride, *N-*ethyl-diisopropylamine and acetic acid in ethanol at 50 °C to yield the title compound as yellow oil.
MS: 549.3 (M+H)⁺.

### Example 1.09

### {2-[1-(3-Ethoxy-4-methoxy-benzyl)-piperidin-4-ylamino]-benzooxazol-7-yloxy}-acetic acid

In analogy to the procedure described in example 5, {2-[1-(3-ethoxy-4-methoxybenzyl)-piperidin-4-ylamino]-benzooxazol-7-yloxy}-acetic acid methyl ester (example 106) was saponified to yield the title compound as off-white solid.
MS: 456.2 (M-H)⁻.

### Example 110

### {2-[1-(3,5-Diethoxy-4-fluoro-benzyl)-piperidin-4-ylamino]-benzooxazol-7-yloxy}-acetic acid

In analogy to the procedure described in example 5, {2-[1-(3,5-diethoxy-4-fluorobenzyl)-piperidin-4-ylamino]-benzooxazol-7-yloxy}-acetic acid methyl ester (example 107) was saponified to yield the title compound as off-white solid.
MS: 488.2 (M-H)⁻.

### Example 111

### {2-[1-(3,5-Diethoxy-4-pyrrol-1-yl-benzyl)-piperidin-4-ylamino]-benzooxazol-7-yloxy}-acetic acid

In analogy to the procedure described in example 5, {2-[1-(3,5-diethoxy-4-pyrrol-1-yl-benzyl)-piperidin-4-ylamino]-benzooxazol-7-yloxy}-acetic acid methyl ester (example 108) was saponified to yield the title compound as off-white solid.
MS: 535.4 (M-H)⁻.

### Example 112

### (5-Aminomethyl-benzooxazol-2-yl)-[1-(3,5-diethoxy-4-fluoro-benzyl)-piperidin-4-yl]-amine

A]-0.30 g (0.7 mmol) of 2-[1-(3,5-diethoxy-4-fluoro-benzyl)-piperidin-4-ylamino]-benzooxazole-5-carbonitrile (example 112B]) was dissolved at RT in 10 mLof THF and then heated to reflux. 0.16 mL (0.127 g = 1.5 mMol) of borane-dimethyl sulfide complex was added and after 1 hour, the reaction mixture was cooled down to 40 °C. 1.0 mL of hydrochloric acid (25% in water) was added drop by drop and the mixture then heated again at reflux for 30 min. It was subsequently poured into crashed ice, the pH adjusted to 10 with potassium carbonate and extracted twice with EtOAc. The organic phases were washed with water and brine, dried over MgSO₄, filtered and evaporated i.V. and the residue (0.38 g) was purified by chromatography (SiO₂, MeCl₂ / MeOH) to yield 0.20 g of the title compound as orange oil.
MS: 443.2 (M+H)⁺.
The 2-[1-(3,5-diethoxy-4-fluoro-benzyl)-piperidin-4-ylamino]-benzooxazole-5-carbonitrile used in example 112A] was synthesized as follows:

### B] 2-[1-(3,5-Diethoxv-4-fluoro-benzyl)-piperidin-4-ylaminol-benzooxazole-5-carbonitrile

In analogy to the procedure described in example 1A], 2-(piperidin-4-ylamino)-benzooxazole-5-carbonitrile (prepared by treatment of 2-chloro-benzooxazole-5-carbonitrile [Heterocycles (2003), 60(6), 1367-1376] with 4-amino-piperidine-carboxylic acid *tert*-butyl ester in *N*-ethyl-diisopropylamine/MeCN at RT in analogy to the procedure described in example 1C] to yield 4-(5-cyano-benzooxazol-2-ylamino)-piperidine-1-carboxylic acid *tert*-butyl ester; followed by Boc cleavage in analogy to the procedure described in example 1D]) was reacted with 3,5-diethoxy-4-fluorobenzaldehyde (example 2H]), sodium cyanoborohydride, *N*-ethyl-diisopropylamine and acetic acid in ethanol at 50 °C to yield the title compound as light yellow solid.
MS: 439.2 (M+H)⁺.

### Example 113

### 4-{2-[1-(3,5-Diethoxy-4-fluoro-benzyl)-piperidin-4-ylamino]-benzooxazol-7-yloxy}-butyric acid methyl ester

A] In analogy to the procedure described in example 1A], 4-[2-(piperidin-4-ylamino)-benzooxazol-7-yloxy]-butyric acid methyl ester (prepared from 4-[7-(3-methoxycarbonyl-propoxy)-benzooxazol-2-ylamino]-piperidine-1-carboxylic acid *tert-*butyl ester (example 113B]) by cleavage of the Boc protecting group with trifluoroacetic acid in analogy to the procedure described in example 1D]) was reacted with 3,5-diethoxy-4-fluoro-benzaldehyde (example 2H]), sodium cyanoborohydride, *N*-ethyl-diisopropylamine and acetic acid in ethanol at 50 °C to yield the title compound as colorless oil.
MS: 530.2 (M+H)⁺.
The 4-[7-(3-methoxycarbonyl-propoxy)-benzooxazol-2-ylamino]-piperidine-1-carboxylic acid *tert*-butyl ester used in example 113A] was synthesized as follows:

### B] 4-[7-(3-Methoxycarbonyl-propoxy)-benzooxazol-2-ylamino]-piperidine-1-carboxylic acid tert-butyl ester

1.10g (3.3 mMol) of 4-(7-hydroxy-benzooxazol-2-ylamino)-piperidine-1-carboxylic acid *tert*-butyl ester (example 106B]) was dissolved in 45 ml of MeCN at RT; 1.14 g (8.2 mMol) of anhydrous potassium carbonate was added, followed by 0.44 mL (0.63 g = 3.5 mMol) of 4-bromo-butyric acid methyl ester. The reaction mixture was stirred at 50 °C for 22 hours. It was poured into crashed ice and extracted twice with EtOAc. The organic phases were washed with water and brine, dried over MgSO₄, filtered and evaporated i.v. and the residue (1.50 g) was purified by chromatography (SiO₂, MeCl₂ / MeOH) to yield 1.15 g of the title compound as light yellow foam.
MS: 434.3 (M+H)⁺.

### Example 114

### 4-{2-[1-(3,5-Diethoxy-4-pyrrol-1-yl-ben zyl)-piperidin-4-ylamino]-benzooxazol-7-yloxy}-butyric acid methyl ester

In analogy to the procedure described in example 1A], 4-[2-(piperidin-4-ylamino)-benzooxazol-7-yloxy]-butyric acid methyl ester (example 113A]) was reacted with 3,5-diethoxy-4-pyrrol-1-yl-benzaldehyde (example 13C]), sodium cyanoborohydride, *N-*ethyl-diisopropylamine and acetic acid in ethanol at 50 °C to yield the title compound as yellow oil.
MS: 577.3 (M+H)⁺.

### Example 115

### 4- {2-[1-(3-Ethoxy-4-methoxy-benzyl)-piperidin-4-ylamino]-benzooxazol-7-yloxy}-butyric acid methyl ester

In analogy to the procedure described in example 1A], 4-[2-(piperidin-4-ylamino)-benzooxazol-7-yloxy]-butyric acid methyl ester (example 113A]) was reacted with 3-ethoxy-4-methoxybenzaldehyde, sodium cyanoborohydride, *N*-ethyl-diisopropylamine and acetic acid in ethanol at 50 °C to yield the title compound as yellow oil.
MS: 498.2 (M+H)⁺.

### Example 116

### 4- {2-[1-(3,5-Diethoxy-4-fluoro-benzyl)-piperidin-4-ylamino]-benzooxazol-7-yloxy}-butyric acid

In analogy to the procedure described in example 5, 4-{2-[1-(3,5-diethoxy-4-fluoro-benzyl)-piperidin-4-ylamino]-benzooxazol-7-yloxy}-butyric acid methyl ester (example 113) was saponified to yield the title compound as off-white solid.
MS: 516.2 (M+H)⁺.

### Example 117

### 4-{2-[1-(3,5-Diethoxy-4-pyrrol-1-yl-benzyl)-piperidin-4-ylamino]-benzooxazol-7-yloxy}-butyric acid

In analogy to the procedure described in example 5, 4-{2-[1-(3,5-diethoxy-4-pyrrol-1-yl-benzyl)-piperidin-4-ylamino]-benzooxazol-7-yloxy}-butyric acid methyl ester (example 114) was saponified to yield the title compound as colorless foam.
MS: 563.5 (M+H)⁺.

### Example 118

### 4-{2-[1-(3-Ethoxy-4-methoxy-benzyl)-piperidin-4-ylamino]-benzooxazol-7-yloxy}-butyric acid

In analogy to the procedure described in example 5, 4-{2-[1-(3-ethoxy-4-methoxybenzyl)-piperidin-4-ylamino]-benzooxazol-7-yloxy}-butyric acid methyl ester (example 115) was saponified to yield the title compound as colorless foam.
MS: 484.3 (M+H)⁺.

### Example 119

### [rac]-3-{2-[1-(3,5-Diethoxy-4-fluoro-benzyl)-piperidin-4-ylamino]-benzooxazol-7-yloxy}-propane-1,2-diol

A] In analogy to the procedure described in example 1A], crude [rac]-3-[2-(piperidin-4-ylamino)-benzooxazol-7-yloxy]-propane-1,2-diol trifluoroacetate (example 119C]) was reacted with 3,5-diethoxy-4-fluoro-benzaldehyde (example 2H]), sodium cyanoborohydride, N-ethyl-diisopropylamine and acetic acid in ethanol at 50 °C to yield the title compound as colorless solid.
MS: 504.3 (M+H)⁺.
The [rac]-3-[2-(piperidin-4-ylamino)-benzooxazol-7-yloxy]-propane-1,2-diol used in example 119A] was synthesized as follows:

### B1 [rac]-4-[7-(2,2-Dimethyl-[1,3]dioxolan-4-ylmethoxy)-benzooxazol-2-ylaminol-piperidine-1-carboxylic acid tert-butyl ester

1.10 g (3.3 mMol) of 4-(7-hydroxy-benzooxazol-2-ylamino)-piperidine-1-carboxylic acid *tert*-butyl ester (example 106B]) was dissolved in 45 ml of MeCN at RT; 1.14 g (8.2 mMol) of anhydrous potassium carbonate was added, followed by 1.08 g (3.5 mmol) of [rac]-2,2-dimethyl-1,3-dioxolan-4-yl-methyl p-toluenesulfonate and 0.27 g (1.6 mMol) of potassium iodide. The reaction mixture was stirred at reflux for 16 hours. It was then poured into crashed ice and extracted twice with EtOAc. The organic phases were washed with water and brine, dried over MgSO₄, filtered and evaporated i.V. and the residue (1.31 g) was purified by chromatography (SiO₂, MeCl₂ / MeOH) to yield 0.84 g of the title compound as a colorless oil.
MS: 448.2 (M+H)⁺.

### C] [rac]-3-[2-(Piperidin-4-ylamino)-benzooxazol-7-yloxyl-propane-1,2-diol trifluoracetate

0.81 g (1.8 mMol) of [rac]-4-[7-(2,2-dimethyl-[1,3]dioxolan-4-ylmethoxy)-benzooxazol-2-ylamino]-piperidine-1-carboxylic acid tert-butyl ester was dissolved in 18 mL of MeCl₂; while stirring, 1.39 mL (2.06 g = 18.1 mMol) of trifluoroacetic acid was added drop by drop. After 20 hours, the reaction mixture was evaporated and dried at high vacuum. 20 mL MeOH was added to the residue; the mixture was stirred for 30 min at RT and subsequently filtered to yield 0.91 g of the crude title compound, which was used whitout further purification.
MS: 308.3 (M+H)⁺.

### Example 120

### [rac]-3-{2-[1-(3,5-Diethoxy-4-pyrrol-1-yl-benzyl)-piperidin-4-ylamino]-benzooxazol-7-yloxy}-propane-1,2-diol

In analogy to the procedure described in example 1A], crude [rac]-3-[2-(piperidin-4-ylamino)-benzooxazol-7-yloxy]-propane-1,2-diol trifluoroacetate (example 119C]) was reacted with 3,5-diethoxy-4-pyrrol-1-yl-benzaldehyde (example 13C]), sodium cyanoborohydride, *N*-ethyl-diisopropylamine and acetic acid in ethanol at 50 °C to yield the title compound as light brown solid.
MS: 551.2 (M+H)⁺.

### Example 121

### [rac]-3-{2-[1-(3-Ethoxy-4-methoxy-benzyl)-piperidin-4-ylamino]-benzooxazol-7-yloxy}-propane-1,2-diol

In analogy to the procedure described in example 1A], crude [rac]-3-[2-(piperidin-4-ylamino)-benzooxazol-7-yloxy]-propane-1,2-diol trifluoroacetate (example 119C]) was reacted with 3-ethoxy-4-methoxybenzaldehyde, sodium cyanoborohydride, *N*-ethyl-diisopropylamine and acetic acid in ethanol at 50 °C to yield the title compound as colorless foam.
MS: 472.0 (M+H)⁺.

### Example 122

### 4-{2-[1-(3,5-Diethoxy-4-fluoro-benzyl)-piperidin-4-ylamino]-benzooxazol-5-yloxy}-butyric acid methyl ester

In analogy to the procedure described in example 1A], 4-[2-(piperidin-4-ylamino)-benzooxazol-5-yloxy]-butynic acid methyl ester (prepared from 4-(5-hydroxy-benzooxazol-2-ylamino)-piperidine-1-carboxylic acid *tert*-butyl ester (example 99B]) and 4-bromo-butyric acid methyl ester in analogy to the procedure described in example 113B] followed by Boc cleavage in with trifluoroacetic acid in analogy to the procedure described in example 1D]) was reacted with 3,5-diethoxy-4-fluoro-benzaldehyde (example 2H]), sodium cyanoborohydride, *N*-ethyl-diisopropylamine and acetic acid in ethanol at 50 °C to yield the title compound as colorless foam.
MS: 530.2 (M+H)⁺.

### Example 123

### 4-{2-[1-(3,5-Diethoxy-4-pyrrol-1-yl-benzyl)-piperidin-4-ylamino]-benzooxazol-5-yloxy}-butyric acid methyl ester

In analogy to the procedure described in example 1A], 4-[2-(piperidin-4-ylamino)-benzooxazol-5-yloxy]-butyric acid methyl ester (example 122) was reacted with 3,5-diethoxy-4-pyrrol-1-yl-benzaldehyde (example 13C]), sodium cyanoborohydride, *N-*ethyl-diisopropylamine and acetic acid in ethanol at 50 °C to yield the title compound as light yellow foam.
MS: 577.3 (M+H)⁺.

### Example 124

### 4-{2-[1-(3-Ethoxy-4-methoxy-benzyl)-piperidin-4-ylamino]-benzooxazol-5-yloxy}-butyric acid methyl ester

In analogy to the procedure described in example 1A], 4-[2-(piperidin-4-ylamino)-benzooxazol-5-yloxy]-butyric acid methyl ester (example 122) was reacted with 3-ethoxy-4-methoxybenzaldehyde, sodium cyanoborohydride, *N*-ethyl-diisopropylamine and acetic acid in ethanol at 50 °C to yield the title compound as colorless foam.
MS:498.1 (M+H)⁺.

### Example 125

### 4-[2-[1-(3,5-Diethoxy-4-fluoro-benzyl)-piperidin-4-ylamino]-benzooxazol-5-yloxy}-butyric acid

In analogy to the procedure described in example 5, 4-{2-[1-(3,5-diethoxy-4-fluoro-benzyl)-piperidin-4-ylamino]-benzooxazol-5-yloxy}-butyric acid methyl ester (example 122) was saponified to yield the title compound as colorless solid.
MS: 514.3 (M-H)⁻.

### Example 126

### 4-{2-[1-(3,5-Diethoxy-4-pyrrol-1-yl-benzyl)-piperidin-4-ylamino]-benzooxazol-5-yloxy}-butyric acid

In analogy to the procedure described in example 5, 4-{2-[1-(3,5-diethoxy-4-pyrrol-1-yl-benzyl)-piperidin-4-ylamino]-benzooxazol-5-yloxy}-butyric acid methyl ester (example 123) was saponified to yield the title compound as colorless solid.
MS: 563.5 (M+H)⁺.

### Example 127

### 4-{2-[1-(3-Ethoxy-4-methoxy-benzyl)-piperidin-4-ylamino]-benzooxazol-5-yloxy}-butyric acid

In analogy to the procedure described in example 5, 4-{2-[1-(3-ethoxy-4-methoxybenzyl)-piperidin-4-ylamino]-benzooxazol-5-yloxy}-butyric acid methyl ester (example 124) was saponified to yield the title compound as yellow solid.
MS: 484.3 (M+H)⁺.

### Example 128

### 1- {2-[1-(3,5-Diethoxy-4-fluoro-benzyl)-piperidin-4-ylamino]-benzooxazol-5-yloxy}-cyclobutanecarboxylic acid ethyl ester

In analogy to the procedure described in example 1A], 1-[2-(piperidin-4-ylamino)-benzooxazol-5-yloxy]-cyclobutanecarboxylic acid ethyl ester (prepared from 4-(5-hydroxy-benzooxazol-2-ylamino)-piperidine-1-carboxylic acid *tert*-butyl ester (example 99B]), 1-bromo-cyclobutanecarboxylic acid ethyl ester and potassium carbonate in *N,N-*dimethylformamide at 100 °C in analogy to the procedure described in example 113B] followed by Boc cleavage in with trifluoroacetic acid in analogy to the procedure described in example 1D]) was reacted with 3,5-diethoxy-4-fluoro-benzaldehyde (example 2H]), sodium cyanoborohydride, *N*-ethyl-diisopropylamine and acetic acid in ethanol at 50 °C to yield the title compound as yellow oil.
MS: 556.3 (M+H)⁺.

### Example 129

### 1-{2-[1-(3,5-Diethoxy-4-pyrrol-1-yl-benzyl)-piperidin-4-ylamino]-benzooxazol-5-yloxy}-cyclobutanecarboxylic acid ethyl ester

In analogy to the procedure described in example 1A], 1-[2-(piperidin-4-ylamino)-benzooxazol-5-yloxy]-cyclobutanecarboxylic acid ethyl ester (example 128) was reacted with 3,5-diethoxy-4-pyrrol-1-yl-benzaldehyde (example 13C]), sodium cyanoborohydride, *N*-ethyl-diisopropylamine and acetic acid in ethanol at 50 °C to yield the title compound as yellow oil.
MS: 603.4 (M+H)⁺.

### Example 130

### 1-{2-[1-(3,5-Diethoxy-4-fluoro-benzyl)-piperidin-4-yiamino]-benzooxazol-5-yloxy}-cyclobutanecarboxylic acid

In analogy to the procedure described in example 5, 1-{2-[1-(3,5-diethoxy-4-fluoro-benzyl)-piperidin-4-ylamino]-benzooxazol-5-yloxy}-cyclobutanecarboxylic acid ethyl ester (example 128) was saponified to yield the title compound as light yellow solid.
MS: 528.2 (M+H)⁺.

### Example 131

### 1-{2-[1-(3,5-Diethoxy-4-pyrrol-1-yl-benzyl)-piperidin-4-ylamino]-benzooxazol-5-yloxy}-cyclobutanecarboxylic acid

In analogy to the procedure described in example 5, 1-{2-[1-(3,5-diethoxy-4-pyrrol-1-yl-benzyl)-piperidin-4-ylamino]-benzooxazol-5-yloxy}-cyclobutanecarboxylic acid ethyl ester (example 129) was saponified to yield the title compound as light yellow solid.
MS: 575.4 (M+H)⁺.

### Example 132

### N-{2-[1-(3,5-Diethoxy-4-fluoro-benzyl)-piperidin-4-ylamino]-benzooxazol-5-ylmethyl }-acetamide

In analogy to the procedure described in example 6], (5-aminomethyl-benzooxazol-2-yl)-[1-(3,5-diethoxy-4-fluoro-benzyl)-piperidin-4-yl]-amine (example 112) was reacted with acetic acid, N-(3-dimethylamino-propyl)-*N*'-ethyl-carbodiimide-hydrochloride and *N,N*-dimethyl-4-aminopyridine in *N,N-*dimethylformamide to yield the title compound as colorless solid.
MS: 485.3 (M+H)⁺.

### Example 133

### N-{2-[1-(3,5-Diethoxy-4-fluoro-benzyl)-piperidin-4-ylamino]-benzooxazol-5-ylmethyl}-malonamic acid ethyl ester

In analogy to the procedure described in example 6], (5-aminomethyl-benzooxazol-2-yl)-[1-(3,5-diethoxy-4-fluoro-benzyl)-piperidin-4-yl]-amine (example 112) was reacted with malonic acid monoethyl ester, *N*-(3-dimethylamino-propyl)-*N'-*ethyl-carbodiimide-hydrochloride and *N,N*-dimethyl-4-aminopyridine in *N,N-*dimethylformamide to yield the title compound as light yellow oil.
MS: 557.3 (M+H)⁺.

### Example 134

### (S)-N-{2-[1-(3,5-Diethoxy-4-fluoro-benzyl)-piperidin-4-ylamino]-benzooxazol-5-ylmethyl}-2-hydroxy-propionamide

In analogy to the procedure described in example 6], (5-aminomethyl-benzooxazol-2-yl)-[1-(3,5-diethoxy-4-fluoro-benzyl)-piperidin-4-yl]-amine (example 112) was reacted with (S)-2-hydroxy-propionic acid, *N*-(3-dimethylamino-propyl)-*N*'-ethyl-carbodiimide-hydrochloride and *N,N*-dimethyl-4-aminopyridine in *N,N-*dimethylformamide to yield the title compound as light yellow solid.
MS: 513.3 (M-H)⁻.

### Example 135

### [1-(3,5-Diethoxy-4-fluoro-benzyl)-piperidin-4-yl]-(4-methyl-oxazolo[5,4-c]pyridin-2-yl)-amine

A] In analogy to the procedure described in example 1A], (4-methyl-oxazolo[5,4-c]pyridin-2-yl)-piperidin-4-yl-amine (example 135B]) was reacted with 3,5-diethoxy-4-fluoro-benzaldehyde (example 2H]), sodium cyanoborohydride, *N*-ethyl-diisopropylamine and acetic acid in ethanol at 50 °C to yield the title compound as colorless oil.
MS: 429.3 (M+H)⁺.
The (4-methyl-oxazolo[5,4-c]pyridin-2-yl)-piperidin-4-yl-amine used in example 135A] was synthesized as follows:

### B] (4-Methyl-oxazolo[5,4-c]pyridin-2-yl)-piperidin-4-yl-amine

The title compound has been prepared by the following reaction sequence: i) hydrogenation of 2-methyl-4-nitro-pyridin-3-ol [Journal of Organic Chemistry (1968), 33(1), 478-80] with H₂ and Pd/C (10%) in MeOH for 1 h at RT in analogy to the procedure described in example 10D] to give 4-amino-2-methyl-pyridin-3-ol; ii) reaction with potassium ethyl xanthogenate in MeOH at reflux for 31 h in analogy to the procedure described in example 10E] to give 4-methyl-oxazolo[5,4-*c*]pyridine-2-thiol; iii) reaction in thionylchloride with a catalytic amount of *N,N-*dimethylformamide at reflux to give 2-chloro-4-methyl-oxazolo[5,4-c]pyridine in analogy to the procedure described in example 1B]; iv) condensation with 4-amino-piperidine-carboxylic acid *tert-*butyl ester in *N*-ethyl-diisopropylamine / dichloromethane at RT in analogy to the procedure described in example 1C] to yield 4-(4-methyl-oxazolo[5,4-*c*]pyridin-2-ylamino)-piperidine-1-carboxylic acid *tert*-butyl ester; v) Boc cleavage in analogy to the procedure described in example 1D].

### Example 136

### [1-(3,5-Diethoxy-4-pyrrol-1-yl-benzyl)-piperidin-4-yl]-(4-methyl-oxazolo[5,4-c]pyridin-2-yl)-amine

In analogy to the procedure described in example 1A], (4-methyl-oxazolo[5,4-c]pyridin-2-yl)-piperidin-4-yl-amine (example 135B]) was reacted with 3,5-diethoxy-4-pyrrol-1-yl-benzaldehyde (example 13C]), sodium cyanoborohydride, *N*-ethyl-diisopropylamine and acetic acid in ethanol at 50 °C to yield the title compound as light yellow oil.
MS:476.1 (M+H)⁺.

### Example 137

### [1-(3,5-Diisopropoxy-benzyl)-piperidin-4-yl]-(4-methyl-oxazolo[5,4-c]pyridin-2-yl)-amine

In analogy to the procedure described in example 1A], (4-methyl-oxazolo[5,4-c]pyridin-2-yl)-piperidin-4-yl-amine (example 135B]) was reacted with 3,5-diisopropoxy-benzaldehyde (prepared in analogy to the procedure described in example 2F], by reaction of 3,5-dihydroxybenzaldehyde with 2-iodo-propane in DMF using K₂CO₃ as base), sodium cyanoborohydride, *N*-ethyl-diisopropylamine and acetic acid in ethanol at 50 °C to yield the title compound as colorless foam.
MS: 439.3 (M+H)⁺.

### Example 138

### N-{2-[1-(3,5-Diethoxy-4-fluoro-benzyl)-piperidin-4-ylamino]-benzooxazol-5-ylmethyl}-malonamic acid

In analogy to the procedure described in example 5, *N*-{2-[1-(3,5-diethoxy-4-fluoro-benzyl)-piperidin-4-ylamino]-benzooxazol-5-ylmethyl}-malonamic acid ethyl ester (example 133) was saponified to yield the title compound as light yellow solid.
MS: 529.2 (M+H)⁺.

### Example 139

### [rac]-3-{2-1-(3,5-Diethoxy-4-fluoro-benzyl)-piperidin-4-ylamino]-benzooxazol-5-yloxy}-propane-1,2-diol

In analogy to the procedure described in example 1A], crude [rac]-3-[2-(piperidin-4-ylamino)-benzooxazol-5-yloxy]-propane-1,2-diol trifluoroacetate (prepared from 4-(5-hydroxy-benzooxazol-2-ylamino)-piperidine-1-carboxylic acid *tert*-butyl ester (example 99B]) and [rac]-2,2-dimethyl-1,3-dioxolan-4-yl-methyl p-toluenesulfonate followed by cleavage of the protective groups in analogy to the procedures described in examples 119B-C]) was reacted with 3,5-diethoxy-4-fluoro-benzaldehyde (example 2H]), sodium cyanoborohydride, *N*-ethyl-diisopropylamine and acetic acid in ethanol at 50 °C to yield the title compound as light yellow foam.
MS: 504.3 (M+H)⁺.

### Example 140

### [rac]-3-{2-[1-(3,5-Diethoxy-4-pyrrol-1-yl-benzyl)-piperidin-4-ylamino]-benzooxazol-5-yloxy}-propane-1,2-diol

In analogy to the procedure described in example 1A], crude [rac]-3-[2-(piperidin-4-ylamino)-benzooxazol-5-yloxy]-propane-1,2-diol trifluoroacetate (example 139) was reacted with 3,5-diethoxy-4-pyrrol-1-yl-benzaldehyde (example 13C]), sodium cyanoborohydride, *N*-ethyl-diisopropylamine and acetic acid in ethanol at 50 °C to yield the title compound as light yellow solid.
MS: 551.3 (M+H)⁺.

### Example 141

### [rac]-3-{2-[1-(3,5-Diisopropoxy-benzyl)-piperidin-4-ylamino]-benzooxazol-5-yloxy]-propane-1,2-diol

In analogy to the procedure described in example 1A], crude [rac]-3-[2-(piperidin-4-ylamino)-benzooxazol-5-yloxy]-propane-1,2-diol trifluoroacetate (example 139) was reacted with 3,5-diisopropoxy-benzaldehyde (prepared in analogy to the procedure described in example 2F], by reaction of 3,5-dihydroxybenzaldehyde with 2-iodo-propane in DMF using K₂CO₃ as base), sodium cyanoborohydride, *N*-ethyl-diisopropylamine and acetic acid in ethanol at 50 °C to yield the title compound as light yellow solid.
MS: 514.4 (M+H)⁺.

### Example 142

### [1-(3,5-Diethoxy-4-pyrrol-1-yl-benzyl)-piperidin-4-yl]-[5-(1H-tetrazol-5-ylmethoxy)-benzooxazol-2-yl]-amine

A] In analogy to the procedure described in example 1A], piperidin-4-yl-[5-(1*H-*tetrazol-5-ylmethoxy)-benzooxazol-2-yl]-amine hydrochloride (example 142C]) was reacted with 3,5-diethoxy-4-pyrrol-1-yl-benzaldehyde (example 13C]), sodium cyanoborohydride, *N*-ethyl-diisopropylamine and acetic acid in ethanol at 50°C to yield the title compound as light brown solid.
MS: 559.4 (M+H)⁺.
The piperidin-4-yl-[5-(1*H*-tetrazol-5-ylmethoxy)-benzooxazol-2-yl]-amine hydrochloride used in example 142A] was synthesized as follows:

### B] (1-Trityl-1H-tetrazol-5-yl)-methanol and/or (2-trityl-2H-tetrazol-5-yl)-methanol

2.55 g (25.5 mMol) of (1*H*-tetrazol-5-yl)-methanol (PCT Int. Appl. WO 98/14450 A1) was suspended under Ar in 30 mLofTHF at RT; while stirring, 2.71 g (1.05 eq.) of triethylamine was added. Then, 7.45 g (1.05 eq.) of triphenyl-chloromethane dissolved in 30 mL of THF was added at 40 °C within 5 min. Subsequently, the reaction mixture was stirred at 40 °C for 2 h. Then, it was cooled down, poured into 50 mL of ice cold water and extracted tree times with 100 mL of ethylacetate; the organic phases were washed with water, dried over MgSO₄, filtered and evaporated i.V. The crude product was purified by chromatography (silicagel, eluent: gradient of ethylacetate / heptane) to yield 7.3 g of the title compound as colorless cristals.
MS: 342.1 (M)⁺.

### C] Piperidin-4-yl-[5-1H-tetrazol-5-ylmethoxy)-benzooxazol-2-yl]-amine hydrochloride

0.52 g (0.9 mMol) of piperidin-4-yl-[5-(1-trityl-1*H*-tetrazol-5-ylmethoxy)-benzooxazol-2-yl]-amine and/or piperidin-4-yl-[5-(2-trityl-2*H*-tetrazol-5-ylmethoxy)-benzooxazol-2-yl]-amine (prepared from 4-(5-hydroxy-benzooxazol-2-ylamino)-piperidine-1-carboxylic acid *tert*-butyl ester (example 99B]) and (1-trityl-1*H*-tetrazol-5-yl)-methanol and/or (2-trityl-2*H*-tetrazol-5-yl)-methanol, triphenylphosphine , di*-tert-*butyl azodicarboxylate in THF in analogy to example 28G] and subsequent Boc cleavage with trifluoroacetic acid in analogy to example 1D]) were dissolved in 5 ml of MeOH. While stirring. 10.4 ml (46 mMol) of HCl in MeOH (4.5 molar) was added. After two hours, the solvent was removed and the residue was dried in high vacuum at 40 °C to yield 0.64 g of the crude title compound as off-white solid.
MS: 316.2 (M+H)⁺.

### Example 143

### [1-(3,5-Diethoxy-4-fluoro-benzyl)-piperidin-4-yl]-[5-(1H-tetrazol-5-ylmethoxy)-benzooxazol-2-yl] -am ine

In analogy to the procedure described in example 1A], piperidin-4-yl-[5-(1*H-*tetrazol-5-ylmethoxy)-benzooxazol-2-yl]-amine hydrochloride (example 142C]) was reacted with 3,5-diethoxy-4-fluoro-benzaldehyde (example 2H]), sodium cyanoborohydride, *N*-ethyl-diisopropylamine and acetic acid in ethanol at 50 °C to yield the title compound as colorless solid.
MS: 512.2 (M+H)⁺.

### Example 144

### {2-[1-(3,5-Diethoxy-4-fluoro-benzyl)-piperidin-4-ylamino]-oxazolo[5,4-c]pyridin-4-yl}-methanol

A] In analogy to the procedure described in example 1A], [2-(piperidin-4-ylamino)-oxazolo[5,4-*c*]pyridin-4-yl]-methanol (example 144D]) was reacted with 3,5-diethoxy-4-fluoro-benzaldehyde (example 2H]), sodium cyanoborohydride, *N*-ethyl-diisopropylamine and acetic acid in ethanol at 50 °C to yield the title compound as colorless oil.
MS: 445.1 (M+H)⁺.
The [2-(piperidin-4-ylamino)-oxazolo[5,4-*c*]pyridin-4-yl]-methanol used in example 144A] was synthesized as follows:

### B] 4-(4-Methyl-5-oxy-oxazolo[5,4-c]pyridin-2-ylamino)-piperidine-1-carboxylic acid tert-butyl ester

0.33 g (1.0 mMol) of 4-(4-methyl-oxazolo[5,4-*c*]pyridin-2-ylamino)-piperidine-1-carboxylic acid *tert*-butyl ester (example 135B]) was dissolved in 15 ml of MeCl₂ and cooled to 5 °C. 0.49 g (2 mmol) of 3-chloroperbenzoic acid was added and the solution warmed up to ambient temperature and stirred for 16 hours. Then, the reaction mixture was poured into crashed ice, the pH was adjusted to 7-8 with sodium hydrogen carbonate solution and the mixture was extracted twice with MeCl₂. The organic layers were dried over MgSO₄, filtered and evaporated. The crude product was purified by chromatography (silicagel, eluent: gradient of MeCl₂ / MeOH) to yield 0.31 g of the title compound as yellow oil.
MS: 349.4 (M+H)⁺.

### C] 2,2,2-Trifluoro-1-[4-(4-hydroxymethyl-oxazolo[5,4-c]pyridin-2-ylamino)-piperidin-1-yl]-ethanone

0.25 g (0.7 mMol) of 4-(4-methyl-5-oxy-oxazolo[5,4-*c*]pyridin-2-ylamino)-piperidine-1-carboxylic acid *tert*-butyl ester was dissolved under Argon in 3 ml of MeCl₂, 1.49 ml (2.25 g = 10.5 mMol) of trifluoroacetic anhydride was added and the reaction vessel was closed with a septum; this reaction mixture was heated over night at 45 °C, then cooled down to room temperature and evaporated. The crude product was purified by chromatography (silicagel, eluent: gradient of MeCl₂ / MeOH) to yield 0.24g of the title compound as light yellow solid.
MS: 344.0 (M)⁺.

### D] [2-(Piperidin-4-ylamino)-oxazolo[5,4-c]pyridin-4-yl]-methanol

0.22 g (0.6 mMol) of 2,2,2-trifluoro-1-[4-(4-hydroxymethyl-oxazolo[5,4-*c*]pyridin-2-ylamino)-piperidin-1-yl]-ethanone was dissolved at room temperature in 23 mL of MeOH; while stirring, a solution of 0.46 g (3.3 mMol) of potassium carbonate in 1.3 mL of water was added. After 30 minutes, the methanol was removed by evaporation and the residue extracted ten times with MeCl₂ / 2-propanol (4:1); the organic layers were dried over MgSO₄, filtered and evaporated to yield 0.11 g of the crude title compound as light yellow solid.
MS: 249.0 (M+H)⁺.

### Example 145

### {2-[1-(3,5-Diethoxy-4-pyrrol-1-yl-benzyl)-piperidin-4-ylamino]-oxazolo[5,4-c]pyridin-4-yl}-methanol

In analogy to the procedure described in example 1A], [2-(piperidin-4-ylamino)-oxazolo[5,4-c]pyridin-4-yl]-methanol (example 144D]) was reacted with 3,5-diethoxy-4-pyrrol-1-yl-benzaldehyde (example 13C]), sodium cyanoborohydride, N-ethyl-diisopropylamine and acetic acid in ethanol at 50 °C to yield the title compound as yellow oil.
MS: 492.3 (M+H)⁺.

### Example 146

### {2-[1-(3,5-Diisopropoxy-benzyl)-piperidin-4-ylamino]-oxazolo[5,4-c]pyridin-4-yl}-methanol

In analogy to the procedure described in example 1A], [2-(piperidin-4-ylamino)-oxazolo[5,4-c]pyridin-4-yl]-methanol (example 144D]) was reacted with 3,5-diisopropoxy-benzaldehyde (prepared in analogy to the procedure described in example 2F], by reaction of 3,5-dihydroxybenzaldehyde with 2-iodo-propane in DMF using K₂CO₃ as base), sodium cyanoborohydride, N-ethyl-diisopropylamine and acetic acid in ethanol at 50 °C to yield the title compound as colorless solid.
MS: 455.4 (M+H)⁺.

### Example 147

### {2-[1-(3,5-Diethoxy-4-[1,2,4]triazol-1-yl-benzyl)-piperidin-4-ylamino]-oxazolo[5,4-c]pyridin-4-yl}-methanol

A] In analogy to the procedure described in example 1A], [2-(piperidin-4-ylamino)-oxazolo[5,4-c]pyridin-4-yl]-methanol (example 144D]) was reacted with 3,5-diethoxy-4-[1,2,4]triazol-1-yl-benzaldehyde (example 147B]), sodium cyanoborohydride, *N*-ethyl-diisopropylamine and acetic acid in ethanol at 50 °C to yield the title compound as light yellow solid.
MS: 494.3 (M+H)⁺.
The 3,5-diethoxy-4-[1,2,4]triazol-1-yl-benzaldehyde used in example 147A] was synthesized as follows:

### B] 3,5-Diethoxy-4- 1,2,4]triazol-1-yl-benzaldehyde

5.00 g (23.6 mMol) of 3,5-diethoxy-4-fluoro-benzaldehyde (example 2H]), 3.25 g (= 2.0 eq.) of 1,2,4-triazole and 6.51 g (= 2.0 eq.) of potassium carbonate were dissolved under argon in 50 mL of DMSO; the reaction mixture was stirred for 1 hour at 110 °C. Then, it was cooled down to ambient temperature, poured into crashed ice and extracted twice with ethyl acetate. The organic phases were washed with water, dried over MgSO₄, filtered and evaporated i.V. The crude product was purified by chromatography (silicagel, eluent: gradient of n-heptane / ethyl acetate) to yield 5.28 a of the title compound as colorless solid.
MS: 261.9 (M+H)⁺.

### Example 148

### {2-[1-(3,5-Diethoxy-4-fluoro-benzyl)-piperidin-4-ylamino]-benzooxazol-5-yl }-acetic acid methyl ester

In analogy to the procedure described in example 1A], [2-(piperidin-4-ylamino)-benzooxazol-5-yl]-acetic acid methyl ester (prepared by condensation of (2-chloro-benzooxazol-5-yl)-acetic acid methyl ester [PCT Int. Appl. WO 2000/006566 A1] with 4-amino-piperidine-carboxylic acid *tert*-butyl ester in *N*-ethyl-diisopropylamine / dichloromethane at RT to 40 °C in analogy to the procedure described in example 1C] to yield 4-(5-methoxycarbonylmethyl-benzooxazol-2-ylamino)-piperidine-1-carboxylic acid *tert*-butyl ester, followed by Boc cleavage in analogy to the procedure described in example 1D]) was reacted with 3,5-diethoxy-4-fluoro-benzaldehyde (example 2H]), sodium cyanoborohydride, *N*-ethyl-diisopropylamine and acetic acid in ethanol at 50 °C to yield the title compound as colorless amorphous solid.
MS: 486.3 (M+H)⁺.

### Example 149

### {2-[1-(3,5-Diethoxy-4-pyrrol-1-yl-benzyl)-piperidin-4-ylamino]-benzooxazol-5-yl}-acetic acid methyl ester

In analogy to the procedure described in example 1A], [2-(piperidin-4-ylamino)-benzooxazol-5-yl]-acetic acid methyl ester (example 148) was reacted with 3,5-diethoxy-4-pyrrol-1-yl-benzaldehyde (example 13C]), sodium cyanoborohydride, *N*-ethyl-diisopropylamine and acetic acid in ethanol at 50 °C to yield the title compound as light brown oil.
MS: 533.4 (M+H)⁺.

### Example 150

### {2-[1-(3,5-Diisopropoxy-benzyl)-piperidin-4-ylamino]-benzooxazol-5-yl}-acetic acid methyl ester

In analogy to the procedure described in example 1A], [2-(piperidin-4-ylamino)-benzooxazol-5-yl]-acetic acid methyl ester (example 148) was reacted with 3,5-diisopropoxy-benzaldehyde (prepared in analogy to the procedure described in example 2F], by reaction of 3,5-dih ydroxybenzaldehyde with 2-iodo-propane in DMF using K₂CO₃ as base), sodium cyanoborohydride, *N*-ethyl-diisopropylamine and acetic acid in ethanol at 50 °C to yield the title compound as colorless amorphous solid.
MS:496.1 (M+H)⁺.

### Example 151

### {2-[1-(3,5-Diethoxy-4-[1,2,4]triazol-1-yl-benzyl)-piperidin-4-ylamino] -ben zooxazol-5-yl}-acetic acid methyl ester

In analogy to the procedure described in example 1A], [2-(piperidin-4-ylamino)-benzooxazol-5-yl]-acetic acid methyl ester (example 148) was reacted with 3,5-diethoxy-4-[1,2,4]triazol-1-yl-benzaldehyde (example 147B]), sodium cyanoborohydride, *N*-ethyl-diisopropylamine and acetic acid in ethanol at 50°C to yield the title compound as colorless amorphous solid.
MS: 535.4 (M+H)⁺.

### Example 152

### {2-[1-(3,5-Diethoxy-4-fluoro-benzyl)-piperidin-4-ylamino]-benzooxazol-5-yl}-acetic acid

In analogy to the procedure described in example 5, {2-[1-(3,5-diethoxy-4-fluorobenzyl)-piperidin-4-ylamino]-benzooxazol-5-yl}-acetic acid methyl ester (example 148) was saponified to yield the title compound as light yellow solid.
MS: 472.0 (M+H)⁺.

### Example 153

### {2-[1-(3,5-Diethoxy-4-pyrrol-1-yl-benzyl)-piperidin-4-ylamino]-benzooxazol-5-yl}-acetic acid

In analogy to the procedure described in example 5, {2-[1-(3,5-diethoxy-4-pyrrol-1-yl-benzyl)-piperidin-4-ylamino]-benzooxazol-5-yl}-acetic acid methyl ester (example 149) was saponified to yield the title compound as yellow solid.
MS: 519.3 (M+H)⁺.

### Example 154

### {2-[1-(3,5-Diisopropoxy-benzyl)-piperidin-4-ylamino]-benzooxazol-5-yl}-acetic acid

In analogy to the procedure described in example 5, {2-[1-(3,5-diisopropoxy-benzyl)-piperidin-4-ylamino]-benzooxazol-5-yl}-acetic acid methyl ester (example 150) was saponified to yield the title compound as colorless solid.
MS: 482.3 (M+H)⁺.

### Example 155

### {2-[1-(3,5-Diethoxy-4-[1,2,4] triazol-1-yl-benzyl)-piperidin-4-ylamino]-benzooxazol-5-yl}-acetic acid

In analogy to the procedure described in example 5, {2-[11-(3,5-diethoxy-4-[1,2,4]triazol-1-yl-benzyl)-piperidin-4-ylamino]-benzooxazol-5-yl}-acetic acid methyl ester (example 151) was saponified to yield the title compound as colorless solid.
MS: 521.3 (M+H)⁺.

### Example 156

### [1-(3,5-Diethoxy-4-fluoro-benzyl)-piperidin-4-yl]-(5-methoxy-benzooxazol-2-yl)-amine

In analogy to the procedure described in example 1A], (5-methoxy-benzooxazol-2-yl)-piperidin-4-yl-amine (example 99B]) was reacted with 3,5-diethoxy-4-fluorobenzaldehyde (example 2H]), sodium cyanoborohydride, *N*-ethyl-diisopropylamine and acetic acid in ethanol at 50 °C to yield the title compound as light yellow solid.
MS: 444.2 (M+H)⁺.

### Example 157

### [1-(3,5-Diethoxy-4-pyrrol-1-yl-ben zyl)-piperidin-4-yl]-(5-methoxy-ben zooxazol-2-yl)-amine

In analogy to the procedure described in example 1A], (5-methoxy-benzooxazol-2-yl)-piperidin-4-yl-amine (example 99B]) was reacted with 3,5-diethoxy-4-pyrrol-1-yl-benzaldehyde (example 13C]), sodium cyanoborohydride, *N*-ethyl-diisopropylamine and acetic acid in ethanol at 50 °C to yield the title compound as light yellow solid.
**MS: 491.2 (M+H)⁺.**

### Example 158

### 2-[1-(3,5-Diethoxy-4-fluoro-benzyl)-piperidin-4-ylamino]-benzooxazole-5-carboxylic acid methyl ester

In analogy to the procedure described in example 1A], 2-(piperidin-4-ylamino)-benzooxazole-5-carboxylic acid methyl ester (prepared by condensation of 2-chloro-benzooxazole-5-carboxylic acid methyl ester (Journal of Organic Chemistry (1996), 61 (10), 3289-97) with 4-amino-piperidine-carboxylic acid *tert*-butyl ester in *N-*ethyl-diisopropylamine / dichloromethane at RT to 40 °C in analogy to the procedure described in example 1C] to yield 2-(1-*tert*-butoxycarbonyl-piperidin-4-ylamino)-benzooxazole-5-carboxylic acid methyl ester, followed by Boc cleavage in analogy to the procedure described in example 1D]) was reacted with 3,5-diethoxy-4-fluoro-benzaldehyde (example 2H]), sodium cyanoborohydride, *N-*ethyl-diisopropylamine and acetic acid in ethanol at 50 °C to yield the title compound as colorless amorphous solid.
**MS: 472.0 (M+H)⁺.**

### Example 159

### 2-[1-(3,5-Diethoxy-4-pyrrol-1-yl-benzyl)-piperidin-4-ylamino]-benzooxazole-5-carboxylic acid methyl ester

In analogy to the procedure described in example 1A], 2-(piperidin-4-ylamino)-benzooxazole-5-carboxylic acid methyl ester (example 158) was reacted with 3,5-diethoxy-4-pyrrol-1-yl-benzaldehyde (example 13C]), sodium cyanoborohydride, *N-*ethyl-diisopropylamine and acetic acid in ethanol at 50°C to yield the title compound as colorless amorphous solid.
**MS: 519.3 (M+H)⁺.**

### Example 160

### 2-[1-(3,5-Diisopropoxy-benzyl)-piperidin-4-ylamino]-benzooxazole-5-carboxylic acid methyl ester

In analogy to the procedure described in example 1A], 2-(piperidin-4-ylamino)-benzooxazole-5-carboxylic acid methyl ester (example 158) was reacted with 3,5-diisopropoxy-benzaldehyde (prepared in analogy to the procedure described in example 2F], by reaction of 3,5-dihydroxybenzaldehyde with 2-iodo-propane in DMF using K₂CO₃ as base), sodium cyanoborohydride, *N-*ethyl-diisopropylamine and acetic acid in ethanol at 50 °C to yield the title compound as light yellow solid.
**MS: 482.3 (M+H)⁺.**

### Example 161

### 2-[1-(3,5-Diethoxy-4-[1,2,4]triazol-1-yl-benzyl)-piperidin-4-ylamino]-benzooxazole-5-carboxylic acid methyl ester

In analogy to the procedure described in example 1A], 2-(piperidin-4-ylamino)-benzooxazole-5-carboxylic acid methyl ester (example 158) was reacted with 3,5-diethoxy-4-[1,2;4]triazol-1-yl-benzaldehyde (example 147B]), sodium cyanoborohydride, *N-*ethyl-diisopropylamine and acetic acid in ethanol at 50 °C to yield the title compound as light yellow solid.
**MS: 521.3 (M+H)⁺.**

### Example 162

### 2-[1-(3,5-Diethoxy-4-fluoro-benzyl)-piperidin-4-ylamino]-benzooxazole-5-carboxylic acid

In analogy to the procedure described in example 5,2-[1-(3,5-diethoxy-4-fluorobenzyl)-piperidin-4-ylamino]-benzooxazole-5-carboxylic acid methyl ester (example 158) was saponified at 50°C to yield the title compound as colorless solid.
**MS: 458.2 (M+H)⁺.**

### Example 163

### 2-[1-(3,5-Diethoxy-4-pyrrol-1-yl-benzyl)-piperidin-4-ylamino]-ben zooxazole-5-carboxylic acid

In analogy to the procedure described in example 5, 2-[1-(3,5-diethoxy-4-pyrrol-1-yl-benzyl)-piperidin-4-ylamino]-benzooxazole-5-carboxylic acid methyl ester (example 159) was saponified at 50°C to yield the title compound as light yellow solid.
**MS: 505.2 (M+H)⁺.**

### Example 164

### 2-[1-(3,5-Diisopropoxy-benzyl)-piperidin-4-ylamino]-benzooxazole-5-carboxylic acid

In analogy to the procedure described in example 5, 2-[1-(3,5-diisopropoxy-benzyl)-piperidin-4-ylamino]-benzooxazole-5-carboxylic acid methyl ester (example 160) was saponified at 50°C to yield the title compound as light yellow solid.
**MS: 468.2 (M+H)⁺.**

### Example 165

### 2-[1-(3,5-Diethoxy-4-[1,2,4]triazol-1-yl-benzyl)-piperidin-4-ylamin o]-ben zooxazole-5-carboxylic acid

In analogy to the procedure described in example 5, 2-[1-(3,5-diethoxy-4-[1,2,4]triazol-1-yl-benzyl)-piperidin-4-ylamino]-benzooxazole-5-carboxylic acid methyl ester (example 161) was saponified at 50°C to yield the title compound as colorless solid.
**MS: 507.3.0 (M+H)⁺.**

### Example 166

### 2-[1-(4-Chloro-3,5-diethoxy-ben zyl)-piperidin-4-ylamino]-ben zooxazole-5-carboxylic acid methyl ester

A] In analogy to the procedure described in example 1A], 2-(piperidin-4-ylamino)-benzooxazole-5-carboxylic acid methyl ester (example 158) was reacted with 4-chloro-3,5-diethoxy-benzaldehyde (prepared from 4-chloro-3,5-diethoxy-benzoic acid ethyl ester (example 166B]) by reduction with di-isobutylaluminium hydride followed by oxidation with MnO₂ in analogy to the procedures described in example 14B]), sodium cyanoborohydride, *N-*ethyl-diisopropylamine and acetic acid in ethanol at 50 °C to yield the title compound as light yellow solid.
**MS: 488.1 (M+H)⁺.**
The 4-chloro-3,5-diethoxy-benzoic acid ethyl ester used in example 166A] was synthesized as follows:

### B1 4-Chloro-3,5-diethoxy-benzoic acid ethyl ester

To a solution of 4-amino-3,5-diethoxy-benzoic acid ethyl ester (5.1g, 20.13 mMol, 1.0 eq.; prepared as described in I. Kompis, A. Wick, Helv. Chim. Acta 1977, 60, 3025-3034) in water (40 mL) and 37% hydrochloric acid (40 mL) at 0 °C was added sodium nitrite (1.67 g, 24.16 mMol, 1.2 eq.). After 10 min, copper(I) chloride (12.0 g, 120.81 mMol, 6.0 eq.) was added, the reaction mixture stirred for an additional 5 h at 0°C and then the ice bath was removed. After stirring for 18 h the crude reaction mixture was adjusted to pH = 8 by addition of a solution of 1 M NaOH and the aqueous layer extraced with ethyl acetate (3 x 100 mL). The combined organic phases were dried over MgSO₄, concentrated by evaporation under reduced pressure and the crude material purified with silica column chromatography using a MPLC system (CombiFlash Companion, Isco Inc.) eluting with a gradient of heptane/ethyl acetate providing 5.0 g (91%) of the title compound as off-white solid.

### MS: 273.3 (M+H)⁺.

### Example 167

### 2-[1-(2,6-Diethoxy-4'-fluoro-biphenyl-4-ylmethyl)-piperidin-4-ylamino]-benzooxazole-5-carboxylic acid methyl ester

A] In analogy to the procedure described in example 1A], 2-(piperidin-4-ylamino)-benzooxazole-5-carboxylic acid methyl ester (example 158) was reacted with 2,6-diethoxy-4'-fluoro-biphenyl-4-carbaldehyde (example 167B]), sodium cyanoborohydride, *N-*ethyl-diisopropylamine and acetic acid in ethanol at 50 °C to yield the title compound as light yellow solid.
MS: 548.3 (M+H)⁺.
The 2,6-diethoxy-4'-fluoro-biphenyl-4-carbaldehyde used in example 167A] was synthesized as follows:

### B1 2,6-Diethoxy-4'-fluoro-biphenyl-4-carbaldehyde

3,5-Diethoxy-4-iodobenzaldehyde (0.760 g, 2.37 mMol, CAS 338454-05-0, PCT Int. Appl. WO 2001/032633A1) was dissolved under Ar in 12 mL of abs. DMF and, treated successively with 4-fluorophenyl boronic acid (0.399 g, 1.2 eq.), K₃PO₄ (0.857 g, 1.7 eq.), and Pd(PPh₃)₄ (0.082 g, 0.03 eq.). The mixture was allowed to react for 16 h at 80°C. Pouring onto crashed ice / NH₄Cl, twofold extraction with AcOEt, washing with brine and water, drying over magnesium sulfate, and evaporation of the solvents, followed by flash chromatography (silica gel, hexane/AcOEt=95/5) left finally 0.505 g of the title compound as light yellow solid.
**MS: 288.2 (M)⁺.**

### Example 168

### 2-[1-(4-Chloro-3,5-diethoxy-benzyl)-piperidin-4-ylamino]-benzooxazole-5-carboxylic acid

In analogy to the procedure described in example 5, 2-[1-(4-chloro-3,5-diethoxy-benzyl)-piperidin-4-ylamino]-benzooxazole-5-carboxylic acid methyl ester (example 166) was saponified at 50°C to yield the title compound as off white solid.
**MS: 474.0 (M+H)⁺.**

### Example 169

### 2-[1-(2,6-Diethoxy-4'-fluoro-biphenyl-4-ylmethyl)-piperidin-4-ylamino]-benzooxazole-5-carboxylic acid

In analogy to the procedure described in examples 5, 2-[1-(2,6-diethoxy-4'-fluoro-biphenyl-4-ylmethyl)-piperidin-4-ylamino]-benzooxazole-5-carboxylic acid methyl ester (example 167) was saponified at 50 °C to yield the title compound as off-white solid.
**MS: 534.3 (M+H)⁺.**

### Example 170

### {2-[1-(3,5-Diethoxy-4-fluoro-benzyl)-piperidin-4-ylamino]-benzooxazol-5-yloxy}-acetonitrile

A] In analogy to the procedure described example 1.A], [2-(piperidin-4-ylamino)-benzooxazol-5-yloxy]-acetonitrile (prepared from 4-(5-cyanomethoxy-benzooxazol-2-ylamino)-piperidine-1-carboxylic acid *tert*-butyl ester (example 170B]) by treatment with trifluoroacetic acid in dichloromethane in analogy to the procedure described in example 1D], obtained as major product) was reacted with 3,5-diethoxy-4-fluoro-benzaldehyde (example 2H]), sodium cyanoborohydride, *N-*ethyl-diisopropylamine and acetic acid in ethanol at 50°C to yield the title compound as yellow gum.
**MS: 469.3 (M+H)⁺.**
The 4-(5-cyanomethoxy-benzooxazol-2-ylamino)-piperidine-1-carboxylic acid *tert*-butyl ester used in example 170A] was synthesized as follows:

### B1 4-(5-Cyanomethoxy-benzooxazol-2-ylamino)-piperidine-1-carboxylic acid tert-butyl ester

3.67 g (11.0 mMol) of 4-(5-hydroxy-benzooxazol-2-ylamino)-piperidine-1-carboxylic acid *tert*-butyl ester (example 99B]) was dissolved in 100 mL of acetone and 2.31 g (16.5 mMol) of anhydrous potassium carbonate was added, followed by.0.83 mL (1.50 g = 12.1 mMol) of bromoacetonitrile. The reaction mixture was stirred for 4 hours at reflux. After cooling down to RT, the crude reaction mixture was concentrated by evaporation under reduced pressure. The residue was then poured into crashed ice, acidified to pH 2-3 with HCl (2N) and extracted twice with EtOAc. The organic phases were washed with water and brine, dried over MgSO₄, filtered and evaporated i.V. and the residue (3.92 g) was purified by chromatography (SiO₂ MeCl₂ / MeOH) to yield 2.32 g of the title compound as light yellow foam.
**MS: 373.3 (M+H)⁺.**

### Example 171

### N-tert-Butyl-2-{2-[1-(3,5-diethoxy-4-fluoro-benzyl)-piperidin-4-ylamino]-benzooxazol-5-yloxy}-acetamide

In analogy to the procedure described example 1A], *N-tert*-butyl-2-[2-(piperidin-4-ylamino)-benzooxazol-5-yloxy]-acetamide (prepared from 4-(5-cyanomethoxy-benzooxazol-2-ylamino)-piperidine-1-carboxylic acid *tert*-butyl ester (example 170B] by treatment with trifluoroacetic acid in dichloromethane in analogy to the procedure described in example 1D], obtained as minor product) was reacted with 3,5-diethoxy-4-fluoro-benzaldehyde (example 2H]), sodium cyanoborohydride, *N-*ethyl-diisopropylamine and acetic acid in ethanol at 50°C to yield the title compound as colorless solid.
**MS: 543.5 (M+H)⁺.**

### Example 172

### {2-[1-(3,5-Diisopropoxy-benzyl)-piperidin-4-ylamino]-benzooxazol-5-yloxy}-acetonitrile

In analogy to the procedure described example 1A], [2-(piperidin-4-ylamino)-benzooxazol-5-yloxy]-acetonitrile (example 170A]) was reacted with 3,5-diisopropoxy-benzaldehyde (prepared in analogy to the procedure described in example 2F], by reaction of 3,5-dihydroxybenzaldehyde with 2-iodo-propane in DMF using K₂CO₃ as base), sodium cyanoborohydride, *N-*ethyl-diisopropylamine and acetic acid in ethanol at 50 °C to yield the title compound as yellow gum.
**MS: 479.2 (M+H)⁺.**

### Example 173

### N-tert-Butyl-2-{2-[1-(3,5-diisopropoxy-benzyl)-piperidin-4-ylamino]-benzooxazol-5-yloxy}-acetamide

In analogy to the procedure described example 1A], *N-tert*-butyl-2-[2-(piperidin-4-ylamino)-benzooxazol-5-yloxy]-acetamide (example 171) was reacted with 3,5-diisopropoxy-benzaldehyde (prepared in analogy to the procedure described in example 2F], by reaction of 3,5-dihydroxybenzaldehyde with 2-iodo-propane in DMF using K₁CO₂ as base), sodium cyanoborohydride, *N*-ethyl-diisopropylamine and acetic acid in ethanol at 50 °C to yield the title compound as colorless solid.
**MS: 553.4 (M+H)⁺.**

### Example 174

### {2-[1-(4-Chloro-3,5-diethoxy-benzyl)-piperidin-4-ylamino]-ben zooxazol-5-yloxy}-acetonitrile

In analogy to the procedure described example 1A], [2-(piperidin-4-ylamino)-benzooxazol-5-yloxy]-acetonitrile (example 170A]) was reacted with 4-chloro-3,5-diethoxy-benzaldehyde (example 166), sodium cyanoborohydride, *N-*ethyl-diisopropylamine and acetic acid in ethanol at 50 °C to yield the title compound as yellow gum.

### MS: 485.3 (M+H)⁺.

### Example 175

### N-tert-Butyl-2-{2-[1-(4-chloro-3,5-diethoxy-benzyl)-piperidin-4-ylamino]-benzooxazol-5-yloxy}-acetamide

In analogy to the procedure described example 1A], *N-tert*-butyl-2-[2-(piperidin-4-ylamino)-benzooxazol-5-yloxy]-acetamide (example 171) was reacted with 4-chloro-3,5-diethoxy-benzaldehyde (example 166), sodium cyanoborohydride, *N-*ethyl-diisopropylamine and acetic acid in ethanol at 50 °C to yield the title compound as light yellow solid.
**MS: 559.3 (M+H)⁺.**

### Example 176

### {2-[1-(2,6-Diethoxy-4'-fluoro-biphenyl-4-ylmethyl)-piperidin-4-ylamino]-benzooxazol-5-yloxy}-acetonitrile

In analogy to the procedure described example 1A], [2-(piperidin-4-ylamino)-benzooxazol-5-yloxy]-acetonitrile (example 170A]) was reacted with 2,6-diethoxy-4'-fluoro-biphenyl-4-carbaldehyde (example 167B]), sodium cyanoborohydride, *N-*ethyl-diisopropylamine and acetic acid in ethanol at 50 °C to yield the title compound as yellow gum,
**MS: 545.3 (M+H)⁺.**

### Example 177

### N-tert-Butyl-2-{2-[ 1-(2,6-diethoxy-4'-fluoro-biphenyl-4-ylmethyl)-piperidin-4-ylamino]-benzooxazol-5-yloxy}-acetamide

In analogy to the procedure described example 1A], *N-tert*-butyl-2-[2-(piperidin-4-ylamino)-benzooxazol-5-yloxy]-acetamide (example 171) was reacted with 2,6-diethoxy-4'-fluoro-biphenyl-4-carbaldehyde (example 167B]), sodium cyanoborohydride, *N-*ethyl-diisopropylamine and acetic acid in ethanol at 50 °C to yield the title compound as light yellow solid.
**MS: 619.4 (M+H)⁺.**

### Example 178

### 2-{2-[1-(2,6-Diethoxy-4'-fluoro-biphenyl-4-ylmethyl)-piperidin-4-ylamino]-benzooxazol-5-yloxy}-acetamide

A] In analogy to the procedure described example 1A], 2-[2-(piperidin-4-ylamino)-benzooxazol-5-yloxy]-acetamide (prepared from 4-(5-carbamoylmethoxy-benzooxazol-2-ylamino)-piperidine-1-carboxylic acid *tert*-butyl ester (example 178B]) by treatment with trifluoroacetic acid in analogy the procedure described in example 1D]) was reacted with 2,6-diethoxy-4'-fluoro-biphenyl-4-carbaldehyde (example 167B]), sodium cyanoborohydride, *N-*ethyl-diisopropylamine and acetic acid in ethanol at 50 °C to yield the title compound as colorless solid.
**MS: 563.4 (M+H)⁺.**
The 4-(5-carbamoylmethoxy-benzooxazol-2-ylamino)-piperidine-1-carboxylic acid *tert-*butyl ester used in example 178A] was synthesized as follows:

### B1 4-(5-Carbamoylmethoxy-benzooxazol-2-ylamino)-piperidine-1-carboxylic acid tert-butyl ester

1.08 g (2.9 mMol) of 4-(5-cyanomethoxy-benzooxazol-2-ylamino)-piperidine-1-carboxylic acid *tert*-butyl ester (example 170B]) and 0.081 g (0.6 mMol) of potassium carbonate were suspended under argon in 5 mL of DMSO at RT; while stirring, 0.50 mL (0.56 g = 2.0 eq.) of hydrogen peroxide solution (35% in water) was added below 25 °C and stirring continued at ambient temperature for 20 hours. Then, the reaction mixture was poured into crashed ice and extracted three times with MeCl₂ / 2-propanol (4:1); the organic phases were evaporated i.V. The crude product was purified by chromatography (SiO₂ MeCl₂ / MeOH) to yield 0.56 g of the title compound title compound as colorless solid.
**MS: 391.0 (M+H)⁺.**

### Example 179

### 2-{2-[1-(3,5-Diisopropoxy-benzyl)-piperidin-4-ylamino]-benzooxazol-5-yloxy}-acetamide

In analogy to the procedure described example 1A], 2-[2-(piperidin-4-ylamino)-benzooxazol-5-yloxy]-acetamide (example 178) was reacted with 3,5-diisopropoxy-benzaldehyde (prepared in analogy to the procedure described in example 2F], by reaction of 3,5-dihydroxybenzaldehyde with 2-iodo-propane in DMF using K₂CO₃ as base), sodium cyanoborohydride, *N-*ethyl-diisopropylamine and acetic acid in ethanol at 50°C to yield the title compound as-light yellow solid.
**MS:491.1 (M+H)⁺.**

### Example 180

### 2-{2-[1-(4-Chloro-3,5-diethoxy-benzyl)-piperidin-4-ylamino]-benzooxazol-5-yloxy}-acetamide

In analogy to the procedure described example 1A], 2-[2-(piperidin-4-ylamino)-benzooxazol-5-yloxy]-acetamide (example 178) was reacted with 4-chloro-3,5-diethoxy-benzaldehyde (example 166), sodium cyanoborohydride, *N-*ethyl-diisopropylamine and acetic acid in ethanol at 50°C to yield the title compound as colorless solid.
**MS: 503.1 (M+H)⁺.**

### Example 181

### 2-{2-[1-(3,5-Diethoxy-4-fluoro-benzyl)-piperidin-4-ylamino]-benzooxazol-5-yloxy}-acetamide

In analogy to the procedure described example 1A], 2-[2-(piperidin-4-ylamino)-benzooxazol-5-yloxy]-acetamide (example 178) was reacted with 3,5-diethoxy-4-fluorobenzaldehyde (example 2H]), sodium cyanoborohydride, *N*-ethyl-diisopropylamine and acetic acid in ethanol at 50 °C to yield the title compound as colorless solid.
**MS: 487.2 (M+H)⁺.**

### Example 182

### [rac]-3-{2-[1-(2,6-Diethoxy-4'-fluoro-biphenyl-4-ylmethyl)-piperidin-4-ylamino]-benzooxazol-5-yloxy}-propane-1,2-diol

In analogy to the procedure described in example 1A], crude [rac]-3-[2-(piperidin-4-ylamino)-benzooxazol-5-yloxy]-propane-1,2-diol trifluoroacetate (example 139) was reacted with 2,6-diethoxy-4'-fluoro-biphenyl-4-carbaldehyde (example 167B]), sodium cyanoborohydride, *N-*ethyl-diisopropylamine and acetic acid in ethanol at 50 °C to yield the title compound as light brown foam.
**MS: 580.2 (M+H)⁺.**

### Example 183

### [rac]-3-{2-[1-(4-Chloro-3,5-diethoxy-benzyl)-piperidin-4-ylamino]-benzooxazol-5-yloxy}-propane-1,2-diol

In analogy to the procedure described in example 1A], crude [rac]-3-[2-(piperidin-4-ylamino)-benzooxazol-5-yloxy]-propane-1,2-diol trifluoroacetate (example 139) was reacted with 4-chloro-3,5-diethoxy-benzaldehyde (example 166), sodium cyanoborohydride, *N-*ethyl-diisopropylamine and acetic acid in ethanol at 50 °C to yield the title compound as colorless foam.
**MS: 520.3 (M+H)⁺.**

### Example 184

### 2-{2-[1-(3,5-Diisopropoxy-benzyl)-piperidin-4-ylamino]-benzooxazol-5-yl}-ethanol

0.46 g (0.93 mMol) of {2-[1-(3,5-diisopropoxy-benzyl)-piperidin-4-ylamino]-benzooxazol-5-yl}-acetic acid methyl ester (example 150]) was suspended under argon in 30 mL of THF at RT and the mixture cooled down to 0 °C; while stirring, 1.86 mL (2.0 eq.) of a lithium aluminium hydride solution (1M in THF) was added drop by drop below 5°C. The reaction mixture was then warmed up to RT. After 1 hour, 1 mL of cold water was added, subsequently, it was acidified with HCl (1N) to pH 3-4, poured into crashed ice and extracted three times with MeCl₂. The organic phases were washed with water and brine, dried over MgSO₄ filtered and evaporated i.V. and the residue (0.34 g) was purified by chromatography (SiO₂ MeCl₂ / MeOH) to yield 0.28 g of the title compound as light yellow oil.
**MS: 468.2 (M+H)⁺.**

### Example 185

### 2-{2-[1-(3,5-Diethoxy-4-fluoro-benzyl)-piperidin-4-ylamino]-benzooxazol-5-yl}-ethanol

In analogy to the procedure described in example 184, {2-[1-(3,5-diethoxy-4-fluoro-benzyl)-piperidin-4-ylamino]-benzooxazol-5-yl}-acetic acid methyl ester (example 148) was reduced with lithium aluminium hydride in tetrahydrofuran to give the title compound as off-white solid.
**MS: 458.2 (M+H)⁺.**

### Example 186

### {2-[1-(3,5-Diethoxy-4-fluoro-benzyl)-piperidin-4-ylamino]-benzooxazol-5-yl}-methanol

In analogy to the procedure described in example 184], 2-[1-(3,5-diethoxy-4-fluoro-benzyl)-piperidin-4-ylamino]-benzooxazole-5-carboxylic acid methyl ester (example 158) was reduced with lithium aluminium hydride in tetrahydrofuran to give the title compound as colorless solid.
**MS: 444.2 (M+H)⁺.**

### Example 187

### [5-(2-Amino-ethoxy)-benzooxazol-2-yl]-[1-(3,5-diethoxy-4-fluoro-benzyl)-piperidin-4-yl]-amine

In analogy to the procedure described in example 112A], {2-[1-(3,5-diethoxy-4-fluoro-benzyl)-piperidin-4-ylamino]-benzooxazol-5-yloxy}-acetonitrile (example 170) was reduced with of borane-dimethyl sulfide complex in tetrahydrofuran to give the title compound as light brown oil.
**MS: 473.4 (M+H)⁺.**

### Example 188

### 2-[1-(2,6-Diethoxy-4'-fluoro-biphenyl-4-ylmethyl)-piperidin-4-ylamino]-benzooxazole-6-carboxylic acid

In analogy to the procedure described in example 1A], 2-(piperidin-4-ylamino)-benzooxazole-6-carboxylic acid methyl ester (example 55) was reacted with with 2,6-diethoxy-4'-fluoro-biphenyl-4-carbaldehyde (example 167B]), sodium cyanoborohydride, *N-*ethyl-diisopropylamine and acetic acid in ethanol at 50°C to yield 2-[1-(2,6-diethoxy-4'-fluoro-biphenyl-4-ylmethyl)-piperidin-4-ylamino]-benzooxazole-6-carboxylic acid methyl ester, which was subsequently saponified in analogy to the procedure described in example 5 to give the title compound as off-white amorphous solid.
**MS: 534.3 (M+H)⁺.**

### Example 189

### 2-[1-(4-Chloro-3,5-diethoxy-benzyl)-piperidin-4-ylamino]-benzooxazole-6-carboxylic acid

In analogy to the procedure described in example 1A], 2-(piperidin-4-ylamino)-benzooxazole-6-carboxylic acid methyl ester (example 55) was reacted with 4-chloro-3,5-diethoxy-benzaldehyde (example 166), sodium cyanoborohydride, *N-*ethyl-diisopropylamine and acetic acid in ethanol at 50 °C to yield 2-[1-(4-chloro-3,5-diethoxy-benzyl)-piperidin-4-ylamino]-benzooxazole-6-carboxylic acid methyl ester, which was subsequently saponified in analogy to the procedure described in example 5 to give the title compound as colorless amorphous solid.
**MS: 474.4 (M+H)⁺.**

### Example 190

### 2-[1-(3,5-Diisopropoxy-benzyl)-piperidin-4-ylamino]-benzooxazole-6-carboxylic acid

In analogy to the procedure described in example 1A], 2-(piperidin-4-ylamino)-benzooxazole-6-carboxylic acid methyl ester (example 55) was reacted with with 3,5-diisopropoxy-benzaldehyde (prepared in analogy to the procedure described in example 2F], by reaction of 3,5-dihydroxybenzaldehyde with 2-iodo-propane in DMF using K₂CO₃ as base), sodium cyanoborohydride, *N-*ethyl-diisopropylamine and acetic acid in ethanol at 50 °C to yield 2-[1-(3,5-diisopropoxy-benzyl)-piperidin-4-ylamino]-benzooxazole-6-carboxylic acid methyl ester, which was subsequently saponified in analogy to the procedure described in example 5 to give the title compound as colorless amorphous solid.
**MS: 468.5 (M+H)⁺.**

### Example 191

### 2-[1-(4-Chloro-3,5-diethoxy-benzyl)-piperidin-4-ylamino]-benzooxazol-5-ol

In analogy to the procedure described in example 1A], 2-(piperidin-4-ylamino)-benzooxazol-5-ol hydrochloride (example 191B]) was reacted with 4-chloro-3,5-diethoxy-benzaldehyde (example 166), sodium cyanoborohydride, *N-*ethyl-diisopropylamine and acetic acid in ethanol at 50 °C to give the title compound as light red solid.
**MS:446.1 (M+H)⁺.**
The 2-(piperidin-4-ylamino)-benzooxazol-5-ol hydrochloride used in example 191A] was synthesized as follows:

### B] 2-(Piperidin-4-ylamino)-benzooxazol-5-ol hydrochloride

0.33 g 4-(5-hydroxy-benzooxazol-2-ylamino)-piperidine-1-carboxylic acid *tert-*butyl ester (example 99B]) was suspended in 10 mL of EtOH at RT; while stirring, 1.25 mL(2.0 eq.) of HCl/ dioxane (4M) was added and the reaction heated up to reflux to achieve a clear solution; then, it was cooled down to 60 °C and stirred for four hours at that temperature and subsequently evaporated i.V. The residue was dried for 5 hours at RT (in high vacuum) to give 0.32 g of the title compound as brown solid.
**MS: 234.1 (M+H)⁺.**

### Example 192

### 3-{2-[1-(3,5-Diethoxy-4-fluoro-benzyl)-piperidin-4-ylamino]-benzooxazol-5-yloxy}-propan-1-ol

In analogy to the procedure described in example 1A], 3-[2-(piperidin-4-ylamino)-benzooxazol-5-yloxy]-propan-1-ol hydrochloride (prepared by i) alkylation of 4-(5-hydroxy-benzooxazol-2-ylamino)-piperidine-1-carboxylic acid *tert*-butyl ester (example 99B]) with 2-(3-bromo-propoxy)-tetrahydro-pyran and potassium carbonate in *N,N-*dimethylformamide at 100 °C in analogy to the procedure described in example 113B] to give 4-{5-[3-(tetrahydro-pyran-2-yloxy)-propoxy]-benzooxazol-2-ylamino}-piperidine-1-carboxylic acid *tert*-butyl ester; ii) Boc and THP cleavage with HCl / dioxane (4 molar) in EtOH between RT and 60 °C in analogy to the procedure described in example 191B]) was reacted with 3,5-diethoxy-4-fluoro-benzaldehyde (example 2H]), sodium cyanoborohydride, *N-*ethyl-diisopropylamine and acetic acid in ethanol at 50 °C to give the title compound as off-white solid.
**MS:488.1 (M+H)⁺.**

### Example 193

### 3-{2-[1-(3,5-Diisopropoxy-benzyl)-piperidin-4-ylamino]-benzooxazol-5-yloxy}-propan-1-ol

In analogy to the procedure described in example 1A], 3-[2-(piperidin-4-ylamino)-benzooxazol-5-yloxy]-propan-1-ol hydrochloride (example 192) was reacted with 3,5-diisopropoxy-benzaldehyde (prepared in analogy to the procedure described in example 2F], by reaction of 3,5-dihydroxybenzaldehyde with 2-iodo-propane in DMF using K₂CO₃ as base), sodium cyanoborohydride, *N-*ethyl-diisopropylamine and acetic acid in ethanol at 50 °C to give the title compound as colorless solid.
**MS: 498.2 (M+H)⁺.**

### Example 194

### 3-{2-[1-(4-Chloro-3,5-diethoxy-benzyl)-piperidin-4-ylamino]-benzooxazol-5-yloxy}-propan-1-ol

In analogy to the procedure described in example 1A], 3-[2-(piperidin-4-ylamino)-benzooxazol-5-yloxy]-propan-1-ol hydrochloride (example 192) was reacted with 4-chloro-3,5-diethoxy-benzaldehyde (example 166), sodium sodium cyanoborohydride, *N-*ethyl-diisopropylamine and acetic acid in ethanol at 50 °C to give the title compound as off-white solid.
**MS: 504.2 (M+H)⁺.**

### Example 195

### 3-{2-[1-(2,6-Diethoxy-4'-fluoro-biphenyl-4-ylmethyl)-piperidin-4-ylamino]-benzooxazol-5-yloxy}-propan-1-ol

In analogy to the procedure described in example 1A], 3-[2-(piperidin-4-ylamino)-benzooxazol-5-yloxy]-propan-1-ol hydrochloride (example 192) was reacted with 2,6-diethoxy-4'-fluoro-biphenyl-4-carbaldehyde (example 167B]), sodium cyanoborohydride, *N-*ethyl-diisopropylamine and acetic acid in ethanol at 50°C to give the title compound as colorless solid.
**MS: 564.4 (M+H)⁺.**

### Example 196

### 2-{2-[1-(3,5-Diethoxy-4-fluoro-benzyl)-piperidin-4-ylamino]-benzooxazol-5-yloxy}-ethanol

In analogy to the procedure described in example 1A], 2-[2-(piperidin-4-ylamino)-benzooxazol-5-yloxy]-ethanol hydrochloride (prepared by i) alkylation of 4-(5-hydroxy-benzooxazol-2-ylamino)-piperidine-1-carboxylic acid *tert*-butyl ester (example 99B]) with 2-(2-bromo-ethoxy)-tetrahydro-pyran and potassium carbonate in *N,N-*dimethylformamide at 100°C in analogy to the procedure described in example 113B] to give 4-{5-[2-(tetrahydro-pyran-2-yloxy)-ethoxy]-benzooxazol-2-ylamino}-piperidine-1-carboxylic acid *tert*-butyl ester; ii) Boc and THP cleavage with HCl / dioxane (4 molar) in EtOH between RT and 60 °C in analogy to the procedure described in example 191B]) was reacted with 3,5-diethoxy-4-fluoro-benzaldehyde (example 2H]), sodium cyanoborohydride, *N-*ethyl-diisopropylamine and acetic acid in ethanol at 50°C to give the title compound as yellow amorphous solid.
**MS: 474.1 (M+H)⁺.**

### Example 197

### 2-{2-[1-(2,6-Diethoxy-4'-fluoro-biphenyl-4-ylmethyl)-piperidin-4-ylamino]-benzooxazol-5-yloxy}-ethanol

In analogy to the procedure described in example 1A], 2-[2-(piperidin-4-ylamino)-benzooxazol-5-yloxy]-ethanol hydrochloride (example 196) was reacted with 2,6-diethoxy-4'-fluoro-biphenyl-4-carbaldehyde (example 167B]), sodium cyanoborohydride, *N-*ethyl-diisopropylamine and acetic acid in ethanol at 50°C to give the title compound as yellow amorphous solid.
**MS: 550.3 (M+H)⁺.**

### Example 198

### [1-(3,5-Diethoxy-4-fluoro-benzyl)-piperidin-4-yl]-[5-(3-methoxy-propoxy)-benzooxazol-2-yl]-amine

In analogy to the procedure described in example 1A], [5-(3-methoxy-propoxy)-benzooxazol-2-yl]-piperidin-4-yl-amine (prepared by i) alkylation of 4-(5-hydroxy-benzooxazol-2-ylamino)-piperidine-1-carboxylic acid *tert*-butyl ester (example 99B]) with 1-bromo-3-methoxy-propane and potassium carbonate in *N*,*N-*dimethylformamide at 100°C in analogy to the procedure described in example 113B] to give 4-[5-(3-methoxy-propoxy)-benzooxazol-2-ylamino]-piperidine-1-carboxylic acid *tert*-butyl ester; ii) Boc cleavage with HCl / dioxane (4 molar) in EtOH between RT and 60 °C in analogy to the procedure described in example 191B]) was reacted with 3,5-diethoxy-4-fluorobenzaldehyde (example 2H]), sodium cyanoborohydride, *N-*ethyl-diisopropylamine and acetic acid in ethanol at 50°C to give the title compound as colorless oil.
**MS: 502.1 (M+H)⁺.**

### Example 199

### [1-(3,5-Diisopropoxy-benzyl)-piperidin-4-yl]-[5-(3-methoxy-propoxy)-benzooxazol-2-yl]-amine

In analogy to the procedure described in example 1A], [5-(3-methoxy-propoxy)-benzooxazol-2-yl]-piperidin-4-yl-amine (example 198) was reacted with 3,5-diisopropoxy-benzaldehyde (prepared in analogy to the procedure described in example 2F], by reaction of 3,5-dihydroxybenzaldehyde with 2-iodo-propane in DMF using K₂CO₃ as base), sodium cyanoborohydride, *N-*ethyl-diisopropylamine and acetic acid in ethanol at 50 °C to give the title compound as colorless oil.
**MS: 512.3 (M+H)⁺.**

### Example 200

### [1-(4-Chloro-3,5-diethoxy-benzyl)-piperidin-4-yl]-[5-(3-methoxy-propoxy)-benzooxazol-2-yl]-amine

In analogy to the procedure described in example 1A], [5-(3-methoxy-propoxy)-benzooxazol-2-yl]-piperidin-4-yl-amine (example 198) was reacted with 4-chloro-3,5-diethoxy-benzaldehyde (example 166), sodium cyanoborohydride, *N-*ethyl-diisopropylamine and acetic acid in ethanol at 50 °C to give the title compound as colorless amorphous solid.
**MS: 518.2 (M+H)⁺.**

### Example 201

### [1-(2,6-Diethoxy-4'-fluoro-biphenyl-4-ylmethyl)-piperidin-4-yl]-[5-(3-methoxy-propoxy)-benzooxazol-2-yl]-amine

In analogy to the procedure described in example 1A], [5-(3-methoxy-propoxy)-benzooxazol-2-yl]-piperidin-4-yl-amine (example 198) was reacted with 2,6-diethoxy-4'-fluoro-biphenyl-4-carbaldehyde (example 167B]), sodium cyanoborohydride, *N-*ethyl-diisopropylamine and acetic acid in ethanol at 50 °C to give the title compound as colorless oil.
**MS: 578.3 (M+H)⁺.**

### Example 202

### [1-(4-Chloro-3,5-diethoxy-benzyl)-piperidin-4-yl]-[5-(3-methanesulfonyl-propoxy)-ben zooxazol-2-yl]-amine

A] In analogy to the procedure described example 1A], [5-(3-methanesulfonyl-propoxy)-benzooxazol-2-yl]-piperidin-4-yl-amine hydrochloride (prepared from 4-[5-(3-methanesulfonyl-propoxy)-benzooxazol-2-ylamino]-piperidine-1-carboxylic acid *tert*-butyl ester (example 202B]) by Boc cleavage with HCl / dioxane (4 molar) in EtOH between RT and 60 °C in analogy to the procedure described in example 191B]) was reacted with 4-chloro-3,5-diethoxy-benzaldehyde (example 166), sodium cyanoborohydride, *N-*ethyl-diisopropylamine and acetic acid in ethanol at 50 °C to yield the title compound as amorphous colorless solid.
**MS: 566.3 (M+H)⁺.**
The 4-[5-(3-methanesulfonyl-propoxy)-benzooxazol-2-ylamino]-piperidine-1-carboxylic acid *tert*-butyl ester used in example 202A] was synthesized as follows:

### B1 4-[5-(3-Methanesulfonyl-propoxy)-benzooxazol-2-ylamino]-piperidine-1-carboxylic acid tert-butyl ester

0.83 g (2.5 mMol) of 4-(5-hydroxy-benzooxazol-2-ylamino)-piperidine-1-carboxylic acid *tert*-butyl ester (example 99B]), 0.30 g (1.10 eq.) of 3-methylsulfanyl-propan-1-ol and 0.86 g (1.30 eq.) of triphenylphosphine were dissolved at RT in 25 mL of THF; a solution of 0.73 g (1.25 eq.) of di-*tert*-butyl azodicarboxylate in 10 mL of THF was added drop by drop. Then, the reaction mixture was stirred at RT for 96 h, the solvent was evaporated i.V. and the residue (3.05 g) was purified by chromatography (SiO₂, n-heptane / AcOEt) to yield 0.26 g of 4-[5-(3-methylsulfanyl-propoxy)-benzooxazol-2-ylamino]-piperidine-1-carboxylic acid *tert*-butyl ester as a light yellow oil (not pure). This product was oxidized with 0.29 g (2 eq.) of m-chloro perbenzoic acid in 3 mL of chloroform for 20 hours at 4 °C. The reaction mixture was then poured into cold water / sodium carbonate solution and extracted twice with chloroform. The organic phases were washed with water and brine, dried over MgSO₄ filtered and evaporated i.V. and the residue (0.26 g) was purified by chromatography (SiO₂, MeCl₂ / MeOH) to yield 0.12 g of the title compound as a off-white solid.
**MS: 454.3 (M+H)⁺.**

### Example 203

### Methanesulfonic acid 2-[1-(3,5-diethoxy-4-fluoro-benzyl)-piperidin-4-ylamino]-benzooxazol-5-yl ester

A] In analogy to the procedure described example 1A], methanesulfonic acid 2-(piperidin-4-ylamino)-benzooxazol-5-yl ester hydrochloride (prepared from 4-(5-methanesulfonyloxy-benzooxazol-2-ylamino)-piperidine-1-carboxylic acid *tert*-butyl ester (example 203B]) by Boc cleavage with HCl / dioxane (4 molar) in EtOH between RT and 60 °C in analogy to the procedure described in example 191B]) was reacted with 3,5-diethoxy-4-fluoro-benzaldehyde (example 2H]), sodium cyanoborohydride, *N-*ethyl-diisopropylamine and acetic acid in ethanol at 50 °C to yield the title compound as light red oil.
**MS: 506.4 (M-H)⁻.**
The 4-(5-methanesulfonyloxy-benzooxazol-2-ylamino)-piperidine-1-carboxylic acid *tert-*butyl ester used in example 203A] was synthesized as follows:

### B] 4-(5-Methanesulfonyloxy-benzooxazol-2-ylamino)-piperidine-1-carboxylic acid tert-butyl ester

1.00 g (3.0 mMol) of 4-(5-hydroxy-benzooxazol-2-ylamino)-piperidine-1-carboxylic acid *tert*-butyl ester (example 99B]) was suspended in 25 mL of MeCl₂, then 0.79 mL (0.59 g = 1.5 eq.) of *N-*ethyl diisopropylamine was added and the mixture cooled down to 10 °C. While stirring, 0.26 mL (0.39 g, 1.1 eq.) of methanesulfonyl chloride was added drop by drop and the reaction then warmed up to RT. After 20 hours, it was poured into crashed ice, acidified with HCl (2N) to pH 2-3 and extracted twice with MeCl₂. The organic phases were washed with water and brine, dried over MgSO₄ filtered and evaporated i.V. and the residue (1.25 g) was purified by chromatography (SiO₂, MeCl₂ / MeOH) to yield 1.09 g of the title compound as a off-white amorphous solid.
**MS: 412.1 (M+H)⁺.**

### Example 204

### Methanesulfonic acid 2-[1-(3,5-diisopropoxy-benzyl)-piperidin-4-ylamino]-benzooxazol-5-yl ester

In analogy to the procedure described example 1A], methanesulfonic acid 2-(piperidin-4-ylamino)-benzooxazol-5-yl ester hydrochloride (example 203) was reacted with 3,5-diisopropoxy-benzaldehyde (prepared in analogy to the procedure described in example 2F], by reaction of 3,5-dihydroxybenzaldehyde with 2-iodo-propane in DMF using K₂CO₃ as base), sodium cyanoborohydride, *N-*ethyl-diisopropylamine and acetic acid in ethanol at 50°C to yield the title compound as light red oil.
**MS: 518.5 (M+H)⁺.**

### Example 205

### Methanesulfonic acid 2-[1-(4-chloro-3,5-diethoxy-benzyl)-piperidin-4-ylamino]-benzooxazol-5-yl ester

In analogy to the procedure described example 1A], methanesulfonic acid 2-(piperidin-4-ylamino)-benzooxazol-5-yl ester hydrochloride (example 203) was reacted with 4-chloro-3,5-diethoxy-benzaldehyde (example 166), sodium cyanoborohydride, *N-*ethyl-diisopropylamine and acetic acid in ethanol at 50°C to yield the title compound as light red oil.
**MS: 522.3 (M-H)⁻.**

### Example 206

### Methanesulfonic acid 2-[1-(2,6-diethoxy-4'-fluoro-biphenyl-4-ylmethyl)-piperidin-4-ylamino]-benzooxazol-5-yl ester

In analogy to the procedure described example 1A], methanesulfonic acid 2-(piperidin-4-ylamino)-benzooxazol-5-yl ester hydrochloride (example 203) was reacted with 2,6-diethoxy-4'-fluoro-biphenyl-4-carbaldehyde (example 167B]), sodium cyanoborohydride, *N*-ethyl-diisopropylamine and acetic acid in ethanol at 50 °C to yield the title compound as light red oil.
**MS: 582.3 (M-H)⁻.**

### Example 207

### [1-(3,5-Diethoxy-4-fluoro-benzyl)-piperidin-4-yl]-[5-(3-[1,2,4]triazol-1-yl-propoxy)-benzooxazol-2-yl]-amine

A] In analogy to the procedure described example 1A], piperidin-4-yl-[5-(3-[1,2,4]triazol-1-yl-propoxy)-benzooxazol-2-yl]-amine (prepared from 4-[5-(3-[1,2,4]triazol-1-yl-propoxy)-benzooxazol-2-ylamino]-piperidine-1-carboxylic acid *tert-*butyl ester (example 207C]) by Boc cleavage with HCl / dioxane (4 molar) in EtOH between RT and 60 °C in analogy to the procedure described in example 191B]) was reacted with 3,5-diethoxy-4-fluoro-benzaldehyde (example 2H]), sodium cyanoborohydride, *N-*ethyl-diisopropylamine and acetic acid in ethanol at 50 °C to yield the title compound as colorless amorphous solid.
**MS: 539.4 (M+H)⁺.**
The 4-[5-(3-[1,2,4]triazol-1-yl-propoxy)-benzooxazol-2-ylamino]-piperidine-1-carboxylic acid *tert*-butyl ester used in example 207A] was synthesized as follows:

### B] 4-[5-(3-Bromo-propoxy)-benzooxazol-2-ylamino]-piperidine-1-carboxylic acid tert-butyl ester

1.00 g (3.0 mMol) of 4-(5-hydroxy-benzooxazol-2-ylamino)-piperidine-1-carboxylic acid *tert*-butyl ester (example 99B]) was dissolved under argon in 30 mL of DMF at RT. While stirring, 2.09 g (5.0 eq.) of anhydrous potassium carbonate and 1.55 mL (3.06 g = 5.0 eq.) of 1,3-dibromo-propane were added and the reaction heated up to 100°C. After 2 hours, it was cooled to RT and stirred over night at RT. It was then poured into crashed ice, acidified with HCl (2N) to pH 2-3 and extracted four times with ether. The organic phases were washed with water and brine, dried over MgSO₄, filtered and evaporated i.V. and the residue (0.81 g) was purified by chromatography (SiO₂, MeCl₂ / MeOH) to yield 0.21 g of the title compound as a yellow oil.
**MS: 454.2 (M+H)⁺.**

### C] 4-[5-(3-[1,2,4]Triazol-1-yl-propoxy)-benzooxazol-2-ylamino]-peridine-1-carboxylic acid tert-butyl ester

0.019 g (0.4 mMol) of sodium hydride dispersion (55% in mineral oil) was suspended under argon in 2.0 mL of DMF; while stirring, 0.033 g (0.5 mMol) of 1,2,4-triazoie was added at RT; after 30 min, a solution of 0.18 g (0.4 mMol) of 4-[5-(3-bromo-propoxy)-benzooxazol-2-ylamino]-piperidine-1-carboxylic acid *tert*-butyl ester in 1.0 mL of DMF was added drop by drop. After stirring for 16 hours, the reaction mixture was poured into crashed ice and extracted twice with AcOEt, the organic phases were washed with water and brine, dried over MgSO₄, filtered and evaporated i.V. and the residue (0.18 g) was purified by chromatography (SiO₂, MeCl₂ / MeOH) to yield 0.12 g of the title compound as light yellow oil.
**MS: 443.1 (M+H)⁺.**

### Example 208

### [1-(3,5-Diisopropoxy-benzyl)-piperidin-4-yl]-[5-(3-[1,2,4]triazol-1-yl-propoxy)-benzooxazol-2-yl]-amine

In analogy to the procedure described example 1A], piperidin-4-yl-[5-(3-[1,2,4]triazol-1-yl-propoxy)-benzooxazol-2-yl]-amine (example 207) was reacted with 3,5-diisopropoxy-benzaldehyde (prepared in analogy to the procedure described in example 2F], by reaction of 3,5-dihydroxybenzaldehyde with 2-iodo-propane in DMF using K₂CO₃ as base), sodium cyanoborohydride, *N-*ethyl-diisopropylamine and acetic acid in ethanol at 50 °C to yield the title compound as colorless amorphous solid.
**MS: 549.3 (M+H)⁺.**

### Example 209

### [1-(4-Chloro-3,5-diethoxy-benzyl)-piperidin-4-yl]-[5-(3-[1,2,4]triazol-1-yl-propoxy)-ben zooxazol-2-yl]-am ine

In analogy to the procedure described example 1A], piperidin-4-yl-[5-(3-[1,2,4]triazol-1-yl-propoxy)-benzooxazol-2-yl]-amine (example 207) was reacted with 4-chloro-3,5-diethoxy-benzaldehyde (example 166), sodium cyanoborohydride, *N-*ethyl-diisopropylamine and acetic acid in ethanol at 50 °C to yield the title compound as colorless amorphous solid.
MS: 555.2 (M+H)⁺.

### Example 210

### [1-(2,6-Diethoxy-4'-fluoro-biphenyl-4-ylmethyl)-piperidin-4-yl]-[5-(3-[1,2,4]triazol-1-yl-propoxy)-benzooxazol-2-yl]-amine

In analogy to the procedure described example 1A], piperidin-4-yl-[5-(3-[1,2,4]triazol-1-yl-propoxy)-benzooxazol-2-yl]-amine (example 207) was reacted with 2,6-diethoxy-4'-fluoro-biphenyl-4-carbaldehyde (example 167B]), sodium cyanoborohydride, *N-*ethyl-diisopropylamine and acetic acid in ethanol at 50 °C to yield the title compound as colorless amorphous solid.
**MS: 615.5 (M+H)⁺.**

### Example 211

### 2-[1-(3,5-Diethoxy-4-fluoro-benzyl)-piperidin-4-ylamino]-benzothiazole-6-carboxylic acid ethyl ester

In analogy to the procedure described in example 1A], 2-(piperidin-4-ylamino)-benzothiazole-6-carboxylic acid ethyl ester (prepared by condensation of 2-chloro-benzothiazole-6-carboxylic acid ethyl ester (PCT Int. Appl. WO 2002000633 A1) with 4-amino-piperidine-carboxylic acid *tert*-butyl ester in *N-*ethyl-diisopropylamine / acetonitrile between 60 °C and reflux in analogy to the procedure described in example 1C] to yield 2-(1-*tert*-butoxycarbonyl-piperidin-4-ylamino)-benzothiazole-6-carboxylic acid ethyl ester; followed by Boc cleavage with HCl/dioxane (4 molar) in EtOH at 60°C in analogy to the procedure described in example 191B]) was reacted with 3,5-diethoxy-4-fluoro-benzaldehyde (example 2H]), sodium cyanoborohydride, *N-*ethyl-diisopropylamine and acetic acid in ethanol at 50 °C to yield the title compound as colorless oil.
**MS: 502.1 (M+H)⁺.**

### Example 212

### 2- [1-(3,5-Diisopropoxy-benzyl)-piperidin-4-ylamino]-benzothiazole-6-carboxylic acid ethyl ester

In analogy to the procedure described in example 1A], 2-(piperidin-4-ylamino)-benzothiazole-6-carboxylic acid ethyl ester (example 211) was reacted with 3,5-diisopropoxy-benzaldehyde (prepared in analogy to the procedure described in example 2F], by reaction of 3,5-dihydroxybenzaldehyde with 2-iodo-propane in DMF using K₂CO₃ as base), sodium cyanoborohydride, *N-*ethyl-diisopropylamine and acetic acid in ethanol at 50 °C to yield the title compound as colorless amorphous solid.
**MS: 512.2 (M+H)⁺.**

### Example 213

### 2-[1-(4-Chloro-3,5-diethoxy-benzyl)-piperidin-4-ylamino]-benzothiazole-6-carboxylic acid ethyl ester

In analogy to the procedure described in example 1A], 2-(piperidin-4-ylamino)-benzothiazole-6-carboxylic acid ethyl ester (example 211) was reacted with 4-chloro-3,5-diethoxy-benzaldehyde (example 166), sodium cyanoborohydride, *N-*ethyl-diisopropylamine and acetic acid in ethanol at 50°C to yield the title compound as colorless amorphous solid.
**MS: 518.1 (M+H)⁺.**

### Example 214

### 2-[1-(2,6-Diethoxy-4'-fluoro-biphenyl-4-ylmethyl)-piperidin-4-ylamino]-benzothiazole-6-carboxylic acid ethyl ester

In analogy to the procedure described in example 1A], 2-(piperidin-4-ylamino)-benzothiazole-6-carboxylic acid ethyl ester (example 211) was reacted with 2,6-diethoxy-4'-fluoro-biphenyl-4-carbaldehyde (example 167B]), sodium cyanoborohydride, *N-*ethyl-diisopropylamine and acetic acid in ethanol at 50°C to yield the title compound as colorless amorphous solid.
**MS: 578.2 (M+H)⁺.**

### Example 215

### 2-[1-(3,5-Diethoxy-4-pyrrol-1-yl-benzyl)-piperidin-4-ylamino]-benzoth iazole-6-carboxylic acid ethyl ester

In analogy to the procedure described in example 1A], 2-(piperidin-4-ylamino)-benzothiazole-6-carboxylic acid ethyl ester (example 211) was reacted with 3,5-diethoxy-4-pyrrol-1-yl-benzaldehyde (example 13C]), sodium cyanoborohydride, *N-*ethyl-diisopropylamine and acetic acid in ethanol at 50°C to yield the title compound as light yellow amorphous solid.
**MS: 549.3 (M+H)⁺.**

### Example 216

### 2-[1-(3,5-Diethoxy-4-fluoro-benzyl)-piperidin-4-ylamino]-benzothiazole-6-carboxylic acid

In analogy to the procedure described in example 5,2-[1-(3,5-diethoxy-4-fluorobenzyl)-piperidin-4-ylamino]-benzothiazole-6-carboxylic acid ethyl ester (example 211) was saponified to yield the title compound as light yellow solid.
**MS: 472.1 (M-H)⁻.**

### Example 217

### 2-[1-(3,5-Diisopropoxy-benzyl)-piperidin-4-ylamino]-benzothiazole-6-carboxylic acid

In analogy to the procedure described in example 5,2-[1-(3,5-diisopropoxy-benzyl)-piperidin-4-ylamino]-benzothiazole-6-carboxylic acid ethyl ester (example 212) was saponified to yield the title compound as colorless solid.
**MS: 482.4 (M-H)⁻.**

### Example 218

### 2-[1-(4-Chloro-3,5-diethoxy-benzyl)-piperidin-4-ylamino]-benzothiazole-6-carboxylic acid

In analogy to the procedure described in example 5,2-[1-(4-chloro-3,5-diethoxy-benzyl)-piperidin-4-ylamino]-benzothiazole-6-carboxylic acid ethyl ester (example 213) was saponified to yield the title compound as colorless solid.
**MS: 488.2 (M-H)⁻.**

### Example 219

### 2-[1-(2,6-Diethoxy-4'-fluoro-biphenyl-4-ylmethyl)-piperidin-4-ylamino]-benzothiazole-6-carboxylic acid

In analogy to the procedure described in example 5, 2-[1-(2,6-diethoxy-4'-fluoro-biphenyl-4-ylmethyl)-piperidin-4-ylamino]-benzothiazole-6-carboxylic acid ethyl ester (example 214) was saponified to yield the title compound as colorless solid.
MS: 548.3 (M-H)⁻.

### Example 220

### 2-[1-(3,5-Diethoxy-4-pyrrol-1-yl-benzyl)-piperidin-4-ylamino)-benzothiazole-6-carboxylic acid

In analogy to the procedure described in example 5,2-[1-(3,5-diethoxy-4-pyrrol-1-yl-benzyl)-piperidin-4-ylamino]-benzothiazole-6-carboxylic acid ethyl ester (example 215) was saponified to yield the title compound as colorless solid.
MS: 519.3 (M-H)-.

### Example 221

### 2-[1-(3,5-Diisopropoxy-benzyl)-piperidin-4-ylamino]-benzothiazole-5-carboxylic acid methyl ester

A] In analogy to the procedure described in example 1A], 2-(piperidin-4-ylamino)-benzothiazole-5-carboxylic acid methyl ester (prepared by condensation of 2-chloro-benzothiazole-5-carboxylic acid methyl ester (example 221B]) with 4-amino-piperidine-carboxylic acid *tert*-butyl ester in *N*-ethyl-diisopropylamine / acetonitrile at reflux in analogy to the procedure described in example 1C] to yield 2-(1-*tert*-butoxycarbonyl-piperidin-4-ylamino)-benzothiazole-5-carboxylic acid methyl ester; followed by Boc cleavage with HCl / dioxane (4 molar) in EtOH at 60 °C in analogy to the procedure described in example 191B]) was reacted with 3,5-diisopropoxy-benzaldehyde (prepared in analogy to the procedure described in example 2F], by reaction of 3,5-dihydroxybenzaldehyde with 2-iodo-propane in DMF using K₂CO₃ as base), sodium cyanoborohydride, *N*-ethyl-diisopropylamine and acetic acid in ethanol at 50 °C to yield the title compound as colorless amorphous solid.
MS: 498.4 (M+H)⁺.
The 2-chloro-benzothiazole-5-carboxylic acid methyl ester used in example 221A] was synthesized as follows:

### B] 2-Chloro-benzothiazole-5-carboxylic acid methyl ester

1.93 g (16.8 mMol) of *tert*-butyl nitrite and 1.35 g of copper(II)-chloride were suspended in 40 mL of MeCN and the mixture heated up to 65°C; after 10 min, a solution of 3.50 g (16.8 mMol) of 2-amino-benzothiazole-5-carboxylic acid methyl ester [PCT Int. Appl. WO 2004 / 067529 A1] in 80 mL of MeCN was added carefully at 65 °C. After 40 min, the gas evolution was complete; the reaction mixture was then cooled down to RT and poured into crashed ice, acidified with HCl (2N) to pH 2 and extracted twice with AcOEt; the organic phases were washed with water and brine, dried over MgSO₄, filtered and evaporated i.V. and the residue (1.8 g) was purified by chromatography (SiO₂, n-heptane / AcOEt) to yield 1.12 g of the title compound as colorless solid.
MS:227.1(M)⁺.

### Example 222

### 2-[1-(4-Chloro-3,5-diethoxy-benzyl)-piperidin-4-ylamino]-benzothiazole-5-carboxylic acid methyl ester

In analogy to the procedure described in example 1A], 2-(piperidin-4-ylamino)-benzothiazole-5-carboxylic acid methyl ester (example 221) was reacted with 4-chloro-3,5-diethoxy-benzaldehyde (example 166), sodium cyanoborohydride, *N*-ethyl-diisopropylamine and acetic acid in ethanol at 50 °C to yield the title compound as colorless amorphous solid.
MS: 504.3 (M+H)⁺.

### Example 223

### 2-[1-(2,6-Diethoxy-4'-fluoro-biphenyl-4-ylmethyl)-piperidin-4-ylamino]-benzothiazole-5-carboxylic acid methyl ester

In analogy to the procedure described in example 1A], 2-(piperidin-4-ylamino)-benzothiazole-5-carboxylic acid methyl ester (example 221) was reacted with 2,6-diethoxy-4'-fluoro-biphenyl-4-carbaldehyde (example 167B]), sodium cyanoborohydride, *N*-ethyl-diisopropylamine and acetic acid in ethanol at 50 °C to yield the title compound as colorless amorphous solid.
MS: 564.4 (M+H)⁺.

### Example 224

### 2-[1-(3,5-Diethoxy-4-pyrrol-1-yl-benzyl)-piperidin-4-ylamino]-benzothiazole-5-carboxylic acid methyl ester

In analogy to the procedure described in example 1A], 2-(piperidin-4-ylamino)-benzothiazole-5-carboxylic acid methyl ester (example 221) was reacted with 3,5-diethoxy-4-pyrrol-1-yl-benzaldehyde (example 13C]), sodium cyanoborohydride, *N-*ethyl-diisopropylamine and acetic acid in ethanol at 50°C to yield the title compound as light yellow amorphous solid.
MS: 535.3 (M+H)⁺.

### Example 225

### 2-[1-(3,5-Diisopropoxy-benzyl)-piperidin-4-ylamino]-benzothiazole-5-carboxylic acid

In analogy to the procedure described in example 5,2-[1-(3,5-diisopropoxy-benzyl)-piperidin-4-ylamino]-benzothiazole-5-carboxylic acid methyl ester (example 221) was saponified to yield the title compound as colorless solid.
MS: 484.4 (M+H)⁺.

### Example 226

### 2-[1-(4-Chloro-3,5-diethoxy-benzyl)-piperidin-4-ylamino]-benzothiazole-5-carboxylic acid

In analogy to the procedure described in example 5, 2-[1-(4-chloro-3,5-diethoxy-benzyl)-piperidin-4-ylamino]-benzothiazole-5-carboxylic acid methyl ester (example 222) was saponified to yield the title compound as colorless solid.
MS: 490.3 (M+H)⁺.

### Example 227

### 2-[1-(2,6-Diethoxy-4'-fluoro-biphenyl-4-ylmethyl)-piperidin-4-ylamino]-benzothiazole-5-carboxylic acid

In analogy to the procedure described in example 5, 2-[1-(2,6-diethoxy-4'-fluoro-biphenyl-4-ylmethyl)-piperidin-4-ylamino]-benzothiazole-5-carboxylic acid methyl ester (example 223) was saponified to yield the title compound as colorless solid.
MS: 550.2 (M+H)⁺.

### Example 228

### 2-[1-(3,5-Diethoxy-4-pyrrol-1-yl-benzyl)-piperidin-4-ylamino]-benzothiazole-5-carboxylic acid

In analogy to the procedure described in example 5, 2-[1-(3,5-diethoxy-4-pyrrol-1-yl-benzyl)-piperidin-4-ylamino]-benzothiazole-5-carboxylic acid methyl ester (example 224) was saponified to yield the title compound as light brown solid.
MS: 521.3 (M+H)⁺.

### Example 229

### {2-[1-(2,6-Diethoxy-4'-fluoro-biphenyl-4-ylmethyl)-piperidin-4-ylamino]-oxazolo[5,4-c]pyridin-4-yl}-methanol

A] In analogy to the procedure described in example 1A], [2-(piperidin-4-ylamino)-oxazolo[5,4-c]pyridin-4-yl]-methanol (example 144D]) was reacted with 2,6-diethoxy-4'-fluoro-biphenyl-4-carbaldehyde (example 167B]), sodium cyanoborohydride, *N*-ethyl-diisopropylamine and acetic acid in ethanol at 50°C to yield the title compound as colorless amorphous solid.
MS: 521.3 (M+H)⁺.

### Example 230

### {2-[1-(4-Chloro-3,5-diethoxy-benzyl)-piperidin-4-ylamino]-oxazolo[5,4-c]pyridin-4-yl}-methanol

A] In analogy to the procedure described in example 1A], [2-(piperidin-4-ylamino)-oxazolo[5,4-*c*]pyridin-4-yl]-methanol (example 144D]) was reacted with 4-chloro-3,5-diethoxy-benzaldehyde (example 166), sodium cyanoborohydride, *N*-ethyl-diisopropylamine and acetic acid in ethanol at 50 °C to yield the title compound as colorless amorphous solid.
MS:461.1(M+H)⁺.

### Example A

Film coated tablets containing the following ingredients can be manufactured in a conventional manner:

| Ingredients | Per tablet | |
|---|---|---|
| Kernel: | | |
| Compound of formula (I) | 10.0 mg | 200.0 mg |
| Microcrystalline cellulose | 23.5 mg | 43.5 mg |
| Lactose hydrous | 60.0 mg | 70.0 mg |
| Povidone K30 | 12.5 mg | 15.0 mg |
| Sodium starch glycolate | 12.5 mg | 17.0 mg |
| Magnesium stearate | 1.5 mg | 4.5 mg |
| (Kernel Weight) | 120.0 mg | 350.0 mg |
| Film Coat: | | |
| Hydroxypropyl methyl cellulose | 3.5 mg | 7.0 mg |
| Polyethylene glycol 6000 | 0.8 mg | 1.6 mg |
| Talc | 1.3 mg | 2.6 mg |
| Iron oxyde (yellow) | 0.8 mg | 1.6 mg |
| Titanium dioxide | 0.8 mg | 1.6mg |

The active ingredient is sieved and mixed with microcristalline cellulose and the mixture is granulated with a solution of polyvinylpyrrolidone in water. The granulate is mixed with sodium starch glycolate and magesium stearate and compressed to yield kernels of 120 or 350 mg respectively. The kernels are lacquered with an aqueous solution / suspension of the above mentioned film coat.

### Example B

Capsules containing the following ingredients can be manufactured in a conventional manner:

| Ingredients | Per capsule |
|---|---|
| Compound of formula (I) | 25.0 mg |
| Lactose | 150.0 mg |
| Maize starch | 20.0 mg |
| Talc | 5.0 mg |

The components are sieved and mixed and filled into capsules of size 2.

### Example C

Injection solutions can have the following composition:

| | |
|---|---|
| Compound of formula (I) | 3.0 mg |
| Gelatine | 150.0 mg |
| Phenol | 4.7 mg |
| Sodium carbonate | to obtain a final pH of 7 |
| Water for injection solutions | ad 1.0 ml |

### Example D

Soft gelatin capsules containing the following ingredients can be manufactured in a conventional manner:

### Capsule contents

| | |
|---|---|
| Compound of formula (I) | 5.0 mg |
| Yellow wax | 8.0 mg |
| Hydrogenated soya bean oil | 8.0 mg |
| Partially hydrogenated plant oils | 34.0 mg |
| Soya bean oil | 110.0 mg |
| Weight of capsule contents | 165.0 mg |
| Gelatin capsule | |
| Gelatin | 75.0 mg |
| Glycerol 85 % | 32.0 mg |
| Karion 83 | 8.0 mg (dry matter) |
| Titanium dioxide | 0.4 mg |
| Iron oxide yellow | 1.1 mg |

The active ingredient is dissolved in a warm melting of the other ingredients and the mixture is filled into soft gelatin capsules of appropriate size. The filled soft gelatin capsules are treated according to the usual procedures.

### Example E

Sachets containing the following ingredients can be manufactured in a conventional manner:

| | |
|---|---|
| Compound of formula (I) | 50.0 mg |
| Lactose, fine powder | 1015.0 mg |
| Microcristalline cellulose (AVICEL PH 102) | 1400.0 mg |
| Sodium carboxymethyl cellulose | 14.0 mg |
| Polyvinylpyrrolidone K30 | 10.0 mg |
| Magnesiumstearate | 10.0 mg |
| Flavoring additives | 1.0 mg |

The active ingredient is mixed with lactose, microcristalline cellulose and sodium carboxymethyl cellulose and granulated with a mixture of polyvinylpyrrolidone in water. The granulate is mixed with magnesiumstearate and the flavouring additives and filled into sachets.

## Claims

1. Compounds of the general formula 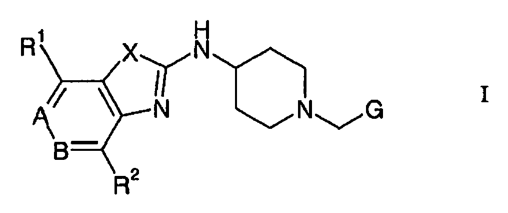 wherein
X is S or O;
A is CR³ and B is CR⁴, or
A is N or N⁺-O⁻ and B is CR⁴, or
B is N or N⁺-O⁻ and A is CR³;
R¹ and R² are independently selected from the group consisting of hydrogen, halogen, cyano, nitro, C₁₋₇-alkyl, hydroxy-C₁₋₇-alkyl, C₁₋₇-alkoxy-C₁₋₇-alkyl, C₁₋₇-alkoxy, hydroxy-C₂₋₇-alkoxy, dihydroxy-C₃₋₇-alkoxy, carboxy-C₁₋₇-alkoxy, C₁₋₇-alkoxycarbonyl-C₁₋₇-alkoxy, carboxy-C₁₋₇-alkyl, C₁₋₇-alkoxycarbonyl-C₁₋₇-alkyl, 1*H*-tetrazol-5-yl-C₁₋₇-alkoxy, pyridinyl-C₁₋₇-alkoxy, -NR⁵R⁶, -NHCOR⁷, -NHSO₂R⁸, -SO₂NR⁹R¹⁰, 1*H*-tetrazol-5-yl, unsubstituted phenyl and phenyl substituted by one to three substituents selected from C₁₋₇-alkyl, C₃₋₇-cycloalkyl, halogen, halogen-C₁₋₇-alkyl and C₁₋₇-alkoxy; and
R² can additionally also be selected from the group consisting of carboxy, C₁₋₇-alkoxycarbonyl and -CONR¹¹R¹²;
R⁵ and R⁶ independently from each other are selected from the group consisting of hydrogen, C₁₋₇-alkyl, hydroxy-C₁₋₇-alkyl and C₃₋₇-cycloalkyl;
R⁷ is selected from the group consisting of C₁₋₇-alkyl, C₃₋₇-cycloalkyl, halogen-C₁₋₇-alkyl, hydroxy-C₁₋₇-alkyl, C₁₋₇-alkoxy-C₁₋₇-alkyl, carboxy-C₁₋₇-alkyl, C₁₋₇-alkoxycarbonyl-C₁₋₇-alkyl, 1*H*-tetrazol-5-yl-C₁₋₇-alkyl, unsubstituted phenyl, phenyl substituted by one to three groups selected from the group consisting of C₁₋₇-alkyl, C₃₋₇-cycloalkyl, C₁₋₇-alkoxy, halogen-C₁₋₇-alkyl and halogen, unsubstituted heteroaryl, heteroaryl substituted by one or two groups selected from C₁₋₇-alkyl, C₃₋₇-cycloalkyl, C₁₋₇-alkoxy, halogen-C₁₋₇-alkyl or halogen;
unsubstituted heteroaryl-C₁₋₇-alkyl and heteroaryl-C₁₋₇-alkyl, wherein the heteroaryl is substituted by one or two groups selected from C₁₋₇-alkyl, C₃₋₇-cycloalkyl, C₁₋₇-alkoxy, halogen-_{C1-7}-alkyl and halogen;
R⁸ is selected from the group consisting of C₁₋₇-alkyl, C₃₋₇-cycloalkyl, unsubstituted heteroaryl and heteroaryl substituted by one or two groups selected from C₁₋₇-alkyl, C₃₋₇-cycloalkyl, C₁₋₇-alkoxy, halogen-C₁₋₇-alkyl and halogen;
R⁹ and R¹⁰ independently from each other are selected from the group consisting of hydrogen, C₁₋₇-alkyl, C₃₋₇-cycloalkyl, unsubstituted heteroaryl and heteroaryl substituted by one or two groups selected from C₁₋₇-alkyl, C₃₋₇-cycloalkyl, C₁₋₇-alkoxy, halogen-C₁₋₇-alkyl and halogen; or R⁹ and R¹⁰ together with the nitrogen atom they are attached to form a pyrrolidine or a piperidine ring;
R¹¹ is selected from the group consisting of hydrogen, C₁₋₇-alkyl and C₃₋₇-cycloalkyl;
R¹² is selected from the group consisting of hydrogen, C₁₋₇-alkyl, C₃₋₁-cycloalkyl, halogen-C₁₋₇-alkyl, hydroxy-C₁₋₇-alkyl, C₁₋₇-alkoxy-C₁₋₇-alkyl, carboxy-C₁₋₇-alkyl, C₁₋₇-alkoxycarbonyl-C₁₋₇-alkyl, unsubstituted phenyl, phenyl substituted by one to three groups selected from the group consisting of C₁₋₇-alkyl, C₃₋₇-cycloalkyl, C₁₋₇-alkoxy, halogen-C₁₋₇-alkyl and halogen, unsubstituted heteroaryl, heteroaryl substituted by one or two groups selected from C₁₋₇-alkyl, C₃₋₇-cycloalkyl, C₁₋₇-alkoxy, halogen-C₁₋₇-alkyl or halogen;
one of R³ and R⁴ is selected from the group consisting of hydrogen, C₁₋₇-alkyl and C₁₋₇- alkoxy, or is absent in case one of A or B is N or N⁺-O⁻, and
the other one of R³ and R⁴ is selected from the group consisting of hydrogen, C₁₋₇-alkyl, C₁₋₇-alkoxy, hydroxy, hydroxy-C₁₋₇-alkyl, cyano-C₁₋₇-alkoxy, C₃₋₇-cycloalkyloxy wherein the cycloalkyl group is substituted by carboxy or C₁₋₇-alkoxy-carbonyl, carboxy, C₁₋₇-alkoxy-carbonyl, carboxy-C₁₋₇-alkoxy, C₁₋₇-alkoxy-carbonyl-C₁₋₇-alkyl, carboxy-C₁₋₇-alkyl, C₁₋₇-alkoxy-carbonyl-C₁₋₇-alkoxy, 1*H-*tetrazol-5-yl-C₁₋₇-alkoxy, triazolyl-C₁₋₇-alkoxy, C₁₋₇-alkylsulfonyloxy, C₁₋₇-alkylsulfonyl-C₁₋₇-alkoxy, hydroxy-C₂₋₇-alkoxy, dihydroxy-C₃₋₇-alkoxy, C₁₋₇alkoxy-C₁₋₇-alkoxy, -(CH₂)ₙ-NR¹³R¹⁴, -(CH₂)ₙ-NHCOR¹⁵, amino-C₁₋₇-alkoxy, aminocarbonyl-C₁₋₇-alkoxy, C₁₋₇-alkylaminocarbonyl-C₁₋₇-alkoxy and 1*H*-tetrazol-5-yl;
R¹³ and R¹⁴ independently from each other are selected from the group consisting of hydrogen, C₁₋₇-alkyl and C₃₋₇-cycloalkyl;
R¹⁵ is selected from the group consisting of C₁₋₇-alkyl, C₃₋₇-cycloalkyl, halogen-C₁₋₇-alkyl, hydroxy-C₁₋₇-alkyl, C₁₋₇-alkoxy-C₁₋₇-alkyl, carboxy-C₁₋₇-alkyl and C₁₋₇-alkoxycarbonyl-C₁₋₇-alkyl;
n is an integer selected from 1,2 and 3;
provided that benzooxazoles and benzothiazoles wherein R¹, R², R³ and R⁴ are hydrogen are excluded;
G is selected from the groups 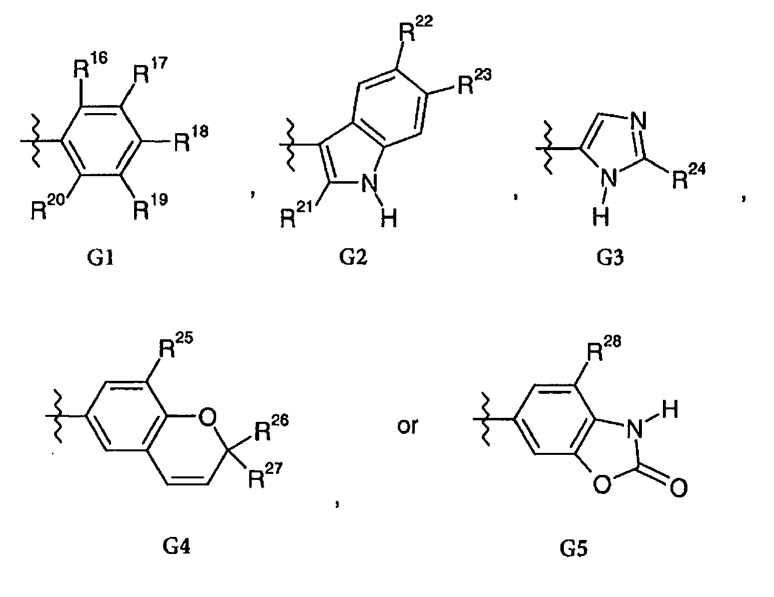
wherein
R¹⁶ is hydrogen or halogen;
R¹⁷ is selected from the group consisting of C₁₋₇-alkoxy, C₂₋₇-alkenyloxy, C₃₋₇-cycloalkyloxy, -NR²⁹R³⁰, halogen-C₁₋₇-alkoxy, C₁₋₇-alkoxy-C₁₋₇-alkoxy, C₁₋₇-alkoxy-C₁₋₇-alkyl; R²⁹ and R³⁰ independently from each other are hydrogen or C₁₋₇-alkyl;
R¹⁸ is selected from the group consisting of hydrogen, C₁₋₇-alkyl, halogen-C₁₋₇-alkyl, hydroxy, C₁₋₇-alkoxy, halogen-C₁₋₇-alkoxy, C₃₋₇-cycloalkyloxy, halogen, pyrrolo, imidazolyl, triazolyl, -CO₂R³¹, -NR³²R³³, -SOR³⁴; unsubstituted phenyl and phenyl substituted by one to two groups selected from the group consisting of C₁₋₇-alkyl, halogen-C₁₋₇-akyl, halogen-C₁₋₇-alkoxy, C₁₋₇-alkoxy and halogen;
R³ is hydrogen or C₁₋₇-alkyl;
R³² and R³³ independently from each other are hydrogen or C₁₋₇-alkyl;
R³⁴ is C₁₋₇-alkyl;
R¹⁹ is selected from the group consisting of hydrogen, C₁₋₇-alkyl, halogen, C₁₋₇-alkoxy, C₁₋₇-alkenyloxy, -O-tetrahydropyranyl, C₃₋₇-cycloalkyloxy, halogen-C₁₋₇-alkoxy, C₁₋₇-alkoxy-C₁₋₇-alkoxy, and C₁₋₇-alkoxy-C₁₋₇-alkyl;
R²⁰ is hydrogen or halogen;
R²¹ is hydrogen or C₁₋₇-alkyl;
R²² and R²³ independently from each other are selected from the group consisting of hydrogen, C₁₋₇-alkyl, C₁₋₇-alkoxy and nitro;
R²⁴ is unsubstituted phenyl or phenyl substituted by one to two groups selected from the group consisting of C₁₋₇-alkyl, C₁₋₇-alkoxy and halogen;
R²⁵ is C₁₋₇-alkoxy;
R²⁶ and R²⁷ independently from each other are C₁₋₇-alkyl;
R²⁸ is C₁₋₇-alkoxy;
and pharmaceutically acceptable salts thereof.

2. Compounds of formula I according to claim 1, wherein X is O.

3. Compounds of formula I according to claims 1 or 2, wherein A is CR³ and B is CR⁴ and wherein
one of R³ and R⁴ is selected from the group consisting of hydrogen, C₁₋₇-alkyl and C₁₋₇-alkoxy and
the other one of R³ and R⁴ is selected from the group consisting of hydrogen, C₁₋₇-alkyl, C₁₋₇-alkoxy, hydroxy, hydroxy-C₁₋₇-alkyl, cyano-C₁₋₇-alkoxy, C₃₋₇-cycloalkyloxy wherein the cycloalkyl group is substituted by carboxy or C₁₋₇-alkoxy-carbonyl, carboxy, C₁₋₇-alkoxy-carbonyl, carboxy-C₁₋₇-alkoxy, C₁₋₇-alkoxy-carbonyl-C₁₋₇-alkyl, carboxy-C₁₋₇-alkyl, C₁₋₇-alkoxy-carbonyl-C₁₋₇-alkoxy, 1*H*-tetrazol-5-yl-C₁₋₇-alkoxy, triazolyl-C₁₋₇-alkoxy, C₁₋₇-alkylsulfonyloxy, C₁₋₇-alkylsulfonyl-C₁₋₇-alkoxy, hydroxy-C₂₋₇-alkoxy, dihydroxy-C₃₋₇-alkoxy, C₁₋₇-alkoxy-C₁₋₇-alkoxy, -(CH₂)ₙ-NR¹³R¹⁴, -(CH₂)ₙ-NHCOR¹⁵, amino-C₁₋₇-alkoxy, aminocarbonyl-C₁₋₇-alkoxy, C₁₋₇-alkylaminocarbonyl-C₁₋₇-alkoxy and 1*H*-tetrazol-5-yl, and wherein R¹³, R¹⁴, R¹⁵ and n are as defined in claim 1.

4. Compounds of formula I according to any one of claims 1 to 3, wherein R³ is hydrogen and R⁴ is selected from the group consisting of C₁₋₇-alkyl, C₁₋₇-alkoxy, hydroxy, hydroxy-C₁₋₇-alkyl, cyano-C₁₋₇-alkoxy, C₃₋₇-cycloalkyloxy wherein the cycloalkyl group is substituted by carboxy or C₁₋₇-alkoxy-carbonyl, carboxy, C₁₋₇-alkoxy-carbonyl, carboxy-C₁₋₇-alkoxy, C₁₋₇-alkoxy-carbonyl-C₁₋₇-alkyl, carboxy-C₁₋₇-alkyl, C₁₋₇-alkoxy-carbonyl-C₁₋₇-alkoxy, 1*H*-tetrazol-5-yl-C₁₋₇-alkoxy, triazolyl-C₁₋₇-alkoxy, C₁₋₇-alkylsulfonyloxy, C₁₋₇-alkylsulfonyl-C₁₋₇-alkoxy, hydroxy-C₂₋₇-alkoxy, dihydroxy-C₃₋₇-alkoxy, C₁₋₇-alkoxy-C₁₋₇-alkoxy, -(CH₂)ₙ-NR¹³R¹⁴, -(CH₂)ₙ-NHCOR¹⁵, amino-C₁₋₇-alkoxy, aminocarbonyl-C₁₋₇-alkoxy, C₁₋₇-alkylaminocarbonyl-C₁₋₇-alkoxy and 1*H*-tetrazol-5-yl, and wherein R¹³, R¹⁴, R¹⁵ and n are as defined in claim 1.

5. Compounds of formula I according to any one of claim 1 to 3, wherein R⁴ is hydrogen and R³ is selected from the group consisting of C₁₋₇-alkyl, C₁₋₇-alkoxy, hydroxy-C₁₋₇-alkyl, carboxy, C₁₋₇-alkoxy-carbonyl, carboxy-C₁₋₇-alkoxy, C₁₋₇-alkoxycarbonyl-C₁₋₇-alkyl, carboxy-C₁₋₇-akyl, C₁₋₇-alkoxy-carbonyl-C₁₋₇-alkoxy, 1*H*-tetrazol-5-yl-C₁₋₇-alkoxy, hydroxy-C₂₋₇-alkoxy, dihydroxy-C₃₋₇-alkoxy, -(CH₂)ₙ-NR¹³R¹⁴, (CH₂)ₙ-NHCOR¹⁵, and 1*H*-tetrazol-5-yl, and wherein R¹³, R¹⁴, R¹⁵ and n are as defined in claim 1.

6. Compounds of formula I according to any one of claims 1 to 3, wherein R³ and R⁴ are hydrogen.

7. Compounds of formula I according to claims 1 or 2, wherein A is N, B is CR⁴ and R⁴ is selected from the group consisting of hydrogen, C₁₋₇-alkyl, C₁₋₇-alkoxy, hydroxy, hydroxy-C₁₋₇-alkyl, cyano-C₁₋₇-alkoxy, C₃₋₇-cycloalkyloxy wherein the cycloalkyl group is substituted by carboxy or C₁₋₇-alkoxy-carbonyl, carboxy, C₁₋₇-alkoxycarbonyl, carboxy-C₁₋₇-alkoxy, C₁₋₇-alkoxy-carbonyl-C₁₋₇-alkyl, carboxy-C₁₋₇-alkyl, C₁₋₇-alkoxy-carbonyl-C₁₋₇-alkoxy, 1*H*-tetrazol-5-yl-C₁₋₇-alkoxy, triazolyl-C₁₋₇-alkoxy, C₁₋₇-alkylsulfonyloxy, C₁₋₇-alkylsulfonyl-C₁₋₇-alkoxy, hydroxy-C₂₋₇-alkoxy, dihydroxy-C₃₋₇-alkoxy, C₁₋₇-alkoxy-C₁₋₇-alkoxy, -(CH₂)ₙ-NR¹³R¹⁴, -(CH₂)ₙ-NHCOR¹⁵, amino-C₁₋₇-alkoxy, aminocarbonyl-C₁₋₇-alkoxy, C₁₋₇-alkylaminocarbonyl-C₁₋₇-alkoxy and 1*H*-tetrazol-5-yl, and wherein R¹³, R¹⁴, R¹⁵ and n are as defined in claim 1.

8. Compounds of formula I according to any one of claims 1,2 or 7, wherein A is N and B is CH.

9. Compounds of formula I according to claims 7 or 8, wherein R¹ and R² are hydrogen.

10. Compounds of formula I according to any one of claims 1 to 8, wherein
R¹ is selected from the group consisting of halogen, cyano, nitro, C₁₋₇-alkyl, hydroxy-C₁₋₇-alkyl, C₁₋₇-alkoxy-C₁₋₇-alkyl, C₁₋₇-alkoxy, hydroxy-C₂₋₇-alkoxy, dihydroxy-C₃₋₇-alkoxy, carboxy-C₁₋₇-alkoxy, C₁₋₇-alkoxycarbonyl-C₁₋₇-alkoxy, carboxy-C₁₋₇-alkyl, C₁₋₇-alkoxycarbonyl-C₁₋₇-alkyl, 1*H*-tetrazol-5-yl-C₁₋₇-alkoxy, pyridinyl-C₁₋₇-alkoxy, -NR⁵R⁶, -NHCOR⁷, -NHSO₂R⁸, -SO₂NR⁹R¹⁰, 1*H*-tetrazol-5-yl, unsubstituted phenyl and phenyl substituted by one to three substituents selected from C₁₋₇-alkyl, C₃₋₇-cycloalkyl, halogen, halogen-C₁₋₇-alkyl and C₁₋₇-alkoxy; and wherein R⁵ to R¹⁰ are as defined in claim 1, and
R² is hydrogen.

11. Compounds of formula I according to claim 10, wherein R¹ is selected from the group consisting of halogen, nitro, C₁₋₇-alkyl, hydroxy-C₁₋₇-alkyl, C₁₋₇-alkoxy, dihydroxy-C₃₋₇-alkoxy, carboxy-C₁₋₇-alkoxy, C₁₋₇-alkoxycarbonyl-C₁₋₇-alkoxy,-NR⁵R⁶, -NHCOR⁷, -NHSO₂R⁸, -SO₂NR⁹R¹⁰, unsubstituted phenyl and phenyl substituted by one to three substituents selected from C₁₋₇-alkyl, C₃₋₇-cycloalkyl, halogen, halogen-C₁₋₇-alkyl and C₁₋₇-alkoxy; and wherein R⁵ to R¹⁰ are as defined in claim 1.

12. Compounds of formula I according to claims 10 or 11, wherein R¹ is -NR⁵R⁶ or -NHCOR⁷, and wherein R⁵ to R⁷ are as defined in claim 1.

13. Compounds of formula I according to any one of claims 1 to 8, wherein
R¹ is hydrogen and
R² is selected from the group consisting of halogen, cyano, nitro, C₁₋₇-alkyl, hydroxy-C₁₋₇-alkyl, C₁₋₇-alkoxy-C₁₋₇-alkyl, C₁₋₇-alkoxy, hydroxy-C₂₋₇-alkoxy, dihydroxy-C₃₋₇-alkoxy, carboxy-C₁₋₇-alkoxy,C₁₋₇-alkoxycarbonyl-C₁₋₇-alkoxy, carboxy-C₁₋₇-alkyl, C₁₋₇-alkoxycarbonyl-C₁₋₇-alkyl,1*H*-tetrazol-5-yl-C₁₋₇-alkoxy, pyridinyl-C₁₋₇-alkoxy, -NR⁵R⁶, -NHCOR⁷, -NHSO₂R⁸, -SO₂NR⁹R¹⁰, carboxy, C₁₋₇-alkoxycarbonyl, -CONR¹¹R¹², 1*H*-tetrazol-5-yl, unsubstituted phenyl and phenyl substituted by one to three substituents selected from C₁₋₇-alkyl, C₃₋₇-cycloalkyl, halogen, halogen-C₁₋₇-alkyl and C₁₋₇-alkoxy; and wherein R⁵ to R¹² are as defined in claim 1.

14. Compounds of formula I according to claim 13, wherein R² is selected from the group consisting of halogen, nitro, C₁₋₇-alkoxy, pyridinyl-C₁₋₇-alkoxy, -NR⁵R⁶, - NHCOR⁷, carboxy, C₁₋₇-alkoxycarbonyl, -CONR¹¹R¹²; and wherein R⁵ to R⁷, R¹¹ and R¹² are as defined in claim 1.

15. Compounds of formula I according to claim 14, wherein R² is -NHCOR⁷ and R⁷ is as defined in claim 1.

16. Compounds of formula I according to any one of claims 1 to 15, wherein G is 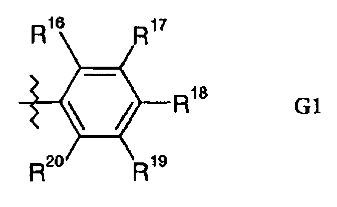 and wherein
R¹⁶ is hydrogen or halogen;
R¹⁷ is selected from the group consisting of C₁₋₇-allkoxy, C₂₋₇-alkenyloxy. C₃₋₇-cycloalkyloxy, -NR²⁹R³⁰, halogen-C₁₋₇-alkoxy, C₁₋₇-alkoxy-C₁₋₇-alkoxy, C₁₋₇-alkoxy-C₁₋₇-alkyl; R²⁹ and R³⁰ independently from each other are hydrogen or C₁₋₇-alkyl;
R¹⁸ is selected from the group consisting of hydrogen, C₁₋₇-alkyl, halogen-C₁₋₇-alkyl, hydroxy, C₁₋₇-alkoxy, halogen-C₁₋₇-alkoxy, C₃₋₇-cycloalkyloxy, halogen, pyrrolo, imidazolyl, triazolyl, -CO₂R³¹, -NR³²R³³, -SOR³⁴; unsubstituted phenyl and phenyl substituted by one to two groups selected from the group consisting of C₁₋₇-alkyl, hatogen-C₁₋₇-alkyl, halogen-C₁₋₇-alkoxy, C₁₋₇-alkoxy and halogen;
R³¹ is hydrogen or C₁₋₇-alkyl;
R³² and R³³ independently from each other are hydrogen or C₁₋₇-alkyl;
R³⁴ is C₁₋₇-alkyl;
R¹⁹ is selected from the group consisting of hydrogen, C₁₋₇-alkyl, halogen, C₁₋₇-alkoxy, C₂₋₇-alkenyloxy, -O-tetrahydropyranyl, C₃₋₇-cycloalkyloxy, halogen-C₁₋₇-alkoxy, C₁₋₇-alkoxy-C₁₋₇-alkoxy, and C₁₋₇-alkoxy-C₁₋₇-alkyl; and
R²⁰ is hydrogen or halogen.

17. Compounds of formula I according to any of claims 1 to 16, wherein R¹⁷ is C₁₋₇-alkoxy or halogen-C₁₋₇-alkoxy.

18. Compounds of formula I according to any of claims 1 to 17, wherein R¹⁷ is ethoxy, isopropyloxy or isobutyloxy.

19. Compounds of formula I according to any one of claims 1 to 18, wherein R¹⁸ is selected from the group consisting of hydrogen, C₁₋₇-alkyl, hydroxy, C₁₋₇-alkoxy, halogen, pyrrolo, imidazolyl, triazolyl, -NR³²R³³ and -SOR³⁴, and R³² and R³³ are independently from each other hydrogen or C₁₋₇-alkyl, and R³⁴ is C₁₋₇-alkyl.

20. Compounds of formula I according to any one of claims 1 to 19, wherein R¹⁸ is selected from the group consisting of hydrogen, halogen, pyrrolo, triazolyl and -NR³²R³³, wherein R³² and R³³ are hydrogen.

21. Compounds of formula I according to claim 1, wherein
X is S or O;
A is CR³ and B is CR⁴, or
A is N or N⁺-O⁻ and B is CR⁴, or
B is N or N⁺-O⁻ and A is CR³;
R¹ and R² are independently selected from the group consisting of hydrogen, halogen, cyano, nitro, C₁₋₇-alkyl, hydroxy-C₁₋₇-alkyl, C₁₋₇-alkoxy-C₁₋₇-alkyl, C₁₋₇-alkoxy, hydroxy-C₂₋₇-alkoxy, dihydroxy-C₃₋₇-akoxy, carboxy-C₁₋₇-alkoxy, C₁₋₇-alkoxycarbonyl-C₁₋₇-alkoxy, carboxy-C₁₋₇-alkyl, C₁₋₇-alkoxycarbonyl-C₁₋₇-alkyl, 1*H*-tetrazol-5-yl-C₁₋₇-alkoxy, pyridinyl-C₁₋₇-alkoxy, -NR⁵R⁶, -NHCOR⁷, -NHSO₂R⁸, -SO₂NR⁹R¹⁰, 1*H*-tetrazol-5-yl, unsubstituted phenyl and phenyl substituted by one to three substituents selected from C₁₋₇-alkyl, C₃₋₇-cycloalkyl, halogen, halogen-C₁₋₇-alkyl and C₁₋₇-alkoxy; and
R² can additionally also be selected from the group consisting of carboxy, C₁₋₇-alkoxycarbonyl and -CONR¹¹R¹²;
R⁵ and R⁶ independently from each other are selected from the group consisting of hydrogen, C₁₋₇-alkyl, hydroxy-C₁₋₇-alkyl and C₃₋₇-cycloalkyl;
R⁷ is selected from the group consisting of C₁₋₇-alkyl, C₃₋₇-cycloalkyl, halogen-C₁₋₇-alkyl, hydroxy-C₁₋₇-alkyl, C₁₋₇-alkoxy-C₁₋₇-alkyl, carboxy-C₁₋₇-alkyl, C₁₋₇-alkoxycarbonyl-C₁₋₇-alkyl, 1*H*-tetrazol-5-yl-C₁₋₇- alkyl, unsubstituted phenyl, phenyl substituted by one to three groups selected from the group consisting of C₁₋₇-alkyl, C₃₋₇-cycloalkyl, C₁₋₇-alkoxy, halogen-C₁₋₇-alkyl and halogen, unsubstituted heteroaryl, heteroaryl substituted by one or two groups selected from C₁₋₇-alkyl, C₃₋₇-cycloalkyl, C₁₋₇-alkoxy, halogen-C₁₋₇-alkyl or halogen; unsubstituted heteroaryl-C₁₋₇-akyl and heteroaryl-C₁₋₇-alkyl, wherein the heteroaryl is substituted by one or two groups selected from C₁₋₇-alkyl, C₃₋₇- cycloalkyl, C₁₋₇-alkoxy, halogen-C₁₋₇-alkyl and halogen;
R⁸ is selected from the group consisting of C₁₋₇-alkyl, C₃₋₇-cycloalkyl, unsubstituted heteroaryl and heteroaryl substituted by one or two groups selected from C₁₋ ₇-alkyl, C₃₋₇-cycloakyl, C₁₋₇-alkoxy, halogen-C₁₋₇-alkyl or halogen;
R⁹ and R¹⁰ independently from each other are selected from the group consisting of hydrogen, C₁₋₇-alkyl, C₃₋₇-cycloalkyl, unsubstituted heteroaryl and heteroaryl substituted by one or two groups selected from C₁₋₇-alkyl, C₃₋₇-cycloalkyl, C₁₋₇-alkoxy, halogen-C₁₋₇-alkyl or halogen; or R⁹ and R¹⁰ together with the nitrogen atom they are attached to form a pyrrolidine or a piperidine ring;
R¹¹ is selected from the group consisting of hydrogen, C₁₋₇-alkyl and C₃₋₇- cycloalkyl;
R¹² is selected from the group consisting of hydrogen, C₁₋₇-alkyl, C₃₋₇-cycloalkyl, halogen-C₁₋₇-alkyl, hydroxy-C₁₋₇-alkyl, C₁₋₇-alkoxy-C₁₋₇-alkyl, carboxy-C₁₋₇-alkyl, C₁₋₇-alkoxycarbonyl-C₁₋₇-alkyl, unsubstituted phenyl, phenyl substituted by one to three groups selected from the group consisting of C₁₋₇-alkyl, C₃₋₇-cycloalkyl, C₁₋₇-alkoxy, halogen-C₁₋₇-alkyl and halogen, unsubstituted heteroaryl, heteroaryl substituted by one or two groups selected from C₁₋₇-alkyl, C₃₋₇-cycloalkyl, C₁₋₇-alkoxy, halogen-C₁₋₇-alkyl or halogen;
one of R³ and R⁴ is selected from the group consisting of hydrogen, C₁₋₇-alkyl and C₁₋₇- alkoxy, or is absent in case one of A or B is N or N⁺-O⁻, and
the other one of R³ and R⁴ is selected from the group consisting of hydrogen, C₁₋₇-alkyl, C₁₋₇-alkoxy, hydroxy-C₁₋₇-alkyl, carboxy, C₁₋₇-alkoxy-carbonyl, carboxy-C₁₋₇-alkoxy, C₁₋₇-alkoxy-carbonyl-C₁₋₇-alkyl, carboxy-C₁₋₇-alkyl, C₁₋₇-alkoxy-carbonyl-C₁₋₇-alkoxy, 1*H*-tetrazol-5-yl-C₁₋₇-alkoxy, hydroxy-C₂₋₇-alkoxy, dihydroxy-C₃₋₇-alkoxy, -(CH₂)ₙ-NR¹³R¹⁴, -(CH₂)ₙ-NHCOR¹⁵, and 1*H*-tetrazol-5-yl;
R¹³ and R¹⁴ independently from each other are selected from the group consisting of hydrogen, C₁₋₇-alkyl and C₃₋₇-cycloalkyl;
R¹⁵ is selected from the group consisting of C₁₋₇-alkyl, C₃₋₇-cycloalkyl, halogen-C₁₋₇-alkyl, hydroxy-C₁₋₇-alkyl, C₁₋₇-alkoxy-C₁₋₇-alkyl, carboxy-C₁₋₇-alkyl and C₁₋₇-alkoxycarbonyl-C₁₋₇-alkyl;
n is an integer selected from 1,2 and 3;
provided that benzooxazoles and benzothiazoles wherein R¹, R², R³ and R⁴ are hydrogen are excluded;
G is selected from the groups 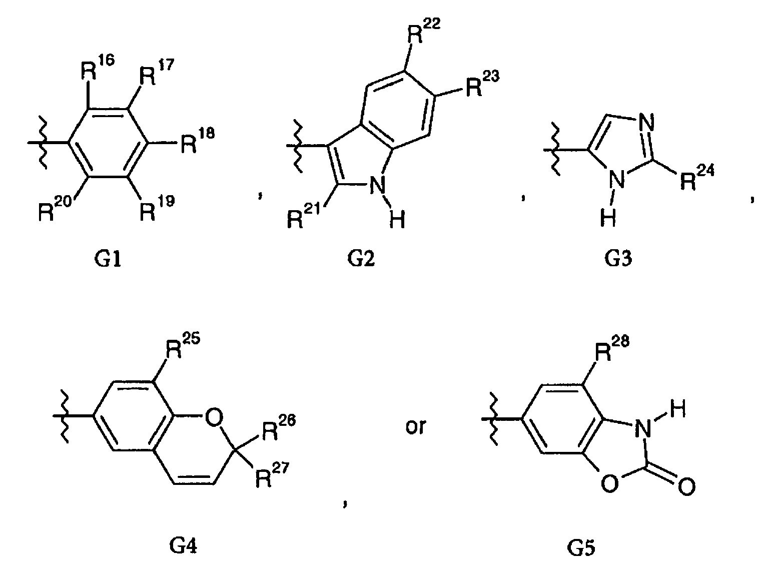
wherein
R¹⁶ is hydrogen or halogen;
R¹⁷ is selected from the group consisting of C₁₋₇-alkoxy, C₂₋₇-alkenyloxy, C₃₋₇-cycloalkyloxy, -NR²⁹R³⁰, halogen-C₁₋₇-alkoxy, C₁₋₇-alkoxy-C₁₋₇-alkoxy, C₁₋₇-alkoxy-C₁₋₇-alkyl; R²⁹ and R³⁰ independently from each other are hydrogen or C₁₋₇-alkyl;
R¹⁸ is selected from the group consisting of hydrogen, C₁₋₇-alkyl, halogen-C₁₋₇-alkyl, hydroxy, C₁₋₇-alkoxy, halogen-C₁₋₇-alkoxy, C₃₋₇-cycloalkyloxy, halogen, pyrrolo, -CO₂R³¹, -NR³²R³³, -SOR³⁴; unsubstituted phenyl and phenyl substituted by one to two groups selected from the group consisting of C₁₋₇-alkyl, halogen-C₁₋₇-alkyl, halogen-C₁₋₇-alkoxy, C₁₋₇-alkoxy and halogen;
R³¹ is hydrogen or C₁₋₇-alkyl;
R³² and R³³ independently from each other are hydrogen or C₁₋₇-alkyl;
R³⁴ is C₁₋₇-alkyl;
R¹⁹ is selected from the group consisting of hydrogen, C₁₋₇-alkyl, halogen, C₁₋₇-alkoxy, C₂₋₇-alkenyloxy, -O-tetrahydropyranyl, C₃₋₇-cyloalkyloxy, halogen-C₁₋₇-alkoxy, C₁₋₇-alkoxy-C₁₋₇-alkoxy, and C₁₋₇-alkoxy-C₁₋₇-alkyl;
R²⁰ is hydrogen or halogen;
R²¹ is hydrogen or C₁₋₇-alkyl;
R²² and R²³ independently from each other are selected from the group consisting of hydrogen, C₁₋₇-alkyl, C₁₋₇-alkoxy and nitro;
R²⁴ is unsubstituted phenyl or phenyl substituted by one to two groups selected from the group consisting of C₁₋₇-alkyl, C₁₋₇-alkoxy and halogen;
R²⁵ is C₁₋₇-alkoxy;
R²⁶ and R²⁷ independently from each other are C₁₋₇-alkyl;
R²⁸ is C₁₋₇-alkoxy;
and pharmaceutically acceptable salts thereof.

22. Compounds of formula I according to claim 1, selected from the group consisting of
[1-(3-ethoxy-4-methoxy-benzyl)-piperidin-4-yl]-(7-nitro-benzooxazol-2-yl)-amine, [1-(3,5-diethoxy-4-fluoro-benzyl)-piperidin-4-yl]-(7-nitro-benzooxazol-2-yl)-amine,
*N²-*[1-(3,5-diethoxy-4-fluoro-benzyl)-piperidin-4-yl]-benzooxazole-2,7-diamine,
*N-*{2-[1-(3,5-diethoxy-4-fluoro-benzyl)-piperidin-4-ylamino]-benzooxazol-7-yl}-2-methoxy-acetamide,
*N*-{2-[1-(3,5-diethoxy-4-fluoro-benzyl)-piperidin-4-ylamino]-benzooxazol-7-yl}-succinamic acid,
pyrimidine-5-carboxylic acid {2-[1-(3,5-diethoxy-4-fluoro-benzyl)-piperidin-4-ylamino]-benzooxazol-7-yl}-amide,
*N*-(2-[1-(3,5-diethoxy-4-fluoro-benzyl)-piperidin-4-ylamino]-benzooxazol-7-yl}-2-pyridin-3-yl-acetamide,
1-methyl-1H-imidazole-4-sulfonic acid {2-[1-(3,5-diethoxy-4-fluoro-benzyl)-piperidin-4-ylamino]-benzooxazol-7-yl}-amide,
2-[1-(3,5-diethoxy-4-fluoro-benzyl)-piperidin-4-ylamino]-benzooxazol-7-yl}-methanesulfonamide,
[1-(3,5-diethoxy-4-fluoro-benzyl)-piperidin-4-yl]-[7-(pyrrolidine-1-sulfonyl)-benzooxazol-2-yl] -amine,
[1-(3,5-diethoxy-benzyl)-piperidin-4-yl]-[7-(pyrrolidine-1-sulfonyl)-benzooxazol-2-yl]-amine,
[1-(3-ethoxy-4-methoxy-benzyl)-piperidin-4-yl]-[7-(pyrrolidine-1-sulfonyl)-benzooxazol-2-yl]-amine,
[1-(3,5-diethoxy-4-pyrrol-1-yl-benzyl)-piperidin-4-yl]-[7-(pyrrolidine-1-sulfonyl)-benzooxazol-2-yl]-amine,
[1-(4-chloro-3-ethoxy-benzyl)-piperidin-4-yl]-[7-(pyrrolidine-1-sulfonyl)-benzooxazol-2-yl]-amine,
[1-(3-ethoxy-4-methoxy-benzyl)-piperiain-4-yl]-(4-nitro-benzooxazol-2-yl)-amine, [1-(3,5-diethoxy-4-pyrrol-1-yl-benzyl)-piperidin-4-yl]-(4-nitro-benzooxazol-2-yl)-amine,
[1-(3,5-diethoxy-4-fluoro-benzyl)-piperidin-4-yl]-(4-nitro-benzooxazol-2-yl)-amine, *N*²-[1-(3,5-diethoxy-4-fluoro-benzyl)-piperidin-4-yl]-benzooxazole-2,4-diamine,
*N*-{2-[1-(3,5-diethoxy-4-fluoro-benzyl)-piperidin-4-ylamino]-benzooxazol-4-yl}-2-methoxy-acetamide,
*N*-{2-[1-(3,5-diethoxy-4-fluoro-benzyl)-piperidin-4-ylamino]-benzooxazol-4-yl}-succinamic acid methyl ester,
*N*-{2-[1-(3,5-diethoxy-4-fluoro-benzyl)-piperidin-4-ylamino]-benzooxazol-4-yl}-succinamic acid,
pyrimidine-5-carboxylic acid {2-[1-(3,5-diethoxy-4-fluoro-benzyl)-piperidin-4-ylamino]-benzooxazol-4-yl}-amide,
*N*²-[1-(3,5-diethoxy-4-pyrrol-1-yl-benzyl)-piperidin-4-yl]-benzooxazole-2,4-diamine,
*N*-{2-[1-(3,5-diethoxy-4-pyrrol-1-yl-benzyl)-piperidin-4-ylamino]-benzooxazol-4-yl}-2-methoxy-acetamide,
*N*-{2-[1-(3,5-diethoxy-4-fluoro-benzyl)-piperidin-4-ylamino]-benzooxazol-4-yl}-2-(1H-tetrazol-5-yl)-acetamide,
*N*-{2-[1-(3,5-diethoxy-4-pyrrol-1-yl-benzyl)-piperidin-4-ylamino]-benzooxazol-4-yl}-succinamic acid methyl ester,
*N*-{2-[1-(3,5-diethoxy-4-pyrrol-1-yl-benzyl)-piperidin-4-ylamino]-benzooxazol-4-yl}-succinamic acid,
[1-(3-ethoxy-4-methoxy-benzyl)-piperidin-4-yl]-[4-(pyridin-4-ylmethoxy)-benzooxazol-2-yl)-amine,
[1-(3,5-diethoxy-4-pyrrol-1-yl-benzyl)-piperidin-4-yl]-[4-(pyridin-4-ylmethoxy)-benzooxazol-2-yl] -amine,
[1-(3,5-diethoxy-4-fluoro-benzyl)-piperidin-4-yl]-[4-(pyridin-4-ylmethoxy)-benzooxazol-2-yl] -amine,
2-[1-(3-ethoxy-4-methoxy-benzyl)-piperidin-4-ylamino]-benzooxazole-4-carboxylic acid methyl ester,
2-[1-(3,5-diethoxy-4-pyrrol-1-yl-benzyl)-piperidin-4-ylamino]-benzooxazole-4-carboxylic acid methyl ester,
2-[1-(3,5-diethoxy-4-fluoro-benzyl)-piperidin-4-ylamino]-benzooxazole-4-carboxylic acid methyl ester,
2-[1-(3-ethoxy-4-methoxy-benzyl)-piperidin-4-ylamino]-benzooxazole-4-carboxylic acid,
2-[1-(3,5-diethoxy-4-pyrrol-1-yl-benzyl)-piperidin-4-ylamino]-benzooxazole-4-carboxylic acid,
2-[1-(3,5-diethoxy-4-fluoro-benzyl)-piperidin-4-ylamino]-benzooxazole-4-carboxylic acid,
2-[1-(3-ethoxy-4-methoxy-benzyl)-piperidin-4-ylamino]-4-benzooxazole-4-carboxylic acid pyridin-3-ylamide,
2-[1-(3,5-diethoxy-4-fluoro-benzyl)-piperidin-4-ylamino]-benzooxazole-4-carboxylic acid pyridin-3-ylamide,
2-[1-(3,5-diethoxy-4-pyrrol-1-yl-benzyl)-piperidin-4-ylamino]-benzooxazole-4-carboxylic acid pyridin-3-ylamide,
2-[1-(3-ethoxy-4-methoxy-benzyl)-piperidin-4-ylamino]-benzooxazole-4-carboxylic acid (2-hydroxy-ethyl)-amide,
2-[1-(3,5-diethoxy-4-fluoro-benzyl)-piperidin-4-ylamino]-benzooxazole-4-carboxylic acid (2-hydroxy-ethyl)-amide,
2-[1-(3,5-diethoxy-4-pyrrol-1-yl-benzyl)-piperidin-4-ylamino]-benzooxazole-4-carboxylic acid (2-hydroxy-ethyl)-amide,
({2-[1-(3-ethoxy-4-methoxy-benzyl)-piperidin-4-ylamino]-benzooxazole-4-carbonyl}-amino)-acetic acid methyl ester,
({2-[1-(3,5-diethoxy-4-fluoro-benzyl)-piperidin-4-ylamino]-benzooxazole-4-carbonyl}-amino)-acetic acid methyl ester,
({2-[1-(3,5-diethoxy-4-pyrrol-1-yl-benzyl)-piperidin-4-ylamino]-benzooxazole-4-carbonyl}-amino)-acetic acid methyl ester,
({2-[1-(3-ethoxy-4-methoxy-benzyl)-piperidin-4-ylamino]-benzooxazole-4-carbonyl}-amino)-acetic acid,
({2-[1-(3,5-diethoxy-4-fluoro-benzyl)-piperidin-4-ylamino]-benzooxazole-4-carbonyl}-amino)-acetic acid,
({2-[1-(3,5-diethoxy-4-pyrrol-1-yl-benzyl)-piperidin-4-ylamino]-benzooxazole-4-carbonyl}-amino)-acetic acid,
[1-(3-ethoxy-4-methoxy-benzyl)-piperidin-4-yl]-(4-iodo-benzooxazol-2-yl)-amine, [1-(3,5-diethoxy-4-fluoro-benzyl)-piperidin-4-yl]-(4-iodo-benzooxazol-2-yl)-amine, [1-(3,5-diethoxy-4-pyrrol-1-yl-benzyl)-piperidin-4-yl]-(4-iodo-benzooxazol-2-yl)-amine,
(7-bromo-benzooxazol-2-yl)-[1-(3-ethoxy-4-methoxy-benzyl)-piperidin-4-yl]-amine, (7-bromo-benzooxazol-2-yl)-[1-(3,5-diethoxy-4-fluoro-benzyl)-piperidin-4-yl]-amine,
(7-bromo-benzooxazol-2-yl)-[1-(3,5-diethoxy-4-pyrrol-1-yl-benzyl)-piperidin-4-yl]-amine,
2-[1-(3-ethoxy-4-hydroxy-benzyl)-piperidin-4-ylamino]-benzooxazole-6-carboxylic acid methyl ester,
2-[1-(3-ethoxy-4-methoxy-benzyl)-piperidin-4-ylamino]-benzooxazole-6-carboxylic acid methyl ester,
2-[1-(8-ethoxy-2,2-dimethyl-2*H*-chromen-6-ymethyl)-piperidin-4-ylamino]-benzooxazole-6-carboxylic acid methyl ester,
2-[1-(3-isobutoxy-4-methoxy-benzyl)-piperidin-4-ylamino]-benzooxazole-6-carboxylic acid methyl ester,
2-{1-[3-(2-fluoro-ethoxy)-4-methoxy-benzyl]-piperidin-4-ylamino}-benzooxazole-6-carboxylic acid methyl ester,
2-[1-(3-ethoxy-4-methyl-benzyl)-piperidin-4-ylamino]-benzooxazole-6-carboxylic acid methyl ester,
2-[1-(4-chloro-3-ethoxy-benzyl)-piperidin-4-ylamino]-benzooxazole-6-carboxylic acid methyl ester,
2-[1-(4-amino-3,5-diethoxy-benzyl)-piperidin-4-ylamino]-benzooxazole-6-carboxylic acid methyl ester,
2-[1-(3,5-diethoxy-4-fluoro-benzyl)-piperidin-4-ylamino]-benzooxazole-6-carboxylic acid methyl ester,
2-[1-(3-ethoxy-4-hydroxy-benzyl)-piperidin-4-ylamino]-benzooxazole-6-carboxylic acid,
2-[1-(3-ethoxy-4-methoxy-benzyl)-piperidin-4-ylamino]-benzooxazole-6-carboxylic acid,
2-[1-(8-ethoxy-2,2-dimethyl-2*H*-chromen-6-ylmethyl)-piperidin-4-ylamino]-benzooxazole-6-carboxylic acid,
2-[1-(3-isobutoxy-4-methoxy-benzyl)-piperidin-4-ylamino]-benzooxazole-6-carboxylic acid,
2-{1-[3-(2-fluoro-ethoxy)-4-methoxy-benzyl]-piperidin-4-ylamino}-benzooxazole-6-carboxylic acid,
2-[1-(3-ethoxy-4-methyl-benzyl)-piperidin-4-ylamino]-benzooxazole-6-carboxylic acid,
2-[1-(4-chloro-3-ethoxy-benzyl)-piperidin-4-ylamino]-benzooxazole-6-carboxylic acid, 2-[1-(4-amino-3,5-diethoxy-benzyl)-piperidin-4-ylamino]-benzooxazole-6-carboxylic acid,
2-[1-(3,5-diethoxy-4-fluoro-benzyl)-piperidin-4-ylamino]-benzooxazole-6-carboxylic acid,
[1-(3-ethoxy-4-fluoro-benzyl)-piperidin-4-yl]-(4-nitro-benzooxazol-2-yl)-amine, [1-(4-chloro-3-ethoxy-benzyl)-piperidin-4-yl]-(4-nitro-benzooxazol-2-yl)-amine, [1-(3-ethoxy-4-methyl-benzyl)-piperidin-4-yl]-(4-nitro-benzooxazol-2-yl)-amine, 2-[1-(3,5-diethoxy-4-fluoro-benzyl)-piperidin-4-ylamino]-benzooxazole-7-sulfonic acid amide,
2-[1-(3-ethoxy-4-fluoro-benzyl)-piperidin-4-ylamino]-benzooxazole-7-sulfonic acid amide,
2-[1-(3-ethoxy-4-methoxy-benzyl)-piperidin-4-ylamino]-benzooxazole-7-sulfonic acid amide,
[1-(3,5-diethoxy-4-fluoro-benzyl)-piperidin-4-yl]-(7-methoxy-benzooxazol-2-yl)-amine,
[1-(3,5-diethoxy-4-pyrrol-1-yl-benzyl)-piperidin-4-yl]-(7-methoxy-benzooxazol-2-yl)-amine,
[1-(3,5-diethoxy-4-fluoro-benzyl)-piperidin-4-yl]-oxazolo[5,4-*c*]pyridin-2-yl-amine, [1-(4-chloro-3-ethoxy-benzyl)-piperidin-4-yl]-oxazolo[5,4-*c*]pyridin-2-yl-amine,
[1-(3-ethoxy-4-methyl-benzyl)-piperidin-4-yl]-oxazolo [5,4-*c*] pyridin-2-yl-amine, [1-(3-ethoxy-4-methoxy-benzyl)-piperidin-4-yl]-oxazolo[5,4-*c*]pyridin-2-ylamine, [1-(3-isobutoxy-4-methoxy-benzyl)-piperidin-4-yl]-oxazolo[5,4-*c*]pyridin-2-yl-amine, 4-ethoxy-6-[4-(oxazolo[5,4-*c*]pyridin-2-ylamino)-piperidin-1-ylmethyl]-3H-benzooxazol-2-one,
[rac]-[1-(3,5-diethoxy-4-methanesulfinyl-benzyl)-piperidin-4-yl]-oxazolo [5,4-*c*]pyridin-2-yl-amine,
[1-(3-ethylamino-4-methoxy-benzyl)-piperidin-4-yl]-oxazolo[5,4-*c*]pyridin-2-yl-amine, [1-(8-ethoxy-2,2-dimethyl-2*H*-chromen-6-ylmethyl)-piperidin-4-yl]-oxazolo [5,4-*c*]pyridin-2-yl-amine,
[1-(4-methoxy-3-propoxy-benzyl)-piperidin-4-yl]-oxazolo[5,4-*c*] pyridin-2-yl-amine, {1-[3-(2-fluoro-ethoxy)-4-methoxy-benzyl]-piperidin-4-yl}-oxazolo[5,4-*c*]pyridin-2-yl-amine,
2-ethoxy-4-[4-(oxazolo[5,4-*c*]pyridin-2-ylamino)-piperidin-1-ylmethyl]-phenol, [1-(4-amino-3,5-diethoxy-benzyl)-piperidin-4-yl]-oxazolo[5,4-*c*]pyridin-2-yl-amine, [1-(3,5-diisopropoxy-benzyl)-piperidin-4-yl]-oxazolo[5,4-*c*]pyridin-2-yl-amine,
[1-(3-ethoxy-4-fluoro-benzyl)-piperidin-4-yl]-oxazolo[5,4-*c*]pyridin-2-yl-amine,
[1-(3-ethoxy-4-isopropoxy-benzyl)-piperidin-4-yl]-oxazolo[5,4-*c*] pyridin-2-yl-amine, oxazolo [5,4-*c*]pyridin-2-yl-[1-(2-phenyl-3H-imidazol-4-ylmethyl)-piperidin-4-yl]-amine,
[1-(2-methyl-5-nitro-1H-indol-3-ylmethyl)-piperidin-4-yl]-oxazolo[5,4-*c*]pyridin-2-yl-amine,
{2-[1-(3-ethoxy-4-methoxy-benzyl)-piperidin-4-ylamino]-benzooxazol-5-yloxy}-acetic acid methyl ester,
{2-[1-(3,5-diethoxy-4-fluoro-benzyl)-piperidin-4-ylamino]-benzooxazol-5-yloxy}-acetic acid methyl ester,
{2-[1-(3,5-diethoxy-4-pyrrol-1-yl-benzyl)-piperidin-4-ylamino]-benzooxazol-5-yloxy}-acetic acid methyl ester,
{2-[1-(3-ethoxy-4-methoxy-benzyl)-piperidin-4-ylamino]-benzooxazol-5-yloxy}-acetic acid,
{2-[1-(3,5-diethoxy-4-fluoro-benzyl)-piperidin-4-ylamino]-benzooxazol-5-yloxy}-acetic acid,
{2-[1-(3,5-diethoxy-4-pyrrol-1-yl-benzyl)-piperidin-4-ylamino]-benzooxazol-5-yloxy}-acetic acid,
[1-(3,5-diethoxy-4-fluoro-benzyl)-piperidin-4-yl]-(7-phenyl-benzooxazol-2-yl)-amine, {2-[1-(3-ethoxy-4-methoxy-benzyl)-piperidin-4-ylamino]-benzooxazol-7-yloxy}-acetic acid methyl ester,
{2-[1-(3,5-diethoxy-4-fluoro-benzyl)-piperidin-4-ylamino]-benzooxazol-7-yloxy}-acetic acid methyl ester,
{2-[1-(3,5-diethoxy-4-pyrrol-1-yl-benzyl)-piperidin-4-ylamino]-benzooxazol-7-yloxy}-acetic acid methyl ester,
{2-[1-(3-ethoxy-4-methoxy-benzyl)-piperidin-4-ylamino]-benzooxazol-7-yloxy}-acetic acid,
{2-[1-(3,5-diethoxy-4-fluoro-benzyl)-piperidin-4-ylamino]-benzooxazol-7-yloxy}-acetic acid,
{2-[1-(3,5-diethoxy-4-pyrrol-1-yl-benzyl)-piperidin-4-ylamino]-benzooxazol-7-yloxy}-acetic acid,
(5-aminomethyl-benzooxazol-2-yl)-[1-(3,5-diethoxy-4-fluoro-benzyl)-piperidin-4-yl]-amine,
4-{2-[1-(3,5-diethoxy-4-fluoro-benzyl)-piperidin-4-ylamino]-benzooxazol-7-yloxy}-butyric acid methyl ester,
4-{2-[1-(3,5-diethoxy-4-pyrrol-1-yl-benzyl)-piperidin-4-ylamino]-benzooxazol-7-yloxy}-butyric acid methyl ester,
4-{2-[1-(3-ethoxy-4-methoxy-benzyl)-piperidin-4-ylamino]-benzooxazol-7-yloxy}-butyric acid methyl ester,
4-{2-[1-(3,5-diethoxy-4-fluoro-benzyl)-piperidin-4-ylamino]-benzooxazol-7-yloxy}-butyric acid,
4-{2-[1-(3,5-diethoxy-4-pyrrol-1-yl-benzyl)-piperidin-4-ylamino]-benzooxazol-7-yloxy}-butyric acid,
4-{2-[1-(3-ethoxy-4-methoxy-benzyl)-piperidin-4-ylamino]-benzooxazol-7-yloxy}-butyric acid,
[rac]-3-{2-[1-(3,5-diethoxy-4-fluoro-benzyl)-piperidin-4-ylamino]-benzooxazol-7-yloxy}-propane-1,2-diol,
[rac]-3-{2-[1-(3,5-diethoxy-4-pyrrol-1-yl-benzyl)-piperidin-4-ylamino] -benzooxazol-7-yloxy}-propane-1,2-diol,
[rac]-3-{2-[1-(3-ethoxy-4-methoxy-benzyl)-piperidin-4-ylamino]-benzooxazol-7-yloxy}-propane-1,2-diol,
4-{2-[1-(3,5-diethoxy-4-fluoro-benzyl)-piperidin-4-ylamino]-benzooxazol-5-yloxy}-butyric acid methyl ester,
4-{2-[1-(3,5-diethoxy-4-pyrrol-1-yl-benzyl)-piperidin-4-ylamino]-benzooxazol-5-yloxy}-butyric acid methyl ester,
4-{2-[1-(3-ethoxy-4-methoxy-benzyl)-piperidin-4-ylamino]-benzooxazol-5-yloxy}-butyric acid methyl ester,
4-{2-[1-(3,5-diethoxy-4-fluoro-benzyl)-piperidin-4-ylamino]-benzooxazol-5-yloxy}-butyric acid,
4-{2-[1-(3,5-diethoxy-4-pyrrol-1-yl-benzyl)-piperidin-4-ylamino]-benzooxazol-5-yloxy}-butyric acid,
4-{2-[1-(3-ethoxy-4-methoxy-benzyl)-piperidin-4-ylamino]-benzooxazol-5-yloxy}-butyric acid,
1-{2-[1-(3,5-diethoxy-4-fluoro-benzyl)-piperidin-4-ylamino]-benzooxazol-5-yloxy}-cyclobutanecarboxylic acid ethyl ester,
1-{2-[1-(3,5-diethoxy-4-pyrrol-1-yl-benzyl)-piperidin-4-ylamino]-benzooxazol-5-yloxy}-cyclobutanecarboxylic acid ethyl ester,
1-{2-[1-(3,5-diethoxy-4-fluoro-benzyl)-piperidin-4-ylamino]-benzooxazol-5-yloxy}-cyclobutanecarboxylic acid,
1-{2-[1-(3,5-diethoxy-4-pyrrol-1-yl-benzyl)-piperidin-4-ylamino]-benzooxazol-5-yloxy}-cyclobutanecarboxylic acid,
*N*-{2-[1-(3,5-diethoxy-4-fluoro-benzyl)-piperidin-4-ylamino]-benzooxazol-5-ylmethyl}-acetamide,
N-{2-[1-(3,5-diethoxy-4-fluoro-benzyl)-piperidin-4-ylamino]-benzooxazol-5-ylmethyl}-malonamic acid ethyl ester,
(S)-*N*-{2-[1-(3,5-diethoxy-4-fluoro-benzyl)-piperidin-4-ylamino]-benzooxazol-5-ylmethyl}-2-hydroxy-propionamide,
[1-(3,5-diethoxy-4-fluoro-benzyl)-piperidin-4-yl]-(4-methyl-oxazolo[5,4-c]pyridin-2-yl)-amine,
[1-(3,5-diethoxy-4-pyrrol-1-yl-benzyl)-piperidin-4-yl]-(4-methyl-oxazolo[5,4-c] pyridin-2-yl) -amine,
[1-(3,5-diisopropoxy-benzyl)-piperidin-4-yl]-(4-methyl-oxazolo[5,4-c]pyridin-2-yl)-amine,
*N*-{2-[1-(3,5-diethoxy-4-fluoro-benzyl)-piperidin-4-ylamino]-benzooxazol-5-ylmethyl}-malonamic acid,
[rac]-3-{2-[1-(3,5-diethoxy-4-fluoro-benzyl)-piperidin-4-ylamino]-benzooxazol-5-yloxy}-propane-1,2-diol,
[rac]-3-{2-[1-(3,5-diethoxy-4-pyrrol-1-yl-benzyl)-piperidin-4-ylamino]-benzooxazol-5-yloxy}-propane-1,2-diol,
[rac]-3-{2-[1-(3,5-diisopropoxy-benzyl)-piperidin-4-ylamino]-benzooxazol-5-yloxy}-propane-1,2-diol,
[1-(3,5-diethoxy-4-pyrrol-1-yl-benzyl)-piperidin-4-yl]-[5-(1H-tetrazol-5-ylmethoxy)-benzooxazol-2-yl]-amine,
[1-(3,5-diethoxy-4-fluoro-benzyl)-piperidin-4-yl]-[5-(1H-tetrazol-5-ylmethoxy)-benzooxazol-2-yl]-amine,
{2-[1-(3,5-diethoxy-4-fluoro-benzyl)-piperidin-4-ylamino]-oxazolo[5,4-c]pyridin-4-yl}-methanol,
{2-[1-(3,5-diethoxy-4-pyrrol-1-yl-benzyl)-piperidin-4-ylamino]-oxazolo [5,4-c] pyridin-4-yl}-methanol,
{2-[1-(3,5-diisopropoxy-benzyl)-piperidin-4-ylamino]-oxazolo[5,4-c]pyridin-4-yl}-methanol,
{2-[1-(3,5-diethoxy-4-[1,2,4]triazol-1-yl-benzyl)-piperidin-4-ylamino]-oxazolo[5,4-c]pyridin-4-yl}-methanol,
{2-[1-(3,5-diethoxy-4-fluoro-benzyl)-piperidin-4-ylamino]-benzooxazol-5-yl}-acetic acid methyl ester,
{2-[1-(3,5-diethoxy-4-pyrrol-1-yl-benzyl)-piperidin-4-ylamino]-benzooxazol-5-yl}-acetic acid methyl ester,
{2-[1-(3,5-diisopropoxy-benzyl)-piperidin-4-ylamino]-benzooxazol-5-yl}-acetic acid methyl ester,
{2-[1-(3,5-diethoxy-4-[1,2,4]triazol-1-yl-benzyl)-piperidin-4-ylamino]-benzooxazol-5-yl}-acetic acid methyl ester,
{2-[1-(3,5-diethoxy-4-fluoro-benzyl)-piperidin-4-ylamino]-benzooxazol-5-yl}-acetic acid,
{2-[1-(3,5-diethoxy-4-pyrrol-1-yl-benzyl)-piperidin-4-ylamino]-benzooxazol-5-yl}-acetic acid,
{2-[1-(3,5-diisopropoxy-benzyl)-piperidin-4-ylamino]-benzooxazol-5-yl}-acetic acid, {2-[1-(3,5-diethoxy-4-[1,2,4] triazol-1-yl-benzyl)-piperidin-4-ylamino]-benzooxazol-5-yl}-acetic acid,
[1-(3,5-diethoxy-4-fluoro-benzyl)-piperidin-4-yl]-(5-methoxy-benzooxazol-2-yl)-amine,
[1-(3,5-diethoxy-4-pyrrol-1-yl-benzyl)-piperidin-4-yl]-(5-methoxy-benzooxazol-2-yl)-amine,
2-[1-(3,5-diethoxy-4-fluoro-benzyl)-piperidin-4-ylamino]-benzooxazole-5-carboxylic acid methyl ester,
2-[1-(3,5-diethoxy-4-pyrrol-1-yl-benzyl)-piperidin-4-ylamino]-benzooxazole-5-carboxylic acid methyl ester,
2-[1-(3,5-diisopropoxy-benzyl)-piperidin-4-ylamino]-benzooxazole-5-carboxylic acid methyl ester,
2-[1-(3,5-diethoxy-4-[1,2,4]triazol-1-yl-benzyl)-piperidin-4-ylamino]-benzooxazole-5-carboxylic acid methyl ester,
2-[1-(3,5-diethoxy-4-fluoro-benzyl)-piperidin-4-ylamino]-benzooxazole-5-carboxylic acid,
2-[1-(3,5-diethoxy-4-pyrrol-1-yl-benzyl)-piperidin-4-ylamino]-benzooxazole-5-carboxylic acid,
2-[1-(3,5-diisopropoxy-benzyl)-piperidin-4-ylamino]-benzooxazole-5-carboxylic acid, 2-[1-(3,5-diethoxy-4-[1,2,4]triazol-1-yl-benzyl)-piperidin-4-ylamino]-benzooxazole-5-carboxylic acid,
2-[1-(4-chloro-3,5-diethoxy-benzyl)-piperidin-4-ylamino]-benzooxazole-5-carboxylic acid methyl ester,
2-[1-(2,6-diethoxy-4'-fluoro-biphenyl-4-ylmethyl)-piperidin-4-ylamino]-benzooxazole-5-carboxylic acid methyl ester,
2-[1-(4-chloro-3,5-diethoxy-benzyl)-piperidin-4-ylamino]-benzooxazole-5-carboxylic acid,
2-[1-(2,6-diethoxy-4'-fluoro-biphenyl-4-ylmethyl)-piperidin-4-ylamino]-benzooxazole-5-carboxylic acid,
{2-[1-(3,5-diethoxy-4-fluoro-benzyl)-pipendin-4-y!amino]-benzooxazol-5-yloxy}-acetonitrile,
*N*-tert-butyl-2-{2-[1-(3,5-diethoxy-4-fluoro-benzyl)-piperidin-4-ylamino]-benzooxazol-5-yloxy}-acetamide,
{2-[1-(3,5-diisopropoxy-benzyl)-piperidin-4-ylamino]-benzooxazol-5-yloxy}-acetonitrile,
*N*-tert-butyl-2-{2-[1-(3,5-diisopropoxy-benzyl)-piperidin-4-ylamino]-benzooxazol-5-yloxy}-acetamide,
{2-[1-(4-chloro-3,5-diethoxy-benzyl)-piperidin-4-ylamino]-benzoaxazol-5-yloxy}-acetonitrile,
*N*-tert-butyl-2-{2-[1-(4-chloro-3,5-diethoxy-benzyl)-piperidin-4-ylamino]-benzooxazol-5-yloxy}-acetamide,
{2-[1-(2,6-diethoxy-4'-fluoro-biphenyl-4-ylmethyl)-piperidin-4-ylamino]-benzooxazol-5-yloxy}-acetonitrile,
*N*-tert-butyl-2-{2-[1-(2,6-diethoxy-4'-fluoro-biphenyl-4-ylmethyl)-piperidin-4-ylamino]-benzooxazol-5-yloxy}-acetamide,
2-{2-[1-(2,6-diethoxy-4'-fluoro-biphenyl-4-ylmethyl)-pipcridin-4-ylamino]-benzooxazol-5-yloxy}-acetamide,
2-{2-[1-(3,5-diisopropoxy-benzyl)-piperidin-4-ylamino]-benzooxazol-5-yloxy}-acetamide,
2-{2-[1-(4-chloro-3,5-diethoxy-benzyl)-piperidin-4-ylamino]-benzooxazol-5-yloxy}-acetamide,
2-{2-[1-(3,5-diethoxy-4-fluoro-benzyl)-piperidin-4-ylamino]-benzooxazol-5-yloxy}-acetamide,
[rac]-3-{2-[1-(2,6-diethoxy-4'-fluoro-biphenyl-4-ylmethyl)-piperidin-4-ylamino]-benzooxazol-5-yloxy}-propane-1,2-diol,
[rac]-3-{2-[1-(4-chloro-3,5-diethoxy-benzyl)-piperidin-4-ylamino]-benzooxazol-5-yloxy}-propane-1,2-diol,
2-{2-[1-(3,5-diisopropoxy-benzyl)-piperidin-4-ylamino]-benzooxazol-5-yl}-ethanol, 2-{2-[1-(3,5-diethoxy-4-fluoro-benzyl)-piperidin-4-ylamino]-benzooxazol-5-yl}-ethanol,
{2-[1-(3,5-diethoxy-4-fluoro-benzyl)-piperidin-4-ylamino]-benzooxazol-5-yl}-methanol,
[5-(2-amino-ethoxy)-benzooxazol-2-yl]-[1-(3,5-diethoxy-4-fluoro-benzyl)-piperidin-4-yl]-amine,
2-[1-(2,6-diethoxy-4'-fluoro-biphenyl-4-ylmethyl)-piperidin-4-ylamino]-benzooxazole-6-carboxylic acid,
2-[1-(4-chloro-3,5-diethoxy-benzyl)-piperidin-4-ylamino]-benzooxazole-6-carboxylic acid,
2-[1-(3,5-diisopropoxy-benzyl)-piperidin-4-ylamino]-benzooxazole-6-carboxylic acid, 2-[1-(4-chloro-3,5-diethoxy-benzyl)-piperidin-4-ylamino]-benzooxazol-5-ol,
3-{2-[1-(3,5-diethoxy-4-fluoro-benzyl)-piperidin-4-ylamino]-benzooxazol-5-yloxy}-propan-1-ol,
3-{2-[1-(3,5-diisopropoxy-benzyl)-piperidin-4-ylamino]-benzooxazol-5-yloxy}-propan-1-ol,
3-{2-[1-(4-chloro-3,5-diethoxy-benzyl)-piperidin-4-ylamino]-benzooxazol-5-yloxy}-propan-1-ol,
3-{2-[1-(2,6-diethoxy-4'-fluoro-biphenyl-4-ylmethyl)-piperidin-4-ylamino]-benzooxazol-5-yloxy}-propan-1-ol,
2-{2-[1-(3,5-diethoxy-4-fluoro-benzyl)-piperidin-4-ylamino]-benzooxazol-5-yloxy}-ethanol,
2-{2-[1-(2,6-diethoxy-4'-fluoro-biphenyl-4-ylmetyl)-piperidin-4-ylamino]-benzooxazol-5-yloxy}-ethanol,
[1-(3,5-diethoxy-4-fluoro-benzyl)-piperidin-4-yl]-[5-(3-methoxy-propoxy)-benzooxazol-2-yl]-amine,
[1-(3,5-diisopropoxy-benzyl)-piperidin-4-yl]-[5-(3-methoxy-propoxy)-benzooxazol-2-yl]-amine,
[1-(4-chloro-3,5-diethoxy-benzyl)-piperidin-4-yl]-[5-(3-methoxy-propoxy)-benzooxazol-2-yl]-amine,
[1-(2,6-diethoxy-4'-fluoro-biphenyl-4-ylmethyl)-piperidin-4-yl]-[5-(3-methoxy-propoxy)-benzooxazol-2-yl]-amine,
[1-(4-chloro-3,5-diethoxy-benzyl)-piperidin-4-yl]-[5-(3-methanesulfonyl-propoxy)-benzooxazol-2-y]-amine,
methanesulfonic acid 2-[1-(3,5-diethoxy-4-fluoro-benzyl)-piperidin-4-ylamino]-benzooxazol-5-yl ester,
methanesulfonic acid 2-[1-(3,5-diisopropoxy-benzyl)-piperidin-4-ylamino]-benzooxazol-5-yl ester,
methanesulfonic acid 2-[1-(4-chloro-3,5-diethoxy-benzyl)-piperidin-4-ylamino]-benzooxazol-5-yl ester,
methanesulfonic acid 2-[1-(2,6-diethoxy-4'-fluoro-biphenyl-4-ylmethyl)-piperidin-4-ylamino]-benzooxazol-5-yl ester,
[1-(3,5-diethoxy-4-fluoro-benzyl)-piperidin-4-yl]-[5-(3-[1,2,4]triazol-1-yl-propoxy)-benzooxazol-2-yl] -amine,
[1-(3,5-diisopropoxy-benzyl)-piperidin-4-yl]-[5-(3-[1,2,4]triazol-1-yl-propoxy)-benzooxazol-2-yl]-amine,
[1-(4-chloro-3,5-diethoxy-benzyl)-piperidin-4-yl]-[5-(3-[1,2,4]triazol-1-yl-propoxy)-benzooxazol-2-yl]-amine,
[1-(2,6-diethoxy-4'-fluoro-biphenyl-4-ylmethyl)-piperidin-4-yl]-[5-(3-[1,2,4] triazol-1-yl-propoxy)-benzooxazol-2-yl]-amine,
2-[1-(3,5-diethoxy-4-fluoro-benzyl)-piperidin-4-ylamino]-benzothiazole-6-carboxylic acid ethyl ester,
2-[1-(3,5-diisopropoxy-benzyl)-piperidin-4-ylamino]-benzothiazole-6-carboxylic acid ethyl ester,
2-[1-(4-chloro-3,5-diethoxy-benzyl)-piperidin-4-ylamino]-benzothiazole-6-carboxylic acid ethyl ester,
2-[1-(2,6-diethoxy-4'-fluoro-biphenyl-4-ylmethyl)-piperidin-4-ylamino]-benzothiazole-6-carboxylic acid ethyl ester,
2-[1-(3,5-diethoxy-4-pyrrol-1-yl-benzyl)-piperidin-4-ylamino]-benzothiazole-6-carboxylic acid ethyl ester,
2-[1-(3,5-diethoxy-4-fluoro-benzyl)-piperidin-4-ylamino]-benzothiazole-6-carboxylic acid,
2-[1-(3,5-diisopropoxy-benzyl)-piperidin-4-ylamino]-benzothiazole-6-carboxylic acid, 2-[1-(4-chloro-3,5-diethoxy-benzyl)-piperidin-4-ylamino]-benzothiazole-6-carboxylic acid,
2-[1-(2,6-diethoxy-4'-fluoro-biphenyl-4-ylmethyl)-piperidin-4-ylamino]-benzothiazole-6-carboxylic acid,
2-[1-(3,5-diethoxy-4-pyrrol-1-yl-benzyl)-piperidin-4-ylamino]-benzothiazole-6-carboxylic acid,
2-[1-(3,5-diisopropoxy-benzyl)-piperidin-4-ylamino]-benzothiazole-5-carboxylic acid methyl ester,
2-[1-(4-chloro-3,5-diethoxy-benzyl)-piperidin-4-ylamino]-benzothiazole-5-carboxylic acid methyl ester,
2-[1-(2,6-diethoxy-4'-fluoro-biphenyl-4-ylmethyl)-piperidin-4-ylamino]-benzothiazole-5-carboxylic acid methyl ester,
2-[1-(3,5-diethoxy-4-pyrrol-1-yl-benzyl)-piperidin-4-ylamino]-benzothiazole-5-carboxylic acid methyl ester,
2-[1-(3,5-diisopropoxy-benzyl)-piperidin-4-ylamino]-benzothiazole-5-carboxylic acid, 2-[1-(4-chloro-3,5-diethoxy-benzyl)-piperidin-4-ylamino]-benzothiazole-5-carboxylic acid,
2-[1-(2,6-diethoxy-4'-fluoro-biphenyl-4-ylmethyl)-piperidin-4-ylamino]-benzothiazole-5-carboxylic acid,
2-[1-(3,5-diethoxy-4-pyrrol-1-yl-benzyl)-piperidin-4-ylamino]-benzothiazole-5-carboxylic acid,
{2-[1-(2,6-diethoxy-4'-fluoro-biphenyl-4-ylmethyl)-piperidin-4-ylamino]-oxazolo[5,4-c]pyridin-4-yl}-methanol,
{2-[1-(4-chloro-3,5-diethoxy-benzyl)-piperidin-4-ylamino]-oxazolo[5,4-c]pyridin-4-yl}-methanol,
and pharmaceutically acceptable salts thereof.

23. Compounds of formula I according to claim 1, selected from the group consisting of
*N*²-[1-(3,5-diethoxy-4-fluoro-benzyl)-piperidin-4-yl]-benzooxazole-2,7-diamine, *N*-{2-[1-(3,5-diethoxy-4-fluoro-benzyl)-piperidin-4-ylamino]-benzooxazol-7-yl}-succinamic acid,
*N*-{2-[1-(3,5-diethoxy-4-fluoro-benzyl)-piperidin-4-ylamino]-benzooxazol-4-yl}-succinamic acid,
2-[1-(4-chloro-3-ethoxy-benzyl)-piperidin-4-ylamino]-benzooxazole-6-carboxylic acid, 2-[1-(4-amino-3,5-diethoxy-benzyl)-piperidin-4-ylamino]-benzooxazole-6-carboxylic acid,
2-[1-(3,5-diethoxy-4-fluoro-benzyl)-piperidin-4-ylamino]-benzooxazole-6-carboxylic acid,
[1-(3,5-diethoxy-4-fluoro-benzyl)-piperidin-4-yl]-oxazolo[5,4-*c*]pyridin-2-yl-amine, [1-(3,5-diisopropoxy-benzyl)-piperidin-4-yl]-oxazolo[5,4-*c*] pyridin-2-yl-amine,
{2-[1-(3,5-diethoxy-4-fluoro-benzyl)-piperidin-4-ylamino]-benzooxazol-5-yloxy}-acetic acid,
{2-[1-(3,5-diethoxy-4-pyrrol-1-yl-benzyl)-piperidin-4-ylamino]-benzooxazol-5-yloxy}-acetic acid,
[1-(3,5-diethoxy-4-fluoro-benzyl)-piperidin-4-yl]-(7-phenyl-benzooxazol-2-yl)-amine, 4-{2-[1-(3,5-diethoxy-4-pyrrol-1-yl-benzyl)-piperidin-4-ylamino]-benzooxazol-7-yloxy}-butyric acid,
[rac]-3-{2-[1-(3,5-diethoxy-4-fluoro-benzyl)-piperidin-4-ylamino]-benzooxazol-7-yloxy}-propane-1,2-diol,
4-{2-[1-(3,5-diethoxy-4-fluoro-benzyl)-piperidin-4-ylamino]-benzooxazol-5-yloxy}-butyric acid,
[rac]-3-{2-1-(3,5-diethoxy-4-fluoro-benzyl)-piperidin-4-ylamino]-benzooxazol-5-yloxy}-propane-1,2-diol,
{2-[1-(3,5-diisopropoxy-benzyl)-piperidin-4-ylamino]-oxazolo[5,4-c]pyridin-4-yl}-methanol,
2-[1-(4-chloro-3,5-diethoxy-benzyl)-piperidin-4-ylamino]-benzooxazole-5-carboxylic acid,
2-[1-(2,6-diethoxy-4'-fluoro-biphenyl-4-ylmethyl)-piperidin-4-ylamino]-benzooxazole-5-carboxylic acid,
{2-[1-(2,6-diethoxy-4'-fluoro-biphenyl-4-ylmethyl)-piperidin-4-ylamino]-benzooxazol-5-yloxy}-acetonitrile,
*N*-*tert*-butyl-2-{2-[1-(2,6-diethoxy-4'-fluoro-biphenyl-4-ylmethyl)-piperidin-4-ylamino]-benzooxazol-5-yloxy}-acetamide,
2-{2-[1-(2,6-diethoxy-4'-fluoro-biphenyl-4-ylmethyl)-piperidin-4-ylamino]-benzooxazol-5-yloxy}-acetamide,
[rac]-3-{2-[1-(2,6-diethoxy-4'-fluoro-biphenyl-4-ylmethyl)-piperidin-4-ylamino]-benzooxazol-5-yloxy}-propane-1,2-diol,
2-[1-(2,6-diethoxy-4'-fluoro-biphenyl-4-ylmethyl)-piperidin-4-ylamino]-benzooxazole-6-carboxylic acid,
2-[1-(4-chloro-3,5-diethoxy-benzyl)-piperidin-4-ylamino]-benzooxazole-6-carboxylic acid,
3-{2-[1-(2,6-diethoxy-4'-fluoro-biphenyl-4-ylmethyl)-piperidin-4-ylamino]-benzooxazol-5-yloxy}-propan-1-ol,
2-{2-[1-(2,6-diethoxy-4'-fluoro-biphenyl-4-ylmethyl)-piperidin-4-ylamino]-benzooxazol-5-yloxy}-ethanol,
[1-(2,6-diethoxy-4'-fluoro-biphenyl-4-ylmethyl)-piperidin-4-yl]-[5-(3-methoxy-propoxy)-benzooxazol-2-yl]-amine,
[1-(4-chloro-3,5-diethoxy-benzyl)-piperidin-4-yl]-[5-(3-methanesulfonyl-propoxy)-benzooxazol-2-yl]-amine,
methanesulfonic acid 2-[1-(2,6-diethoxy-4'-fluoro-biphenyl-4-ylmethyl)-piperidin-4-ylamino]-benzooxazol-5-yl ester,
[1-(4-chloro-3,5-diethoxy-benzyl)-piperidin-4-yl]-[5-(3-[1,2,4] triazol-1-yl-propoxy)-benzooxazol-2-yl] -amine,
[1-(2,6-diethoxy-4'-fluoro-biphenyl-4-ylmethyl)-piperidin-4-yl]-[5-(3-[1,2,4]triazol-1-yl-propoxy)-benzooxazol-2-yl]-amine,
2-[1-(4-chloro-3,5-diethoxy-benzyl)-piperidin-4-ylamino]-benzothiazole-6-carboxylic acid,
2-[1-(2,6-diethoxy-4'-fluoro-biphenyl-4-ylmethyl)-piperidin-4-ylamino]-benzothiazole-6-carboxylic acid,
2-1-(4-chloro-3,5-diethoxy-benzyl)-piperidin-4-ylamino]-benzothiazole-5-carboxylic acid,
2-[1-(2,6-diethoxy-4'-fluoro-biphenyl-4-ylmethyl)-piperidin-4-ylamino]-benzothiazole-5-carboxylic acid,
and pharmaceutically acceptable salts thereof.

24. A process for the manufacture of compounds according to any one of claims 1 to 23, which process comprises
reacting a compound of the general formula 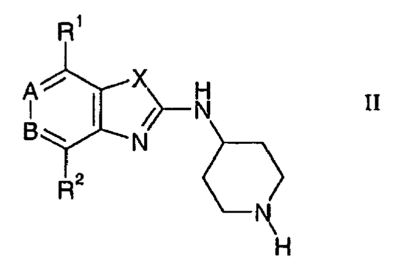 wherein A, B, X, R¹ and R² are as defined in claim 1, with an aldehyde of the formula 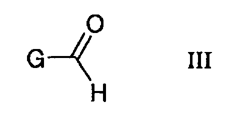 wherein G is as defined in claim 1,
by employing a reducing agent to obtain a compound of the formula 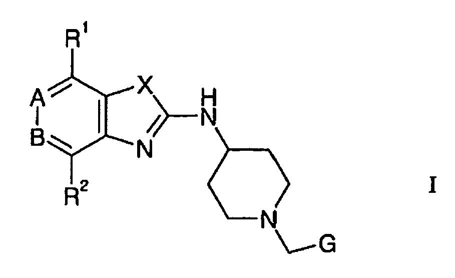 and, if desired,
converting the compound of formula I into a pharmaceutically acceptable salt.

25. Pharmaceutical compositions comprising a compound according to any one of claims 1 to 23 as well as a pharmaceutically acceptable carrier and/or adjuvant.

26. Compounds according to any one of claims 1 to 23 for use as therapeutically active substances.

27. Compounds according to any one of claims 1 to 23 for use as therapeutically active substances for the treatment and/or prevention of diseases which are associated with the modulation of SST receptors subtype 5.

28. The use of compounds according to any one of claims 1 to 23 for the preparation of medicaments for the treatment and/or prevention of diseases which are associated with the modulation of SST receptors subtype 5.

29. The use according to claim 28 for the treatment and/or prevention of diabetes mellitus, particularly type 2 diabetes mellitus, impaired fasting glucose, impaired glucose tolerance, micro- and macrovascular diabetic complications, post-transplantational in type 1 diabetes mellitus, gestational diabetes, obesity, inflammatory bowel diseases such as Crohn's disease or ulcerative colitis, malabsorption, autoimmune diseases such as rheumatoid arthritis, osteoarthritis, psoriasis and other skin disorders, and immunodeficiences.

30. The use according to claim 28 for the treatment and/or prevention of diabetes mellitus, particularly type 2 diabetes mellitus, impaired fasting glucose, impaired glucose tolerance.

## Patentansprüche

1. Verbindungen der allgemeinen Formel 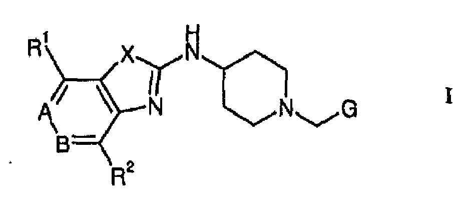 wobei
X S oder O ist;
A CR³ ist und B CR⁴ ist oder A N oder N⁺-O⁻ ist und B CR⁴ ist oder B N oder N⁺-O⁻ ist und A CR³ ist;
R¹ und R² unabhängig voneinander ausgewählt sind aus der Gruppe bestehend aus Wasserstoff, Halogen, Cyano, Nitro, C₁₋₇-Alkyl, Hydroxy-C₁₋₇-alkyl, C₁₋₇-Alkoxy-C₁₋₇-alkyl, C₁₋₇-Alkoxy, Hydroxy-C₂₋₇-alkoxy, Dihydroxy-C₃₋₇-alkoxy, carboxy-C₁₋₇-alkoxy, C₁₋₇-Alkoxycarbonyl-C₁₋₇-alkoxy, Carboxy-C₁₋₇-alkyl, C₁₋₇-Alkoxycarbonyl-C₁₋₇-alkyl, 1H-Tetrazol-5-yl-C₁₋₇-alkoxy, Pyridinyl-C₁₋₇-alkoxy, -NR⁵R⁶, -NHCOR⁷, -NHSO₂R⁸, -SO₂NR⁹R¹⁰, 1H-Tetrazol-5-yl, unsubstituiertem Phenyl und Phenyl, das mit einem bis drei Substituenten substituiert ist, ausgewählt aus C₁₋₇-Alkyl, C₃₋₇-Cycloalkyl, Halogen, Halogen-C₁₋₇-alkyl und C₁₋₇-Alkoxy; und
R² zusätzlich ausgewählt sein kann aus der Gruppe bestehend aus Carboxy, C₁₋₇-Alkoxycarbonyl und -CONR¹¹R¹²;
R⁵ und R⁶ unabhängig voneinander ausgewählt sind aus der Gruppe bestehend aus Wasserstoff, C₁₋₇-Alkyl, Hydroxy-C₁₋₇-alkyl und C₃₋₇-Cycloalkyl,
R⁷ ausgewählt ist aus der Gruppe bestehend aus C₁₋₇-Alkyl, C₃₋₇Cycloalkyl, Halogen-C₁₋₇-alkyl, Hydroxy-C₁₋₇-alkyl, C₁₋₇-Alkoxy-C₁₋₇-alkyl, Carboxy-C₁₋₇-alkyl, C₁₋₇-Alkoxycarbonyl-C₁₋₇-alkyl, 1H-Tetrazol-5-yl-C₁₋₇-alkyl, unsubstituiertem Phenyl, Phenyl, das mit einer bis drei Gruppen substituiert ist, ausgewählt aus der Gruppe bestehend aus C₁₋₇-Alkyl, C₃₋₇-Cycloalkyl, C₁₋₇-Alkoxy, Halogen-C₁₋₇- alkyl und Halogen, unsubstituiertem Heteroaryl, Heteroaryl, das mit einer oder zwei Gruppen substituiert ist, ausgewählt aus der Gruppe bestehend aus C₁₋₇- Alkyl, C₃₋₇-Cycloalkyl, C₁₋₇-Alkoxy, Halogen-C₁₋₇-alkyl oder Halogen; unsubstituiertem Heteroaryl-C₁₋₇-alkyl und Heteroaryl-C₁₋₇-alkyl, wobei das Heteroaryl mit einer oder zwei Gruppen substituiert ist, ausgewählt aus C₁₋₇- Alkyl, C₃₋₇-Cycloalkyl, C₁₋₇-Alkoxy, Halogen-C₁₋₇-alkyl und Halogen;
R⁸ ausgewählt ist aus der Gruppe bestehend aus C₁₋₇-Alkyl, C₃₋₇-Cycloalkyl, unsubstituiertem Heteroaryl und Heteroaryl, das mit einer oder zwei Gruppen substituiert ist, ausgewählt aus C₁₋₇-Alkyl, C₃₋₇-Cycloalkyl, C₁₋₇-Alkoxy, Halogcn-C₁₋₇-alkyl und Halogen;
R⁹ und R¹⁰ unabhängig voneinander ausgewählt sind aus der Gruppe bestehend aus Wasserstoff, C₁₋₇-Alkyl, C₃₋₇-Cycloalkyl, unsubstituiertem Heteroaryl und Heteroaryl, das mit einer oder zwei Gruppen substituiert ist, ausgewählt aus C₁₋₇-Alkyl, C₃₋₇-Cycloalkyl, C₁₋₇-Alkoxy, Halogen-C₁₋₇-alkyl und Halogen; oder R⁹ und R¹⁰ zusammen mit dem Stickstoffatom an das sie gebunden sind einen Pyrrolidin- oder einen Piperidinring bilden;
R¹¹ ausgewählt ist aus der Gruppe bestehend aus Wasserstoff, C₁₋₇-Alkyl und C₃₋₇-Cycloalkyl;
R¹² ausgewählt ist aus der Gruppe bestehend aus Wasserstoff, C₁₋₇-Alkyl, C₃₋₇-Cycloalkyl, Halagen-C₁₋₇-alkyl, Hydroxy-C₁₋₇-alkyl, C₁₋₇-Alkoxy-C₁₋₇- alkyl, Carboxy-C₁₋₇-alkyl, C₁₋₇-Alkoxycarbonyl-C₁₋₇-alkyl, unsubstituiertem Phenyl, Phenyl, das mit einer bis drei Gruppen substituiert ist, ausgewählt aus der Gruppe bestehend aus C₁₋₇-Alkyl, C₃₋₇-Cycloalkyl, C₁₋₇-Alkoxy, Halogen- C₁₋₇-alkyl und Halogen, unsubstituiertem Heteroaryl, Heteroaryl, das mit einer oder zwei Gruppen substituiert ist, ausgewählt aus C₁₋₇-Alkyl, C₃₋₇-Cycloalkyl, C₁₋₇-Alkoxy, Halogen-C₁₋₇-alkyl oder Halogen;
einer von R³ und R⁴ ausgewählt ist aus der Gruppe bestehend aus Wasserstoff, C₁₋₇-Alkyl und C₁₋₇-Alkoxy oder im Falle, dass einer von A oder B N oder N⁺-O⁻ ist, nicht vorhanden ist und
der andere von R³ und R⁴ ausgewählt ist aus der Gruppe bestehend aus Wasserstoff, C₁₋₇-Alkyl, C₁₋₇-Alkoxy, Hydroxy, Hydroxy-C₁₋₇-alkyl, Cyano-C₁₋₇-alkoxy, C₃₋₇-Cycloalkyloxy, wobei die Cycloalkylgruppe substituiert ist mit Carboxy oder C₁₋₇-Alkoxycarbonyl, Carboxy, C₁₋₇-Alkoxycarbonyl, Carboxy-C₁₋₇-alkoxy, C₁₋₇-Alkoxycarbonyl-C₁₋₇-alkyl, Carboxy-C₁₋₇-alkyl, C₁₋₇-Alkoxycarbonyl-C₁₋₇-alkoxy, 1H-Tetrazol-5-yl-C₁₋₇-alkoxy, Triazolyl-C₁₋₇-alkoxy, C₁₋₇-Alkylsulfonyloxy, C₁₋₇-Alkylsulfonyl-C₁₋₇-alkoxy, Hydroxy-C₂₋₇-alkoxy, Dihydroxy-C₃₋₇-alkoxy, C₁₋₇-Alkoxy-C₁₋₇-alkoxy, -(CH₂)n-NR¹³R¹⁴, -(CH₂)ₙ-NHCOR¹⁵, Amino-C₁₋₇-alkoxy, Aminocarbonyl-C₁₋₇-alkoxy, C₁₋₇-Alkylanlinocarbonyl-C₁₋₇-alkoxy und 1H-Tetrazol-5-yl;
R¹³ und R¹⁴ unabhängig voneinander ausgewählt sind aus der Gruppe bestehend aus Wasserstoff, C₁₋₇-Alkyl und C₃₋₇-Cycloalkyl;
R¹⁵ ausgewählt ist aus der Gruppe bestehend aus C₁₋₇-Alkyl, C₃₋₇-Cycloalkyl, Halogen-C₁₋₇-alkyl, Hydroxy-C₁₋₇-alkyl, C₁₋₇-Alkoxy-C₁₋₇- alkyl, Carboxy-C₁₋₇-alkyl und C₁₋₇-Alkoxycarbonyl-C₁₋₇-alkyl;
n eine ganze Zahl ist, ausgewählt aus 1, 2 und 3; Wasserstoff vorausgesetzt, dass Benzooxazole und Benzothiazole, bei denen R¹, R², R³ und R⁴ sind, ausgeschlossen sind;
G ausgewählt ist aus den Gruppen 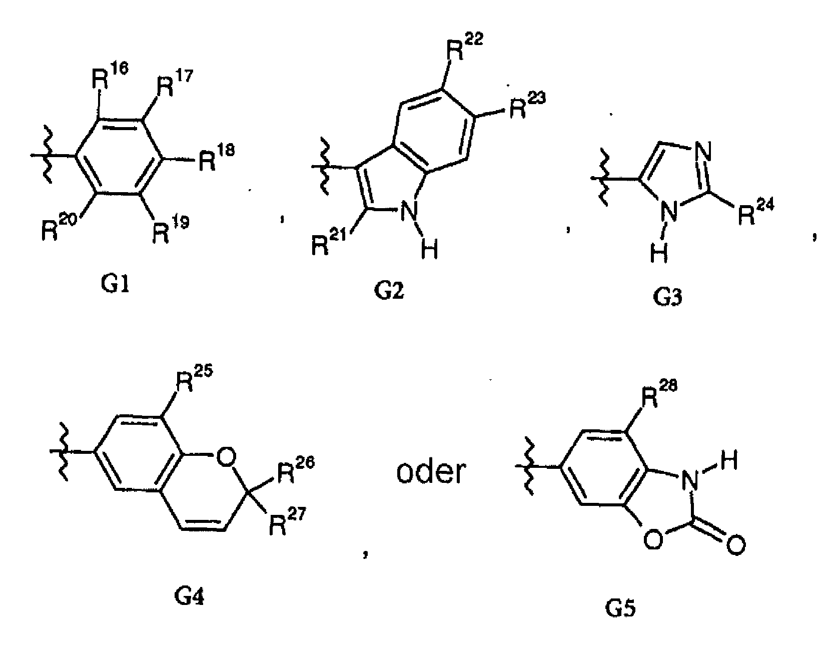
wobei
R¹⁶ Wasserstoff oder Halogen ist;
R¹⁷ ausgewählt ist aus der Gruppe bestehend aus C₁₋₇-Alkoxy, C₂₋₇-Alkenyloxy, C₃₋₇-Cycloalkyloxy, -NR²⁹R³⁰ Halogen-C₁₋₇-alkoxy, C₁₋₇-Alkoxy-C₁₋₇-alkoxy, C₁₋₇-Alkoxy-C₁₋₇-alkyl,
R²⁹ und R³⁰ unabhängig voneinander Wasserstoff oder C₁₋₇-Alkyl sind;
R¹⁸ ausgewählt ist aus der Gruppe bestehend aus Wasserstoff, C₁₋₇-Alkyl, Halogen-C₁₋₇- alkyl, Hydroxy, C₁₋₇-Alkoxy, Halogen-C₁₋₇-alkoxy, C₃₋₇-Cycloalkyloxy, Halogen, Pyrrolo, Imidazolyl, Triazolyl, -CO₂R³¹, -NR³²R³³, -SOR³⁴; unsubstituiertem Phenyl und Phenyl, das mit einer bis zwei Gruppen substituiert ist, ausgewählt aus der Gruppe bestehend aus C₁₋₇-Alkyl, Halogen-C₁₋₇-alkyl, Halogen-C₁₋₇-alkoxy, C₁₋₇-Alkoxy und Halogen;
R³¹ Wasserstoff oder C₁₋₇-Alkyl ist;
R³² und R³³ unabhängig voneinander Wasserstoff oder C₁₋₇-Alkyl sind;
R³⁴ C₁₋₇-Alkyl ist;
R¹⁹ ausgewählt ist aus der Gruppe bestehend aus Wasserstoff, C₁₋₇-Alkyl, Halogen, C₁₋₇-Alkoxy, C₂₋₇-Alkenyloxy, -O-Tetrahydropyranyl, C₃₋₇-Cycloalkyloxy, Hatogen-C₁₋₇-alkoxy, C₁₋₇-Alkoxy-C₁₋₇-alkoxy und C₁₋₇-Alkoxy-C₁₋₇-alkyl;
R²⁰ Wasserstoff oder Halogen ist;
R²¹ Wasserstoff oder C₁₋₇-Alkyl ist;
R²² und R²³ unabhängig voneinander ausgewählt sind aus der Gruppe bestehend aus Wasserstoff, C₁₋₇-Alkyl, C₁₋₇-Alkoxy und Nitro;
R²⁴ unsubstituiertes Phenyl ist oder Phenyl, das mit einer bis zwei Gruppen substituiert ist, ausgewählt aus der Gruppe bestehend aus C₁₋₇-Alkyl, C₁₋₇-Alkoxy und Halogen;
R²⁵ C₁₋₇-Alkoxy ist;
R²⁶ und R²⁷ unabhängig voneinander C₁₋₇-Alkyl sind;
R²⁸ C₁₋₇-Alkoxy ist;
und pharmazeutisch verträgliche Salze davon.

2. Verbindungen der Formel I gemäss Anspruch 1, wobei X O ist.

3. Verbindungen der Formel I gemäss Anspruch 1 oder 2, wobei A CR³ ist und B CR⁴ ist und wobei
einer von R³ und R⁴ ausgewählt ist aus der Gruppe bestehend aus Wasserstoff, C₁₋₇-Alkyl und C₁₋₇-Alkoxy und
der andere von R³ und R⁴ ausgewählt ist aus der Gruppe bestehend aus Wasserstoff, C₁₋₇-Alkyl, C₁₋₇-Alkoxy, Hydroxy, Hydroxy-C₁₋₇-alkyl, Cyano-C₁₋₇-alkoxy, C₃₋₇-Cycloalkyloxy, wobei die Cycloalkylgruppe substituiert ist mit Carboxy oder C₁₋₇-Alkoxycarbonyl, Carboxy, C₁₋₇-Alkoxycarbonyl, Carboxy-C₁₋₇-alkoxy, C₁₋₇-Alkoxycarbonyl-C₁₋₇-alkyl, Carboxy-C₁₋₇-alkyl, C₁₋₇-Alkoxycarbonyl-C₁₋₇-alkoxy, 1H-Tetrazol-5-yl-C₁₋₇alkoxy, Triazolyl-C₁₋₇-alkoxy, C₁₋₇-Alkylsulfonyloxy, C₁₋₇-Alkylsulfonyl-C₁₋₇-alkoxy, Hydroxy-C₂₋₇-alkoxy, Dihydroxy-C₃-₇-alkoxy, C₁₋₇-Alkoxy-C₁₋₇-alkoxy, -(CH₂)ₙ-NR¹³R¹⁴, -(CH₂)ₙ-NHCOR¹⁵, Amino-C₁₋₇-alkoxy, Aminocarbonyl-C₁₋₇-alkoxy, C₁₋₇-Alkylaminocarbonyl-C₁₋₇-alkoxy und 1H-Tetrazol-5-yl und wobei R¹³, R¹⁴, R¹⁵ und n wie in Anspruch 1 definiert sind.

4. Verbindungen der Formel I gemäss einem der Ansprüche 1 bis 3, wobei R³ Wasserstoff ist und R⁴ ausgewählt ist aus der Gruppe bestehend aus C₁₋₇-Alkyl, C₁₋₇-Alkoxy, Hydroxy, Hydroxy-C₁₋₇-alkyl, Cyano-C₁₋₇alkoxy, C₃₋₇-Cycloalkyloxy, wobei die Cycloalkylgruppe substituiert ist mit Carboxy oder C₁₋₇-Alkoxycarbonyl, Carboxy, C₁₋₇-Alkoxycarbonyl, Carboxy-C₁₋₇-alkoxy, C₁₋₇-Alkoxycarbonyl-C₁₋₇-alkyl, Carboxy-C₁₋₇-alkyl, C₁₋₇-Alkoxycarbonyl-C₁₋₇-alkoxy, 1H-Tetrazol-5-yl-C₁₋₇-alkoxy, Triazolyl-C₁₋₇-alkoxy, C₁₋₇-Alkylsulfonyloxy, C₁₋₇-Alkylsulfonyl-C₁₋₇-alkoxy, Hydroxy-C₂₋₇-alkoxy, Dihydroxy-C₃₋₇-alkoxy, C₁₋₇-Alkoxy-C₁₋₇-alkoxy, -(CH₂)ₙ-NR¹³R¹⁴, -(CH₂)ₙ-NHCOR¹⁵, Amino-C₁₋₇-alkoxy, Aminocarbonyl-C₁₋₇-alkoxy, C₁₋₇-Alkylaminocarbonyl-C₁₋₇-alkoxy und 1H-Tetrazol-5-yl und wobei R¹³, R¹⁴, R¹⁵ und n wie in Anspruch 1 definiert sind.

5. Verbindungen der Formel I gemäss einem der Ansprüche 1 bis 3, wobei R⁴ Wasserstoff ist und R³ ausgewählt ist aus der Gruppe bestehend aus C₁₋₇-Alkyl, C₁₋₇-Alkoxy, Hydroxy-C₁₋₇-alkyl, Carboxy, C₁₋₇-Alkoxycarbonyl, Carboxy-C₁₋₇-alkoxy, C₁₋₇-Alkoxycarbonyl-C₁₋₇-alkyl, Carboxy-C₁₋₇-alkyl, C₁₋₇-Alkoxycarbonyl-C₁₋₇-alkoxy, 1H-Tetrazol-5-yl-C₁₋₇-alkoxy, Hydroxy-C₂₋₇-alkoxy, Dihydroxy-C₃₋₇-alkoxy, -(CH₂)ₙ-NR¹³R¹⁴, -(CH₂)ₙ-NHCOR¹⁵ und 1-H-Tetrazol-5-yl und wobei R¹³, R¹⁴, R¹⁵ und n wie in Anspruch definiert sind.

6. Verbindungen der Formel I gemäss einem der Ansprüche 1 bis 3, wobei R³ und R⁴ Wasserstoff sind.

7. Verbindungen der Formel I gemäss Anspruch 1 oder 2, wobei A N ist, B CR⁴ ist und R⁴ ausgewählt ist aus der Gruppe bestehend aus Wasserstoff, C₁₋₇-Alkyl, C₁₋₇-Alkoxy, Hydroxy, Hydroxy-C₁₋₇-alkyl, Cyano-C₁₋₇-alkoxy, C₃₋₇-Cycloalkyloxy, wobei die Cycloalkylgruppe substituiert ist mit Carboxy oder C₁₋₇-Alkoxycarbonyl, Carboxy, C₁₋₇-Alkoxycarbonyl, Carboxy-C₁₋₇-alkoxy, C₁₋₇-Alkoxycarbonyl-C₁₋₇-alkyl, Carboxy-C₁₋₇-alkyl, C₁₋₇-Alkoxycarbonyl-C₁₋₇-alkoxy, 1H-Tetrazol-5-yl-C₁₋₇-alkoxy, Triazolyl-C₁₋₇-alkoxy, C₁₋₇-Alkylsulfonyloxy, C₁₋₇-Alkylsulfonyl-C₁₋₇-alkoxy, Hydroxy-C₂₋₇-alkoxy, Dihydroxy-C₃₋₇-alkoxy, C₁₋₇-Alkoxy-C₁₋₇-alkoxy, -(CH₂)ₙ-NR¹³R¹⁴, -(CH₂)ₙ-NHCOR¹⁵, Amino-C₁₋₇-alkoxy, Aminocarbonyl-C₁₋₇-alkoxy, C₁₋₇-Alkylaminocarbonyl-C₁₋₇-alkoxy und 1H-Tetrazol-5-yl und wobei R¹³, R¹⁴, R¹⁵ und n wie in Anspruch 1 definiert sind.

8. Verbindungen der Formel I gemäss einem der Ansprüche 1, 2 oder 7, wobei A N ist und B CH ist.

9. Verbindungen der Formel I gemäss Anspruch 7 oder 8, wobei R¹ und R² Wasserstoff sind.

10. Verbindungen der Formel I gemäss einem der Ansprüche 1 bis 8, wobei R¹ ausgewählt ist aus der Gruppe bestehend aus Halogen, Cyano, Nitro, C₁₋₇-Alkyl, Hydroxy-C₁₋₇-alkyl, C₁₋₇-Alkoxy-C₁₋₇-alkyl, C₁₋₇-Alkoxy, Hydroxy-C₂₋₇-alkoxy, Dihydroxy-C₃₋₇-alkoxy, Carboxy-C₁₇-alkoxy, C₁₋₇-Alkoxycarbonyl-C₁₋₇-alkoxy, Carboxy-C₁₋₇-alkyl, C₁₋₇-Alkoxycarbonyl-C₁₋₇-alkyl, 1H-Tetrazol-5-yl-C₁₋₇-alkoxy, Pyridinyl-C₁₋₇-alkoxy, -NR⁵R⁶, -NHCOR⁷, -NHSO₂R⁸, - SO₂NR⁹R¹⁰, 1H-Tetrazol-5-yl, unsubstituiertem Phenyl und Phenyl, das mit einem bis drei Substituenten substituiert ist, ausgewählt aus C₁₋₇-Alkyl, C₃₋₇-Cycloalkyl, Halogen, Halogen-C₁₋₇-alkyl, und C₁₋₇-Alkoxy, und wobei R⁵ bis R¹⁰ wie in Anspruch 1 definiert sind, und R² Wasserstoff ist.

11. Verbindungen der Formel I gemäss Anspruch 10, wobei *R¹* ausgewählt ist aus der Gruppe bestehend aus Halogen, Nitro, C₁₋₇-Alkyl, Hydroxy-C₁₋₇-alkyl, C₁₋₇-Alkoxy, Dihydroxy-C₃₋₇-alkoxy, Carboxy-C₁₋₇-alkoxy, C₁₋₇-Alkoxycarbotiyl-C₁₋₇-alkoxy, -NR⁵R⁶, -NHCOR⁷, -NHSO₂R⁸, -SO₂NR⁹R¹⁰, unsubstituiertem Phenyl und Phenyl, das mit einem bis drei Substituenten substituiert ist, ausgewählt aus C₁₋₇-Alkyl, C₃₋₇-Cycloalkyl, Halogen, Halogen-C₁₋₇-alkyl, und C₁₋₇-Alkoxy; und wobei R⁵ bis R¹⁰ wie in Anspruch 1 definiert sind.

12. Verbindungen der Formel I gemäss Anspruch 10 oder 11, wobei R¹ -NR⁵R⁶ oder-NHCOR⁷ ist und wobei R⁵ bis R⁷ wie in Anspruch 1 definiert sind.

13. Verbindungen der Formel I gemäss einem der Ansprüche 1 bis 8, wobei R¹ Wasserstoff ist und
R² ausgewählt ist aus der Gruppe bestehend aus Halogen, Cyano, Nitro, C₁₋₇-Alkyl, Hydroxy-C₁₋₇-alkyl, C₁₋₇-Alkoxy-C₁₋₇-alkyl, C₁₋₇-Alkoxy, Hydroxy-C₂₋₇-alkoxy, Dihydroxy-C₃₋₇-alkoxy, Carboxy-C₁₋₇-alkoxy, C₁₋₇-Alkoxycarbonyl-C₁₋₇-alkoxy, Carboxy-C₁₋₇-alkyl, C₁₋₇-Alkoxycarbonyl-C₁₋₇-alkyl, 1H-Tetrazol-5-yl-C₁₋₇-alkoxy, Pyridinyl-C₁₋₇-alkoxy, -NR⁵R⁶, -NHCOR⁷, -NHSO₂R⁸, -SO₂NR⁹R¹⁰, Carboxy, C₁₋₇-Alkoxycarbonyl, -CONR¹¹R¹², 1H-Tetrazol-5-yl, unsubstituiertem Phenyl und Phenyl, das mit einem bis drei Substituenten substituiert ist, ausgewählt aus C₁₋₇-Alkyl, C₃₋₇-Cycloalkyl, Halogen, Halogen-C₁₋₇-alkyl und C₁₋₇-Alkoxy; und wobei R⁵ bis R¹² wie in Anspruch 1 definiert sind.

14. Verbindungen der Formel I gemäss Anspruch 13, wobei R² ausgewählt ist aus der Gruppe bestehend aus Halogen, Nitro, C₁₋₇-Alkoxy, Pyridinyl-C₁₋₇-alkoxy, -NR⁵R⁶, -NHCOR⁷, Carboxy, C₁₋₇-Alkoxycarbonyl, -CONR¹¹R¹² und wobei R⁵ bis R⁷, R¹¹ und R¹² wie in Anspruch 1 definiert sind.

15. Verbindungen der Formel I gemäss Anspruch 14, wobei R² -NHCOR⁷ ist und R⁷ wie in Anspruch 1 definiert ist.

16. Verbindungen der Formel I gemäss einem der Ansprüche 1 bis 15, wobei G 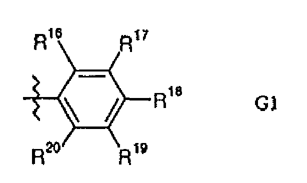 ist und wobei
R¹⁶ Wasserstoff oder Halogen ist;
R¹⁷ ausgewählt ist aus der Gruppe bestehend aus C₁₋₇-Alkoxy, C₂₋₇-Alkenyloxy, C₃₋₇-Cycloalkyloxy, -NR²⁹R³⁰, Halogen-C₁₋₇-alkoxy, C₁₋₇-Alkoxy-C₁₋₇-alkoxy, C₁₋₇- Alkoxy-C₁₋₇-alkyl; R²⁹ und R³⁰ unabhängig voneinander Wasserstoff oder C₁₋₇-Alkyl sind; sind;
R¹⁸ ausgewählt ist aus der Gruppe bestehend aus Wasserstoff, C₁₋₇-Alkyl, Halogen-C₁₋₇- alkyl, Hydroxy, C₁₋₇-Alkoxy, Halogen-C₁₋₇-alkoxy, C₃₋₇-Cycloalkyloxy, Halogen, Pyrrolo, Imidazolyl, Triazolyl, -CO₂R³¹, -NR³²R³³, -SOR³⁴; unsubstituiertem Phenyl und Phenyl, das mit einer bis zwei Gruppen substituiert ist, ausgewählt aus der Gruppe bestehend aus C₁₋₇Alkyl, Halogen-C₁₋₇-alkyl, Halogen-C₁₋₇-alkoxy, C₁₋₇-Alkoxy und Halogen;
R³¹ Wasserstoff oder C₁₋₇-Alkyl ist;
R³² und R³³ unabhängig voneinander Wasserstoff oder C₁₋₇-Alkyl sind;
R³⁴ C₁₋₇-Alkyl ist;
R¹⁹ ausgewählt ist aus der Gruppe bestehend aus Wasserstoff, C₁₋₇-Alkyl, Halogen, C₁₋₇- Alkoxy, C₂₋₇-Alkenloxy, -O-Tetrahydropyranyl, C₃₋₇-Cycloalkyloxy, Halogen-C₁₋₇- alkoxy, C₁₋₇-Alkoxy-C₁₋₇-alkoxy und C₁₋₇-Alkoxy-C₁₋₇-alkyl, und
R²⁰ Wasserstoff oder Halogen ist.

17. Verbindungen der Formel I gemäss einem der Ansprüche 1 bis 16, wobei R¹⁷ C₁₋₇-Alkoxy oder Halogcn-C₁₋₇-Alkoxy ist.

18. Verbindungen der Formel I gemäss einem der Ansprüche 1 bis 17, wobei R¹⁷ Ethoxy, Isopropyloxy oder Isobutyloxy ist.

19. Verbindungen der Formel I gemäss einem der Ansprüche 1 bis 18, wobei R¹⁸ ausgewählt ist aus der Gruppe bestehend aus Wasserstoff, C₁₋₇-Alkyl, Hydroxy, C₁₋₇-Alkoxy, Halogen, Pyrrolo, Imidazolyl, Triazolyl, -NR³²R³³ und -SOR³⁴ und R³² und R³³ unabhängig voneinander Wasserstoff oder C₁₋₇-Alkyl sind und R³⁴ C₁₋₇-Alkyl ist.

20. Verbindungen der Formel I gemäss einem der Ansprüche 1 bis 19, wobei R¹⁸ ausgewählt ist aus der Gruppe bestehend aus Wasserstoff, Halogen, Pyrrolo, Triazolyl und -NR³²R³³, wobei R³² und R³³ Wasserstoff sind.

21. Verbindungen der Formel I gemäss Anspruch 1, wobei
X S oder O ist
A CR³ ist und B CR⁴ ist oder A N oder N⁺-O⁻ ist und B CR⁴ ist oder B N oder N⁺-O⁻ ist und A CR³ ist;
R¹ und R² unabhängig voneinander ausgewählt sind aus der Gruppe bestehend aus Wasserstoff, Halogen, Cyano, Nitro, C₁₋₇-Alkyl, Hydroxy-C₁₋₇-alkyl, C₁₋₇-Alkoxy-C₁₋₇-alkyl, C₁₋₇-Alkoxy, Hydroxy-C₂₋₇-alkoxy, Dihydroxy-C₃₋₇-alkoxy, Carboxy-C₁₋₇-alkoxy, C₁₋₇-Alkoxycarbonyl-C₁₋₇-alkoxy, Carboxy-C₁₋₇-alkyl, C₁₋₇-Alkoxycarbonyl-C₁₋₇-alkyl, 1H-Tetrazol-5-yl-C₁₋₇-alkoxy, Pyridinyl-C₁₋₇-alkoxy, -NR⁵R⁶, -NHCOR⁷, -NHSO₂R⁸, -SO₂NR⁹R10, 1H-Tetrazol-5-yl, unsubstituiertem Phenyl und Phenyl, das mit einem bis drei Substittuenten substituiert ist, ausgewählt aus C₁₋₇-Alkyl, C₃₋₇-Cycloalkyl, Halogen, Halogen-C₁₋₇-alkyl und C₁₋₇-Alkoxy; und
R² zusätzlich ebenfalls ausgewählt sein kann, aus der Gruppe bestehend aus Carboxy, C₁₋₇-Alkoxycarbonyl und -CONR¹¹R¹²; R⁵ und R⁶ unabhängig voneinander ausgewählt sind aus der Gruppe bestehend aus Wasserstoff, C₁₋₇-Alkyl, Hydroxy-C₁₋₇-alkyl und C₃₋₇-cycloalkyl;
R⁷ ausgewählt ist aus der Gruppe bestehend aus C₁₋₇-Alkyl, C₃₋₇-Cycloalkyl, Halogen-C₁₋₇-alkyl, Hydroxy-C₁₋₇-alkyl, C₁₋₇-Alkoxy-C₁₋₇-alkyl, Carboxy-C₁₋₇-alkyl, C₁₋₇-Alkoxycarbonyl-C₁₋₇-alkyl, 1H-Tetrazol-5-yl-C₁₋₇-alkyl, unsubstituiertem Phenyl, Phenyl, das mit einer bis drei Gruppen substituiert ist, ausgewählt aus der Gruppe bestehend aus C₁₋₇-Alkyl, C₃₋₇-Cycloalkyl, C₁₋₇-Alkoxy, Halogen-C₁₋₇-alkyl und Halogen, unsubstituiertem Heteroaryl, Heteroaryl, das mit einer oder zwei Gruppen substituiert ist, ausgewählt aus C₁₋₇-Alkyl, C₃₋₇-Cycloalkyl, C₁₋₇-Alkoxy, Halogen-C₁₋ ₇-alkyl oder Halogen; unsubstituiertem Heteroaryl-C₁₋₇-alkyl und Heteroaryl-C₁₋₇-alkyl, wobei das Heteroaryl, mit einer oder zwei Gruppen substituiert ist, ausgewählt aus C₁₋₇-Alkyl, C₃₋₇-Cycloalkyl, C₁₋₇-Alkoxy, Halogen-C₁₋₇-alkyl und Halogen;
R⁸ ausgewählt ist aus der Gruppe bestehend aus C₁₋₇-Alkyl, C₃₋₇-Cycloalkyl, unsubstituiertem Heteroaryl und Heteroaryl, das mit einer oder zwei Gruppen substituiert ist, ausgewählt aus C₁₋₇-Alkyl, C₃₋₇-Cycloalkyl, C₁₋₇-Alkoxy, Halogen-C₁₋ ₇-alkyl oder Halogen;
R⁹ und R¹⁰ unabhängig voneinander ausgewählt sind aus der Gruppe bestehend aus Wasserstoff, C₁₋₇-Alkyl, C₃₋₇-Cycloalkyl, unsubstituiertem Heteroaryl und Heteroaryl, das mit einer oder zwei Gruppen substituiert ist, ausgewählt aus C₁₋₇-Alkyl, C₃₋₇- Cycloalkyl, C₁₋₇-Alkoxy, Halogen-C₁₋₇-alkyl oder Halogen; oder R⁹ und R¹⁰ zusammen mit dem Stickstoffatom an das sie gebunden sind einen Pyrrolidin- oder einen Piperidinring bilden;
R¹¹ ausgewählt ist aus der Gruppe bestehend aus Wasserstoff, C₁₋₇-Alkyl und C₃₋₇-Cycloalkyl,
R¹² ausgewählt ist aus der Gruppe bestehend aus Wasserstoff, C₁₋₇-Alkyl, C₃₋₇-Cycloalkyl, Halogen-C₁₋₇-alkyl, Hydroxy-C₁₋₇-alkyl, C₁₋₇-Alkoxy-C₁₋₇-alkyl, Carboxy-C₁₋₇-alkyl, C₁₋₇-Alkoxycarbonyl-C₁₋₇-alkyl, unsubstituiertem Phenyl, Phenyl, das mit einer bis drei Gruppen substituiert ist, ausgewählt aus der Gruppe bestehend aus C₁₋₇-Alkyl, C₃₋ ₇-Cycloalkyl, C₁₋₇-Alkoxy, Halogen-C₁₋₇-alkyl und Halogen, unsubstituiertem Heteroaryl, Heteroaryl, das mit einer oder zwei Gruppen substituiert ist, ausgewählt aus C₁₋₇-Alkyl, C₃₋₇-Cycloalkyl, C₁₋₇-Alkoxy, Halogen-C₁₋₇-alkyl oder Halogen;
einer von R³ und R⁴ ausgewählt ist aus der Gruppe bestehend aus Wasserstoff, C₁₋₇-Alkyl und C₁₋₇-Alkoxy oder im Falle, dass einer von A oder B N oder N⁺-O⁻ ist, nicht vorhanden ist und
der andere und von R³ und R⁴ ausgewählt ist aus der Gruppe bestehend aus Wasserstoff, C₁₋₇-Alkyl, C₁₋₇-Alkoxy, Hydroxy-C₁₋₇-alkyl, Carboxy, C₁₋₇-Alkoxycarbonyl, Carboxy-C₁₋₇-alkoxy, C₁₋₇-Alkoxycarbonyl-C₁₋₇-alkyl, Carboxy-C₁₋₇-alkyl, C₁₋₇-Alkoxycarbonyl-C₁₋₇-alkoxy, 1H-Tetrazol-5-yl-C₁₋₇-alkoxy, Hydroxy-C₂₋₇-alkoxy, Dihydroxy-C₃₋₇-alkoxy, -(CH₂)ₙ-NR¹³R¹⁴, -(CH₂)ₙ-NHCOR¹⁵, 1H-Tetrazol-5-yl;
R¹³ und R¹⁴ unabhängig voneinander ausgewählt sind aus der Gruppe bestehend aus Wasserstoff, C₁₋₇-Alkyl und C₃₋₇-Cycloalkyl;
R¹⁵ ausgewählt ist aus der Gruppe bestehend aus C₁₋₇-Alkyl, C₃₋₇-Cycloalkyl, Halogen-C₁₋₇-alkyl, Hydroxy-C₁₋₇-alkyl, C₁₋₇-Alkoxy-C₁₋₇- alkyl, Carboxy-C₁₋₇-alkyl und C₁₋₇-Alkoxycarbonyl-C₁₋₇-alkyl;
n eine ganze Zahl ist, ausgewählt aus 1, 2 und 3;
mit der Maßgabe, dass Benzooxazole und Benzothiazole, bei denen R¹, R², R³ und R⁴ Wasserstoff sind, ausgeschlossen sind;
G ausgewählt ist aus den Gruppen 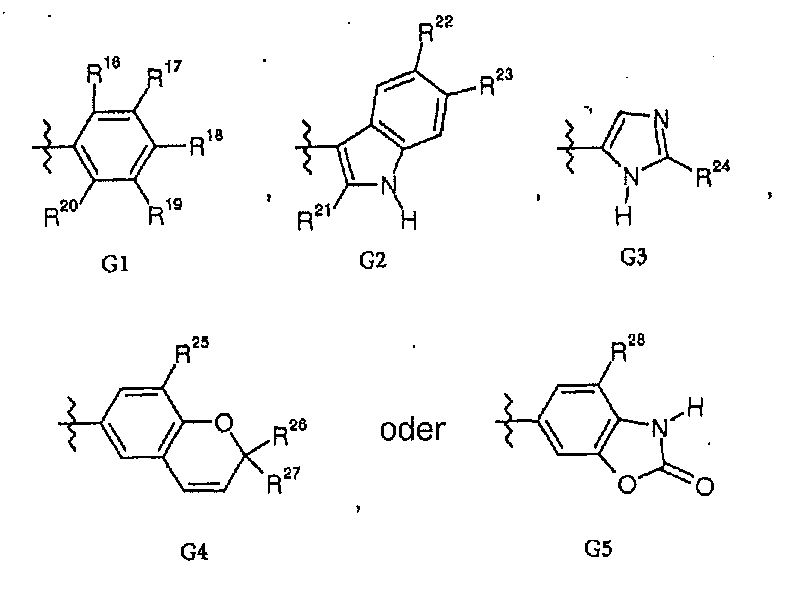
wobei
R¹⁶ Wasserstoff oder Halogen ist;
R¹⁷ ausgewählt ist aus der Gruppe bestehend aus C₁₋₇-Alkoxy, C₂₋₇-Alkenyloxy, C₃₋₇-Cycloalkyloxy, -NR²⁹R³⁰, Halogen-C₁₋₇-alkoxy, C₁₋₇-Alkoxy-C₁₋₇-alkoxy, C₁₋₇-Alkoxy-C₁₋₇-alkyl;
R²⁹ und R³⁰ unabhängig voneinander Wasserstoff oder C₁₋₇-Alkyl sind;
R¹⁸ ausgewählt ist aus der Gruppe bestehend aus Wasserstoff, C₁₋₇-Alkyl, Halogen-C₁₋₇- alkyl, Hydroxy, C₁₋₇-Alkoxy, Halogen-C₁₋₇-alkoxy, C₃₋₇Cycloalkyloxy, Halogen, Pyrrolo, -CO₂R³¹, -NR³²R³³, -SOR³⁴; unsubstituiertem Phenyl und Phenyl, das mit einer bis zwei Gruppen substituiert ist, ausgewählt aus der Gruppe bestehend aus C₁₋₇- Alkyl, Halogen-C₁₋₇-alkyl, Halogen-C₁₋₇-alkoxy, C₁₋₇-Alkoxy und Halogen;
R³¹ Wasserstoff oder C₁₋₇-Alkyl ist;
R³² und R³³ unabhängig voneinander Wasserstoff oder C₁₋₇-Alkyl sind; R³⁴ C₁₋₇-Alkyl ist;
R¹⁹ ausgewählt ist aus der Gruppe bestehend aus Wasserstoff, C₁₋₇-Alkyl, Halogen, C₁₋₇-Alkoxy, C₂₋₇-Alkenyloxy, -O-Tetrahydropyranyl, C₃₋₇-Cycyloalkyloxy, Halogen-C₁₋₇-alkoxy, C₁₋₇-Alkoxy-C₁₋₇-alkoxy und C₁₋₇-Alkoxy-C₁₋₇-alkyl;
R²⁰ Wasserstoff oder Halogen ist;
R²¹ Wasserstoff oder C₁₋₇-Alkyl ist;
R²² und R²³ unabhängig voneinander ausgewählt sind aus der Gruppe bestehend aus Wasserstoff, C₁₋₇-Alkyl, C₁₋₇-Alkoxy und Nitro;
R²⁴ unsubstituiertes Phenyl ist oder Phenyl, das mit einer bis zwei Gruppen substituiert ist, ausgewählt aus der Gruppe bestehend aus C₁₋₇-Alkyl, C₁₋₇-Alkoxy und Halogen;
R²⁵ C₁₋₇-Alkoxy ist;
R²⁶ und R²⁷ unabhängig voneinander C₁₋₇-Alkyl sind;
R²⁸ C₁₋₇-Alkoxy ist;
und pharmazeutisch verträgliche Salze davon.

22. Verbindungen der Formel I gemäss Anspruch 1, ausgewählt aus der Gruppe bestehend aus
[1-(3-Ethoxy-4-methoxybenzyl)piperidin-4-yl]-(7-nitrobenzooxazol-2-yl)amin,
[1-(3,5-Diethoxy-4fluorbenzyl)piperidin-4-yl](7-nitrobenzooxazol-2-yl)amin,
N²-[1-(3,5-Diethoxy-4-fluorbenzyl)piperidin-4-yl]benzooxazol-2,7-diamin,
N-{2-[1-(3,5-Diethoxy-4-fluorbenzyl)piperidin-4-ylamino]benzooxazol-7-yl}-2-methoxyacetamid,
N-{2-[1-(3,5-Diethoxy-4-fluorbenzyl)piperidin-4-ylamino]benzooxazol-7-yl}succinamidsäure,
Pyrimidin-5-carbonsäure {2-[1-(3,5-diethoxy-4-fluorbenzyl)piperidin-4-ylamino]-benzooxazol-7-yl}amid,
N-{2-[1-(3,5-Diethoxy-4-fluorbenzyl)piperidin-4-ylamino]-benzooxazol-7-yl}-2-pyridin-3-yl-acetamid,
1-Methyl-1H-imidazol-4-sulfonsäure {2-[1-(3,5-diethoxy-4-fluorbenzyl)piperidin-4-ylamino]-benzooxazol-7-yl}amid,
2-[1-(3,5-Diethoxy-4-fluorbenzyl)piperidin-4-ylamino]-benzooxazol-7-yl}methansulfonamid,
[1-(3,5-Diethoxy-4-fluorbenzyl)piperidin-4-yl]-[7-(pyrrolidin-1-sulfonyl)benzooxazol-2-yl]amin,
[1-(3,5-Diethoxybenzyl)piperidin-4-yl]-[7-(pyrrolidin-1-sulfonyl)benzooxazol-2-yl]amin,
[1-(3-Ethoxy-4-methoxybenzyl)piperidin-4-yl]-[7-(pyrrolidin-1-sulfonyl)benzooxazol-2-yl]amin,
[1-(3,5-Diethoxy-4-pyrrol-1-yl-benzyl)piperidin-4-yl]-[7-(pyrrolidin-1-sulfonyl)benzooxazol-2-yl]amin,
[1-(4-Chlor-3-ethoxybenzyl)piperidin-4-yl]-[7-(pyrrolidin-1-sulfonyl)benzooxazol-2-yl]amin,
[1-(3-Ethoxy-4-methoxybenzyl)piperidin-4-yl]-(4-nitrobenzooxazol-2-yl)amin,
[1-(3,5-Diethoxy-4-pyrrol-1-yl-benzyl)piperidin-4-yl]-(4-nitrobenzooxazol-2-yl)amin,
[1-(3,5-Diethoxy-4-fluorbenzyl)piperidin-4-yl]-(4-nitrobenzooxazol-2-yl)amin,
N²-[1-(3,5-Diethoxy-4-fluorbenzyl)piperidin-4-yl]-benzooxazol-2,4-diamin,
N-{2-[1-(3,5-Diethoxy-4-fluorbenzyl)piperidin-4-ylamino]-benzooxazol-4-yl}-2-methoxyacetamid,
N-{2-[1-(3,5-Diethoxy-4-fluorbenzyl)piperidin-4-ylamino]-benzooxazol-4-yl}succinamidsäuremethylester,
N-{2-[1-(3,5-Diethoxy-4-fluorbenzyl)piperidin-4-ylamino]benzooxazal-4-yl} succinamidsäure,
Pyrimidin-5-carbonsäure {2-[1-(3,5-diethoxy-4-fluorbenzyl)piperidin-4-ylamino]benzooxazol-4-yl}amid,
N²-[1-(3,5-Diethoxy-4-pyrrol-1-yl-benzyl)piperidin-4-yl]benzooxazol-2,4-diamin,
N-{2-[1-(3,5-Diethoxy-4-pyrrol-1-yl-benzyl)piperidin-4-ylamino]benzooxazol-4-yl}-2-methoxyacetamid,
N-{2-[1-(3,5-Diethoxy-4-fluorbenzyl)piperidin-4-ylamino]benzooxazol-4-yl}-2-(1H-tetrazol-5-yl)acetamid,
N-{2-[1-(3,5-Diethoxy-4-pyrrol-1-yl-benzyl)piperidin-4-ylamino]benzooxazol-4-yl}succinamidsäuremethylester,
N-{2-[1-(3,5-Diethoxy-4-pyrrol-1-yl-benzyl)piperidin-4-ylamino]benzooxazol-4-yl}succinamidsäure,
[1-(3-Ethoxy-4-methoxybenzyl)piperidin-4-yl]-[4-(pyridin-4-ylmethoxy)benzooxazol-2-yl]amin,
[1-(3,5-Diethoxy-4-pyrrol-1-yl-benzyl)piperidin-4-yl]-[4-(pyridin-4-ylmethoxy)benzooxazol-2-yl]amin,
[1-(3,5-Diethoxy-4-fluorbenzyl)piperidin-4-yl]-[4-(pyridin-4-ylmethoxy)benzooxazol-2-yl]amin,
2-[1-(3-Ethoxy-4-methoxybenzyl)piperidin-4-ylamino]benzooxazol-4-carbonsäuremethylester,
2-[1-(3,5-Diethoxy-4-pyrrol-1-yl-benzyl)piperidin-4-ylamino]benzooxazol-4-carbonsäuremethylester,
2-[1-(3,5-Diethoxy-4-fluorbenzyl)piperidin-4-ylamino]benzooxazol-4-carbonsäuremethylester,
2-[1-(3-Ethoxy-4-methoxybenzyl)piperidin-4-ylamino]benzooxazol-4-carbonsäure,
2-[1-(3,5-Diethoxy-4-pyrrol-1-yl-benzyl)piperidin-4-ylamino]benzooxazol-4-carbonsäure,
2-[1-(3,5-Diethoxy-4-fluorbenzyl)piperidin-4-ylamino]benzooxazol-4-carbonsäure,
2-[1-(3-Ethoxy-4-methoxybenzyl)piperidin-4-ylamino]benzooxazol-4-carbonsäurepyridin-3-ylamid,
2-[1-(3,5-Diethoxy-4-fluorbenzyl)piperidin-4-ylamino]benzooxazol-4-carbonsäurepyridin-3-ylamid,
2-[1-(3,5-Diethoxy-4-pyrrol-1-yl-benzyl)piperidin-4-ylamino]benzooxazol-4-carbonsäurepyridin-3-ylamid,
2-[1-(3-Ethoxy-4-methoxybenzyl)piperidin-4-ylamino]benzooxazol-4-carbonsäure(2-hydroxyethyl)amid,
2-[1-(3,5-Diethoxy-4-fluorbenzyl)piperidin-4-ylamino]benzooxazol-4-carbonsäure(2-hydroxyethyl)amid,
2-[1-(3,5-Diethoxy-4-pyrrol-1-yl-benzyl)piperidin-4-ylamino]benzooxazol-4-carbonsäure(2-hydroxyethyl)amid,
({2-[1-(3-Ethoxy-4-methoxybenzyl)piperidin-4-ylamino]benzooxazol-4-carbonyl}amino)essigsäuremethylester,
({2-[1-(3,5-Diethoxy-4-fluorbenzyl)piperidin-4-ylamino]benzooxazol-4-carbonyl}amino)essigsäuremethylester,
({2-[1-(3,5-Diethoxy-4-pyrrol-1-yl-benzyl)piperidin-4-ylamino]benzooxazol-4-carbonyl}amino)essigsäuremethylester,
({2-[1-(3-Ethoxy-4-methoxybenzyl)piperidin-4-ylamino]benzooxazol-4-carbonyl}amino)essigsäure,
({2-[1-(3,5-Diethoxy-4-fluorbenzyl)piperidin-4-ylamino]benzooxazol-4-carbonyl}amino)essigsäure,
({2-[1-(3,5-Diethoxy-4-pyrrol-1-yl-benzyl)piperidin-4-ylamino]benzooxazol-4-carbonyl}amino)essigsäure,
[1-(3-Ethoxy-4-methoxybenzyl)piperidin-4-yl]-(4-Jodbenzooxazol-2-yl)amin,
[1-(3,5-Diethoxy-4-fluorbenzyl)piperidin-4-yl]-(4-Jodbenzooxazol-2-yl)amin,
[1-(3,5-Diethoxy-4-pyrrol-1-yl-benzyl)piperidin-4-yl]-(4-Jodbenzooxazol-2-yl)amin,
(7-Brombenzooxazol-2-yl)-[1-(3-ethoxy-4-methoxybenzyl)piperidin-4-yl]amin,
(7-Brombenzooxazol-2-yl)-[1-(3,5-Diethoxy-4-fluorbenzyl)piperidin-4-yl]amin,
(7-Brombenzooxazol-2-yl)-[1-(3,5-Diethoxy-4-pyrrol-1-yl-benzyl)piperidin-4-yl]amin,
2-[1-(3-Ethoxy-4-hydroxybenzyl)piperidin-4-ylamino]benzooxazol-6-carbonsäuremethylester,
2-[1-(3-Ethoxy-4-methoxybenzyl)piperidin-4-ylamino]benzooxazol-6-carbonsäuremethylester,
2-[1-(8-Ethoxy-2,2-dimethyl-2H-chromen-6-ylmethyl)piperidin-4-ylamino]benzooxazol-6-carbonsäuremethylester,
2-[1-(3-Isobutoxy-4-methoxybenzyl)piperidin-4-ylamino]benzooxazol-6-carbonsäuremethylester,
2-{1-[3-(2-Fluorethoxy)-4-methoxybenzyl]piperidin-4-ylamino}benzooxazol-6-carbonsäuremethylester,
2-[1-(3-Ethoxy-4-methylbenzyl)piperidin-4-ylamino]benzooxazol-6-carbonsäuremethylester,
2-[1-(4-Chlor-3-ethoxybenzyl)piperidin-4-ylamino]benzooxazol-6-carbonsäuremethylester,
2-[1-(4-Amino-3,5-diethoxybenzyl)piperidin-4-ylamino]benzooxazol-6-carbonsäuremethylester,
2-[1-(3,5-Diethoxy-4-fluorbenzyl)piperidin-4-ylamino]benzooxazol-6-carbonsäuremethylester,
2-[1-(3-Ethoxy-4-hydroxybenzyl)piperidin-4-ylamino]benzooxazol-6-carbonsäure,
2-[1-(3-Ethoxy-4-methoxybenzyl)piperidin-4-ylamino]benzooxazol-6-carbonsäure,
2-[1-(8-Ethoxy-2,2-dimethyl-2H-chromen-6-ylmethyl)piperidin-4-ylamino]benzooxazol-6-carbonsäure,
2-[1-(3-Isobutoxy-4-methaxybenzyl)piperidin-4-ylamino]benzooxazol-6-carbonsäure,
2-{1-[3-(2-Fluorethoxy)-4-methoxybenzyl]piperidin-4-ylamino}benzooxazol-6-carbonsäure,
2-[1-(3-Ethoxy-4-methylbenzyl)piperidin-4-ylamino]benzooxazol-6-carbonsäure,
2-[1-(4-Chlor-3-ethoxybenzyl)piperidin-4-ylamino]benzooxazol-6-carbonsäure,
2-[1-(4-Amino-3,5-diethoxybenzyl)piperidin-4-ylamino]benzooxazol-6-carbonsäure,
2-[1-(3,5-Diethoxy-4-fluorbenzyl)piperidin-4-ylamino]benzooxazol-6-carbonsäure,
[1-(3-Ethoxy-4-fluorbenzyl)piperidin-4-yl]-(4-nitrobenzooxazol-2-yl)amin,
[1-(4-chlor-3-ethoxybenzyl)piperidin-4-yl]-(4-nitrobenzooxazol-2-yl)amin,
[1-(3-Ethoxy-4-methylbenzyl)piperidin-4-yl]-(4-nitrobenzooxazol-2-yl)amin,
2-[1-(3,5-Diethoxy-4-fluorbenzyl)piperidin-4-ylamino]benzooxazol-7-sulfonsäureamid,
2-[1-(3-Ethoxy-4-fluorbenzyl)piperidin-4-ylamino]benzooxazol-7-sulfonsäureamid,
2-[1-(3-Ethoxy-4-methoxybenzyl)piperidin-4-ylamino]benzooxazol-7-sulfonsäureamid,
[1-(3,5-Diethoxy-4-fluorbenzyl)piperidin-4-yl]-(7-methoxybenzooxazol-2-yl)amin,
[1-(3,5-Diethoxy-4-pyrrol-1-yl-benzyl)piperidin-4-yl]-(7-methoxybenzooxazol-2-yl)amin,
[1-(3,5-Diethoxy-4-fluorbenzyl)piperidin-4-yl]oxazolo[5,4-c]pyridin-2-yl-amin,
[1-(4-Chlor-3-ethoxybenzyl)piperidin-4-yl]oxazolo[5,4-c]pyridin-2-yl-amin,
[1-(3-Ethoxy-4-methylbenzyl)piperidin-4-yl]oxazolo[5,4-c]pyridin-2-yl-amin,
[1-(3-Ethoxy-4-methoxybenzyl)piperidin-4-yl]oxazolo[5,4-c]pyridin-2-yl-amin,
[1-(3-Isobutoxy-4-methoxybenzyl)piperidin-4-yl]oxazolo[5,4-c]pyridin-2-yl-amin,
4-Ethoxy-6-[4-(oxazolo[5,4-c]pyridin-2-ylamino)piperidin-1-ylmethyl]-3H-benzooxazol-2-on,
[rac]-[1-(3,5-Diethoxy-4-methansulfinylbenzyl)piperidin-4-yl]oxazolo[5,4-c]pyridin-2-ylamin,
[1-(3-Ethylamino-4-methoxybenzyl)piperidin-4-yl]oxazolo[5,4-c]pyridin-2-yl-amin,
[1-(8-Ethoxy-2,2,dimethyl-2H-chromen-6-ylmethyl)piperidin-4-yl]oxazolo[5,4-c]pyridin-2-yl-amin,
[1-(4-Methoxy-3-propoxybenzyl)piperidin-4-yl]oxazolo[5,4-c]pyridin2-yl-amin,
{1-[3-(2-Fluorethoxy)-4-methoxybenzyl]piperidin-4-yl}oxazolo[5,4-c]pyridin-2-yl-amin,
2-Ethoxy-4-[4-(oxazolo[5,4-c]pyridin-2-ylamino)piperidin-1-ylmethyl]phenol,
[1-(4-Amino-3,5-diethoxybenzyl)piperidin-4-yl]oxazolo[5,4-c]pyridin-2-yl-amin,
[1-(3,5-Diisopropoxybenzyl)piperidin-4-yl]oxazolo[5,4-c]pyridin-2-yl-amin,
[1-(3-Ethoxy-4-fluorbenzyl)piperidin-4-yl]oxazolo[5,4-c]pyridin-2-yl-amin,
[1-(3-Ethoxy-4-isopropoxybenzyl)piperidin-4-yl]oxazolo[5,4-c]pyridin-2-yl-amin,
Oxazolo[5,4-c]pyridin-2-yl-[1-(2-phenyl-3H-imidazol-4-ylmethyl)piperidin-4-yl]amin,
[1-(2-Methyl-5-nitro-1-H-indol-3-ylmethyl)piperidin-4-yl]oxazolo[5,4-c]pyridin-2-yl-amin,
{2-[1-(3-Ethoxy-4-methoxybenzyl)piperidin-4-ylaminol]benzooxazol-5-yloxy}essigsäuremethylester,
{2-[1-(3,5-Diethoxy-4-fluorbenzyl)piperidin-4-ylamino]benzooxazol-5-yloxy}essigsäuremethylester,
{2-[1-(3,5-Diethoxy-4-pyrrol-1-yl-benzyl)piperidin-4-ylamino]benzooxazol-5-yloxy}essigsäuremethylester,
{2-[1-(3-Ethoxy-4-methoxybenzyl)piperidin-4-ylamino]benzooxazol-5-yloxy}essigsäure,
{2-[1-(3,5-Diethoxy-4-fluorbenzyl)piperidin-4-ylamino]benzooxazol-5-yloxy}essigsäure,
{2-[1-(3,5-Diethoxy-4-pyrrol-1-yl-benzyl)piperidin-4-ylamino]benzooxazol-5-yloxy}essigsäure,
[1-(3,5-Diethoxy-4-fluorbenzyl)piperidin-4-yl]-(7-phenylbenzooxazol-2-yl)amin,
{2-[1-(3-Ethoxy-4-methoxybenzyl)piperidin-4-ylamino]benzooxazol-7-yloxy}essigsäuremethylester,
{2-[1-(3,5-Diethoxy-4-fluorbenzyl)piperidin-4-ylamino]benzooxazol-7-yloxy}essigsäuremethylester,
{2-[1-(3,5-Diethoxy-4-pyrrol-1-yl-benzyl)piperidin-4-ylamino]benzooxazol-7-yloxy}essigsäuremethylester,
{2-[1-(3-Ethoxy-4-methoxybenzyl)piperidin-4-ylamino]benzooxazol-7-yloxy}essigsäure,
{2-[1-(3,5-Diethoxy-4-fluorbenzyl)piperidin-4-ylamino]benzooxazol-7-yloxy}essigsäure,
{2-[1-(3,5-Diethoxy-4-pyrrol-1-yl-benzyl)piperidin-4-ylamino]benzooxazol-7-yloxy}essigsäure,
(5-Aminomethylbenzooxazol-2-yl)-[1-(3,5-diethoxy-4-fluorbenzyl)piperidin-4-yl]amin,
4-{2-[1-(3,5-Diethoxy-4-fluorbenzyl)piperidin-4-ylamino]benzooxazol-7-yloxy}buttersäuremethylester,
4-{2-[1-(3,5-Diethoxy-4-pyrrol-1-yl-benzyl)piperidin-4-ylamino]benzooxazol-7-yloxy}buttersäuremethylester,
4-{2-[1-(3-Ethoxy-4-methoxybenzyl)piperidin-4-ylamino]benzooxazol-7-yloxy}buttersäuremethylester,
4-{2-[1-(3,5-Diethoxy-4-fluorbenzyl)piperidin-4-ylamino]benzooxazol-7-yloxy}buttersäure,
4-{2-[1-(3,5-Diethoxy-4-pyrrol-1-yl-benzyl)piperidin-4-ylamino]benzooxazol-7-yloxy}buttersäure,
4-{2-[1-(3-Ethoxy-4-methoxybenzyl)piperidin-4-ylamino]benzooxazol-7-yloxy}buttersäure,
[rac]-3-{2-[1-(3,5-Diethoxy-4-fluorbenzyl)piperidin-4-ylamino]benzooxazol-7-yloxy}propan-1,2-diol,
[rac]-3-{2-[1-(3,5-Diethoxy-4-pyrrol-1-yl-benzyl)piperidin-4-ylamino]benzooxazol-7-yloxy}propan-1,2-diol,
[rac]-3-{2-[1-(3-Ethoxy-4-methoxybenzyl)piperidin-4-ylamino]benzooxazol-7-yloxy}propan-1,2-diol,
4-{2-[1-(3,5-Diethoxy-4-fluorbenzyl)piperidin-4-ylamino]benzooxazol-5-yloxy}buttersäuremethylester,
4-{2-[1-(3,5-Diethoxy-4-porrol-1-yl-benzyl)piperidin-4-ylamino]benzooxazol-5-yloxy}buttersäuremethylester,
4-{2-[1-(3-Ethoxy-4-methoxybenzyl)piperidin-4-ylamino]benzooxazol-5-yloxy}buttersäuremethylester,
4-{2-[1-(3,5-Diethoxy-4-fluorbenzyl)piperidin-4-ylamino]benzooxazol-5-yloxy}buttersäure,
4-{2-[1-(3,5-Diethoxy-4-pyrrol-1-yl-benzyl)piperidin-4-ylamino]benzooxazol-5-yloxy}buttersäure,
4-{2-1-(3-Ethoxy-4-methoxybenzyl)piperidin-4-ylamino]benzooxazol-5-yloxy}buttersäure,
1-{2-[1-(3,5-Diethoxy-4-fluorbenzyl)piperidin-4-ylamino]benzooxazol-5-yloxy}cyclobutancarbonsäureethylester,
1-{2-[1-(3,5-Diethoxy-4-pyrrol-1-yl-benzyl)piperidin-4-ylamino]benzooxazol-5-yloxy}cyclobutancarbonsäureethylester,
1-{2-1-(3,5-Diethoxy-4-fluorbenzyl)piperidin-4-ylamino]benzooxazol-5-yloxy}cyclobutancarbonsäure,
1-{2-[1-(3,5-Diethoxy-4-pyrrol-1-yl-benzyl)piperidin-4-ylamino]benzooxazol-5-yloxy}cyclobutancarbonsäure,
N-{2-[1-(3,5-Diethoxy-4-fluorbenzyl)piperidin-4-ylamino]benzooxazol-5-ylmethyl}acetamid,
N-{2-[1-(3,5-Diethoxy-4-fluorbenzyl)piperidin-4-ylamino]benzooxazol-5-ylmethyl}malonamidsäureethylester,
(S)-N-{2-[1-(3,5-Diethoxy-4-fluorbenzyl)piperidin-4-ylamino]benzooxazol-5-ylmethyl}-2-hydroxypropionamid,
[1-(3,5-Diethoxy-4-fluorbenzyl)piperidin-4-yl]-(4-methyloxazolo[5,4-c]pyridin-2-yl)amin,
[1-(3,5-Diethoxy-4-pyrrol-1-yl-benzyl)piperidin-4-yl]-(4-methyloxazolo[5,4-c]pyridin-2-yl)amin,
[1-(3,5-Diisopropoxybenzyl)piperidin-4-yl]-(4-methyloxazolo[5,4-c]pyridin-2-yl)amin,
N-{2-[1-(3,5-Diethoxy-4-fluorbenzyl)piperidin-4-ylamino]benzooxazol-5-ylmethyl}malonamidsäure,
[rac]-3-{2-[1-(3,5-Diethoxy-4-fluorbenzyl)piperidin-4-ylamino]benzooxazol-5-yloxy}propan-1,2-diol,
[rac]-3-{2-[1-(3,5-Diethoxy-4-pyrrol-1-yl-benzyl)piperidin-4-ylamino]benzooxazol-5-yloxy}propan-1,2-diol,
[rac]-3-{2-[1-(3,5-Diisoprapoxybenzyl)piperidin-4-ylamino]benzooxazol-5-yloxy}propan-1,2-diol,
[1-(3,5-Diethoxy-4-pyrrol-1-yl-benzyl)piperidin-4-yl]-[5-(1H-tetrazol-5-ylmethoxy)benzooxazol-2-yl]amin,
[1-(3,5-Diethoxy-4-fluorbenzyl)piperidin-4-yl]-[5-(1H-tetrazol-5-ylmethoxy)benzooxazol-2-yl]amin,
{2-[1-(3,5-Diethoxy-4-fluorbenzyl)piperidin-4-ylamino]oxazolo[5,4-c]pyridin-4-yl}methanol,
{2-[1-(3,5-Diethoxy-4-pyrrol-1-yl-benzyl)piperidin-4-ylamino]oxazolo[5,4-c]pyridin-4-yl}methanol,
{2-[1-(3,5-Diisopropoxybenzyl)piperidin-4-ylamino]oxazolo[5,4-c]pyridin-4-yl}methanol,
{2-[1-(3,5-Diethoxy-4-[1,2,4]triazol-1-yl-benzyl)piperidin-4-ylamino]oxazolo[5,4-c]pyridin-4-yl}methanol,
{2-[1-(3,5-Diethoxy-4-fluorbenzyl)piperidin-4-ylamino]benzooxazol-5-yl}essigsäuremethylester,
{2-[1-(3,5-Diethoxy-4-pyrrol-1-yl-benzyl)piperidin-4-ylamino]benzooxazol-5-yl}essigsäuremethylester,
{2-[1-(3,5-Diisopropoxybenzyl)piperidin-4-ylamino]benzooxazol-5-yl}essigsäuremethylester,
{2-[1-(3,5-Diethoxy-4-[1,2,4]triazol-1-ylbenzyl)piperidin-4-ylamino]benzooxazol-5-yl}essigsäuremethylester,
{2-[1-(3,5-Diethoxy-4-fluorbenzyl)piperidin-4-ylamino]benzooxazol-5-yl}essigsäure,
{2-[1-(3,5-Diethoxy-4-pyrrol-1-yl-benzyl)piperidin-4-ylamino]benzooxazol-5-yl}essigsäure,
{2-[1-(3,5-Diisopropoxybenzyl)piperidin-4-ylamino]benzooxazol-5-yl}essigsäure,
{2-[1-(3,5-Diethoxy-4-[1,2,4]triazol-1-yl-benzyl)piperidin-4-ylamino]benzooxazol-5-yl}essigsäure,
[1-(3,5-Diethoxy-4-fluorbenzyl)piperidin-4-yl]-(5-methoxybenzooxazol-2-yl)amin,
[1-(3,5-Diethoxy-4-pyrrol-1-yl-benzyl)piperidin-4-yl]-(5-methoxybenzooxazol-2-yl)amin,
2-[1-(3,5-Diethoxy-4-fluorbenzyl)piperidin-4-ylamino]benzooxazol-5-carbonsäuremethylester,
2-[1-(3,5-Diethoxy-4-pyrrol-1-yl-benzyl)piperidin-4-ylamino]benzooxazol-5-carbonsäuremethylester,
2-[1-(3,5-Diisopropoxybenzyl)piperidin-4-ylamino]benzooxazol-5-carbonsäuremethylester,
2-[1-(3,5-Diethoxy-4-[1,2,4]triazol-1-yl-benzyl)piperidin-4-ylamino]benzooxazol-5-carbonsäuremethylester,
2-[1-(3,5-Diethoxy-4-fluorbenzyl)piperidin-4-ylamino]benzooxazol-5-carbonsäure,
2-[1-(3,5-Diethoxy-4-pyrrol-1-yl-benzyl)piperidin-4-ylamino]benzooxazol-5-carbonsäure,
2-[1-(3,5-Diisopropoxybenzyl)piperidin-4-ylamino]benzooxazol-5-carbonsäure,
2-[1-(3,5-Diethoxy-4-[1,2,4]triazol-1-yl-benzyl)piperidin-4-ylamino]benzooxazol-5-carbonsäure,
2-[1-(4-Chlor-3,5-diethoxybenzyl)piperidin-4-ylamino]benzooxazol-5-carbonsäuremethylester,
2-[1-(2,6-Diethoxy-4'-fluorbiphenyl-4-ylmethyl)piperidin-4-ylamino]benzooxazol-5-carbonsäuremethylester,
2-[1-(4-Chlor-3,5-diethoxybenzyl)piperidin-4-ylamino]benzooxazol-5-carbonsäure,
2-[1-(2,6-Diethoxy-4'-fluorbiphenyl-4-ylmethyl)piperidin-4-ylamino]benzooxazol-5-carbonsäure,
{2-[1-(3,5-Diethoxy-4-fluorbenzyl)piperidin-4-ylamino]benzooxazol-5-yloxy}acetonitril,
N-tert-butyl-2-{2-[1-(3,5-Diethoxy-4-fluorbenzyl)piperidin-4-ylamino]benzooxazol-5-yloxy}acetamid,
{2-[1-(3,5-Diisopropoxybenzyl)piperidin-4-ylamino]benzooxazol-5-yloxy}acetonitril,
N-tert-butyl-2-{2-[1-(3,5-Diisopropoxybenzyl)piperidin-4-ylamino]benzooxazol-5-yloxy}acetamid,
{2-[1-(4-chlor-3,5-diethoxybenzyl)piperidin-4-ylamino]benzooxazol-5-yloxy}acetonitril,
N-tert-butyl-2-{2-[1-(4-Chlor-3,5-diethoxybenzyl)piperidin-4-ylamino]benzooxazol-5-yloxy}acetamid,
{2-[1-(2,6-Diethoxy-4'-fluorbiphenyl-4-ylmethyl)piperidin-4-ylamino]benzooxazol-5-yloxy}acetonitril,
N-tert-butyl-2-{2-[1-(2,6-Diethoxy-4'-fluorbiphenyl-4-ylmethyl)piperidin-4-ylamino]benzooxazol-5-yloxy}acetamid,
2-{2-[1-(2,6-Diethoxy-4'-fluorbiphenyl-4-ylmethyl)piperidin-4-ylamino]benzooxazol-5-yloxy}acetamid,
2-{2-[1-(3,5-Diisopropoxybenzyl)piperidin-4-ylamino]benzooxazol-5-yloxy}acetamid,
2-{2-[1-(4-Chlor-3,5-diethoxybenzyl)piperidin-4-ylamino]benzooxazol-5-yloxy}acetamid,
2-{2-[1-(3,5-Diethoxy-4-fluorbenzyl)piperidin-4-ylamino]benzooxazol-5-yloxy}acetamid,
[rac]-3-{2-[1-(2,6-Diethoxy-4'-fluorbiphenyl-4-ylmethyl)piperidin-4-ylamino]benzooxazol-5-yloxy}propan-1,2-diol,
[rac]-3-{2-[1-(4-Chlor-3,5-Diethoxybenzyl)piperidin-4-ylamino]benzooxazol-5-yloxy}propan-1,2-diol,
2-{2-[1-(3,5-Diisopropoxybenzyl)piperidin-4-ylamino]benzooxazol-5-yl}ethanol,
2-{2-[1-(3,5-Diethoxy-4-fluorbenzyl)piperidin-4-ylamino]benzooxazol-5-yl}ethanol,
{2-[1-(3,5-Diethoxy-4-fluorbenzyl)piperidin-4-ylamino]benzooxazol-5-yl}methanol,
[5-(2-Aminoethoxy)benzooxazol-2-yl]-[1-(3,5-diethoxy-4-fluorbenzyl)piperidin-4-yl]amin,
2-[1-(2,6-Diethoxy-4'-fluorbiphenyl-4-ylmethyl)piperidin-4-ylamino]benzooxazol-6-carbonsäure,
2-[1-(4-Chlor-3,5-diethoxybenzyl)piperidin-4-ylamino]benzooxazol-6-carbonsäure,
2-[1-(3,5-Diisopropoxybenzyl)piperidin-4-ylamino]benzooxazol-6-carbonsäure,
2-[1-(4-Chlor-3,5-diethoxybenzyl)piperidin-4-ylamino]benzooxazol-5-ol,
3-{2-[1-(3,5-Diethoxy-4-fluorbenzyl)piperidin-4-ylamino]benzooxazol-5-yloxy}propan-1-ol,
3-{2-[1-(3,5-Diisopropoxybenzyl)piperidin-4-ylamino]benzooxazol-5-yloxy}pzopan-1-ol,
3-{2-[1-(4-Chlor-3,5-diethoxybenzyl)piperidin-4-ylamino]benzooxazol-5-yloxy}propan-1-ol,
3-{2-[1-(2,6-Diethoxy-4'-fluorbiphenyl-4-ylmethyl)piperidin-4-ylamino]benzooxazol-5-yloxy}propan-1-ol,
2-{2-[1-(3,5-Diethoxy-4-fluorbenzyl)piperidin-4-ylamino]benzooxazol-5-yloxy}ethanol,
2-{2-[1-(2,6-Diethoxy-4'-fluorbiphenyl-4-ylmethyl)piperidin-4-ylamino]benzooxazol-5-yloxy}ethanol,
[1-(3,5-Diethoxy-4-fluorbenzyl)piperidin-4-yl]-[5-(3-methoxypropoxy)benzooxazol-2-yl]amin,
[1-(3,5-Diisopropoxybenzyl)piperidin-4-yl]-[5-(3-methoxypropoxy)benzooxazol-2-yl]amin,
[1-(4-Chlor-3,5-diethoxybenzyl)piperidin-4-yl]-[5-(3-methoxypropoxy)benzooxazol-2-yl]amin,
[1-(2,6-Diethoxy-4'-fluorbiphenyl-4-ylmethyl)piperidin-4-yl]-[5-(3-methoxypropoxy)benzooxazol-2-yl]amin,
[1-(4-Chlor-3,5-diethoxybenzyl)piperidin-4-yl]-[5-(3-methansulfonylpropoxy)benzooxazol-2-yl]amin,
Methansulfonsäure 2-[1-(3,5-diethoxy-4-fluorbenzyl)piperidin-4-ylamino]benzooxazol-5-ylester,
Methansulfonsäure 2-[1-(3,5-diisopropoxybenzyl)piperidin-4-ylamino]benzooxazol-5-ylester,
Methansulfonsäure 2-[1-(4-chlor-3,5-diethoxybenzyl)piperidin-4-ylamino]benzooxazol-5-ylester,
Methansulfonsäure 2-[1-(2,6-diethoxy-4'-fluorbiphenyl-4-ylmethyl)piperidin-4-ylaminol]benzooxazol-5-ylester,
[1-(3,5-Diethoxy-4-fluorbenzyl)piperidin-4-yl]-[5-(3-[1,2,4]triazol-1-yl-propoxy)benzooxazol-2-yl]amin,
[1-(3,5-Diisopropoxybenzyl)piperidin-4-yl]-[5-(3-[1,2,4]triazol-1-yl-propoxy)benzooxazol-2-yl]amin,
[1-(4-Chlor-3,5-diethoxybenzyl)piperidin-4-yl]-[5-(3-[1,2,4]triazol-1-yl-propoxy)benzooxazol-2-yl]amin,
[1-(2,6-Diethoxy-4'-fluorbiphenyl-4-ylmethyl)piperidin-4-yl]-[5-(3-[1,2,4]triazol-1-yl-propoxy)benzooxazol-2-yl]amin,
2-[1-(3,5-Diethoxy-4-fluorbenzyl)piperidin-4-ylamino]benzothiazol-6-carbonsäureethylester,
2-[1-(3,5-Diisopropoxybenlzyl)piperidin-4-ylamino]benzothiazol-6-carbonsäureethylester,
2-[1-(4-chlor-3,5-Ddiethoxybenzyl)piperidin-4-ylamino]benzothiazol-6-carbonsäureethylester,
2-[1-(2,6-Diethoxy-4'-fluorbiphenyl-4-ylmethyl)piperidin-4-ylamino]benzothiazol-6-carbonsäureethylester,
2-[1-(3,5-Diethoxy-4-pyrrol-1-ylbenzyl)piperidin-4-ylamino]benzothiazol-6-carbonsäureethylester,
2-[1-(3,5-Diethoxy-4-fluorbenzyl)piperidin-4-ylamino]benzothiazol-6-carbonsäure,
2-[1-(3,5-Diisopropoxybenzyl)piperidin-4-ylamino]benzothiazol-6-carbonsäure,
2-[1-(4-Chlor-3,5-diethoxybenzyl)piperidin-4-ylamino]benzothiazol-6-carbonsäure,
2-[1-(2,6-Diethoxy-4'-fluorbiphenyl-4-ylmethyl)piperidin-4-ylamino]benzothiazol-6-carbonsäure,
2-[1-(3,5-Diethoxy-4-pyrrol-1-yl-benzyl)piperidin-4-ylamino]benzothiazol-6-carbonsäure,
2-[1-(3,5-Diisopropoxybenzyl)piperidin-4-ylamino]benzothiazol-5-carbonsäuremethylester,
2-[1-(4-Chlor-3,5-diethoxybenzyl)piperidin-4-ylamino]benzothiazol-5-carbonsäuremethylester,
2-[1-(2,6-Diethoxy-4'-fluorbiphenyl-4-ylmethyl)piperidin-4-ylamino]benzothiazol-5-carbonsäuremethylester,
2-[1-(3,5-Diethoxy-4-pyrrol-1-yl-benzyl)piperidin-4-ylamino]benzothiazol-5-carbonsäuremethylester,
2-[1-(3,5-Diisopropoxybenzyl)piperidin-4-ylamino]benzothiazol-5-carbonsäure,
2-[1-(4-Chlor-3,5-diethoxybenzyl)piperidin-4-ylamino]benzothiazol-5-carbonsäure,
2-[1-(2,6-Diethoxy-4'-fluorbiphenyl-4ylmethyl)piperidin-4-ylamino]benzothiazol-5-carbonsäure,
2-[1-(3,5-Diethoxy-4-pyrrol-1-yl-benzyl)piperidin-4-ylamino]benzothiazol-5-carbonsäure,
{2-[1-(2,6-Diethoxy-4'-fluorbiphenyl-4ylmethyl)piperidin-4-ylamino]oxazolo[5,4-c]pyridin-4-yl}methanol,
{2-[1-(4-Chlor-3,5-diethoxybenzyl)piperin-4-ylamino]oxazolo[5,4-c]pyridin-4-yl}methanol,
und pharmazeutisch verträgliche Salze davon.

23. Verbindungen der Formel I gemäss Anspruch 1 ausgewählt aus der Gruppe bestehend aus
N²-[1-(3,5-Diethoxy-4-fluorbenzyl)piperidin-4-yl]benzooxazol-2,7-diamin,
N{2-[1-(3,5-Diethoxy-4-fluorbenzyl)piperidin-4-ylamino]benzooxazol-7-yl}succinamidsäure,
N{2-[1-(3,5-Diethoxy-4-fluorbenzyl)piperidin-4-ylamino]benzooxazol-4-yl}succinamidsäure,
2-[1-(4-Chlor-3-ethoxybenzyl)piperidin-4-ylamino]benzooxazol-6-carbonsäure,
2-[1-(4-Amino-3,5-diethoxybenzyl)piperidin-4-ylamino]benzooxazol-6-carbonsäure,
2-[1-(3,5-Diethoxy-4-fluorbenzyl)piperidin-4-ylamino]benzooxazol-6-carbonsäure,
[1-(3,5-Diethoxy-4-fluorbenzyl)piperidin-4-yl]oxazolo[5,4-c]pyridin-2-yl-amin,
[1-(3,5-Diisapropoxybenzyl)piperidin-4-yl]oxazolo[5,4-c]pyridin-2-yl-amin,
{2-[1-(3,5-Diethoxy-4-fluorbenzyl)piperidin-4-ylamino]benzooxazol-5-yloxy}essigsäure,
{2-[1-(3,5-Diethoxy-4-pyrrol-1-yl-benzyl)piperidin-4-ylamino]benzooxazol-5-yloxy}essigsäure,
[1-(3,5-Diethoxy-4-fluorbenzyl)piperidin-4-yl]-(7-phenylbenzooxazol-2-yl)amin,
4-{2-[1-(3,5-Diethoxy-4-pyrrol-1-yl-benzyl)piperidin-4-ylamino]benzooxazol-7-yloxy} buttersäure,
[rac]-3-{2-[1-(3,5-Diethoxy-4-fluorbenzyl)piperidin-4-ylamino]benzooxazol-7-yloxy}propan-1,2-diol,
4-{2-[1-(3,5-Diethoxy-4-fluorbenzyl)piperidin-4-ylamino]benzooxazol-5-yloxy}buttersäure,
[rac]-3-{2-[1-(3,5-Diethoxy-4-fluorbenzyl)piperidin-4-ylamino]benzooxazol-5-yloxy}propan-1,2-diol,
{2-[1-(3,5-Diisopropoxybenzyl)piperidin-4-ylamino]oxazolo[5,4-c]pyridin-4-yl}methanol,
2-[1-(4-Chlor-3,5-diethoxybenzyl)piperidin-4-ylamino]benzooxazol-5-carbonsäure,
2-[1-(2,6-Diethoxy-4'-fluorbiphenyl-4-ylmethyl)piperidin-4-ylamino]benzooxazol-5-carbonsäure,
{2-[1-(2,6-Diethoxy-4'-fluorbiphenyl-4-ylmethyl)piperidin-4-ylamino]benzooxazol-5-yloxy}acetonitril,
N-tert-butyl-2-{2-[1-(2,6-Diethoxy-4'-fluorbiphenyl-4-ylmethyl)piperidin-4-ylamino]benzooxazol-5-yloxy}acetamid,
2-{2-[1-(2,6-Diethoxy-4'-fluorbiphenyl-4-ylmethyl)piperidin-4-ylamino]benzooxazol-5-yloxy}acetamid,
[rac]-3-{2-[1-(2,6-Diethoxy-4'-fluorbiphenyl-4-ylmethyl)piperidin-4-ylamino]benzooxazol-5-yloxy}propan-1,2-diol,
2-[1-(2,6-Diethoxy-4'-fluorbiphenyl-4-ylmethyl)piperidin-4-ylamino]benzooxazol-6-carbonsäure,
2-[1-(4-chlor-3,5-diethoxybenzyl)piperidin-4-ylamino]benzooxazol-6-carbonsäure,
3-{2-[1-(2,6-Diethoxy-4'-fluorbiphenyl-4-ylmethyl)piperidin-4-ylamino]benzooxazol-5-yloxy}propan-1-ol;
2-{2-[1-(2,6-Diethoxy-4'-fluorbiphenyl-4-ylmethyl)piperidin-4-ylamino]benzooxazol-5-yloxy}ethanol,
[1-(2,6-Diethoxy-4'-fluorbiphenyl-4-ylmethyl)piperidin-4-yl]-[5-(3-methoxypropoxy)benzooxazol-2-yl]amin,
[1-(4-Chlor-3,5-diethoxybenzyl)piperidin-4-yl]-[5-(3-methansulfonylpropoxy)benzooxazol-2-yl]amin,
Methansulfonsäure 2-[1-(2,6-Diethoxy-4'-ffuorbiphenyl-4-ylmethyl)piperidin-4-ylamino]benzooxazol-5-ylester
[1-(4-Chlor-3,5-diethoxybenzyl)piperidin-4-yl]-[5-(3-[1,2,4]triazol-1-yl-propoxy)benzooxazol-2-yl]amin,
[1-(2,6-Diethoxy-4'-fluorbiphenyl-4-ylmethyl)piperidin-4-yl]-[5-(3-[1,2,4]triazol-1-yl-propoxy)benzooxazol-2-yl]amin,
2-[1-(4-Chlor-3,5-diethoxybenzyl)piperidin-4-ylamino]benzothiazol-6-carbonsäure,
2-[1-(2,6-Diethoxy-4'-fluorbiphenyl-4-ylmethyl)piperidin-4-ylamino]benzothiazol-6-carbonsäure,
2-[1-(4-Chlor-3,5-diethoxybenzyl)piperidin-4-ylamino]benzothiazol-5-carbonsäure,
2-[1-(2,6-Diethoxy-4'-fluorbiphenyl-4-ylmethyl)piperidin-4-ylamino]benzothiazol-5-carbonsäure,
und pharmazeutisch verträgliche Salze davon.

24. Verfahren zur Herstellung von Verbindungen gemäss einem der Ansprüche 1 bis 23, wobei das Verfahren umfasst
Umsetzen einer Verbindung der allgemeinen Formel 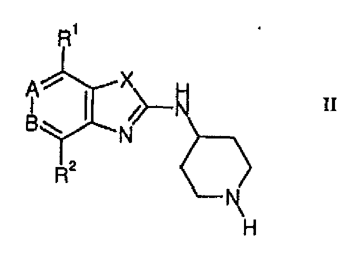 wobei A, B, X, R¹ und R² wie in Anspruch 1 definiert sind, mit einem Aldehyden der Formel 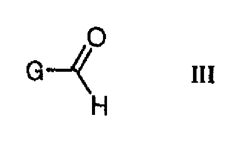 wobei G wie in Anspruch 1 definiert ist,
unter Verwenden eines Reduktionsmittels, um eine Verbindung der Formel 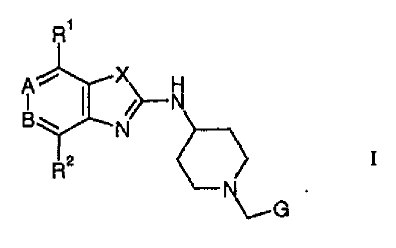 zu erhalten,
und falls es gewünscht wird,
Überführen der Verbindung der Formel I in ein pharmazeutisch verträgliches Salz.

25. Arzneimittel umfassend eine Verbindung gemäss einem der Ansprüche 1 bis 23 sowie einen pharmazeutisch verträglichen Träger und/oder Hilfsstoff.

26. Verbindungen gemäss einem der Ansprüche 1 bis 23 zur Verwendung als therapeutisch wirksame Substanzen.

27. Verbindungen gemäss einem der Ansprüche 1 bis 23 zur Verwendung als therapeutisch wirksame Substanzen zur Behandlung und/oder Prophylaxe von Krankheiten, die mit der Modulation von SST-Rezeptoren Subtyp 5 verbunden sind.

28. Verwendung von Verbindungen gemäss einem der Ansprüche 1 bis 23 zur Herstellung von Medikamenten zur Behandlung und/oder Prophylaxe von Krankheiten, die mit der Modulation von SST-Rezeptoren Subtyp 5 verbunden sind.

29. Verwendung gemäss Anspruch 28 zur Behandlung und /oder Prophylaxe von Diabetes mellitus, insbesondere Typ 2 Diabetes Mellitus, beeinträchtigte Fastenglukose, beeinträchtigte Glukosetoleranz, mikro-und makrovaskuläre diabetische Komplikationen, posttransplantatorisch in Typ 1 Diabetes mellitus, gestationale Diabetes, Fettleibigkeit, entzündliche Darmerkrankungen, wie Croln'sche Krankheit oder Colitis ulcerosa, Malabsorption, Autoimmunkrankheiten, wie rheumatoide Arthritis, Osteoarthritis, Psoriasis und andere Hautstörungen und Immunschwächen.

30. Verwendung gemäss Anspruch 28 zur Behandlung und /oder Prophylaxe von Diabetes mellitus, insbesondere Typ 2 Diabetes Mellitus, beeinträchtigte Fastenglukose, beeinträchtigte Glukosetoleranz.

## Revendications

1. Composés de formule générale 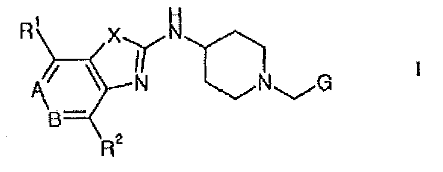 dans laquelle
X représente S ou O ;
A représente un groupe CR³ et B représente un groupe CR⁴, ou A représente N ou un groupe N⁺ - O⁻ et B représente un groupe CR⁴, ou bien B représente N ou un groupe N⁺ - O⁻ et A représente un groupe CR³ ;
R¹ et R² sont choisis indépendamment dans le groupe consistant en : hydrogène, halogène, des groupes cyano, nitro, alkyle en C₁ à C₇, hydroxyalkyle en C₁ à C₇, (alkoxy en C₁ à C₇) (alkyle en C₁ à C₇), alkoxy en C₁ à C₇, hydroxyalkoxy en C₂ à C₇, dihydroxy- alkoxy en C₃ à C₇, carboxy (alkoxy en C₁ à C₇), (alkoxy en C₁ à C₇) carbonyl (alkoxy en C₁ à C₇), carboxy (alkyle en C₁ à C₇), (alkoxy en C₁ à C₇)- carbonyl(alkyle en C₁ à C₇), 1*H*-tétrazole-5-yl-(alkoxy en C₁ à C₇), pyridinyl- (alkoxy en C₁ à C₇), -NR⁵R⁶, NHCOR⁷, -NHSO₂R⁸, -SO₂NR⁹R¹⁰, 1*H*-tétrazole-5-yle, phényle non substitué et phényle substitué avec un à trois substituants choisis entre des substituants alkyle en C₁ à C₇, cycloalkyle en C₃ à C₇, halogéno, halogéno-alkyle en C₁ à C₇ et alkoxy en C₁ à C₇ ; et
R² peut être choisi en outre dans le groupe consistant en des groupes carboxy, (alkoxy en C₁ à C₇) carbonyle et -CONR¹¹R¹² ;
R⁵ et R⁶, indépendamment l'un de l'autre, sont choisis dans le groupe consistant en l'hydrogène, des groupes alkyle en C₁ à C₇, hydroxyalkyle en C₁ à C₇ et cycloalkyle en C₃ à C₇ ;
R⁷ est choisi dans le groupe consistant en des groupes alkyle en C₁ à C₇, cycloalkyle en C₃ à C₇, halogéno-alkyle en C₁ à C₇, hydroxyalkyle en C₁ à C₇, (alkoxy en C₁ à C₇) (alkyle en C₁ à C₇), carboxy (alkyle en C₁ à C₇), (alkoxy en C₁ à C₇) carbonyl (alkyle en C₁ à C₇), 1*H*-tétrazole-5-yl-(alkyle en C₁ à C₇), phényle non substitué, phényle substitué avec un à trois groupes choisis dans le groupe consistant en des groupes alkyle en C₁ à C₇, cycloalkyle en C₃ à C₇, alkoxy, en C₁ à C₇, halogéno-alkyle en C₁ à C₇ et halogéno, hétéroaryle non substitué, hétéroaryle substitué avec un ou deux groupes choisis entre des groupes alkyle en C₁ à C₇, cycloalkyle en C₃ à C₇, alkoxy en C₁ à C₇, halogéno-alkyle en C₁ à C₇ et halogéno ; hétéroaryl(alkyle en C₁ à C₇) non substitué et hétéroaryl(alkyle en C₁ à C₇), dans lequel le groupe hétéroaryle est substitué avec un ou deux groupes choisis entre des groupes alkyle en C₁ à C₇, cycloalkyle en C₃ à C₇, alkoxy en C₁ à C₇, halogéno-alkyle en C₁ à C₇ et halogéno ;
R⁸ est choisi dans le groupe consistant en des groupes alkyle en C₁ à C₇, cycloalkyle en C₃ à C₇, hétéroaryle non substitué et hétéroaryle substitué avec un ou deux groupes choisis entre les groupes alkyle en C₁ à C₇, cycloalkyle en C₃ à C₇, alkoxy en C₁ à C₇, halogéno-alkyle en C₁ à C₇, et halogène ;
R⁹ et R¹⁰, indépendamment l'un de l'autre, sont choisis dans le groupe consistant en un atome d'hydrogène, des groupes alkyle en C₁ à C₇, cycloalkyle en C₃ à C₇, hétéroaryle non substitué et hétéroaryle substitué avec un ou deux groupes choisis entre des groupes alkyle en C₁ à C₇, cycloalkyle en C₃ à C₇, alkoxy en C₁ à C₇, halogéno-alkyle en C₁ à C₇ et halogéno ; ou bien R⁹ et R¹⁰, conjointement avec l'atome d'azote auquel ils sont fixés, forment un noyau pyrrolidine ou un noyau pipéridine ;
R¹¹ est choisi dans le groupe consistant en un atome d'hydrogène, des groupes alkyle en C₁ à C₇ et cycloalkyle en C₃ à C₇ ;
R¹² est choisi dans le groupe consistant en un atome d'hydrogène, des groupes alkyle en C₁ à C₇, cycloalkyle en C₃ à C₇, halogéno-alkyle en C₁ à C₇, hydroxyalkyle en C₁ à C₇, (alkoxy en C₁ à C₇) - (alkyle en C₁ à C₇), carboxy(alkyle en C₁ à C₇), (alkoxy en C₁ à C₇)carbonyl(alkyle en C₁ à C₇), phényle non substitué, phényle substitué avec un à trois groupes choisis dans le groupe consistant en des groupes alkyle en C₁ à C₇, cycloalkyle en C₃ à C₇, alkoxy en C₁ à C₇, halogéno-alkyle en C₁ à C₇ et halogéno, hétéroaryle non substitué, hétéroaryle substitué avec un ou deux groupes choisis entre des groupes alkyle en C₁ à C₇, cycloalkyle en C₃ à C₇, alkoxy en C₁ à C₇, halogéno-alkyle en C₁ à C₇ et halogène ;
l'un de R³ et R⁴ est choisi dans le groupe consistant en un atome d'hydrogène, des groupes alkyle en C₁ à C₇ et alkoxy en C₁ à C₇, ou bien est absent dans le cas où un de A et B représente N ou un groupe N⁺ - O⁻, et
l'autre de R³ et R⁴ est choisi dans le groupe consistant en un atome d'hydrogène, des groupes alkyle en C₁ à C₇, alkoxy en C₁ à C₇, hydroxy, hydroxyalkyle en C₁ à C₇, cyano (alkoxy en C₁ à C₇), cycloalkyloxy en C₃ à C₇ dans lequel le groupe cycloalkyle est substitué avec un substituant carboxy ou (alkoxy en C₁ à C₇)- carbonyle ; carboxy, (alkoxy en C₁ à C₇) carbonyle, carboxy(alkoxy en C₁ à C₇), (alkoxy en C₁ à C₇) carbonyl (alkyle en C₁ à C₇), carboxy (alkyle en C₁ à C₇), (alkoxy en C₁ à C₇) carbonyl (alkoxy en C₁ à C₇), 1H-tétrazole-5-yl-(alkoxy en C₁ à C₇), triazolyl-(alkoxy en C₁ à C₇), alkylsulfonyloxy en C₁ à C₇, (alkyle en C₁ à C₇)sulfonyl(alkoxy en C₁ à C₇), hydroxyalkoxy en C₂ à C₇, dihydroxyalkoxy en C₃ à C₇, (alkoxy en C₁ à C₇) (alkoxy en C₁ à C₇), - (CH₂)ₙ-NR¹³R¹⁴, -(CH₂)ₙ-NHCOR¹⁵, amino-alkoxy en C₁ à C₇, aminocarbonyl(alkoxy en C₁ à C₇), (alkyl en C₁ à C₇) aminocarbonyl (alkoxy en C₁ à C₇) et 1*H*-tétrazole-5-yle ;
R¹³ et R¹⁴, indépendamment l'un de l'autre, sont choisis dans le groupe consistant en un atome d'hydrogène, des groupes alkyle en C₁ à C₇, et cycloalkyle en C₃ à C₇,
R¹⁵ est choisi dans le groupe consistant en des groupes alkyl en C₁ à C₇, cycloalkyle en C₃ à C₇, halogénoalkyle en C₁ à C₇, hydroxyalkyle en C₁ à C₇, (alkoxy en C₁ à C₇)(alkyle en C₁ à C₇), carboxy(alkyle en C₁ à C₇) et (alkoxy en C₁ à C₇)carbonyl(alkyle en C₁ à C₇),
n représente un nombre entier choisi entre 1, 2 et 3 ;
sous réserve que des benzooxazoles et benzothiazoles dans lesquels R¹, R², R³ et R⁴ représentent des atomes d'hydrogène, soient exclus ;
G est choisi entre les groupes 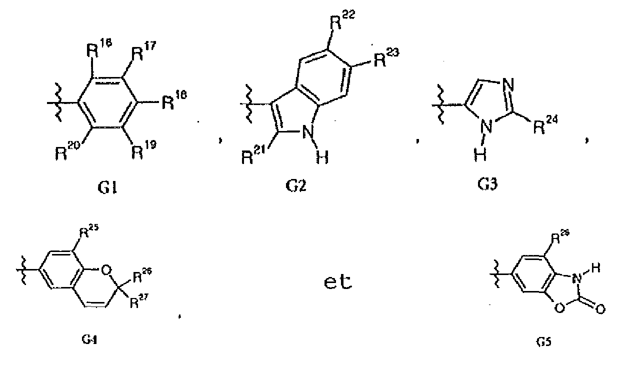
dans lesquels
R¹⁶ représente un atome d'hydrogène ou un atome d'halogène ;
R¹⁷ est choisi dans le groupe consistant en des groupes alkoxy en C₁ à C₇, alcényloxy en C₂ à C₇, cycloalkyloxy en C₃ à C₇, -NR²⁹R³⁰, halogéno-alkoxy en C₁ à C₇, (alkoxy en C₁ à C₇) (alkoxy en C₁ à C₇), (alkoxy en C₁ à C₇)(alkyle en C₁ à C₇) ; R²⁹ et R³⁰, indépendamment l'un de l'autre, représentent un atome d'hydrogène ou un groupe alkyle en C₁ à C₇ ;
R¹⁸ est choisi dans le groupe consistant en un atome d'hydrogène, des groupes alkyle en C₁ à C₇, halogéno- alkyle en C₁ à C₇, hydroxy, alkoxy en C₁ à C₇, halogéno-alkoxy en C₁ à C₇, cycloalkyloxy en C₃ à C₇, halogéno, pyrrolo, imidazolyle, triazolyle, -CO₂R³¹, -NR³²R³³, -SOR³⁴ ; phényle non substitué et phényle substitué avec un ou deux groupes choisis dans le groupe consistant en des groupes alkyle en C₁ à C₇, halogéno-alkyle en C₁ à C₇, halogéno-alkoxy en C₁ à C₇, alkoxy en C₁ à C₇ et halogéno ; R³¹ représente un atome d'hydrogène ou un groupe alkyle en C₁ à C₇ ; R³² et R³³, indépendamment l'un de l'autre, représentent un atome d'hydrogène ou un groupe alkyle en C₁ à C_{7 ;} R³⁴ représente un groupe alkyl en C₁ à C₇ ;
R¹⁹ est choisi dans le groupe consistant en un atome d'hydrogène, des groupes alkyl en C₁ à C₇, halogéno, alkoxy en C₁ à C₇, alcényloxy en C₂ à C₇, -O-tétra- hydropyranyle, cycloalkyloxy en C₃ à C₇, halogéno- alkoxy en C₃ à C₇, (alkoxy en C₁ à C₇) (alkoxy en C₁ à C₇) et (alkoxy en C₁ à C₇) (alkyle en C₁ à C₇) ;
R²⁰ représente un atome d'hydrogène ou un atome d'halogène ;
R²¹ représente un atome d'hydrogène ou un groupe alkyle en C₁ à C₇ ;
R²² et R²³, indépendamment l'un de l'autre, sont choisis dans le groupe consistant en un atome d'hydrogène, des groupes alkyle en C₁ à C₇, alkoxy, en C₁ à C₇, et nitro ;
R²⁴ représente un groupe phényle non substitué ou phényle substitué avec un ou deux groupes choisis dans le groupe consistant en des groupes alkyle en C₁ à C₇, alkoxy en C₁ à C₇ et halogéno ;
R²⁵ représente un groupe alkoxy en C₁ à C₇,
R²⁶ et R²⁷, indépendamment l'un de l'autre, représentent un groupe alkyle en C₁ à C₇ ;
R²⁸ représente un groupe alkoxy, en C₁ à C₇ ;
et leurs sels pharmaceutiquement acceptables.

2. Composés de formule I suivant la revendication 1, dans laquelle X représente 0.

3. Composés de formule I, suivant la revendication 1 ou 2, dans lesquels A représente un groupe CR³ et B représente un groupe CR⁴ et dans lesquels
l'un de R³ et R⁴ est choisi dans le groupe consistant en un atome d'hydrogène, des groupes alkyle en C₁ à C₇ et alkoxy en C₁ à C₇,
l'autre de R³ et R⁴ est choisi dans le groupe consistant en un atome d'hydrogène, des groupes alkyle en C₁ à C₇, alkoxy en C₁ à C₇, hydroxy, hydroxyalkyle en C₁ à C₇, cyano(alkoxy en C₁ à C₇), cycloalkyloxy en C₃ à C₇ dans lequel le groupe cycloalkyle est substitué avec un substituant carboxy ou (alkoxy en C₁ à C₇) carbonyle, carboxy, (alkoxy en C₁ à C₇)carbonyle, carboxy (alkoxy en C₁ à C₇), (alkoxy en C₁ à C₇)carbonyl (alkyle en C₁ à C₇), carboxy (alkyle en C₁ à C₇), (alkoxy en C₁ à C₇) - carbonyl (alkoxy en C₁ à C₇), 1*H*-tétrazole-5-yl-(alkoxy en C₁ à C₇), triazolyl-(alkoxy en C₁ à C₇), alkylsulfonyloxy en C₁ à C₇, (alkyle en C₁ à C₇)sulfonyl(alkoxy en C₁ à C₇), hydroxyalkoxy en C₂ à C₇, dihydroxyalkoxy en C₃ à C₇, (alkoxy en C₁ à C₇)(alkoxy en C₁ à C₇), -(CH₂)ₙ-NR¹³R¹⁴, -(CH₂)ₙ-NHCOR¹⁵, amino-alkoxy en C₁ à C₇, aminocarbonyl (alkoxy en C₁ à C₇), (alkyle en C₁ à C₇)aminocarbonyl (alkoxy en C₁ à C₇) et 1*H*-tétrazole-5-yle, et dans lesquels R¹³, R¹⁴, R¹⁵ et n répondent aux définitions figurant dans la revendication 1.

4. Composés de formule I suivant l'une quelconque des revendications 1 à 3, dans lesquels R³ représente un atome d'hydrogène et R⁴ est choisi dans le groupe consistant en des groupes alkyle en C₁ à C₇, alkoxy en C₁ à C₇, hydroxy, hydroxyalkyle en C₁ à C7, cyano(alkoxy en C₁ à C₇), cycloalkyloxy en C₃ à C₇ dans lequel le groupe cycloalkyle est substitué avec un substituant carboxy ou (alkoxy en C₁ à C₇) carbonyle, carboxy, (alkoxy en C₁ à C₇) carbonyle, carboxy(alkoxy en C₁ à C₇), (alkoxy en C₁ à C₇) carbonyl-(alkyle en C₁ à C₇), carboxy (alkyle en C₁ à C₇), (alkoxy en C₁ à C₇) carbonyl (alkoxy en C₁ à C₇), 1*H*-tétrazole-5-yl-(alkoxy en C₁ à C₇), triazolyl-(alkoxy en C₁ à C₇), alkylsulfonyloxy en C₁ à C₇, (alkyle en C₁ à C₇)sulfonyl-(alkoxy en C₁ à C₇), hydroxyalkoxy en C₂ à C₇, dihydroxyalkoxy en C₃ à C₇, (alkoxy en C₁ à C₇) (alkoxy en C₁ à C₇), -(CH₂)ₙ-NR¹³R¹⁴, -(CH2)ₙ-NHCOR¹⁵, amino-alkoxy en C₁ à C₇, aminocarbonyl (alkoxy en C₁ à C₇), (alkyle en C₁ à C₇)-aminocarbonyl(alkoxy en C₁ à C₇) et 1*H*-tétrazole-5-yle, et dans lesquels R¹³, R¹⁴, R¹⁵ et n répondent aux définitions figurant dans la revendication 1.

5. Composés de formule I suivant l'une quelconque des revendications 1 à 3, dans lesquels R⁴ représente un atome d'hydrogène et R³ est choisi dans le groupe consistant en des groupes alkyle en C₁ à C₇, alkoxy en C₁ à C₇, hydroxyalkyle en C₁ à C₇, carboxy, (alkoxy en C₁ à C₇)-carbonyle, carboxy(alkoxy en C₁ à C₇), (alkoxy en C₁ à C₇)-carbonyl(alkyle en C₁ à C₇), carboxy(alkyle en C₁ à C₇), (alkoxy en C₁ à C₇)carbonyl(alkoxy en C₁ à C₇), 1*H-*tétrazole-5-yl-(alkoxy en C₁ à C₇), hydroxyalkoxy en C₂ à C₇, dihydroxyalkoxy en C₃ à C₇, -(CH₂)ₙ-NR¹³R¹⁴, -(CH₂)ₙ-NHCOR¹⁵, et 1*H*-tétrazole-5-yle, et dans lesquels R¹³, R¹⁴, R¹⁵ et n répondent aux définitions figurant dans la revendication 1.

6. Composés de formule I suivant l'une quelconque des revendications 1 à 3, dans lesquels R³ et R⁴ représentent des atomes d'hydrogène.

7. Composés de formule I suivant la revendication 1 ou 2, dans lesquels A représente N, B représente un groupe CR⁴ et R⁴ est choisi dans le groupe consistant en un atome d'hydrogène, des groupes alkyle en C₁ à C₇, alkoxy en C₁ à C₇, hydroxy, hydroxyalkyle en C₁ à C₇, cyano (alkoxy en C₁ à C₇), cycloalkyloxy en C₃ à C₇ dans lequel le groupe cycloalkyle est substitué avec un substituant carboxy ou (alkoxy en C₁ à C₇) carbonyle, carboxy, (alkoxy en C₁ à C₇)-carbonyle, carboxy (alkoxy en C₁ à C₇), (alkoxy en C₁ à C₇)-carbonyl (alkyle en C₁ à C₇), carboxy(alkyle en C₁ à C₇), (alkoxy en C₁ à C₇)carbonyl(alkoxy en C₁ à C₇), 1H-tétrazole-5-yl- (alkoxy en C₁ à C₇), triazolyl-(alkoxy en C₁ à C₇), alkylsulfonyloxy en C₁ à C₇, (alkyle en C₁ à C₇)-sulfonyl (alkoxy en C₁ à C₇*),* hydroxyalkoxy en C₂ à C₇, dihydroxyalkoxy en C₃ à C₇, (alkoxy en C₁ à C₇) (alkoxy en C₁ à C₇), (CH₂)ₙ-NR¹³R¹⁴, -(CH₂)ₙ-NHCOR¹⁵, amino-alkoxy en C₁ à C₇, aminocarbonyl (alkoxy en C₁ à C₇), (alkyle en C₁ à C₇)-aminocarbonyl(alkoxy en C₁ à C₇) et 1*H*-tétrazole-5-yle, et dans lesquels R¹³, R¹⁴, R¹⁵ et n répondent aux définitions figurant dans la revendication 1.

8. Composés de formule I suivant l'une quelconque des revendications 1, 2 et 7, dans lesquels A représente N et B représente un groupe CH.

9. Composés de formule I suivant la revendication 7 ou 8, dans lesquels R¹ et R² représentent des atomes d'hydrogène.

10. Composés de formule I suivant l'une quelconque des
revendications 1 à 8, dans lesquels
R¹ est choisi dans le groupe consistant en des groupes halogéno, cyano, nitro, alkyl en C₁ à C₇, hydroxyalkyle en C₁ à C₇, (alkoxy en C₁ à C₇) (alkyle en C₁ à C₇), alkoxy en C₁ à C₇, hydroxyalkoxy en C₂ à C₇, dihydroxyalkoxy en C₃ à C₇, carboxy (alkoxy en C₁ à C₇), (alkoxy en C₁ à C₇) carbonyl (alkoxy en C₁ à C₇), carboxy(alkyle en C₁ à C₇), (alkoxy en C₁ à C₇) carbonyl (alkyle en C₁ à C₇), 1*H*-tétrazole-5-yl- (alkoxy, en C₁ à C₇), pyridinyl-(alkoxy en C₁ à C₇), -NR⁵R⁶, -NHCOR⁷, -NHSO₂R⁸, -SO₂NR⁹R¹⁰, 1*H*-tétrazole-5- yle, phényle non substitué et phényle substitué avec un à trois substituants choisis entre des substituants alkyle en C₁ à C₇, cycloalkyle en C₃ à C₇, halogéno, halogéno-alkyle en C₁ à C₇ et alkoxy en C₁ à C₇ ; et dans lesquels R⁵ à R¹⁰ répondent aux définitions figurant dans la revendication 1, et
R² représente un atome d'hydrogène.

11. Composés de formule I suivant la revendication 10, dans lesquels R¹ est choisi dans le groupe consistant en des groupes halogéno, nitro, alkyle en C₁ à C₇, hydroxyalkyle en C₁ à C₇, alkoxy en C₁ à C₇, dihydroxyalkoxy en C₃ à C₇, (carboxy en C₁ à C₇) (alkoxy en C₁ à C₇), (alkoxy en C₁ à C₇)carbonyl(alkoxy en C₁ à C₇*),* -NR⁵R⁶, -NHCOR⁷, -NHSO₂R⁸, -SO₂NR⁹R¹⁰, phényle non substitué et phényle substitué avec un à trois substituants choisis entre des substituants alkyle en C₁ à C₇, cycloalkyl en C₃ à C₇, halogéno, halogéno-alkyle en C₁ à C₇ et alkoxy en C₁ à C₇ ; et dans lesquels R⁵ à R¹⁰ répondent aux définitions figurant dans la revendication 1.

12. Composés de formule I suivant la revendication 10 ou 11, dans lesquels R¹ représente un groupe -NR⁵R⁶ ou -NHCOR⁷, et dans lesquels R⁵ à R⁷ répondent aux définitions figurant dans la revendication 1.

13. Composés de formule I suivant l'une quelconque des
revendications 1 à 8, dans lesquels
R¹ représente un atome d'hydrogène, et
R² est choisi dans le groupe consistant en des groupes halogéno, cyano, nitro, alkyle en C₁ à C₇, hydroxyalkyle en C₁ à C₇, (alkoxy en C₁ à C₇) (alkyl en C₁ à C₇), alkoxy en C₁ à C₇, hydroxyalkoxy en C₂ à C₇, dihydroxyalkoxy en C₃ à C₇, carboxy (alkoxy en C₁ à C₇) , (alkoxy en C₁ à C₇)carbonyl(alkoxy en C₁ à C₇), carboxy(alkyle en C₁à C₇), (alkoxy en C₁ à C₇) - carbonyl(alkyle en C₁ à C₇), 1*H*-tétrazole-5-yl-(alkoxy en C₁ à C₇), pyridinyl - (alkoxy en C₁ à C₇), -NR⁵R⁶, -NHCOR⁷, -NHSO₂R⁸, -SO₂NR⁹R¹⁰, carboxy, (alkoxy en C₁ à C₇) carbonyle, -CONR¹¹R¹², 1*H*-tétrazole-5-yle, phényle non substitué et phényle substitué avec un à trois substituants choisis entre des substituants alkyle en C₁ à C₇, cycloalkyle en C₃ à C₇, halogéno, halogéno- alkyle en C₁ à C₇ et alkoxy en C₁ à C₇ ; et dans lesquels R⁵ à R¹² répondent aux définitions figurant dans la revendication 1.

14. Composés de formule I suivant la revendication 13, dans lesquels R² est choisi dans le groupe consistant en des groupes halogéno, nitro, alkoxy en C₁ à C₇, pyridinyl-(alkoxy en C₁ à C₇), -NR⁵R⁶, -NHCOR⁷, carboxy, (alkoxy en C₁ à C₇) carbonyle, -CONR¹¹R¹², et dans lesquels R⁵ à R⁷, R¹¹ et R¹² répondent aux définitions figurant dans la revendication 1.

15. Composés de formule I suivant la revendication 14, dans lesquels R² représente un groupe -NHCOR⁷ et R⁷ répond à la définition figurant dans la revendication 1.

16. Composés de formule I suivant l'une quelconque des
revendications 1 à 15, dans lesquels G représente un groupe 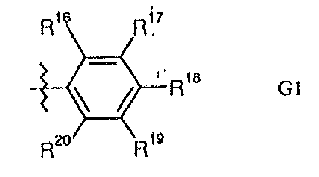 et dans lequel
R¹⁶ représente un atome d'hydrogène ou un atome d'halogène ;
R¹⁷ est choisi dans le groupe consistant en des groupes alkoxy en C₁ à C₇, alcényloxy en C₂ à C₇, cycloalkyloxy en C₃ à C₇, -NR²⁹R³⁰, halogéno-alkoxy en C₁ à C₇, (alkoxy en C₁ à C₇) (alkoxy en C₁ à C₇), (alkoxy en C₁ à C₇) (alkyle en C₁ à C₇) ; R²⁹ et R³⁰, indépendamment l'un de l'autre, représentent un atome d'hydrogène ou un groupe alkyle en C₁ à C₇ ;
R¹⁸ est choisi dans le groupe consistant en un atome d'hydrogène, des groupes alkyl en C₁ à C₇, halogéno- alkyle en C₁ à C₇, hydroxy, alkoxy en C₁ à C₇, halogéno-alkoxy en C₁ à C₇, cycloalkyloxy en C₃ à C₇, halogéno, pyrrolo, imidazolyle, triazolyle, -CO₂R³¹, -NR³²R³³, -SOR³⁴; phényle non substitué et phényle substitué avec un ou deux groupes choisis dans le groupe consistant en des substituants alkyle en C₁ à C₇, halogéno-alkyle en C₁ à C₇, halogéno-alkoxy en C₁ à C₇, alkoxy en C₁ à C₇ et halogéno ; R³¹ représente un atome d'hydrogène ou un groupe alkyle en C₁ à C₇ ; R³² et R³³, indépendamment l'un de l'autre, représentent un atome d'hydrogène ou un groupe alkyle en C₁ à C₇ ; R³⁴ représente un groupe alkyle en C₁ à C₇ ;
R¹⁹ est choisi dans le groupe consistant en un atome d'hydrogène, des groupes alkyle en C₁ à C₇, halogéno, alkoxy en C₁ à C₇, alcényloxy en C₂ à C₇, -O-tétra- hydropyranyle, cycloalkyloxy en C₃ à C₇, halogéno- alkoxy, en C₁ à C₇, (alkoxy en C₁ à C₇) (alkoxy en C₁ à C₇) et (alkoxy en C₁ à C₇) (alkyle en C₁ à C₇) ; et
R²⁰ représente un atome d'hydrogène ou un atome d'halogène.

17. Composés de formule I suivant l'une quelconque des revendications 1 à 16, dans lesquels R¹⁷ représente un groupe alkoxy en C₁ à C₇ ou halogéno-alkoxy en C₁ à C₇.

18. Composés de formule I suivant l'une quelconque des revendications 1 à 17, dans lesquels R¹⁷ représente un groupe éthoxy, isopropyloxy ou isobutyloxy.

19. Composés de formule I suivant l'une quelconque des revendications 1 à 18, dans lesquels R¹⁸ est choisi dans le groupe consistant en un atome d'hydrogène, des groupes alkyle en C₁ à C₇, hydroxy, alkoxy en C₁ à C₇, halogéno, pyrrolo, imidazolyle, triazolyle, -NR³²R³³ et -SOR³⁴, et R³² et R³³ représentent, indépendamment l'un de l'autre, un atome d'hydrogène ou un groupe alkyle en C₁ à C₇, et R³⁴ représente un groupe alkyle en C₁ à C₇.

20. Composés de formule I suivant l'une quelconque des revendications 1 à 19, dans lesquels R¹⁸ est choisi dans le groupe consistant en un atome d'hydrogène, des groupes halogéno, pyrrolo, triazolyle et -NR³²R³³, dans lequel R³² et R³³ représentent des atomes d'hydrogène.

21. Composés de formule I suivant la revendication 1, dans lesquels
X représente S ou O ;
A représente un groupe CR³ et B représente un groupe CR⁴, ou A représente N ou un groupe N⁺-O⁻ et B représente un groupe CR⁴, ou bien B représente N ou un groupe N⁺-O⁻ et A représente un groupe CR³ ;
R¹ et R² sont choisis indépendamment dans le groupe consistant en hydrogène, halogène, des groupes cyano, nitro, alkyle en C₁ à C₇, hydroxyalkyle en C₁ à C₇, (alkoxy en C₁ à C₇)(alkyle en C₁ à C₇), alkoxy en C₁ à C₇, hydroxyalkoxy en C₁ à C₇, dihydroxy- alkoxy en C₃ à C₇, carboxy(alkoxy en C₁ à C₇), (alkoxy en C₁ à C₇)carbonyl(alkoxy en C₁ à C₇), carboxy(alkyle en C₁ à C₇), (alkoxy en C₁ à C₇)- carbonyl(alkyle en C₁ à C₇), 1*H*-tétrazole-5-yl-(alkoxy en C₁ à C₇), pyridinyl- (alkoxy en C₁ à C₇), -NR⁵R⁶, NHCOR⁷, -NHSO₂R⁸, -SO₂NR⁹R¹⁰, 1*H*-tétrazole-5-yle, phényle non substitué et phényle substitué avec un à trois substituants choisis entre des substituants alkyle en C₁ à C₇, cycloalkyle en C₃ à C₇, halogéno, halogéno-alkyle en C₁ à C₇ et alkoxy en C₁ à C₇ ; et
R² peut être choisi en outre dans le groupe consistant en des groupes carboxy, (alkoxy en C₁ à C₇)carbonyle et -CONR¹¹R¹² ; R⁵ et R⁶, indépendamment l'un de l'autre, sont choisis dans le groupe consistant en un atome d'hydrogène, des groupes alkyle en C₁ à C₇, hydroxyalkyle en C₁ à C₇ et cycloalkyle en C₃ à C₇ ;
R⁷ est choisi dans le groupe consistant en des groupes alkyle en C₁ à C₇, cycloalkyl en C₃ à C₇, halogéno-alkyle en C₁ à C₇, hydroxyalkyle en C₁ à C₇, (alkoxy en C₁ à C₇)(alkyle en C₁ à C₇), carboxy(alkyle en C₁ à C₇), (alkoxy en C₁ à C₇)carbonyl(alkyle en C₁ à C₇), 1*H*-tétrazole-5-yl- (alkyle en C₁ à C₇), phényle non substitué, phényle substitué avec un à trois groupes choisis dans le groupe consistant en des groupes alkyle en C₁ à C₇, cycloalkyle en C₃ à C₇, alkoxy en C₁ à C₇, halogéno-alkyle en C₁ à C₇ et halogéno, hétéroaryle non substitué, hétéroaryle substitué avec un ou deux groupes choisis entre des groupes alkyle en C₁ à C₇, cycloalkyle en C₃ à C₇, alkoxy en C₁ à C₇, halogéno-alkyle en C₁ à C₇ et halogéno ; hétéroaryl(alkyle en C₁ à C₇) non substitué et hétéroaryl(alkyle en C₁ à C₇), dans lequel le groupe hétéroaryle est substitué avec un ou deux groupes choisis entre des groupes alkyle en C₁ à C₇, cycloalkyle en C₃ à C₇, alkoxy en C₁ à C₇, halogéno-alkyle en C₁ à C₇ et halogéno ;
R⁸ est choisi dans le groupe consistant en des groupes alkyle en C₁ à C₇, cycloalkyle en C₃ à C₇, hétéroaryle non substitué, hétéroaryle substitué avec un ou deux groupes choisis entre les groupes alkyle en C₁ à C₇, cycloalkyle en C₃ à C₇, alkoxy en C₁ à C₇, halogéno-alkyle en C₁ à C₇, et halogéno ;
R⁹ et R¹⁰, indépendamment l'un de l'autre, sont choisis dans le groupe consistant en un atome d'hydrogène, des groupes alkyle en C₁ à C₇, cycloalkyle en C₃ à C₇, hétéroaryle non substitué et hétéroaryle substitué avec un ou deux groupes choisis entre des groupes alkyle en C₁ à C₇, cycloalkyle en C₃ à C₇, alkoxy en C₁ à C₇, halogéno-alkyle en C₁ à C₇ et halogéno ; ou bien R⁹ et R¹⁰, conjointement avec l'atome d'azote auquel ils sont fixés, forment un noyau pyrrolidine ou pipéridine ;
R¹¹ est choisi dans le groupe consistant en un atome d'hydrogène, des groupes alkyle en C₁ à C₇ et cycloalkyle en C₃ à C₇ ;
R¹² est choisi dans le groupe consistant en un atome d'hydrogène, des groupes alkyl en C₁ à C₇, cycloalkyle en C₃ à C₇, halogéno-alkyle en C₁ à C₇, hydroxyalkyle en C₁ à C₇, (alkoxy en C₁ à C₇) - (alkyle en C₁ à C₇), carboxy (alkyle en C₁ à C₇), (alkoxy en C₁ à C₇)carbonyl(alkyle en C₁ à C₇), phényle non substitué, phényle substitué avec un à trois groupes choisis dans le groupe consistant en des groupes alkyle en C₁ à C₇, cycloalkyle en C₃ à C₇, alkoxy en C₁ à C₇, halogéno-alkyle en C₁ à C₇ et halogéno, hétéroaryle non substitué, hétéroaryle substitué avec un ou deux groupes choisis entre des groupes alkyle en C₁ à C₇, cycloalkyle en C₃ à C₇, alkoxy en C₁ à C₇, halogéno-alkyle en C₁ à C₇ et halogéno ;
l'un de R³ et R⁴ est choisi dans le groupe consistant en un atome d'hydrogène, des groupes alkyle en C₁ à C₇ et alkoxy en C₁ à C₇, ou bien est absent dans le cas où l'un de A et B représente N ou un groupe N⁺-O⁻, et
l'autre de R³ et R⁴ est choisi dans le groupe consistant en un atome d'hydrogène, des groupes alkyle en C₁ à C₇, alkoxy en C₁ à C₇, hydroxyalkyle en C₁ à C₇, carboxy, (alkoxy en C₁ à C₇)carbonyle, carboxy- (alkoxy en C₁ à C₇), (alkoxy en C₁ à C₇)carbonyl(alkyle en C₁ à C₇), carboxy(alkyle en C₁ à C₇), (alkoxy en C₁ à C₇)carbonyl(alkoxy en C₁ à C₇), 1*H*-tétrazole-5-yl-(alkoxy en C₁ à C₇), hydroxyalkoxy en C₂ à C₇, dihydroxyalkoxy en C₃ à C₇, - (CH₂)ₙ-NR¹³R¹⁴, -(CH₂)ₙ-NHCOR¹⁵, et 1*H*-tétrazole-5- yle ;
R¹³ et R¹⁴, indépendamment l'un de l'autre, sont choisis dans le groupe consistant en un atome d'hydrogène, des groupes alkyle en C₁ à C₇ et cycloalkyle en C₃ à C₇,
R¹⁵ est choisi dans le groupe consistant en des groupes alkyle en C₁ à C₇, cycloalkyle en C₃ à C₇, halogéno-alkyle en C₁ à C₇, hydroxyalkyle en C₁ à C₇, (alkoxy, en C₁ à C₇)(alkyle en C₁ à C₇), carboxy(alkyle en C₁ à C₇) et (alkoxy en C₁ à C7) carbonyl(alkyle en C₁ à C₇),
n représente un nombre entier choisi entre 1, 2 et 3 ;
sous réserve que des benzooxazoles et benzothiazoles dans lesquels R¹, R², R³ et R⁴ représentent des atomes d'hydrogène, soient exclus ;
G est choisi entre les groupes 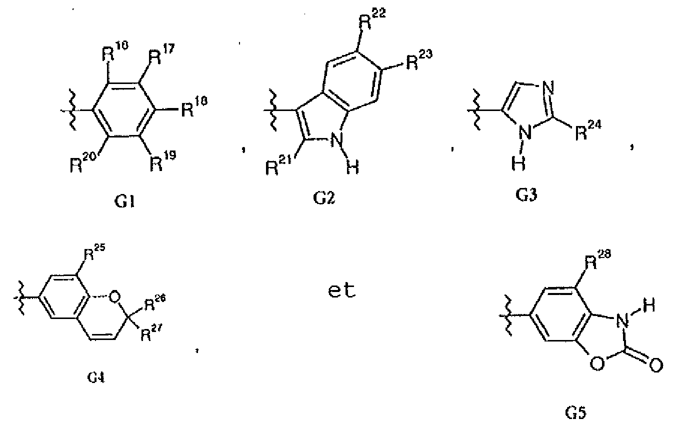
dans lesquels
R¹⁶ représente un atome d' hydrogène ou un atome d'halogène ;
R¹⁷ est choisi dans le groupe consistant en des groupes alkoxy en C₁ à C₇, alcényloxy en C₂ à C₇, cycloalkyloxy en C₃ à C₇, -NR²⁹R³⁰, halogéno-alkoxy en C₁ à C₇, (alkoxy en C₁ à C₇)(alkoxy en C₁ à C₇), (alkoxy en C₁ à C₇)(alkyle en C₁ à C₇) ; R²⁹ et R³⁰, indépendamment l'un de l'autre, représentent un atome d'hydrogène ou un groupe alkyle en C₁ à C₇ ;
R¹⁸ est choisi dans le groupe consistant en un atome d'hydrogène, des groupes alkyle en C₁ à C₇, halogéno- alkyle en C₁ à C₇, hydroxy, alkoxy en C₁ à C₇, halogéno-alkoxy en C₁ à C₇, cycloalkyloxy en C₃ à C₇, halogéno, pyrrolo, CO₂R³¹, - NR³²R³³, -SOR³⁴ ; phényle non substitué et phényle substitué avec un ou deux groupes choisis dans le groupe consistant en des groupes alkyle en C₁ à C₇, halogéno-alkyle en C₁ à C₇, halogéno- alkoxy en C₁ à C₇, alkoxy en C₁ à C₇ et halogéno ; R³¹ représente un atome d'hydrogène ou un groupe alkyle en C₁ à C₇ ; R³² et R³³, indépendamment l'un de l'autre, représentent un atome d'hydrogène ou un groupe alkyle en C₁ à C₇ ; R³⁴ représente un groupe alkyle en C₁ à C₇
R¹⁹ est choisi dans le groupe consistant en un atome d'hydrogène, des groupes alkyle en C₁ à C₇, halogéno, alkoxy en C₁ à C₇, alcényloxy en C₂ à C₇, -O-tétra- hydropyranyle, cycloalkyloxy en C₃ à C₇, halogéno- alkoxy en C₁ à C₇, (alkoxy en C₁ à C₇)(alkoxy en C₁ à C₇) et (alkoxy en C₁ à C₇)(alkyle en C₁ à C₇) ;
R²⁰ représente un atome d'hydrogène ou un atome d'halogène ;
R²¹ représente un atome d'hydrogène ou un groupe alkyle en C₁ à C₇ ;
R²² et R²³, indépendamment l'un de l'autre, sont choisis dans le groupe consistant en un atome d'hydrogène, des groupes alkyle en C₁ à C₇, alkoxy en C₁ à C₇ et nitro ;
R²⁴ représente un groupe phényle non substitué ou phényle substitué avec un ou deux groupes choisis dans le groupe consistant en des groupes alkyle en C₁ à C₇, alkoxy en C₁ à C₇ et halogéno ;
R²⁵ représente un groupe alkoxy en C₁ à C₇,
R²⁶ et R²⁷, indépendamment l' un de l'autre, représentent un groupe alkyle en C₁ à C₇ ;
R²⁸ représente un groupe alkoxy en C₁ à C₇ ;
et leurs sels pharmaceutiquement acceptables.

22. Composés de formule I suivant la revendication 1, choisis dans le groupe consistant en :
la [1-(3-éthoxy-4-méthoxy-benzyl)-pipéridine-4-yl]-(7-nitro-benzooxazole-2-yl)-amine,
la [1-(3,5-diéthoxy-4-fluoro-benzyl)-pipéridine-4-yl]-(7-nitro-benzooxazole-2-yl)-amine,
la *N*²-[1-(3,5-diéthoxy-4-fluoro-benzyl)-pipéridine-4-y1]-benzooxazole-2,7-diamine,
le *N*- {2-[1-(3,5-diéthoxy-4-fluoro-benzyl)-pipéridine-4-ylamino]-benzooxazole-7-yl}-2-méthoxy-acétamide,
l'acide *N*-{2-[1-(3,5-diéthoxy-4-fluoro-benzyl)-pipéridine-4-ylamino]-benzooxazole-7-yl}-succinamique,
le {2-[1-(3,5-diéthoxy-4-fluoro-benzyl)-pipéridine-4-ylamino]-benzooxazole-7-yl}-amide d'acide pyrimidine-5-carboxylique,
le *N*-{2-[1-(3,5-diéthoxy-4-fluoro-benzyl)-pipéridine-4-ylamino]-benzooxazole-7-yl}-2-pyridine-3-yl-acétamide,
le {2-[1-(3,5-diéthoxy-4-fluoro-benzyl)-pipéridine-4-ylamino]-benzooxazole-7-yl}-amide d'acide 1-méthyl-1H-imidazole-4-sulfonique,
le 2-[1-(3,5-diéthoxy-4-fluoro-benzyl)-pipéridine-4-ylamino]-benzooxazole-7-yl]-méthane-sulfonamide,
la [1-(3,5-diéthoxy-4-fluoro-benzyl)-pipéridine-4-yl] - [7-(pyrrolidine-1-sulfonyl)-benzooxazole-2-yl]-amine,
la [1-(3,5-diéthoxy-benzyl)-pipéridine-4-yl]-[7-(pyrrolidine-1-sulfonyl)-benzooxazole-2-yl] -amine,
la [1-(3-éthoxy-4-méthoxy-benzyl)-pipéridine-4-yl] -[7-(pyrrolidine-1-sulfonyl)-benzooxazole-2-yl]-amine,
la [1-(3,5-diéthoxy-4-pyrrole-1-yl-benzyl)-pipéridine-4-yl]-[7-(pyrrolidine-1-sulfonyl)-benzooxazole-2-yl]-amine,
la [1-(4-chloro-3-éthoxy-benzyl)-pipéridine-4-yl]-[7-(pyrrolidine-1-sulfonyl)-benzooxazole-2-yl]-amine,
la [1-(3-éthoxy-4-méthoxy-benzyl)-pipéridine-4-yl]-(4-nitro-benzooxazole-2-yl)-amine,
la [1-(3,5-diéthoxy-4-pyrrole-1-yl-benzyl)-pipéridine-4-yl]-(4-nitro-benzooxazole-2-yl)-amine,
la [1-(3,5-diéthoxy-4-fluoro-benzyl)-pipéridine-4-yl] - (4-nitro-benzooxazole-2-yl)-amine,
la *N*²-[1-(3,5-diéthoxy-4-fluoro-benzyl)-pipéridine-4-yl]-benzooxazole-2,4-diamine,
le *N*-{2-[1-(3,5-diéthoxy-4-fluoro-benzyl)-pipéridine-4-yl-amino]-benzooxazole-4-yl}-2-méthoxy-acétamide,
l'ester méthylique d'acide *N*-{2-[1-(3,5-diéthoxy-4-fluoro-benzyl)-pipéridine-4-yl-amino]-benzooxazole-4-yl} - succinamique,
l'acide *N*-{2-[1-(3,5-diéthoxy-4-fluoro-benzyl)-pipéridine-4-yl-amino]-benzooxazole-4-yl}-succinamique,
le {2-[1-(3,5-diéthoxy-4-fluoro-benzyl)-pipéridine-4-yl-amino]-benzooxazole-4-yl}-amide d'acide pyrimidine-5-carboxylique,
la *N*²-[1-(3,5-diéthoxy-4-pyrrole-l-yl-benzyl)-pipéridine-4-yl]-benzooxazole-2,4-diamine,
le *N*-{2-[1-(3,5-diéthoxy-4-pyrrole-1-yl-benzyl)-pipéridine-4-yl-amino]-benzooxazole-4-yl}-2-méthoxy-acétamide,
le *N*-{2-[1-(3,5-diéthoxy-4-fluoro-benzyl)-pipéridine-4-yl-amino]-benzooxazole-4-yl}-2-(1H-tétrazole-5-yl)-acétamide,
l'ester méthylique d'acide *N*-{2-[1-(3,5-diéthoxy-4-pyrrole-1-yl-benzyl)-pipéridine-4-ylamino] -benzooxazole-4-yl}-succinamique,
l'acide *N*-{2-[1-(3,5-diéthoxy-4-pyrrole-1-yl-benzyl)-pipéridine-4-ylamino]-benzooxazole-4-yl}-succinamique,
la [1-(3-éthoxy-4-méthoxy-benzyl)-pipéridine-4-yl] -[4-(pyridine-4-ylméthoxy)-benzooxazole-2-yl]-amine,
la [1-(3,5-diéthoxy-4-pyrrole-1-yl-benzyl)-pipéridine-4-yl]-[4-(pyridine-4-ylméthoxy)-benzooxazole-2-yl]-amine,
la [1-(3,5-diéthoxy-4-fluoro-benzyl)-pipéridine-4-yl]-[4-(pyridine-4-ylméthoxy)-benzooxazole-2-yl]-amine,
l'ester méthylique d'acide 2-[1-(3-éthoxy-4-méthoxy-benzyl)-pipéridine-4-ylamino]-benzooxazole-4-carboxylique,
l'ester méthylique d'acide 2-[1-(3,5-diéthoxy-4-pyrrole-1-yl-benzyl)-pipéridine-4-ylamino]-benzooxazole-4-carboxylique,
l'ester méthylique d'acide 2-[1-(3,5-diéthoxy-4-fluoro-benzyl)-pipéridine-4-ylamino]-benzooxazole-4-carboxylique,
l'acide 2-[1-(3-éthoxy-4-méthoxy-benzyl)-pipéridine-4-ylamino]-benzooxazole-4-carboxylique,
l'acide 2-[1-(3,5-diéthoxy-4-pyrrole-1-yl-benzyl)-pipéridine-4-ylamino]-benzooxazole-4-carboxylique,
l'acide 2-[1-(3,5-diéthoxy-4-fluoro-benzyl)-pipéridine-4-ylamino]-benzooxazole-4-carboxylique,
le pyridine-3-ylamide d'acide 2-[1-(3-éthoxy-4-méthoxy-benzyl)-pipéridine-4-ylamino]-benzooxazole-4-carboxylique,
le pyridine-3-ylamide d'acide 2-[1-(3,5-diéthoxy-4-fluoro-benzyl)-pipéridine-4-ylamino]-benzooxazole-4-carboxylique,
le pyridine-3-ylamide d'acide 2-[1-(3,5-diéthoxy-4-pyrrole-1-yl-benzyl)-pipéridine-4-ylamino]-benzooxazole-4-carboxylique,
le (2-hydroxy-éthyl)-amide d'acide 2-[1-(3-éthoxy-4-méthoxy-benzyl)-pipéridine-4-ylamino]-benzooxazole-4-carboxylique,
le (2-hydroxy-éthyl)-amide d'acide 2-[1-(3,5-diéthoxy-4-fluoro-benzyl)-pipéridine-4-ylamino]-benzooxazole-4-carboxylique,
le (2-hydroxy-éthyl)-amide d'acide 2-[1-(3,5-diéthoxy-4-pyrrole-1-yl-benzyl)-pipéridine-4-ylamino]-benzooxazole-4-carboxylique,
l'ester méthylique d'acide ({2-[1-(3-éthoxy-4-méthoxy-benzyl)-pipéridine-4-ylamino]-benzooxazole-4-carbonyl}-amino)-acétique,
l'ester méthylique d'acide ({2-[1-(3,5-diéthoxy-4-fluoro-benzyl)-pipéridine-4-ylamino]-benzooxazole-4-carbonyl}-amino)-acétique,
l'ester méthylique d'acide ({2-[1-(3,5-diéthoxy-4-pyrrole-1-yl-benzyl)-pipéridine-4-ylamino]-benzooxazole-4-carbonyl}-amino)-acétique,
l'acide ({2-[1-(3-éthoxy-4-méthoxy-benzyl)-pipéridine-4-ylamino]-benzooxazole-4-carbonyl}-amino)-acétique,
l'acide ({2-[1-(3,5-diéthoxy-4-fluoro-benzyl)-pipéridine-4-ylamino]-benzooxazole-4-carbonyl}-amino)-acétique,
l'acide ({2-[1-(3,5-diéthoxy-4-pyrrole-1-yl-benzyl)-pipéridine-4-ylamino]-benzooxazole-4-carbonyl}-amino)-acétique,
la [1-(3-éthoxy-4-méthoxy-benzyl)-pipéridine-4-yl]-(4-iodo-benzooxazole-2-yl)-amine,
la [1-(3,5-diéthoxy-4-fluoro-benzyl)-pipéridine-4-yl]-(4-iodo-benzooxazole-2-yl)-amine,
la [1-(3,5-diéthoxy-4-pyrrole-1-yl-benzyl)-pipéridine-4-yl]-(4-iodo-benzooxazole-2-yl)-amine,
la (7-bromo-benzooxazole-2-yl)-[1-(3-éthoxy-4-méthoxy-benzyl)-pipéridine-4-yl]-amine,
la (7-bromo-benzooxazole-2-yl)-[1-(3,5-diéthoxy-4-fluoro-benzyl)-pipéridine-4-yl]-amine,
la (7-bromo-benzooxazole-2-yl)-[1-(3,5-diéthoxy-4-pyrrole-1-yl-benzyl)-pipéridine-4-yl]-amine,
l'ester méthylique d'acide 2-[1-(3-éthoxy-4-hydroxy-benzyl)-pipéridine-4-ylamino]-benzooxazole-6-carboxylique,
l'ester méthylique d'acide2-[1-(3-éthoxy-4-méthoxy-benzyl)-pipéridine-4-ylamino]-benzooxazole-6-carboxylique,
l'ester méthylique d'acide de 2-[1-(8-éthoxy-2,2-diméthyl-2*H*-chromène-6-ylméthyl)-pipéridine-4-ylamino]-benzooxazole-6-carboxylique,
l'ester méthylique d'acide 2-[1-(3-isobutoxy-4-méthoxy-benzyl)-pipéridine-4-ylamino]-benzooxazole-6-carboxylique,
l'ester méthylique d'acide 2-{1-[3-(2-fluoro-éthoxy)-4-méthoxy-benzyl]-pipéridine-4-ylamino}-benzooxazole-6-carboxylique,
l'ester méthylique d'acide 2-{1-(3-éthoxy-4-méthyl-benzyl)-pipéridine-4-ylamino]-benzooxazole-6-carboxylique,
l'ester méthylique d'acide 2-[1-(4-choro-3-éthoxy-benzyl)-pipéridine-4-ylamino]-benzooxazole-6-carboxylique,
l'ester méthylique d'acide 2-[1-(4-amino-3,5-diéthoxy-benzyl)-pipéridine-4-ylamino]-benzooxazole-6-carboxylique,
l'ester méthylique d'acide 2-[1-(3,5-diéthoxy-4-fluoro-benzyl)-pipéridine-4-ylamino]-benzooxazole-6-carboxylique,
l'acide 2-[1-(3-éthoxy-4-hydroxy-benzyl)-pipéridine-4-ylamino]-benzooxazole-6-carboxylique,
l'acide 2-[1-(3-éthoxy-4-méthoxy-benzyl) -pipéridine-4-ylamino]-benzooxazole-6-carboxylique,
l'acide 2-[1-(8-éthoxy-2,2-diméthyl-2*H*-chromène-6-yl-méthyl)-pipéridine-4-ylamino] -benzooxazole-6-carboxylique,
l'acide 2-[1-(3-isobutoxy-4-méthoxy-benzyl) -pipéridine-4-ylamino]-benzooxazole-6-carboxylique,
l'acide 2-{1-[3-(2-fluoro-éthoxy)-4-méthoxy-benzyl]-pipéridine-4-ylamino}-benzooxazole-6-carboxylique,
l'acide 2-[1-(3-éthoxy-4-méthyl-benzyl) -pipéridine-4-ylamino]-benzooxazole-6-carboxylique,
l'acide 2-[1-(4-chloro-3-éthoxy-benzyl)-pipéridine-4-ylamino]-benzooxazole-6-carboxylique,
l'acide 2-[1-(4-amino-3,5-diéthoxy-benzyl)-pipéridine-4-ylamino]-benzooxazole-6-carboxylique,
le 2-[1-(3,5-diéthoxy-4-fluoro-benzyl)-pipéridine-4-ylamino]-benzooxazole-6-carboxylique,
la [1-(3-éthoxy-4-fluoro-benzyl)-pipéridine-4-yl]-(4-nitro-benzooxazole-2-yl)-amine,
la [1-(4-chloro-3-éthoxy-benzyl)-pipéridine-4-yl]-(4-nitro-benzoxazole-2-yl)-amine,
la [1-(3-éthoxy-4-méthyl-benzyl)-pipéridine-4-yl]-(4-nitro-benzooxazole-2-yl)-amine,
l'amide d'acide 2-[1-(3,5-diéthoxy-4-fluoro-benzyl)-pipéridine-4-ylamino]-benzooxazole-7-sulfonique,
l'amide d'acide 2-[1-(3-éthoxy-4-fluoro-benzyl)-pipéridine-4-ylamino]-benzooxazole-7-sulfonique,
l'amide d'acide 2-[1-(3-éthoxy-4-méthoxy-benzyl)-pipéridine-4-ylamino]-benzooxazole-7-sulfonique,
la [1-(3,5-diéthoxy-4-fluoro-benzyl)-pipéridine-4-yl]-(7-méthoxy-benzooxazole-2-yl)-amine,
la [1-(3,5-diéthoxy-4-pyrrole-1-yl-benzyl)-pipéridine-4-yl]-(7-méthoxy-benzooxazole-2-yl)-amine,
la [1-(3,5-diéthoxy-4-fluoro-benzyl)-pipéridine-4-yl]-oxazolo [5,4-*c*] pyridine-2-yl-amine,
la [1-(4-chloro-3-éthoxy-benzyl)-pipéridine-4-yl]-oxazolo[5,4-*c*]pyridine-2-yl-amine,
la [1- (3-éthoxy-4-méthyl-benzyl) -pipéridine-4-yl]-oxazolo[5,4-*c*]pyridine-2-yl-amine,
la [1-(3-éthoxy-4-méthoxy-benzyl)-pipéridine-4-yl]-oxazolo [5,4-*c*] pyridine-2-yl-amine,
la [1-(3-isobutoxy-4-méthoxy-benzyl)-pipéridine-4-yl]-oxazolo[5,4-*c*]pyridine-2-yl-amine,
la 4-éthoxy-6-[4-(oxazolo[5,4-*c*]pyridine-2-ylamino)-pipéridine-1-ylméthyl]-3H-benzooxazole-2-one,
la [rac]-[1-(3,5-diéthoxy-4-méthanesulfinyl-benzyl)-pipéridine-4-yl]-oxazolo[5,4,*c*]pyridine-2-yl-amine,
la [1-(3-éthylamino-4-méthoxy-benzyl)-pipéridine-4-yl]-oxazolo[5,4-*c*]pyridine-2-yl-amine,
la [1-(8-éthoxy-2,2-diméthyl-2*H*-chromène-6-ylméthyl)-pipéridine-4-yl]-oxazolo [5,4-*c*]pyridine-2-yl-amine,
la [1-(4-méthoxy-3-propoxy-benzyl)-pipéridine-4-yl]-oxazolo [5,4-*c*] pyridine-2-yl-amine,
la {1-[3-(2-fluoro-éthoxy)-4-méthoxy-benzyl]-pipéridine-4-yl}-oxazolo[5,4-*c*]pyridine-2-yl-amine,
le 2-éthoxy-4-[4-(oxazolo[5,4-*c*]pyridine-2-ylamino)-pipéridine-1-ylméthyl]-phénol,
la [1-(4-amino-3,5-diéthoxy-benzyl)-pipéridine-4-yl]-oxazolo[5,4-*c*]pyridine-2-yl-amine,
la [1-(3,5-diisopropoxy-benzyl)-pipéridine-4-yl] - oxazolo[5,4-*c*]pyridine-2-yl-amine,
la [1-(3-éthoxy-4-fluoro-benzyl)-pipéridine-4-yl]-oxazolo[5,4-*c*]pyridine-2-yl-amine,
la [1-(3-éthoxy-4-isopropoxy-benzyl)-pipéridine-4-yl]-oxazolo[5,4-*c*]pyridine-2-yl-amine,
la oxazolo [5,4-*c*]pyridine-2-yl-[1-(2-phényl-3H-imidazole-4-ylméthyl)-pipéridine-4-yl]-amine,
la [1-(2-méthyl-5-nitro-1H-indole-3-ylméthyl) -pipéridine-4-yl]-oxazolo[5,4-*c*]pyridine-2-yl-amine,
l'ester méthylique d'acide {2-[1-(3-éthoxy-4-méthoxy-benzyl)-pipéridine-4-ylamino]-benzooxazole-5-yloxy]-acétique,
l'ester méthylique d'acide {2-[1-(3,5-diéthoxy-4-fluoro-benzyl)-pipéridine-4-ylamino]-benzooxazole-5-yloxy}-acétique,
l'ester méthylique d'acide {2-[1-(3,5-diéthoxy-4-pyrrole-1-yl-benzyl)-pipéridine-4-ylamino]-benzooxazole-5-yloxy}-acétique,
l'acide {2-[1-(3-éthoxy-4-méthoxy-benzyl)-pipéridine-4-ylamino]-benzooxazole-5-yloxy}-acétique,
l'acide {2-[1-(3,5-diéthoxy-4-fluoro-benzyl)-pipéridine-4-ylamino]-benzooxazole-5-yloxy}-acétique,
l'acide {2-[1-(3,5-diéthoxy-4-pyrrole-1-yl-benzyl) - pipéridine-4-ylamino]-benzooxazole-5-yloxy}-acétique,
la [1-(3,5-diéthoxy-4-fluoro-benzyl)-pipéridine-4-yl] - (7-phényl-benzooxazole-2-yl)-amine,
l'ester méthylique d'acide {2-(1-(3-éthoxy-4-méthoxy-benzyl)-pipéridine-4-ylamino]-benzooxazole-7-yloxy}-acétique,
l'ester méthylique d'acide {2-[1-(3,5-diéthoxy-4-fluoro-benzyl)-pipéridine-4-ylamino]-benzooxazole-7-yloxy}-acétique,
l'ester méthylique d'acide {2-[1-(3,5-diéthoxy-4-pyrrole-1-yl-benzyl)-pipéridine-4-ylamino]-benzooxazole-7-yloxy} -acétique,
l'acide {2-[1-(3-éthoxy-4-méthoxy-benzyl)-pipéridine-4-ylamino]-benzooxazole-7-yloxy}-acétique,
l'acide {2-[1-3,5-diéthoxy-4-fluoro-benzyl) -pipéridine-4-ylamino]-benzooxazole-7-yloxy}-acétique,
l'acide {2-[1-(3,5-diéthoxy-4-pyrrole-1-yl-benzyl) - pipéridine-4-ylamino]-benzooxazole-7-yloxy}-acétique,
la (5-aminométhyl-benzooxazole-2-yl)- [1- (3,5-diéthoxy-4-fluoro-benzyl) -pipéridine-4-yl]-amine,
l'ester méthylique d'acide 4- {2- [1- (3,5-diéthoxy-4-fluoro-benzyl) -pipéridine-4-ylamino] -benzooxazole-7-yloxy} - butyrique,
l'ester méthylique d'acide 4-{2-[1-(3,5-diéthoxy-4-pyrrole-1-yl-benzyl)-pipéridine-4-ylamino]-benzooxazole-7-yloxy}-butyrique,
l'ester méthylique d'acide 4-{2-[1-(3-éthoxy-4-méthoxy-benzyl)-pipéridine-4-ylamino]-benzooxazole-7-yloxy} - butyrique,
l'acide 4-{2-[1-(3,5-diéthoxy-4-fluoro-benzyl)-pipéridine-4-ylamino]-benzooxazole-7-yloxy} -butyrique,
l'acide 4-{2-[1-(3,5-diéthoxy-4-pyrrole-1-yl-benzyl) - pipéridine-4-ylamino]-benzooxazole-7-yloxy}-butyrique,
l'acide 4-{2-[1-(3-éthoxy-4-méthoxy-benzyl)-pipéridine-4-ylamino] -benzooxazole-7-yloxy}-butyrique,
le [rac]-3-{2-[1-(3,5-diéthoxy-4-fluoro-benzyl)-pipéridine-4-ylamino] -benzooxazole-7-yloxy}-propane-1,2-diol,
le [rac]-3-{2-[1-(3,5-diéthoxy-4-pyrrole-1-yl-benzyl) - pipéridine-4-ylamino]-benzooxazole-7-yloxy}-propane-1,2-diol,
le [rac]-3-{2-[1-(3-éthoxy-4-méthoxy-benzyl)-pipéridine-4-ylamino]benzooxazole-7-yloxy}-propane-1,2-diol,
l'ester méthylique d'acide 4-{2-[1-(3,5-diéthoxy-4-fluoro-benzyl)-pipéridine-4-ylamino]-benzooxazole-5-yloxy}-butyrique,
l'ester méthylique d'acide 4-{2-[1-(3,5-diéthoxy-4-pyrrole-1-yl-benzyl)-pipéridine-4-ylamino]-benzooxazole-5-yloxy}-butyrique,
l'ester méthylique d'acide 4-{2-[1-(3-éthoxy-4-méthoxy-benzyl)-pipéridine-4-ylamino]-benzooxazole-5-yloxy}-butyrique,
l'acide 4-{2-[1-(3,5-diéthoxy-4-fluoro-benzyl)-pipéridine-4-ylamino]-benzooxazole-5-yloxy}-butyrique,
l'acide 4-{2-[1-(3,5-diéthoxy-4-pyrrole-1-yl-benzyl)-pipéridine-4-ylamino]-benzooxazole-5-yloxy}-butyrique,
l'acide 4-{2-[1-(3-éthoxy-4-méthoxy-benzyl)-piperidine-4-ylamino]-benzooxazole-5-yloxy}-butyrique,
l'ester éthylique d'acide 1-{2-[1-(3,5-diéthoxy-4-fluoro-benzyl)-pipéridine-4-ylamino]-benzooxazole-5-yloxy} - cyclobutanecarboxylique,
l'ester éthylique d'acide1-{2-[1-(3,5-diéthoxy-4-pyrrole-1-yl-benzyl)-pipéridine-4-ylamino]-benzooxazole-5-yloxy} -cyclobutanecarboxylique,
l'acide 1-{2-[1-(3,5-diéthoxy-4-fluoro-benzyl)-pipéridine-4-ylamino}-benzooxazole-5-yloxy}-cyclobutanecarboxylique,
l'acide 1-{2-[1-(3,5-diéthoxy-4-pyrrole-1-yl-benzyl) - pipéridine-4-ylamino]-benzooxazole-5-yloxy}-cyclobutanecarboxylique,
le *N*-{2-(1-(3,5-diéthoxy-4-fluoro-benzyl)-pipéridine-4-ylamino] -benzooxazole-5-ylméthyl}-acétamide,
l'ester éthylique d'acide *N*-{2-[1-(3,5-diéthoxy-4-fluoro-benzyl)-pipéridine-4-ylamino]-benzooxazole-5-ylméthyl}-malonamique,
le (S)-*N*-{2-[1-(3,5-diéthoxy-4-fluoro-benzyl)-piperidine-4-ylamino]-benzooxazole-5-ylméthyl}-2-hydroxy-propionamide,
la [1-(3,5-diéthoxy-4-fluoro-benzyl)-pipéridine-4-yl] - (4-méthyl-oxazolo[5,4-*c*]pyridine-2-yl)-amine,
la [1-(3,5-diéthoxy-4-pyrrole-1-yl-benzyl)-pipéridine-4-yl]-(4-méthyl-oxazolo[5,4-*c*]pyridine-2-yl)-amine,
la [1-(3,5-diisopropoxy-benzyl)-pipéridine-4-yl]-(4-méthyl-oxazolo[5,4-*c*]pyridine-2-yl)-amine,
l'acide *N*-{2-[1-(3,5-diéthoxy-4-fluoro-benzyl)-pipéridine-4-ylamino]-benzooxazole-5-ylméthyl}-malonamique,
le [rac]-3-{2-[1-(3,5-diéthoxy-4-fluoro-benzyl)-pipéridine-4-ylamino]-benzooxazole-5-yloxy}-propane-1,2-diol,
le [rac]-3-{2-[1-(3,5-diéthoxy-4-pyrrole-1-yl-benzyl) - pipéridine-4-ylamino]-benzooxazole-5-yloxy}-propane-1,2-diol,
le [rac]-3-{2-[1-(3,5-diisopropoxy-benzyl)-pipéridine-4-ylamino]-benzooxazole-5-yloxy}-propane-1,2-diol,
la [1-(3,5-diéthoxy-4-pyrrole-1-yl-benzyl)-pipéridine-4-yl]-[5-(1H-tétrazole-5-ylméthoxy)-benzooxazole-2-yl] -amine,
la [1-(3,5-diéthoxy-4-fluoro-benzyl)-pipéridine-4-yl] - [5-(1H-tétrazole-5-ylméthoxy)-benzooxazole-2-yl]-amine,
le {2-[1-(3,5-diéthoxy-4-fluoro-benzyl)-pipéridine-4-ylamino]-oxazolo[5,4-c]pyridine-4-yl}-méthanol,
le {2-[1-(3,5-diéthoxy-4-pyrrole-1-yl-benzyl)-pipéridine-4-ylamino]-oxazolo[5,4-c]pyridine-4-yl}-méthanol,
le {2-[1-(3,5-diisopropoxy-benzyl)-pipéridine-4-ylamino]-oxazolo[5,4-c]pyridine-4-yl}-méthanol,
le {2-[1-(3,5-diéthoxy-4-[1,2,4]triazole-1-yl-benzyl) - pipéridine-4-ylamino]-oxazolo[5,4-c]pyridine-4-yl}-méthanol,
l'ester méthylique d'acide{2-[1-(3,5-diéthoxy-4-fluoro-benzyl)-pipéridine-4-ylamino]-benzooxazole-5-yl}-acétique,
l'ester méthylique d'acide{2-[1-(3,5-diéthaxy-4-pyrrole-1-yl-benzyl)-pipéridine-4-ylamino]-benzooxazole-5-yl}-acétique,
l'ester méthylique d'acide {2-[1-(3,5-diéthoxy-4-diisopropoxy-benzyl)-pipéridine-4-ylamino]-benzooxazole-5-yl}-acétique,
l'ester méthylique d'acide {2-[1-(3,5-diéthoxy-4-[1,2,4]triazole-1-yl-benzyl)-pipéridine-4-ylamino]-benzooxazole-5-yl}-acétique,
l'acide {2-[1-(3,5-diéthoxy-4-fluoro-benzyl)-pipéridine-4-ylamino]-benzooxazole-5-yl}-acétique,
l'acide {2-[1-(3,5-diéthoxy-4-pyrrole-1-yl-benzyl)-pipéridine-4-ylamino]-benzooxazole-5-yl}-acétique,
l'acide {2-[1-(3,5-diisopropoxy-benzyl)-pipéridine-4-ylamino]-benzooxazole-5-yl}-acétique,
l'acide {2-[1-(3,5-diéthoxy-4-[1,2,4]triazole-1-yl-benzyl)-pipéridine-4-ylamino]-benzooxazole-5-yl}-acétique,
la [1-(3,5-diéthoxy-4-fluoro-benzyl)-pipéridine-4-yl]-(5-méthoxy-benzooxazole-2-yl)-amine,
la [1-(3,5-diéthoxy-4-pyrrole-1-yl-benzyl)-pipéridine-4-yl]-(5-méthoxy-benzooxazole-2-yl)-amine,
l'ester méthylique d'acide 2-[1-(3,5-diéthoxy-4-fluoro-benzyl)-pipéridine-4-ylamino]-benzooxazole-5-carboxylique,
l'ester méthylique d'acide 2-[1-(3,5-diéthoxy-4-pyrrole-1-yl-benzyl)-pipéridine-4-ylamino]-benzooxazole-5-carboxylique,
l'ester méthylique d'acide 2-[1-(3,5-diisopropoxy-benzyl)-pipéridine-4-ylamino]-benzooxazole-5-carboxylique,
l'ester méthylique d'acide 2-[1-(3,5-diéthoxy-4-[1,2,4] triazole-1-yl-benzyl)-pipéridine-4-ylamino]-benzooxazole-5-carboxylique,
l'acide 2-[1-(3,5-diéthoxy-4-fluoro-benzyl)-pipéridine-4-ylamino] -benzooxazole-5-carboxylique,
l'acide 2-[1-(3,5-diéthoxy-4-pyrrole-1-yl-benzyl)-pipéridine-4-ylamino]-benzooxazole-5-carboxylique,
l'acide 2-[1-(3,5-diisopropoxy-benzyl)-pipéridine-4-ylamino]-benzooxazole-5-carboxylique,
l'acide 2-[1-(3,5-diéthoxy-4-[1,2,4]triazole-1-yl-benzyl)-pipéridine-4-ylamino]-benzooxazole-5-carboxylique,
l'ester méthylique d'acide 2-[1-(4-chloro-3,5-diéthoxy-benzyl)-pipéridine-4-ylamino]-benzooxazole-5-carboxylique,
l'ester méthylique d'acide 2-[1-(2,6-diéthoxy-4'-fluoro-biphényl-4-ylméthyl)-pipéridine-4-ylamino]-benzooxazole-5-carboxylique,
l'acide 2-[1-(4-chloro-3,5-diéthoxy-benzyl)-pipéridine-4-ylamino]-benzooxazole-5-carboxylique,
l'acide 2-[1-(2,6-diéthoxy-4'-fluoro-biphényl-4-yl-méthyl)-pipéridine-4-ylamino]-benzooxazole-5-carboxylique,
le {2-[1-(3,5-diéthoxy-4-fluoro-benzyl) -pipéridine-4-ylamino]-benzooxazole-5-yloxy}-acétonitrile,
le *N*-tertio-butyl-2-{2-[1-(3,5-diéthoxy-4-fluoro-benzyl)-pipéridine-4-ylamino]-benzooxazole-5-yloxy}-acétamide,
le {2-[1-(3,5-diisopropoxy-benzyl)-pipéridine-4-ylamino]-benzooxazole-5-yloxy}-acétonitrile,
le *N*-tertio-butyl-2-{2-[1-(3,5-diisopropoxy-benzyl)-pipéridine-4-ylamino]-benzooxazole-5-yloxy}-acétamide,
le {2-[1-(4-chloro-3,5-diéthoxy-benzyl)-pipéridine-4-ylamino]-benzooxazole-5-yloxy}-acétonitrile,
le *N*-tertio-butyl-2-{2-[1-(4-chloro-3,5-diéthoxy-benzyl)-pipéridine-4-ylamino]-benzooxazole-5-yloxy}-acétamide,
le {2-[1-(2,6-diéthoxy-4'-fluoro-biphényl-4-ylméthy) - pipéridine-4-ylamino]-benzooxazole-5-yloxy}-acétonitrile,
le *N*-tertio-butyl-2-{2-[1-(2,6-diéthoxy-4'-fluoro-biphényl-4-ylméthyl)-pipéridine-4-ylamino]-benzooxazole-5-yloxy}-acétamide,
le 2-{2-[1-(2,6-diéthoxy-4'-fluoro-biphényl-4-ylméthyl) - pipéridine-4-ylamino] -benzooxazole-5-yloxy}-acétamide,
le 2-{2-[1-(3,5-diisopropoxy-benzyl)-pipéridine-4-ylamino]-benzooxazole-5-yloxy}-acétamide,
le 2-{2-[1-(4-chloro-3,5-diéthoxy-benzyl)-pipéridine-4-ylamino]-benzooxazole-5-yloxy}-acétamide,
le 2-{2-[1-(3,5-diéthoxy-4-fluoro-benzyl)-pipéridine-4-ylamino]-benzooxazole-5-yloxy}-acétamide,
le [rac]-3-{2-[1-(2,6-diéthoxy-4'-fluoro-biphényl-4-ylméthyl)-pipéridine-4-ylamino]-benzooxazole-5-yloxy}-propane-1,2-diol,
le [rac]-3-{2-[1-(4-chloro-3,5-diéthoxy-benzyl)-pipéridine-4-ylamino]-benzooxazole-5-yloxy}-propane-1,2-diol,
le 2-{2-[1-(3,5-diisopropoxy-benzyl)-pipéridine-4-ylamino]-benzooxazole-5-yl}-éthanol,
le 2-{2-[1-(3,5-diéthoxy-4-fluoro-benzyl)-pipéridine-4-ylamino]-benzooxazole-5-yl}-éthanol,
le {2-[1-(3,5-diéthoxy-4-fluoro-benzyl)-pipéridine-4-ylamino]-benzooxazole-5-yl}-méthanol,
la [5-(2-amino-éthoxy)-benzooxazole-2-yl]-[1-(3,5-diéthoxy-4-fluoro-benzyl)-pipéridine-4-yl]-amine,
l'acide 2-[1-(2,6-diéthoxy-4'-fluoro-biphényl-4-yl-méthyl) -pipéridine-4-ylamino]-benzooxazole-6-carboxylique,
l'acide 2-[1-(4-chloro-3,5-diéthoxy-benzyl)-pipéridine-4-ylamino]-benzooxazole-6-carboxylique,
l'acide 2-[1-(3,5-diisopropoxy-benzyl)-pipéridine-4-ylamino]-benzooxazole-6-carboxylique,
le 2-[1-(4-chloro-3,5-diéthoxy-benzyl)-pipéridine-4-ylamino]-benzooxazole-5-ol,
le 3-{2-[1-(3,5-diéthoxy-4-fluoro-benzyl)-pipéridine-4-ylamino]-benzooxazole-5-yloxy}-propane-1-ol,
le 3-{2-[1-(3,5-diisopropoxy-benzyl)-pipéridine-4-ylamino]-benzooxazole-5-yloxy}-propane1-ol,
le 3-{2-[1-(4-chloro-3,5-diéthoxy-benzyl)-pipéridine-4-ylamino]-benzooxazole-5-yloxy}-propane-1-ol,
le 3-{2-[1-(2,6-diéthoxy-4'-fluoro-biphényl-4-ylméthyl)-pipéridine-4-ylamino]-benzooxazole-5-yloxy}-propane-1-ol,
le 2-{2-[1-(3,5-diéthoxy-4-fluoro-benzyl)-pipéridine-4-ylamino]-benzooxazole-5-yloxy}-éthanol,
le 2-{2-[1-(2,6-diéthoxy-4'-fluoro-biphényl-4-ylméthyl)-pipéridine-4-ylamino]-benzooxazole-5-yloxy}-éthanol,
la [1-(3,5-diéthoxy-4-fluoro-benzyl)-pipé-ridine-4-yl]-[5-(3-méthoxy-propoxy)-benzooxazole-2-yl]-amine,
la [1-(3,5-diisopropoxy-benzyl)-pipéridine-4-yl]-[5-(3-méthoxy-propoxy)-benzooxazole-2-yl]-amine,
la [1-(4-chloro-3,5-diéthoxy-benzyl)-pipéridine-4-yl]-[5-(3-méthoxy-propoxy)-benzooxazole-2-yl]-amine,
la [1-(2,6-diéthoxy-4'-fluoro-biphényl-4-ylméthyl)-pipéridine-4-yl]-[5-(3-méthoxy-propoxy)-benzooxazole-2-yl]-amine,
la [1-(4-chloro-3,5-diéthoxy-benzyl)-pipéridine-4-yl]-[5-(3-méthanesulfonyl-propoxy)-benzooxazole-2-yl]-amine,
l'ester de 2-[1-(3,5-diéthoxy-4-fluoro-benzyl)-pipéridine-4-ylamino]-benzoxazole-5-yle d'acide méthanesulfonique,
l'ester de 2-[1-(3,5-diisopropoxy-benzyl)-pipéridine-4-ylamino]-benzoxazole-5-yle d'acide méthanesulfonique,
l'ester de 2-[1-(4-chloro-3,5-diéthoxy-benzyl)-pipéridine-4-ylamino]-benzoxazole-5-yle d'acide méthanesulfonique,
l'ester de 2-[1-(2,6-diéthoxy-4'-fluoro-biphényl-4-ylméthyl)-pipéridine-4-ylaminol-benzoxazole-5-yle d'acide méthanesulfonique,
la [1-(3,5-diéthoxy-4-fluoro-benzyl)-pipéridine-4-yl]-[5-(3-[1,2,4] triazole-1-yl-propoxy)-benzooxazole-2-yl]-amine,
la [1-(3,5-diisopropoxy-benzyl)-pipéridine-4-yl]-[5-(3-[1,2,4]triazole-1-yl-propoxy)-benzooxazole-2-yl]-amine,
la [1-(4-chloro-3,5-diéthoxy-benzyl)-pipéridine-4-yl]-[5-(3-[1,2,4] triazole-1-yl-propoxy)-benzooxazole-2-yl]-amine,
la [1-(2,6-diéthoxy-4'-fluoro-biphényl-4-ylméthyl)-pipéridine-4-yl]-[5-(3-[1,2,4]triazole-1-yl-propoxy)-benzooxazole-2-yl]-amine,
l'ester éthylique d'acide 2-[1-(3,5-diéthoxy-4-fluoro-benzyl)-pipéridine-4-ylamino]-benzothiazole-6-carboxylique,
l'ester éthylique d'acide 2-[1-(3,5-diisopropoxy-benzyl)-pipéridine-4-ylamino]-benzothiazole-6-carboxylique,
l'ester éthylique d'acide 2-[1-(4-chloro-3,5-diéthoxy-benzyl)-pipéridine-4-ylamino]-benzothiazole-6-carboxylique,
l'ester éthylique d'acide 2-[1-(2,6-diéthoxy-4'-fluoro-biphényl-4-ylméthyl)-pipéridine-4-ylamino]-benzothiazole-6-carboxylique,
l'ester éthylique d'acide 2-[1-(3,5-diéthoxy-4-pyrrole-1-yl-benzyl)-pipéridine-4-ylamino]-benzothiazole-6-carboxylique,
l'acide 2- [1-(3,5-diéthoxy-4-fluoro-benzyl)-pipéridine-4-ylamino]-benzothiazole-6-carboxylique,
l'acide 2-[1-(3,5-diisopropoxy-benzyl)-pipéridine-4-ylamino]-benzothiazole-6-carboxylique,
l'acide 2-[1-(4-chloro-3,5-diéthoxy-benzyl)-pipéridine-4-ylamino]-benzothiazole-6-carboxylique,
l'acide 2-[1-(2,6-diéthoxy-4'-fluoro-biphényl-4-yl-méthyl)-pipéridine-4-ylamino]-benzothiazole-6-carboxylique,
l'acide 2-[1-(3,5-diéthoxy-4-pyrrole-1-yl-benzyl)-pipéridine-4-ylamino]-benzothiazole-6-carboxylique,
l'ester méthylique d'acide 2-[1-(3,5-diisopropoxy-benzyl)-pipéridine-4-ylamino]-benzothiazole-5-carboxylique,
l'ester méthylique d'acide 2-[1-(4-chloro-3,5-diéthoxy-benzyl)-pipéridine-4-ylamino]-benzothiazole-5-carboxylique,
l'ester méthylique d'acide 2-[1-(2,6-diéthoxy-4'-fluoro-biphényl-4-ylméthyl)-pipéridine-4-ylamino]-benzothiazole-5-carboxylique,
l'ester méthylique d'acide 2-[1-(3,5-diéthoxy-4-pyrrole-1-yl-benzyl)-pipéridine-4-ylamino]-benzothiazole-5-carboxylique,
l'acide 2-[1-(3,5-diisopropoxy-benzyl)-pipéridine-4-ylamino]-benzothiazole-5-carboxylique,
l'acide 2- [1-(4-chloro-3,5-diéthoxy-benzyl)-pipéridine-4-ylamino]-benzothiazole-5-carboxylique,
l'acide 2-[1-(2,6-diéthoxy-4'-fluoro-biphényl-4-yl-méthyl)-pipéridine-4-ylamino]-benzothiazole-5-carboxylique,
l'acide 2-[1-(3,5-diéthoxy-4-pyrrole-1-yl-benzyl)-pipéridine-4-ylamino]-benzothiazole-5-carboxylique,
le {2-[1-(2,6-diéthoxy-4'-fluoro-biphényl-4-ylméthyl)-pipéridine-4-ylamino]-oxazolo[5,4-c]pyridine-4-yl}-méthanol,
le {2-[1-(4-chloro-3,5-diéthoxy-benzyl)-pipéridine-4-ylamino]-oxazolo[5,4-c] pyridine-4-yl}-méthanol
et leurs sels pharmaceutiquement acceptables.

23. Composés de formule I suivant la revendication 1, choisis dans le groupe consistant en :
la *N*²-[1-(3,5-diéthoxy-4-fluoro-benzyl)-pipéridine-4-yl]-benzooxazole-2,7-diamine,
l'acide *N*-{2-[1-(3,5-diéthoxy-4-fluoro-benzyl)-pipéridine-4-ylamino]-benzooxazole-7-yl}-succinamique,
l'acide *N*-{2-[1-(3,5-diéthoxy-4-fluoro-benzyl)-pipéridine-4-ylamino]-benzooxazole-4-yl}-succinamique,
l'acide 2-[1-(4-chloro-3-éthoxy-benzyl)-pipéridine-4-ylamino]-benzooxazole-6-carboxylique,
l'acide 2-[1-(4-amino-3,5-diéthoxy-benzyl)-pipéridine-4-ylamino]-benzooxazole-6-carboxylique,
l'acide 2-[1-(3,5-diéthoxy-4-fluoro-benzyl)-pipéridine-4-ylamino]-benzooxazole-6-carboxylique,
la [1-(3,5-diéthoxy-4-fluoro-benzyl)-pipéridine-4-yl]-oxazolo [5,4-c]pyridine-2-yl-amine,
la [1-(3,5-diisopropoxy-benzyl)-pipéridine-4-yl]-oxazolo[5,4-c]pyridine-2-yl-amine,
l'acide {2-[1-(3,5-diéthoxy-4-fluoro-benzyl)-pipéridine-4-ylamino]-benzooxazole-5-yloxy}-acétique,
l'acide {2-[1-(3,5-diéthoxy-4-pyrrole-1-yl-benzyl)-pipéridine-4-ylamino]-benzooxazole-5-yloxy)-acétique,
la [1-(3,5-diéthoxy-4-fluoro-benzyl)-pipéridine-4-yl]-(7-phényl-benzooxazole-2-yl)-amine,
l'acide 4-{2-[1-(3,5-diéthoxy-4-pyrrole-1-yl-benzyl)-pipéridine-4-ylamino]-benzooxazole-7-yloxy}-butyrique,
le [rac]-3-{2-[1-(3,5-diéthoxy-4-fluoro-benzyl)-pipéridine-4-ylamino]-benzooxazole-7-yloxy}-propane-1,2-diol,
l'acide 4-{2-[1- (3,5-diéthoxy-4-fluoro-benzyl) -pipéridine-4-ylamino]-benzooxazole-5-yloxy}-butyrique,
le [rac] -3-{2-[1-(3,5-diéthoxy-4-fluoro-benzyl)-pipéridine-4-ylamino]-benzooxazole-5-yloxy}-propane-1,2-diol,
le {2-[1-(3,5-diisopropoxy-benzyl)-pipéridine-4-ylamino]-oxazolo[5,4-c]pyridine-4-yl}-méthanol,
l'acide 2- [1-(4-chloro-3,5-diéthoxy-benzyl)-pipéridine-4-ylamino]-benzooxazole-5-carboxylique,
l'acide 2-[1-(2,6-diéthoxy-4'-fluoro-biphényl-4-yl-méthyl)-pipéridine-4-ylamino]-benzooxazole-5-carboxylique,
le {2-[1-{2,6-diéthoxy-4'-fluoro-biphényl-4-ylméthyl)-pipéridine-4-ylamino]-benzooxazole-5-yloxy}-acétonitrile,
le *N*-tertio-butyl-2-{2-[1-(2,6-diéthoxy-4'-fluoro-biphényl-4-ylméthyl)-pipéridine-4-ylamino]-benzooxazole-5-yloxy}-acétamide,
le 2-{2-[1-(2,6-diéthoxy-4'-fluoro-biphényl-4-ylméthyl)-pipéridine-4-ylamino]-benzooxazole-5-yloxy}-acétamide,
le [rac] -3-{2-[1-(2,6-diéthoxy-4'-fluoro-biphényl-4-yl-méthyl)-pipéridine-4-ylamino]-benzooxazole-5-yloxy}-propane-1,2-diol,
l'acide 2-[1-(2,6-diéthoxy-4'-fluoro-biphényl-4-yl-méthyl)-pipéridine-4-ylamino]-benzooxazole-6-carboxylique,
l'acide 2- [1-(4-chloro-3,5-diéthoxy-benzyl)-pipéridine-4-ylamino]-benzooxazole-6-carboxylique,
le 3-{2-[1-(2,6-diéthoxy-4'-fluoro-biphényl-4-ylméthyl)-pipéridine-4-ylamino]-benzooxazole-5-yloxy}-propane-1-ol,
le 2-{2-[1-(2,6-diéthoxy-4'-fluoro-biphényl-4-ylméthyl)-pipéridine-4-ylamino]-benzooxazole-5-yloxy}-éthanol,
la [1-(2,6-diéthoxy-4'-fluoro-biphényl-4-ylméthyl)-pipéridine-4-yl]-[5-(3-méthoxy-propoxy)-benzooxazole-2-yl]-amine,
la [1-(4-chloro-3,5-diéthoxy-benzyl)-pipéridine-4-yl]-[5-(3-méthanesulfonyl-propoxy)-benzooxazole-2-yl]-amine,
l'ester de 2-[1-(2,6-diéthoxy-4'-fluoro-biphényl-4-ylméthyl)-pipéridine-4-ylamino]-benzooxazole-5-yle d'acide méthanesulfonique,
la [1-(4-chloro-3,5-diéthoxy-benzyl)-pipéridine-4-yl]-[5-(3-[1,2,4]triazole-1-yl-propoxy)-benzooxazole-2-yl]-amine,
la [1-(2,6-diéthoxy-4'-fluoro-biphényl-4-ylméthyl)-pipéridine-4-yl]-[5-(3-[1,2,4]triazole-1-yl-propoxy)-benzooxazole-2-yl]-amine,
l'acide 2- [1-(4-chloro-3,5-diéthoxy-benzyl)-pipéridine-4-ylamino]-benzothiazole-6-carboxylique,
l'acide 2-[1-(2,6-diéthoxy-4'-fluoro-biphényl-4-yl-méthyl)-pipéridine-4-ylamino]-benzothiazole-6-carboxylique,
l'acide 2- [1-(4-chloro-3,5-diéthoxy-benzyl)-pipéridine-4-ylamino]-benzothiazole-5-carboxylique,
l'acide 2-[1-(2,6-diéthoxy-4'-fluoro-biphényl-4-yl-méthyl)-pipéridine-4-ylamino]-benzothiazole-5-carboxylique,
et leurs sels pharmaceutiquement acceptables.

24. Procédé pour la production de composés suivant l'une quelconque des revendications 1 à 23, procédé qui comprend
la réaction d'un composé de formule générale 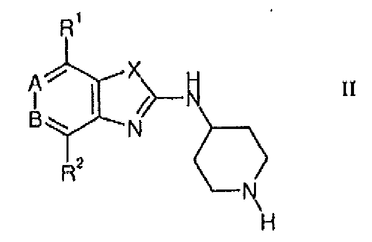 dans laquelle A, B, X, R¹ et R² répondent aux définitions figurant dans la revendication 1,
avec un aldéhyde de formule 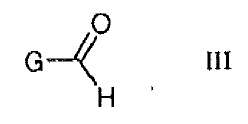 dans laquelle G répond à la définition figurant dans la revendication 1,
en utilisant un agent réducteur, pour obtenir un composé de formule générale 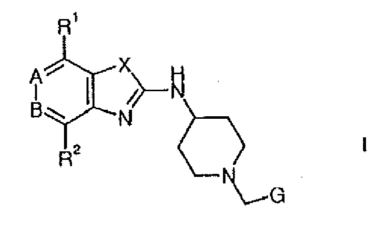 et, si cela est désiré,
la conversion du composé de formule I en un sel pharmaceutiquement acceptable.

25. Compositions pharmaceutiques comprenant un composé suivant l'une quelconque des revendications 1 à 23, ainsi qu'un support et/ou adjuvant pharmaceutiquement acceptable.

26. Composés suivant l'une quelconque des revendications 1 à 23, destinés à être utilisés comme substances thérapeutiquement actives.

27. Composés suivant l'une quelconque des revendications 1 à 23, destinés à être utilisés comme substances thérapeutiquement actives pour le traitement et/ou la prévention de maladies qui sont associées à la modulation des récepteurs SST de sous-type 5.

28. Utilisation de composés suivant l'une quelconque des revendications 1 à 23, pour la préparation de médicaments destinés au traitement et/ou à la prévention de maladies qui sont associées à la modulation des récepteurs SST de sous-type 5.

29. Utilisation suivant la revendication 28, pour le traitement et/ou la prévention du diabète sucré, en particulier du diabète sucré de type 2, du taux entravé de glucose à jeun, de la tolérance entravée au glucose, des complications diabétiques micro- et macro-vasculaires, de l'état après transplantation dans le diabète sucré de type 1, du diabète gestationnel, de l'obésité, de maladies intestinales inflammatoires telles que la maladie de Crohn ou la colite ulcérative, de la malabsorption, de maladies auto-immunes telles que la polyarthrite rhumatoïde, l'arthrose, le psoriasis et d'autres troubles cutanés, et d'immunodéficiences.

30. Utilisation suivant la revendication 28, pour le traitement et/ou la prévention du diabète sucré, en particulier du diabète sucré de type 2, du taux entravé de glucose à jeun, et de la tolérance entravée au glucose.
